# EUROPEAN PATENT APPLICATION

(11) **EP 2 116 604 A1**
(43) Date of publication of application: **11.11.2009**
(21) Application number: 09167071.1
(22) Date of filing: 05.08.2003
(51) Int. Cl.: C12N 15/62, C12N 9/78

(54) **Protein transducing domain/deaminase chimeric proteins, related compounds, and uses thereof**

(30) Priority: 05.08.2002 US 401293 P; 21.10.2002 US 419982 P
(62) Divisional of application: 03767199.7
(71) Applicant: UNIVERSITY OF ROCHESTER, Rochester, NY 14642 (US)
(72) Inventor: Smith, Harold, C., Rochester, NY 14623 (US); Sowden, Mark, P., Penfield, NY 14526 (US); Dewhurst, Stephen, Rochester, NY 14618 (US); Kim, Baek, Rochester, NY 14618 (US); Wedekind, Joseph, Rochester, NY 14642 (US)
(74) Representative: Helbing, Jörg

(57) **Abstract**

Disclosed are compositions for chimeric proteins comprising a protein transduction domain and a deaminase domain, mimetics or analog thereof, and uses of same.

SEQUENCE LISTING

<110> University of Rochester

<120> PROTEIN TRANSDUCING DOMAIN/DEAMINASE CHIMERIC PROTEINS,
      RELATED COMPOUNDS, AND USES THEREOF

<130> 082893ep

<150> EP 03767199.7
<151> 2003-08-05

<150> 60/419,982
<151> 2002-10-21

<150> 60/401,293
<151> 2002-08-05

<160> 49

<170> FastSEQ for Windows Version 4.0

<210> 1
<211> 384
<212> PRT
<213> Artificial Sequence

<220>
<223> Description of Artificial Sequence; note =
      synthetic construct

<400> 1
Met Lys Pro His Phe Arg Asn Thr Val Glu Arg Met Tyr Arg Asp Thr
 1               5                  10                  15
Phe Ser Tyr Asn Phe Tyr Asn Arg Pro Ile Leu Ser Arg Arg Asn Thr
            20                  25                  30
Val Trp Leu Cys Tyr Glu Val Lys Thr Lys Gly Pro Ser Arg Pro Pro
        35                  40                  45
Leu Asp Ala Lys Ile Phe Arg Gly Gln Val Tyr Ser Glu Leu Lys Tyr
    50                  55                  60
His Pro Glu Met Arg Phe Phe His Trp Phe Ser Lys Trp Arg Lys Leu
65                  70                  75                  80
His Arg Asp Gln Glu Tyr Glu Val Thr Trp Tyr Ile Ser Trp Ser Pro
                85                  90                  95
Cys Thr Lys Cys Thr Arg Asp Met Ala Thr Phe Leu Ala Glu Asp Pro
            100                 105                 110
Lys Val Thr Leu Thr Ile Phe Val Ala Arg Leu Tyr Tyr Phe Trp Asp
        115                 120                 125
Pro Asp Tyr Gln Glu Ala Leu Arg Ser Leu Cys Gln Lys Arg Asp Gly
    130                 135                 140
Pro Arg Ala Thr Met Lys Ile Met Asn Tyr Asp Glu Phe Gln His Cys
145                 150                 155                 160
Trp Ser Lys Phe Val Tyr Ser Gln Arg Glu Leu Phe Glu Pro Trp Asn
                165                 170                 175
Asn Leu Pro Lys Tyr Tyr Ile Leu Leu His Ile Met Leu Gly Glu Ile
            180                 185                 190
Leu Arg His Ser Met Asp Pro Pro Thr Phe Thr Phe Asn Phe Asn Asn
        195                 200                 205
Glu Pro Trp Val Arg Gly Arg His Glu Thr Tyr Leu Cys Tyr Glu Val
    210                 215                 220
Glu Arg Met His Asn Asp Thr Trp Val Leu Leu Asn Gln Arg Arg Gly
225                 230                 235                 240

Phe Leu Cys Asn Gln Ala Pro His Lys His Gly Phe Leu Glu Gly Arg
                245                 250                 255
His Ala Glu Leu Cys Phe Leu Asp Val Ile Pro Phe Trp Lys Leu Asp
            260                 265                 270
Leu Asp Gln Asp Tyr Arg Val Thr Cys Phe Thr Ser Trp Ser Pro Cys
        275                 280                 285
Phe Ser Cys Ala Gln Glu Met Ala Lys Phe Ile Ser Lys Asn Lys His
    290                 295                 300
Val Ser Leu Cys Ile Phe Thr Ala Arg Ile Tyr Asp Asp Gln Gly Arg
305                 310                 315                 320
Cys Gln Glu Gly Leu Arg Thr Leu Ala Glu Ala Gly Ala Lys Ile Ser
                325                 330                 335
Ile Met Thr Tyr Ser Glu Phe Lys His Cys Trp Asp Thr Phe Val Asp
            340                 345                 350
His Gln Gly Cys Pro Phe Gln Pro Trp Asp Gly Leu Asp Glu His Ser
        355                 360                 365
Gln Asp Leu Ser Gly Arg Leu Arg Ala Ile Leu Gln Asn Gln Glu Asn
    370                 375                 380


<210> 2
<211> 1155
<212> DNA
<213> Artificial Sequence

<220>
<223> Description of Artificial Sequence; note =
      synthetic construct

<400> 2
atgaagcctc acttcagaaa cacagtggag cgaatgtatc gagacacatt ctcctacaac      60
ttttataata gacccatcct ttctcgtcgg aataccgtct ggctgtgcta cgaagtgaaa     120
acaaagggtc cctcaaggcc ccctttggac gcaaagatct ttcgaggcca ggtgtattcc     180
gaacttaagt accacccaga gatgagattc ttccactggt tcagcaagtg gaggaagctg     240
catcgtgacc aggagtatga ggtcacctgg tacatatcct ggagcccctg cacaaagtgt     300
acaagggata tggccacgtt cctggccgag gacccgaagg ttaccctgac catcttcgtt     360
gcccgcctct actacttctg ggacccagat taccaggagg cgcttcgcag cctgtgtcag     420
aaaagagacg gtccgcgtgc caccatgaag atcatgaatt atgacgaatt tcagcactgt     480
tggagcaagt tcgtgtacag ccaaagagag ctatttgagc cttggaataa tctgcctaaa     540
tattatatat tactgcacat catgctgggg gagattctca gacactcgat ggatccaccc     600
acattcactt tcaactttaa caatgaacct tgggtcagag gacggcatga gacttacctg     660
tgttatgagg tggagcgcat gcacaatgac acctgggtcc tgctgaacca gcgcaggggc     720
tttctatgca accaggctcc acataaacac ggtttccttg aaggccgcca tgcagagctg     780
tgcttcctgg acgtgattcc cttttggaag ctggacctgg accaggacta cagggttacc     840
tgcttcacct cctggagccc ctgcttcagc tgtgcccagg aaatggctaa attcatttca     900
aaaaacaaac acgtgagcct gtgcatcttc actgcccgca tctatgatga tcaaggaaga     960
tgtcaggagg ggctgcgcac cctggccgag gctggggcca aaatttcaat aatgacatac    1020
agtgaattta agcactgctg ggacaccttt gtggaccacc agggatgtcc cttccagccc    1080
tgggatggac tagatgagca cagccaagac ctgagtggga ggctgcgggc cattctccag    1140
aatcaggaaa actga                                                     1155


<210> 3
<211> 198
<212> PRT
<213> Artificial Sequence

<220>
<223> Description of Artificial Sequence; note =
      synthetic construct

<400> 3


<210> 4
<211> 597
<212> DNA
<213> Artificial Sequence

<220>
<223> Description of Artificial Sequence; note =
      synthetic construct

<400> 4
atggacagcc tcttgatgaa ccggaggaag tttctttacc aattcaaaaa tgtccgctgg      60
gctaagggtc ggcgtgagac ctacctgtgc tacgtagtga agaggcgtga cagtgctaca     120
tccttttcac tggactttgg ttatcttcgc aataagaacg gctgccacgt ggaattgctc     180
ttcctccgct acatctcgga ctgggaccta gaccctggcc gctgctaccg cgtcacctgg     240
ttcacctcct ggagcccctg ctacgactgt gcccgacatg tggccgactt tctgcgaggg     300
aaccccaacc tcagtctgag gatcttcacc gcgcgcctct acttctgtga ggaccgcaag     360
gctgagcccg aggggctgcg gcggctgcac cgcgccgggg tgcaaatagc catcatgacc     420
ttcaaagatt atttttactg ctggaatact tttgtagaaa accatgaaag aactttcaaa     480
gcctgggaag ggctgcatga aaattcagtt cgtctctcca gacagcttcg gcgcatcctt     540
ttgcccctgt atgaggttga tgacttacga gacgcatttc gtactttggg actttga        597


<210> 5
<211> 236
<212> PRT
<213> Artificial Sequence

<220>
<223> Description of Artificial Sequence; note =
      synthetic construct

<400> 5
Met Thr Ser Glu Lys Gly Pro Ser Thr Gly Asp Pro Thr Leu Arg Arg
1               5                   10                  15
Arg Ile Glu Pro Trp Glu Phe Asp Val Phe Tyr Asp Pro Arg Glu Leu
            20                  25                  30

Arg Lys Glu Ala Cys Leu Leu Tyr Glu Ile Lys Trp Gly Met Ser Arg
        35                  40                  45
Lys Ile Trp Arg Ser Ser Gly Lys Asn Thr Thr Asn His Val Glu Val
    50                  55                  60
Asn Phe Ile Lys Lys Phe Thr Ser Glu Arg Asp Phe His Pro Ser Ile
65                  70                  75                  80
Ser Cys Ser Ile Thr Trp Phe Leu Ser Trp Ser Pro Cys Trp Glu Cys
                85                  90                  95
Ser Gln Ala Ile Arg Glu Phe Leu Ser Arg His Pro Gly Val Thr Leu
            100                 105                 110
Val Ile Tyr Val Ala Arg Leu Phe Trp His Met Asp Gln Gln Asn Arg
        115                 120                 125
Gln Gly Leu Arg Asp Leu Val Asn Ser Gly Val Thr Ile Gln Ile Met
    130                 135                 140
Arg Ala Ser Glu Tyr Tyr His Cys Trp Arg Asn Phe Val Asn Tyr Pro
145                 150                 155                 160
Pro Gly Asp Glu Ala His Trp Pro Gln Tyr Pro Pro Leu Trp Met Met
                165                 170                 175
Leu Tyr Ala Leu Glu Leu His Cys Ile Ile Leu Ser Leu Pro Pro Cys
            180                 185                 190
Leu Lys Ile Ser Arg Arg Trp Gln Asn His Leu Thr Phe Phe Arg Leu
        195                 200                 205
His Leu Gln Asn Cys His Tyr Gln Thr Ile Pro Pro His Ile Leu Leu
    210                 215                 220
Ala Thr Gly Leu Ile His Pro Ser Val Ala Trp Arg
225                 230                 235


<210> 6
<211> 863
<212> DNA
<213> Artificial Sequence

<220>
<223> Description of Artificial Sequence; note =
      synthetic construct

<400> 6
gatcccagag gaggaagtcc agagacagag caccatgact tctgagaaag gagaagaatc      60
gaaccctggg agtttgacgt cttctatgac cccagagaac ttcgtaaaga ggcctgtctg     120
ctctacgaaa tcaagtgggg catgagccgg aagatctggc gaagctcagg caaaaacacc     180
accaatcacg tggaagttaa ttttataaaa aaatttacgt cagaaagaga ttttcaccca     240
tccatcagct gctccatcac ctggttcttg tcctggagtc cctgctggga atgctcccag     300
gctattagag agtttctgag tcggcaccct ggtgtgactc tagtgatcta cgtagctcgg     360
cttttttggc acatggatca acaaaatcgg caaggtctca gggaccttgt taacagtgga     420
gtaactattc agattatgag agcatcagag tattatcact gctggaggaa ttttgtcaac     480
tacccacctg gggatgaagc tcactggcca caatacccac ctctgtggat gatgttgtac     540
gcactggagc tgcactgcat aattctaagt cttccaccct gtttaaagat ttcaagaaga     600
tggcaaaatc atcttacatt tttcagactt catcttcaaa actgccatta ccaaacgatt     660
ccgccacaca tccttttagc tacagggctg atacatcctt ctgtggcttg gagatgaata     720
ggatgattcc gtgtgtgtac tgattcaaga acaagcaatg atgacccact aaagagtgaa     780
tgccatttag aatctagaaa tgttcacaag gtaccccaaa actctgtagc ttaaaccaac     840
aataaatatg tattacctct ggc                                             863


<210> 7
<211> 192
<212> PRT
<213> Artificial Sequence

<220>
<223> Description of Artificial Sequence; note =
      synthetic construct

<400> 7
Met Glu Asn Arg Trp Gln Val Met Ile Val Trp Gln Val Asp Arg Met
1               5                   10                  15
Arg Ile Lys Thr Trp Lys Ser Leu Val Lys His His Met Tyr Ile Ser
            20                  25                  30
Lys Lys Ala Lys Glu Trp Val Tyr Arg His His Tyr Glu Ser Thr His
        35                  40                  45
Pro Arg Ile Ser Ser Glu Val His Ile Pro Leu Gly Asp Ala Lys Leu
    50                  55                  60
Val Ile Thr Thr Tyr Trp Gly Leu His Thr Gly Glu Arg Glu Trp His
65                  70                  75                  80
Leu Gly Gln Gly Val Ser Ile Glu Trp Arg Lys Lys Arg Tyr Asn Thr
                85                  90                  95
Gln Val Asp Pro Asp Leu Ala Asp Lys Leu Ile His Leu His Tyr Phe
            100                 105                 110
Asp Cys Phe Ser Asp Ser Ala Ile Arg His Ala Ile Leu Gly His Arg
        115                 120                 125
Val Arg Pro Lys Cys Glu Tyr Gln Ala Gly His Asn Lys Val Gly Ser
    130                 135                 140
Leu Gln Tyr Leu Ala Leu Thr Ala Leu Ile Thr Pro Lys Lys Ile Lys
145                 150                 155                 160
Pro Pro Leu Pro Ser Val Arg Lys Leu Thr Glu Asp Arg Trp Asn Lys
                165                 170                 175
Pro Gln Lys Thr Lys Gly His Arg Gly Ser His Thr Met Asn Gly His
            180                 185                 190


<210> 8
<211> 569
<212> DNA
<213> Artificial Sequence

<220>
<223> Description of Artificial Sequence; note =
      synthetic construct

<400> 8
gatggcaggt gatgattgtg tggcaagtag acaggatgag gattaaaaca tggaaaagtt      60
tagtaaaaca ccatatgtat atttcaaaga aagctaagga atgggtctat agacatcact     120
atgaaagcac tcatccaaga ataagttcag aagtacacat cccactaggg gatgctaaat     180
tagtaataac aacatattgg ggtctgcata caggagaaag agaatggcat ctgggtcagg     240
gagtctccat agaatggagg aaaaagagat ataatacaca agtagaccct gacctagcag     300
acaaactaat ccacctgcat tattttgatt gtttttcaga ctctgctata agacatgcca     360
tattaggaca tagagttagg cctaagtgtg aatatcaagc aggacataac aaggtagggt     420
ctctacagta cttggcacta acagcattaa taacaccaaa aaagataaag ccacctttgc     480
ctagtgttag gaaactaaca gaggatagat ggaacaagcc ccagaagacc aagggccaca     540
gagggagcca tacaatgaat ggacactag                                       569


<210> 9
<211> 4
<212> PRT
<213> Artificial Sequence

<220>
<223> Description of Artificial Sequence; note =
      synthetic construct

<400> 9
Arg Gly Tyr Trp
1

<210> 10
<211> 20
<212> DNA
<213> Artificial Sequence

<220>
<223> Description of Artificial Sequence; note =
      synthetic construct

<400> 10
cactttaggg agggctgtcc                                                    20


<210> 11
<211> 20
<212> DNA
<213> Artificial Sequence

<220>
<223> Description of Artificial Sequence; note =
      synthetic construct

<400> 11
ctgtgatcag ctggagatgg                                                    20


<210> 12
<211> 33
<212> DNA
<213> Artificial Sequence

<220>
<223> Description of Artificial Sequence; note =
      synthetic construct

<400> 12
ctcccatggc aaagcctcac ttcagaaaca cag                                     33

<210> 13
<211> 35
<212> DNA
<213> Artificial Sequence


<220>
<223> Description of Artificial Sequence; note =
      synthetic construct

<400> 13
ctcctcgagg ttttcctgat tctggagaat ggccc                                   35


<210> 14
<211> 51
<212> PRT
<213> Artificial Sequence

<220>
<223> Description of Artificial Sequence; note =
      synthetic construct

<400> 14

Gln Gln Asn Arg Gln Gly Leu Arg Asp Leu Val Asn Ser Gly Val Thr
1               5                   10                  15
Ile Gln Ile Met Arg Ala Ser Glu Tyr Tyr His Cys Trp Arg Asn Phe
            20                  25                  30
Val Asn Tyr Pro Pro Gly Asp Glu Ala His Trp Pro Gln Tyr Pro Pro
        35                  40                  45
Leu Trp Met
    50


<210> 15
<211> 51
<212> PRT
<213> Artificial Sequence

<220>
<223> Description of Artificial Sequence; note =
      synthetic construct

<400> 15
Pro Glu Thr Gln Gln Asn Leu Cys Arg Leu Val Gln Glu Gly Ala Gln
1               5                   10                  15
Val Ala Ala Met Asp Leu Tyr Glu Phe Lys Lys Cys Trp Lys Lys Phe
            20                  25                  30
Val Asp Asn Gly Gly Arg Arg Phe Arg Pro Trp Lys Arg Leu Leu Thr
        35                  40                  45
Asn Phe Arg
    50


<210> 16
<211> 48
<212> PRT
<213> Artificial Sequence

<220>
<223> Description of Artificial Sequence; note =
      synthetic construct

<400> 16
Asp Gln Gln Asn Arg Gln Gly Leu Arg Asp Leu Val Asn Ser Gly Val
1               5                   10                  15
Thr Ile Gln Ile Met Arg Ala Ser Glu Tyr Tyr His Cys Trp Arg Asn
            20                  25                  30
Phe Val Asn Tyr Pro Pro Gly Asp Glu Ala His Trp Pro Gln Tyr Pro
        35                  40                  45


<210> 17
<211> 47
<212> PRT
<213> Artificial Sequence

<220>
<223> Description of Artificial Sequence; note =
      synthetic construct

<400> 17
Lys Arg Pro Phe Gln Lys Gly Leu Cys Ser Leu Trp Gln Ser Gly Ile
1               5                   10                  15
Leu Val Asp Val Met Asp Leu Pro Gln Phe Thr Asp Cys Trp Thr Asn
            20                  25                  30
Phe Val Asn Pro Lys Arg Pro Phe Trp Pro Trp Lys Gly Leu Glu

35                  40                  45

<210> 18
<211> 51
<212> PRT
<213> Artificial Sequence

<220>
<223> Description of Artificial Sequence; note =
      synthetic construct

<400> 18
Gln Gln Asn Arg Gln Gly Leu Arg Asp Leu Val Asn Ser Gly Val Thr
1               5                   10                  15
Ile Gln Ile Met Arg Ala Ser Glu Tyr Tyr His Cys Trp Arg Asn Phe
            20                  25                  30
Val Asn Tyr Pro Pro Gly Asp Glu Ala His Trp Pro Gln Tyr Pro Pro
        35                  40                  45
Leu Trp Met
    50

<210> 19
<211> 51
<212> PRT
<213> Artificial Sequence

<220>
<223> Description of Artificial Sequence; note =
      synthetic construct

<400> 19
Pro Glu Asn Gln Gln Asn Leu Cys Arg Leu Val Gln Glu Gly Ala Gln
1               5                   10                  15
Val Ala Ala Met Asp Leu Tyr Glu Phe Lys Lys Cys Trp Lys Lys Phe
            20                  25                  30
Val Asp Asn Gly Gly Arg Arg Phe Arg Pro Trp Lys Lys Leu Leu Thr
        35                  40                  45
Asn Phe Arg
    50

<210> 20
<211> 45
<212> PRT
<213> Artificial Sequence

<220>
<223> Description of Artificial Sequence; note =
      synthetic construct

<400> 20
Asn Arg Gln Gly Leu Arg Asp Leu Val Asn Ser Gly Val Thr Ile Gln
1               5                   10                  15
Ile Met Arg Ala Ser Glu Tyr Tyr His Cys Trp Arg Asn Phe Val Asn
            20                  25                  30
Tyr Pro Pro Gly Asp Glu Ala His Trp Pro Gln Tyr Pro
        35                  40                  45

<210> 21
<211> 44

<212> PRT
<213> Artificial Sequence

<220>
<223> Description of Artificial Sequence; note =
      synthetic construct

<400> 21
Phe Gln Lys Gly Leu Cys Ser Leu Trp Gln Ser Gly Ile Leu Val Asp
1               5                   10                  15
Val Met Asp Leu Pro Gln Phe Thr Asp Cys Trp Thr Asn Phe Val Asn
            20                  25                  30
Pro Lys Arg Pro Phe Trp Pro Trp Lys Gly Leu Glu
        35                  40


<210> 22
<211> 21
<212> DNA
<213> Artificial Sequence

<220>
<223> Description of Artificial Sequence; note =
      synthetic construct

<400> 22
aagtcaaaga aagaaagaca a                                                21


<210> 23
<211> 21
<212> DNA
<213> Artificial Sequence

<220>
<223> Description of Artificial Sequence; note =
      synthetic construct

<400> 23
aagtcaaaga aagaaagaca a                                                21


<210> 24
<211> 39
<212> DNA
<213> Artificial Sequence

<220>
<223> Description of Artificial Sequence; note =
      synthetic construct

<400> 24
ttcaggaagg agcccaggtg gctgccatgg acctatacg                             39


<210> 25
<211> 39
<212> DNA
<213> Artificial Sequence

<220>
<223> Description of Artificial Sequence; note =
      synthetic construct

<400> 25
ttcaggaagg agcccaggtg gctgccatgg acctatacg                                39

<210> 26
<211> 24
<212> DNA
<213> Artificial Sequence

<220>
<223> Description of Artificial Sequence; note =
      synthetic construct

<400> 26
tggtggacgt catggacctc ccac                                                24

<210> 27
<211> 24
<212> DNA
<213> Artificial Sequence

<220>
<223> Description of Artificial Sequence; note =
      synthetic construct

<400> 27
tggtggacgt catggacctc ccac                                                24

<210> 28
<211> 39
<212> DNA
<213> Artificial Sequence

<220>
<223> Description of Artificial Sequence; note =
      synthetic construct

<400> 28
aatggccaag cgccactcaa aggctgcctg ctaagcgag                                39

<210> 29
<211> 39
<212> DNA
<213> Artificial Sequence

<220>
<223> Description of Artificial Sequence; note =
      synthetic construct

<400> 29
aatggccaag cgccactcaa aggctgcctg ctaagcgag                                39

<210> 30
<211> 56
<212> DNA
<213> Artificial Sequence

<220>

<223> Description of Artificial Sequence; note =
      synthetic construct

<400> 30
aaaaaactgc ttacaaattt tagataccag gattctaagc ttcaggagat tctgag          56


<210> 31
<211> 56
<212> DNA
<213> Artificial Sequence

<220>
<223> Description of Artificial Sequence; note =
      synthetic construct

<400> 31
aaaagactgc ttacaaattt tagataccag gattctaagc ttcaggagat tctgag          56


<210> 32
<211> 46
<212> DNA
<213> Artificial Sequence

<220>
<223> Description of Artificial Sequence; note =
      synthetic construct

<400> 32
acaaaaggtc tcccagagac gaggttctgc gtggagggca ggcgag                     46


<210> 33
<211> 46
<212> DNA
<213> Artificial Sequence

<220>
<223> Description of Artificial Sequence; note =
      synthetic construct

<400> 33
acaaaaggtc tcccagagac gaggttctgg gtggagggca ggtgag                     46


<210> 34
<211> 53
<212> DNA
<213> Artificial Sequence

<220>
<223> Description of Artificial Sequence; note =
      synthetic construct

<400> 34
atgggaccat tctgtctggg atgcagccat cgcaaatgct attcaccgat cag             53


<210> 35
<211> 53
<212> DNA
<213> Artificial Sequence

<220>
<223> Description of Artificial Sequence; note =
      synthetic construct

<400> 35
atgggaccat tctgtctggg atgcagccat cgcaaatgct attcaccgat cag          53


<210> 36
<211> 4
<212> RNA
<213> Artificial Sequence

<220>
<223> Description of Artificial Sequence; note =
      synthetic construct

<400> 36
ugau                                                                  4


<210> 37
<211> 20
<212> DNA
<213> Artificial Sequence

<220>
<223> Description of Artificial Sequence; note =
      synthetic construct

<400> 37
ttacctgggt ctatggcagt                                                20


<210> 38
<211> 19
<212> DNA
<213> Artificial Sequence

<220>
<223> Description of Artificial Sequence; note =
      synthetic construct

<400> 38
tgaaggctca gaatccccc                                                 19


<210> 39
<211> 738
<212> PRT
<213> Artificial Sequence

<220>
<223> Description of Artificial Sequence; note =
      synthetic construct

<400> 39
Met Arg Lys Lys Arg Arg Gln Arg Arg Arg Val Asp Ser Leu Leu Met
 1               5                  10                  15
Asn Arg Arg Lys Phe Leu Tyr Gln Phe Lys Asn Val Arg Trp Ala Lys
            20                  25                  30
Gly Arg Arg Glu Thr Tyr Leu Cys Tyr Val Val Lys Arg Arg Asp Ser

35                  40                  45
Ala Thr Ser Phe Ser Leu Asp Phe Gly Tyr Leu Arg Asn Lys Asn Gly
    50                  55                  60
Cys His Val Glu Leu Leu Phe Leu Arg Tyr Ile Ser Asp Trp Asp Leu
65                  70                  75                  80
Asp Pro Gly Arg Cys Tyr Arg Val Thr Trp Phe Thr Ser Trp Ser Pro
                85                  90                  95
Cys Tyr Asp Cys Ala Arg His Val Ala Asp Phe Leu Arg Gly Asn Pro
            100                 105                 110
Asn Leu Ser Leu Arg Ile Phe Thr Ala Arg Leu Tyr Phe Cys Glu Asp
        115                 120                 125
Arg Lys Ala Glu Pro Glu Gly Leu Arg Arg Leu His Arg Ala Gly Val
    130                 135                 140
Gln Ile Ala Ile Met Thr Phe Lys Asp Tyr Phe Tyr Cys Trp Asn Thr
145                 150                 155                 160
Phe Val Glu Asn His Glu Arg Thr Phe Lys Ala Trp Glu Gly Leu His
                165                 170                 175
Glu Asn Ser Val Arg Leu Ser Arg Gln Leu Arg Arg Ile Leu Leu Pro
            180                 185                 190
Leu Tyr Glu Val Asp Asp Leu Arg Asp Ala Phe Arg Thr Leu Gly Leu
        195                 200                 205
His Ala Ala Met Ala Asp Thr Phe Leu Glu His Met Cys Arg Leu Asp
    210                 215                 220
Ile Asp Ser Glu Pro Thr Ile Ala Arg Asn Thr Gly Ile Ile Cys Thr
225                 230                 235                 240
Ile Gly Pro Ala Ser Arg Ser Val Asp Lys Leu Lys Glu Met Ile Lys
                245                 250                 255
Ser Gly Met Asn Val Ala Arg Leu Asn Phe Ser His Gly Thr His Glu
            260                 265                 270
Tyr His Glu Gly Thr Ile Lys Asn Val Arg Glu Ala Thr Glu Ser Phe
        275                 280                 285
Ala Ser Asp Pro Ile Thr Tyr Arg Pro Val Ala Ile Ala Leu Asp Thr
    290                 295                 300
Lys Gly Pro Glu Ile Arg Thr Gly Leu Ile Lys Gly Ser Gly Thr Ala
305                 310                 315                 320
Glu Val Glu Leu Lys Lys Gly Ala Ala Leu Lys Val Thr Leu Asp Asn
                325                 330                 335
Ala Phe Met Glu Asn Cys Asp Glu Asn Val Leu Trp Val Asp Tyr Lys
            340                 345                 350
Asn Leu Ile Lys Val Ile Asp Val Gly Ser Lys Ile Tyr Val Asp Asp
        355                 360                 365
Gly Leu Ile Ser Leu Leu Val Lys Glu Lys Gly Lys Asp Phe Val Met
    370                 375                 380
Thr Glu Val Glu Asn Gly Gly Met Leu Gly Ser Lys Lys Gly Val Asn
385                 390                 395                 400
Leu Pro Gly Ala Ala Val Asp Leu Pro Ala Val Ser Glu Lys Asp Ile
                405                 410                 415
Gln Asp Leu Lys Phe Gly Val Glu Gln Asn Val Asp Met Val Phe Ala
            420                 425                 430
Ser Phe Ile Arg Lys Ala Ala Asp Val His Ala Val Arg Lys Val Leu
        435                 440                 445
Gly Glu Lys Gly Lys His Ile Lys Ile Ile Ser Lys Ile Glu Asn His
    450                 455                 460
Glu Gly Val Arg Arg Phe Asp Glu Ile Met Glu Ala Ser Asp Gly Ile
465                 470                 475                 480
Met Val Ala Arg Gly Asp Leu Gly Ile Glu Ile Pro Ala Glu Lys Val
                485                 490                 495
Phe Leu Ala Gln Lys Met Met Ile Gly Arg Cys Asn Arg Ala Gly Lys
            500                 505                 510
Pro Ile Ile Cys Ala Thr Gln Met Leu Glu Ser Met Ile Lys Lys Pro
        515                 520                 525
Arg Pro Thr Arg Ala Glu Gly Ser Asp Val Ala Asn Ala Val Leu Asp
    530                 535                 540

Gly Ala Asp Cys Ile Met Leu Ser Gly Glu Thr Ala Lys Gly Asp Tyr
545                 550                 555                 560
Pro Leu Glu Ala Val Arg Met Gln His Ala Ile Ala Arg Glu Ala Glu
                565                 570                 575
Ala Ala Met Phe His Arg Gln Gln Phe Glu Glu Ile Leu Arg His Ser
            580                 585                 590
Val His His Arg Glu Pro Ala Asp Ala Met Ala Ala Gly Ala Val Glu
        595                 600                 605
Ala Ser Phe Lys Cys Leu Ala Ala Ala Leu Ile Val Met Thr Glu Ser
    610                 615                 620
Gly Arg Ser Ala His Leu Val Ser Arg Tyr Arg Pro Arg Ala Pro Ile
625                 630                 635                 640
Ile Ala Val Thr Arg Asn Asp Gln Thr Ala Arg Gln Ala His Leu Tyr
                645                 650                 655
Arg Gly Val Phe Pro Val Leu Cys Lys Gln Pro Ala His Asp Ala Trp
            660                 665                 670
Ala Glu Asp Val Asp Leu Arg Val Asn Leu Gly Met Asn Val Gly Lys
        675                 680                 685
Ala Arg Gly Phe Phe Lys Thr Gly Asp Leu Val Ile Val Leu Thr Gly
    690                 695                 700
Trp Arg Pro Gly Ser Gly Tyr Thr Asn Thr Met Arg Val Val Pro Val
705                 710                 715                 720
Pro Leu Glu Tyr Pro Tyr Asp Val Pro Asp Tyr Ala His His His His
                725                 730                 735
His His

<210> 40
<211> 2217
<212> DNA
<213> Artificial Sequence

<220>
<223> Description of Artificial Sequence; note =
      synthetic construct

<400> 40
atgagaaaaa aaagaagaca aagaagaaga gtggacagcc tcttgatgaa ccggaggaag      60
tttctttacc aattcaaaaa tgtccgctgg gctaagggtc ggcgtgagac ctacctgtgc     120
tacgtagtga agaggcgtga cagtgctaca tccttttcac tggactttgg ttatcttcgc     180
aataagaacg gctgccacgt ggaattgctc ttcctccgct acatctcgga ctgggaccta     240
gaccctggcc gctgctaccg cgtcacctgg ttcacctcct ggagcccctg ctacgactgt     300
gcccgacatg tggccgactt tctgcgaggg aaccccaacc tcagtctgag gatcttcacc     360
gcgcgcctct acttctgtga ggaccgcaag gctgagcccg aggggctgcg gcggctgcac     420
cgcgccgggg tgcaaatagc catcatgacc ttcaaagatt atttttactg ctggaatact     480
tttgtagaaa accatgaaag aactttcaaa gcctgggaag ggctgcatga aaattcagtt     540
cgtctctcca gacagcttcg acgaatcctt ttgcccctgt atgaggttga tgacttacga     600
gacgcatttc gtactttggg acttcacgct gccatggcag acacctttct ggagcacatg     660
tgccgcctgg acatcgactc cgagccaacc attgccagaa acaccggcat catctgcacc     720
atcggcccag cctcccgctc tgtggacaag ctgaaggaaa tgattaaatc tggaatgaat     780
gttgcccgcc tcaacttctc gcacggcacc cacgagtatc atgagggcac aattaagaac     840
gtgcgagagg ccacagagag ctttgcctct gacccgatca cctacagacc tgtggctatt     900
gcactggaca ccaagggacc tgaaatccga actggactca tcaagggaag tggcacagca     960
gaggtggagc tcaagaaggg cgcagctctc aaagtgacgc tggacaatgc cttcatggag    1020
aactgcgatg agaatgtgct gtgggtggac tacaagaacc tcatcaaagt tatagatgtg    1080
ggcagcaaaa tctatgtgga tgacggtctc atttccttgc tggttaagga gaaaggcaag    1140
gactttgtca tgactgaggt tgagaacggt ggcatgcttg gtagtaagaa gggagtgaac    1200
ctcccaggtg ctgcggtcga cctgcctgca gtctcagaga aggacattca ggacctgaaa    1260
tttggcgtgg agcagaatgt ggacatggtg ttcgcttcct tcatccgcaa agctgctgat    1320
gtccatgctg tcaggaaggt gctaggggaa aagggaaagc acatcaagat tatcagcaag    1380
attgagaatc acgagggtgt gcgcaggttt gatgagatca tggaggccag cgatggcatt    1440
atggtggccc gtggtgacct gggtattgag atccctgctg aaaaagtctt cctcgcacag    1500
aagatgatga ttgggcgctg caacagggct ggcaaaccca tcatttgtgc cactcagatg    1560

ttggaaagca tgatcaagaa acctcgcccg acccgcgctg agggcagtga tgttgccaat 1620
gcagttctgg atggagcaga ctgcatcatg ctgtctgggg agaccgccaa gggagactac 1680
ccactggagg ctgtgcgcat gcagcacgct attgctcgtg aggctgaggc cgcaatgttc 1740
catcgtcagc agtttgaaga aatcttacgc cacagtgtac accacaggga gcctgctgat 1800
gccatggcag caggcgcggt ggaggcctcc tttaagtgct tagcagcagc tctgatagtt 1860
atgaccgagt ctggcaggtc tgcacacctg gtgtcccggt accgcccgcg ggctcccatc 1920
atcgccgtca cccgcaatga ccaaacagca cgccaggcac acctgtaccg cggcgtcttc 1980
cccgtgctgt gcaagcagcc ggcccacgat gcctgggcag aggatgtgga tctccgtgtg 2040
aacctgggca tgaatgtcgg caaagcccgt ggattcttca agaccgggga cctggtgatc 2100
gtgctgacgg gctggcgccc cggctccggc tacaccaaca ccatgcgggt ggtgcccgtg 2160
ccactcgagt acccctacga cgtgcccgac tacgcccacc accaccacca ccactga 2217

<210> 41
<211> 530
<212> PRT
<213> Artificial Sequence

<220>
<223> Description of Artificial Sequence; note =
      synthetic construct

<400> 41
Met Ser Lys His His Asp Ala Gly Thr Ala Phe Ile Gln Thr Gln Gln
1               5                   10                  15
Leu His Ala Ala Met Ala Asp Thr Phe Leu Glu His Met Cys Arg Leu
            20                  25                  30
Asp Ile Asp Ser Glu Pro Thr Ile Ala Arg Asn Thr Gly Ile Ile Cys
        35                  40                  45
Thr Ile Gly Pro Ala Ser Arg Ser Val Asp Lys Leu Lys Glu Met Ile
    50                  55                  60
Lys Ser Gly Met Asn Val Ala Arg Leu Asn Phe Ser His Gly Thr His
65                  70                  75                  80
Glu Tyr His Glu Gly Thr Ile Lys Asn Val Arg Glu Ala Thr Glu Ser
                85                  90                  95
Phe Ala Ser Asp Pro Ile Thr Tyr Arg Pro Val Ala Ile Ala Leu Asp
            100                 105                 110
Thr Lys Gly Pro Glu Ile Arg Thr Gly Leu Ile Lys Gly Ser Gly Thr
        115                 120                 125
Ala Glu Val Glu Leu Lys Lys Gly Ala Ala Leu Lys Val Thr Leu Asp
    130                 135                 140
Asn Ala Phe Met Glu Asn Cys Asp Glu Asn Val Leu Trp Val Asp Tyr
145                 150                 155                 160
Lys Asn Leu Ile Lys Val Ile Asp Val Gly Ser Lys Ile Tyr Val Asp
                165                 170                 175
Asp Gly Leu Ile Ser Leu Leu Val Lys Glu Lys Gly Lys Asp Phe Val
            180                 185                 190
Met Thr Glu Val Glu Asn Gly Gly Met Leu Gly Ser Lys Lys Gly Val
        195                 200                 205
Asn Leu Pro Gly Ala Ala Val Asp Leu Pro Ala Val Ser Glu Lys Asp
    210                 215                 220
Ile Gln Asp Leu Lys Phe Gly Val Glu Gln Asn Val Asp Met Val Phe
225                 230                 235                 240
Ala Ser Phe Ile Arg Lys Ala Ala Asp Val His Ala Val Arg Lys Val
                245                 250                 255
Leu Gly Glu Lys Gly Lys His Ile Lys Ile Ile Ser Lys Ile Glu Asn
            260                 265                 270
His Glu Gly Val Arg Arg Phe Asp Glu Ile Met Glu Ala Ser Asp Gly
        275                 280                 285
Ile Met Val Ala Arg Gly Asp Leu Gly Ile Glu Ile Pro Ala Glu Lys
    290                 295                 300
Val Phe Leu Ala Gln Lys Met Met Ile Gly Arg Cys Asn Arg Ala Gly
305                 310                 315                 320

Lys Pro Ile Ile Cys Ala Thr Gln Met Leu Glu Ser Met Ile Lys Lys
                325                 330                 335
Pro Arg Pro Thr Arg Ala Glu Gly Ser Asp Val Ala Asn Ala Val Leu
            340                 345                 350
Asp Gly Ala Asp Cys Ile Met Leu Ser Gly Glu Thr Ala Lys Gly Asp
        355                 360                 365
Tyr Pro Leu Glu Ala Val Arg Met Gln His Ala Ile Ala Arg Glu Ala
    370                 375                 380
Glu Ala Ala Met Phe His Arg Gln Gln Phe Glu Glu Ile Leu Arg His
385                 390                 395                 400
Ser Val His His Arg Glu Pro Ala Asp Ala Met Ala Ala Gly Ala Val
                405                 410                 415
Glu Ala Ser Phe Lys Cys Leu Ala Ala Ala Leu Ile Val Met Thr Glu
            420                 425                 430
Ser Gly Arg Ser Ala His Leu Val Ser Arg Tyr Arg Pro Arg Ala Pro
        435                 440                 445
Ile Ile Ala Val Thr Arg Asn Asp Gln Thr Ala Arg Gln Ala His Leu
    450                 455                 460
Tyr Arg Gly Val Phe Pro Val Leu Cys Lys Gln Pro Ala His Asp Ala
465                 470                 475                 480
Trp Ala Glu Asp Val Asp Leu Arg Val Asn Leu Gly Met Asn Val Gly
                485                 490                 495
Lys Ala Arg Gly Phe Phe Lys Thr Gly Asp Leu Val Ile Val Leu Thr
            500                 505                 510
Gly Trp Arg Pro Gly Ser Gly Tyr Thr Asn Thr Met Arg Val Val Pro
        515                 520                 525
Val Pro
    530


<210> 42
<211> 1593
<212> DNA
<213> Artificial Sequence

<220>
<223> Description of Artificial Sequence; note =
      synthetic construct

<400> 42
atgtcgaagc accacgatgc agggaccgct ttcatccaga cccagcagct gcacgctgcc      60
atggcagaca cctttctgga gcacatgtgc cgcctggaca tcgactccga gccaaccatt     120
gccagaaaca ccggcatcat ctgcaccatc ggcccagcct cccgctctgt ggacaagctg     180
aaggaaatga ttaaatctgg aatgaatgtt gcccgcctca acttctcgca cggcacccac     240
gagtatcatg agggcacaat taagaacgtg cgagaggcca cagagagctt tgcctctgac     300
ccgatcacct acagacctgt ggctattgca ctggacacca agggacctga aatccgaact     360
ggactcatca agggaagtgg cacagcagag gtggagctca agaagggcgc agctctcaaa     420
gtgacgctgg acaatgcctt catggagaac tgcgatgaga atgtgctgtg ggtggactac     480
aagaacctca tcaaagttat agatgtgggc agcaaaatct atgtggatga cggtctcatt     540
tccttgctgg ttaaggagaa aggcaaggac tttgtcatga ctgaggttga gaacggtggc     600
atgcttggta gtaagaaggg agtgaacctc ccaggtgctg cggtcgacct gcctgcagtc     660
tcagagaagg acattcagga cctgaaattt ggcgtggagc agaatgtgga catggtgttc     720
gcttccttca tccgcaaagc tgctgatgtc catgctgtca ggaaggtgct aggggaaaag     780
ggaaagcaca tcaagattat cagcaagatt gagaatcacg agggtgtgcg caggtttgat     840
gagatcatgg aggccagcga tggcattatg gtggcccgtg gtgacctggg tattgagatc     900
cctgctgaaa aagtcttcct cgcacagaag atgatgattg ggcgctgcaa cagggctggc     960
aaacccatca tttgtgccac tcagatgttg gaaagcatga tcaagaaacc tcgcccgacc    1020
cgcgctgagg gcagtgatgt tgccaatgca gttctggatg gagcagactg catcatgctg    1080
tctggggaga ccgccaaggg agactaccca ctggaggctg tgcgcatgca gcacgctatt    1140
gctcgtgagg ctgaggccgc aatgttccat cgtcagcagt ttgaagaaat cttacgccac    1200
agtgtacacc acagggagcc tgctgatgcc atggcagcag gcgcggtgga ggcctccttt    1260
aagtgcttag cagcagctct gatagttatg accgagtctg gcaggtctgc acacctggtg    1320
tcccggtacc gcccgcgggc tcccatcatc gccgtcaccc gcaatgacca aacagcacgc    1380

caggcacacc tgtaccgcgg cgtcttcccc gtgctgtgca agcagccggc ccacgatgcc 1440
tgggcagagg atgtggatct ccgtgtgaac ctgggcatga atgtcggcaa agcccgtgga 1500
ttcttcaaga ccggggacct ggtgatcgtg ctgacgggct ggcgccccgg ctccggctac 1560
accaacacca tgcgggtggt gcccgtgcca tga 1593

<210> 43
<211> 9
<212> PRT
<213> Artificial Sequence

<220>
<223> Description of Artificial Sequence; note =
      synthetic construct

<400> 43
Arg Lys Lys Arg Arg Gln Arg Arg Arg
 1               5

<210> 44
<211> 27
<212> DNA
<213> Artificial Sequence

<220>
<223> Description of Artificial Sequence; note =
      synthetic construct

<400> 44
agaaaaaaaa gaagacaaag aagaaga 27

<210> 45
<211> 237
<212> PRT
<213> Artificial Sequence

<220>
<223> Description of Artificial Sequence; note =
      synthetic construct

<400> 45
Met Thr Ser Glu Lys Gly Pro Ser Thr Gly Asp Pro Thr Leu Arg Arg
 1               5                  10                  15
Arg Ile Glu Pro Trp Glu Phe Asp Val Phe Tyr Asp Pro Arg Glu Leu
            20                  25                  30
Arg Lys Glu Ala Cys Leu Leu Tyr Glu Ile Lys Trp Gly Met Ser Arg
        35                  40                  45
Lys Ile Trp Arg Ser Ser Gly Lys Asn Thr Thr Asn His Val Glu Val
    50                  55                  60
Asn Phe Ile Lys Lys Phe Thr Ser Glu Arg Asp Phe His Pro Ser Ile
65                  70                  75                  80
Ser Cys Ser Ile Thr Trp Phe Leu Ser Trp Ser Pro Cys Trp Glu Cys
                85                  90                  95
Ser Gln Ala Ile Arg Glu Phe Leu Ser Arg His Pro Gly Val Thr Leu
            100                 105                 110
Val Ile Leu Tyr Val Ala Arg Leu Phe Trp His Met Asp Gln Gln Asn
        115                 120                 125
Arg Gln Gly Leu Arg Asp Leu Val Asn Ser Gly Val Thr Ile Gln Ile
    130                 135                 140
Met Arg Ala Ser Glu Tyr Tyr His Cys Trp Arg Asn Phe Val Asn Tyr
145                 150                 155                 160

Pro Pro Gly Asp Glu Ala His Trp Pro Gln Tyr Pro Pro Leu Trp Met
                165                 170                 175
Met Leu Tyr Ala Leu Glu Leu His Cys Ile Ile Leu Ser Leu Pro Pro
            180                 185                 190
Cys Leu Lys Ile Ser Arg Arg Trp Gln Asn His Leu Thr Phe Phe Arg
        195                 200                 205
Leu His Leu Gln Asn Cys His Tyr Gln Thr Ile Pro Pro His Ile Leu
    210                 215                 220
Leu Ala Thr Gly Leu Ile His Pro Ser Val Ala Trp Arg
225                 230                 235


<210> 46
<211> 9
<212> PRT
<213> Artificial Sequence

<220>
<223> Description of Artificial Sequence; note =
      synthetic construct

<400> 46
Tyr Pro Tyr Asp Val Pro Asp Tyr Ala
 1               5


<210> 47
<211> 27
<212> DNA
<213> Artificial Sequence

<220>
<223> Description of Artificial Sequence; note =
      synthetic construct

<400> 47
   tacccctacg acgtgcccga ctacgcc                                          27


<210> 48
<211> 429
<212> PRT
<213> Artificial Sequence

<220>
<223> Description of Artificial Sequence; note =
      synthetic construct

<400> 48
Met Gly Pro Phe Cys Leu Gly Cys Ser His Arg Lys Cys Tyr Ser Pro
 1               5                  10                  15
Ile Arg Asn Leu Ile Ser Gln Glu Thr Phe Lys Phe His Phe Lys Asn
            20                  25                  30
Leu Arg Tyr Ala Ile Asp Arg Lys Asp Thr Phe Leu Cys Tyr Glu Val
        35                  40                  45
Thr Arg Lys Asp Cys Asp Ser Pro Val Ser Leu His His Gly Val Phe
    50                  55                  60
Lys Asn Lys Asp Asn Ile His Ala Glu Ile Cys Phe Leu Tyr Trp Phe
65                  70                  75                  80
His Asp Lys Val Leu Lys Val Leu Ser Pro Arg Glu Glu Phe Lys Ile
                85                  90                  95
Thr Trp Tyr Met Ser Trp Ser Pro Cys Phe Glu Cys Ala Glu Gln Val
            100                 105                 110

Leu Arg Phe Leu Ala Thr His His Asn Leu Ser Leu Asp Ile Phe Ser
        115                 120                 125
Ser Arg Leu Tyr Asn Ile Arg Asp Pro Glu Asn Gln Gln Asn Leu Cys
    130                 135                 140
Arg Leu Val Gln Glu Gly Ala Gln Val Ala Ala Met Asp Leu Tyr Glu
145                 150                 155                 160
Phe Lys Lys Cys Trp Lys Lys Phe Val Asp Asn Gly Gly Arg Arg Phe
                165                 170                 175
Arg Pro Trp Lys Lys Leu Leu Thr Asn Phe Arg Tyr Gln Asp Ser Lys
            180                 185                 190
Leu Gln Glu Ile Leu Arg Pro Cys Tyr Ile Pro Val Pro Ser Ser Ser
        195                 200                 205
Ser Ser Thr Leu Ser Asn Ile Cys Leu Thr Lys Gly Leu Pro Glu Thr
    210                 215                 220
Arg Phe Cys Val Glu Gly Arg Arg Val His Leu Leu Ser Glu Glu Glu
225                 230                 235                 240
Phe Tyr Ser Gln Phe Tyr Asn Gln Arg Val Lys His Leu Cys Tyr Tyr
                245                 250                 255
His Gly Met Lys Pro Tyr Leu Cys Tyr Gln Leu Glu Gln Phe Asn Gly
            260                 265                 270
Gln Ala Pro Leu Lys Gly Cys Leu Leu Ser Glu Lys Gly Lys Gln His
        275                 280                 285
Ala Glu Ile Leu Phe Leu Asp Lys Ile Arg Ser Met Glu Leu Ser Gln
    290                 295                 300
Val Ile Ile Thr Cys Tyr Leu Thr Trp Ser Pro Cys Pro Asn Cys Ala
305                 310                 315                 320
Trp Gln Leu Ala Ala Phe Lys Arg Asp Arg Pro Asp Leu Ile Leu His
                325                 330                 335
Ile Tyr Thr Ser Arg Leu Tyr Phe His Trp Lys Arg Pro Phe Gln Lys
            340                 345                 350
Gly Leu Cys Ser Leu Trp Gln Ser Gly Ile Leu Val Asp Val Met Asp
        355                 360                 365
Leu Pro Gln Phe Thr Asp Cys Trp Thr Asn Phe Val Asn Pro Lys Arg
    370                 375                 380
Pro Phe Trp Pro Trp Lys Gly Leu Glu Ile Ile Ser Arg Arg Thr Gln
385                 390                 395                 400
Arg Arg Leu His Arg Ile Lys Glu Ser Trp Gly Leu Gln Asp Leu Val
                405                 410                 415
Asn Asp Phe Gly Asn Leu Gln Leu Gly Pro Pro Met Ser
            420                 425

<210> 49
<211> 1948
<212> DNA
<213> Artificial Sequence

<220>
<223> Description of Artificial Sequence; note =
      synthetic construct

<400> 49
acttggcccg ggaggtcagt ttcacttctg ggggtcttcc atagcctgct cacagaaaat      60
gcaaccccag cgcatggggc ccagagctgg gatgggacca ttctgtctgg gatgcagcca     120
tcgcaaatgc tattcaccga tcagaaacct gatatctcaa gaaacattca aattccactt     180
taagaaccta cgctatgcca tagaccggaa agataccttc ttgtgctatg aagtgactag     240
aaaggactgc gattcacccg tctcccttca ccatggggtc tttaagaaca aggacaacat     300
ccacgctgaa atctgctttt tatactggtt ccatgacaaa gtactgaaag tgctgtctcc     360
gagagaagag ttcaagatca cctggtatat gtcctggagc ccctgtttcg aatgtgcaga     420
gcaggtacta aggttcctgg ctacacacca caacctgagc ctggacatct tcagctcccg     480
cctctacaac atacgggacc cagaaaacca gcagaatctt tgcaggctgg ttcaggaagg     540
agcccaggtg gctgccatgg acctatacga atttaaaaag tgttggaaga agtttgtgga     600
caatggcggc aggcgattca ggccttggaa aaaactgctt acaaatttta gataccagga     660

ttctaagctt caggagattc tgagaccttg ctacatcccg gtcccttcca gctcttcatc 720
cactctgtca aatatctgtc taacaaaagg tctcccagag acgaggttct gcgtggaggg 780
caggcgagtg cacctgctaa gtgaagagga attttactcg cagttttaca accaacgagt 840
caagcatctc tgctactacc acggcatgaa gccctatcta tgctaccagc tggagcagtt 900
caatggccaa gcgccactca aaggctgcct gctaagcgag aaaggcaaac agcatgcaga 960
aatcctcttc cttgataaga ttcggtccat ggagctgagc caagtgataa tcacctgcta 1020
cctcacctgg agcccctgcc caaactgtgc ctggcaactg gcggcattca aaagggatcg 1080
tccagatcta attctgcata tctacacctc ccgcctgtat ttccactgga agaggccctt 1140
ccagaagggg ctgtgttctc tgtggcaatc agggatcctg gtggacgtca tggacctccc 1200
acagtttact gactgctgga caaactttgt gaacccgaaa aggccgtttt ggccatggaa 1260
aggattggag ataatcagca ggcgcacaca aaggcggctc cacaggatca aggagtcctg 1320
gggtctgcaa gatttggtga atgactttgg aaacctacag cttggacccc cgatgtcttg 1380
agaggcaaga agagattcaa gaaggtcttt tggtgacccc cccacccaac cccaagtcta 1440
ggagaccttt tgttctcccg tttgtttccc cttttgtttt atcttttgtt gttttgcttt 1500
gttttgaaga cagagtctca ctgggtagct tgctactctg gaactcacta ctagactaag 1560
ctggccttaa actctaaaat ccacctgcca atgccttctg agagccaggc ttaaggtgtg 1620
cgctgcccac tcccagcctt aacccactgt ggcttttcct tcctctttct tttattatct 1680
ttttatctcc cctcaccctc ccgccatcaa taggtactta attttgtact tgaaattttt 1740
aagttgggcc aggcatggtg gagcagcgtg cctctaatcg caggcaggag gatttccacg 1800
agcttgaggc tagcctgatc tacatagtgg gctccaggac agccagaact acacagagac 1860
cctgtctcaa aaataaattt agatagataa atacataaat aaataaatgg aagaagtcaa 1920
agaaagaaag acaaaaaaaa aaaaaaaa 1948

## Description

This invention was made with government support under Grants DK43738-08 and F49620 awarded by the National Institutes of Health and the United States Air Force. The government has certain rights in the invention. This application claims priority United States Provisional Applications 60/419,982, filed October 21, 2002; and 60/401,293, filed August 5, 2002.

### I. BACKGROUND OF THE INVENTION

1. There are several examples of cellular and viral mRNA editing in mammalian cells. (Grosjean and Benne (1998); Smith (1997) RNA 3: 1105-23). Two examples of such editing mechanisms are the adenosine to inosine and cytidine to uridine conversions. (Grosjean and Benne (1998); Smith (1996) Trends in Genetics 12:418-24; Krough (1994) J. Mol. Biol. 235:1501-31). Editing can also occur on DNA.
2. A to I editing involves a family of adenosine deaminases active on RNA (ADARs). ADARs typically have two or more double stranded RNA binding motifs (DRBM) in addition to a catalytic domain whose tertiary structure positions a histidine and two cysteines for zinc ion coordination and a glutamic acid residue as a proton donor. The catalytic domain is conserved at the level of secondary and tertiary structure among ADARs, cytidine nucleoside/nucleotide deaminases and CDARs but differs markedly from that found in adenosine nucleoside/nucleotide deaminases (Higuchi (1993) Cell 75:1361-70). ADAR editing sites are found predominantly in exons and are characterized by RNA secondary structure encompassing the adenosine(s) to be edited. In human exon A to I editing, RNA secondary structure is formed between the exon and a 3' proximal sequence with the downstream intron (Grosjean and Benne (1998); Smith (1997) RNA 3: 1105-23; Smith (1996) Trends in Genetics 12:418-24; Maas (1996) J. Biol. Chem. 271:12221-26; Reuter (1999) Nature 399:75-80; O'Connell (1997) Current Biol. 7:R437-38). Consequently, A to I editing occurs prior to pre-mRNA splicing in the nucleus. The resultant inosine base pairs with cytosine and codons that have been edited, effectively have an A to G change. ADAR mRNA substrates frequently contain multiple A to I editing sites and each site is selectively edited by an ADAR, such as ADAR1 or ADAR2. ADARs typically function autonomously in editing mRNAs. ADARs bind secondary structure at the editing site through their double stranded RNA binding motifs or DRBMs and perform hydrolytic deamination of adenosine through their catalytic domain.
3. Deaminases play an important role in various disease processes. An example of a cytidine deaminase molecule is Activation Induced Deaminase (AID). AID plays a prominent role in class switch recombination and somatic hypermutation, amongst other functions. Several genetic defects in SHM, which lead to hyper-IgM syndrome, have been described in humans (Durandy Biochemical Society p. 815-818, 2002). In addition to the well known role of CD40-ligand-CD40 interaction, these pathologies demonstrate definitively the requirement of CD40-mediated nuclear factor κB activation and the essential role of AID in an efficient humoral response, which includes class switch recombination and the production of high-affinity antibodies. The present invention is directed to overcoming these deficiencies in the art by providing a chimeric protein capable of transduction into B cells for purposes of treating CSR and SHM, as well as other conditions such as B cell lymphoma.
4. CEM15/APOBEC-3G is another cytidine deaminase and APOBEC-1 homolog. CEM15 has been shown to posess antiviral activity. Current therapies for HIV infected patients target the production of new virus by antiviral agents that prevent replication of the viral RNA genomes into DNA prior to integration of the HIV DNA into chromosomal DNA or the disruption of the production or function of viral encoded proteins that are necessary for production of infectious viral particles. Antiviral agents that target viral replication have blunted the course of disease in patients already infected with HIV but these drugs have side effects due to toxicity and, while extending life for many patients, ultimately fail due to the high mutation frequency of HIV-1. Disruption of viral encoded protein production has not been as effective due largely to the high mutation rate of HIV and its consequence of changing the viral protein to one that retains function but no longer is a target for the therapy. A combination of therapies together with better screening of blood supplies and blood products, improved public education and safe-sex practices has curbed the spread of disease only in developed countries but, even in these countries, exhibit incomplete control over the spread of the virus. Needed in the art is a means of editing RNA or DNA involved in disease processes, like HIV, hyper-IgM syndrome, and other cytidine deaminase related diseases, thus preventing or ameliorating the symptoms, and in the case of retroviral-based diseases, eventually irradicating these diseases.

### II. SUMMARY OF THE INVENTION

5. In accordance with the purposes of this invention, as embodied and broadly described herein, this invention, in one aspect, relates to chimeric proteins comprising a protein transduction domain and a deaminase domain and methods of making and using such chimeric proteins. The present invention is an important improvement over the prior art because of the advantages of protein therapy and delivery as compared to gene therapy.
6. Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### III. BRIEF DESCRIPTION OF THE DRAWINGS

7. The accompanying drawings, which are incorporated in and constitute a part of this specification, illustrate several embodiments of the invention and together with the description, serve to explain the principles of the invention.

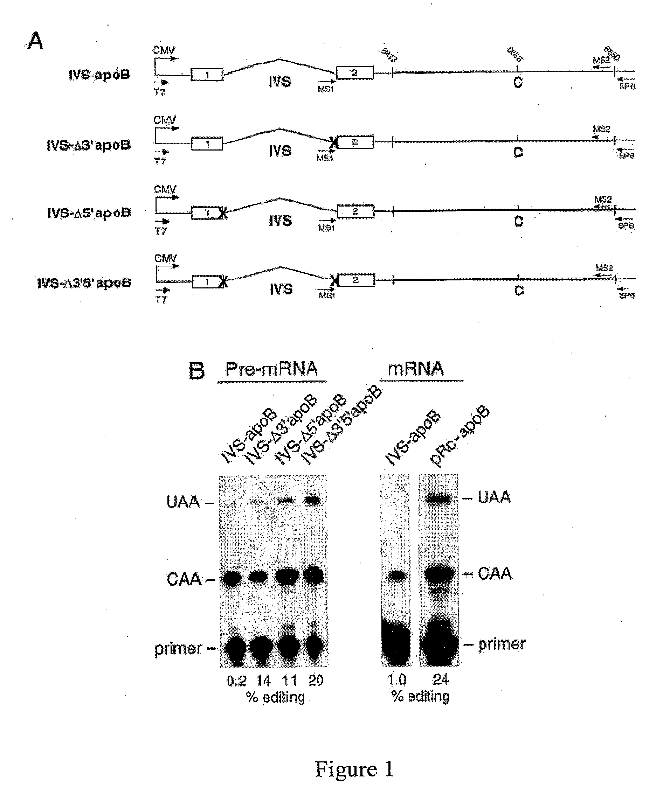

8. Figure 1 shows the effect of introns on editing efficiency. (A) Diagram of the chimeric apoB expression constructs. The intron sequence (IVS) is derived from the adenovirus late leader sequence. Coordinates of the human apoB sequence are shown and the location of PCR amplimers are indicated. X indicates the deleted 5' splice donor or 3' splice acceptor sequences. CMV, cytomegalovirus. (B) Poisoned-primer-extension assays of amplified apoB RNAs. Pre-mRNA and mRNA were amplified with the MS1/MS2 or SP6/T7 amplimers respectively. Editing efficiencies, an average for triplicate transfections, for each RNA are shown beneath. Editing efficiency was determined as the number of counts in edited apoB mRNA (UAA) divided by the sum of counts in UAA plus those in unedited apoB mRNA (CAA) and multiplied by 100.

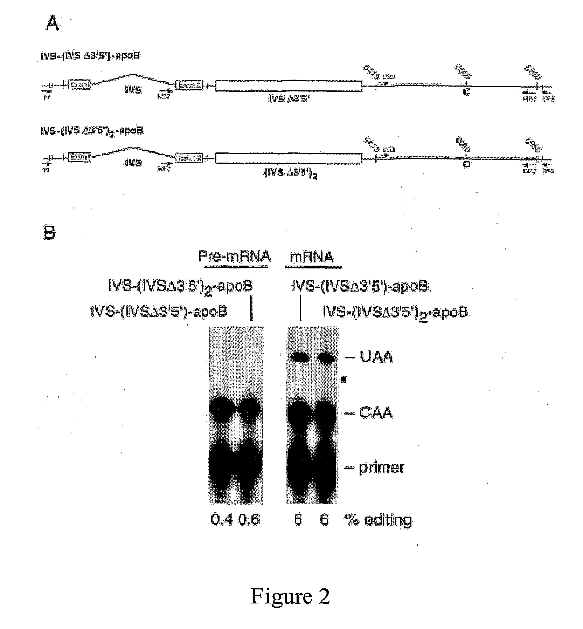

9. Figure 2 shows the effect of intron proximity on editing efficiency. Figure 2a shows a diagram of the chimeric apoB expression constructs. IVS-(IVSΔ3'5')-apoB and IVS-(IVSΔ3'5')₂ -apoB were created by the insertion of one or two copies respectively of the IVSΔ3'5' intron cassette into IVS-apoB. Human apoB coordinates and amplimer annealing sites are indicated (see Figure 1). Figure 2b shows poisoned-primer-extension assays of amplified apoB RNAs. Pre-mRNA and mRNA were amplified with the MS7/MS2 or SP6/T7 amplimers respectively. Editing efficiencies, an average for duplicate transfections, for each RNA are shown beneath.

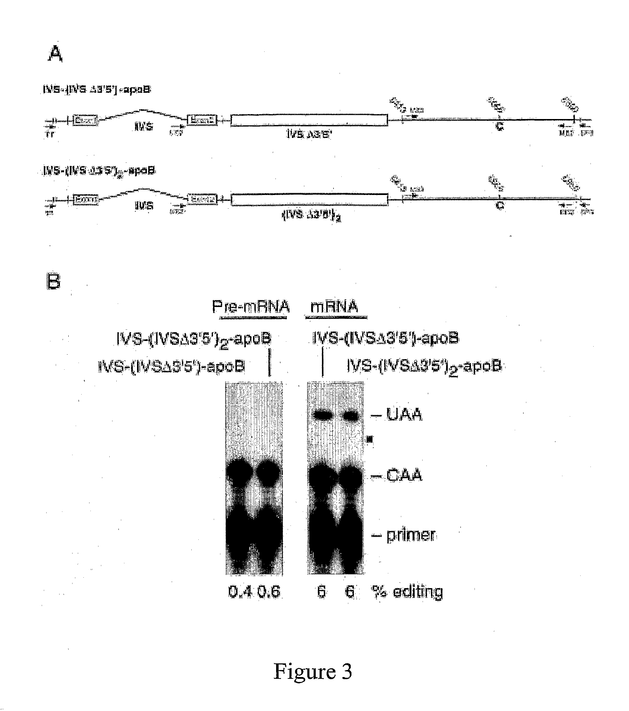

10. Figure 3 shows that the editing sites within introns are poorly utilized. Panel A shows a diagram of the chimeric apoB expression constructs. The apoB editing cassette was inserted as a PCR product into a unique HindIII site 5' of the polypyrimidine tract in IVS-apoB and IVS-Δ3'5'apoB (see Figure 1). Amplimer annealing sites are indicated. Panel B shows poisoned primer extension assays of amplified apoB RNAs. Unspliced pre-mRNA and intron containing RNA were amplified with the Exl/Ex2 or MS D5/MS D6 amplimers respectively. Editing efficiencies, an average for duplicate transfections, for each RNA are shown beneath.

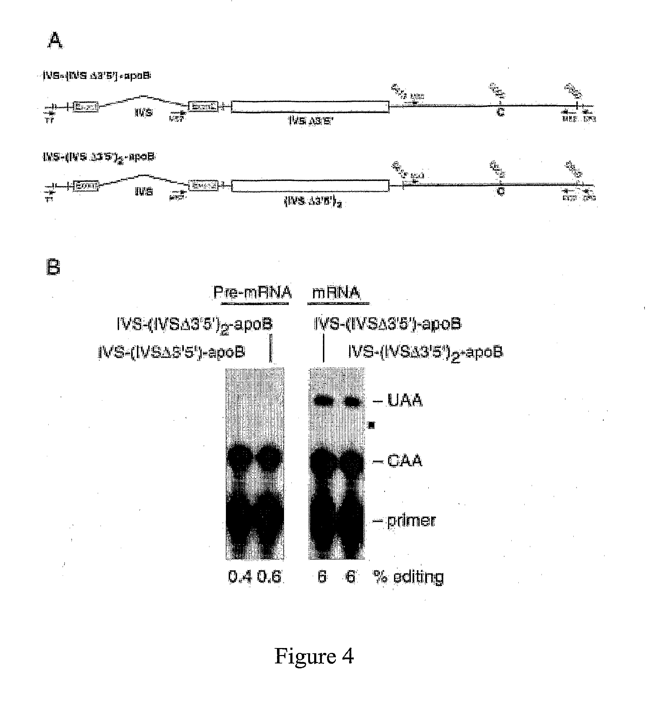

11. Figure 4 shows that editing is regulated by RNA splicing. Figure 4A shows a diagram of the modified CAT reporter construct (CMV128) used in the Rev complementation assay; a gift from Dr Thomas J. Hope of the Salk Institute. The splice donor (SD), splice acceptor (SA), RRE, intron and 3' long tandem repeat (LTR) are from the HIV-1 genome. CMV128 was modified by insertion of the apoB editing cassette as a PCR product into the BamHI site 3' of the CAT gene. Amplimer annealing sites are indicated. Figure 4B shows McArdle cell CAT activity in the absence (Vector) or presence of the Rev transactivator. Values are averages for duplicate experiments. CMVCAT was an assay control transfection. Figure 4C shows poisoned-primer-extension assays of amplified apoB RNAs. 'Intron and exon RNA' was amplified using the EF/MS2 amplimers. Editing efficiencies for each RNA are shown beneath. Promiscuous editing is indicated by '1'.

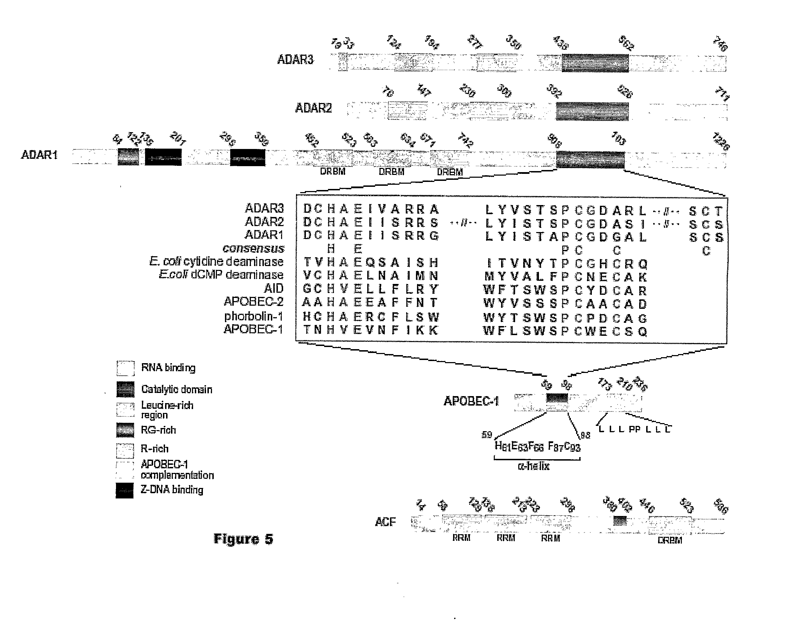

12. Figure 5 shows the adenosine deaminases, cytidine deaminase and cognate RNA binding protein. Conserved residues within the zinc-dependent deaminase domain (ZDD) are shown for the ADARs and APOBEC-1. The catalytic domain of APOBEC-1 is characterized by a ZDD with three zinc ligands (either His or Cys), a glutamic acid, a proline residue and a conserved primary sequence spacing (Mian, I.S., (1998) J Comput Biol. 5:57-72.). The spacing of the terminal cysteine in the primary sequence of ADARs is greater than that seen in cytidine deaminases (represented by as a purple C in the consensus sequence). The ZDD of other deaminases and APOBEC-1 related proteins are shown for comparison along with a consensus ZDD. ADARs bind to their editing sites through double stranded RNA binding domains (DRBM) (Keegan, L.P., (2001) Nat Rev Genet. 2:869-78) and may be catalytically active as homodimer. The indicated residues in the catalytic site of APOBEC-1 bind AU-rich RNA with weak affinity. The leucine rich region (LRR) of APOBEC-1 has been implicated in APOBEC-1 dimerization and shown to be required for editing (Lau, P.P., (1994) Proc Natl Acad Sci USA, 91:8522-6; Oka, K., (1997) J Biol Chem. 272:1456-60) but structural modeling suggests that LRR forms the hydrophobic core of the protein monomer (Navaratnam, N., (1998) J Mol Biol. 275:695-714). ACF complements APOBEC-1 through its APOBEC-1 and RNA bindings activities. The RNA recognition motifs (RRM)s are required for mooring sequence-specific RNA binding and these domains plus sequence flanking them are required for APOBEC-1 interaction and complementation (Blanc, V., (2001) J Biol Chem. 276:46386-93.; Mehta, A., (2002) RNA. 8:69-82). APOBEC-1 complementation activity minimally depends on ACF binding to both APOBEC-1 and mooring sequence RNA. A broad APOBEC-1 complementation region is indicated that is inclusive of all regions implicated in this activity (Blanc, V (2001) J Biol Chem. 276:46386-93.; Mehta, A., (2002) RNA. 8:69-82.).

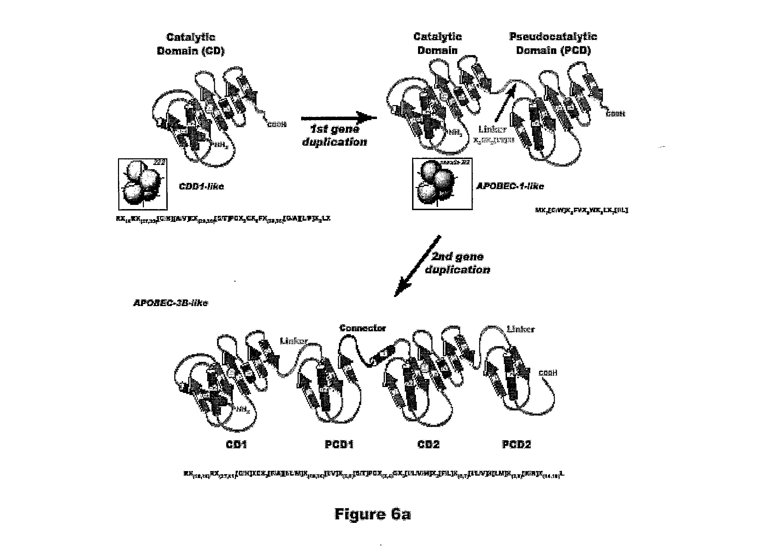

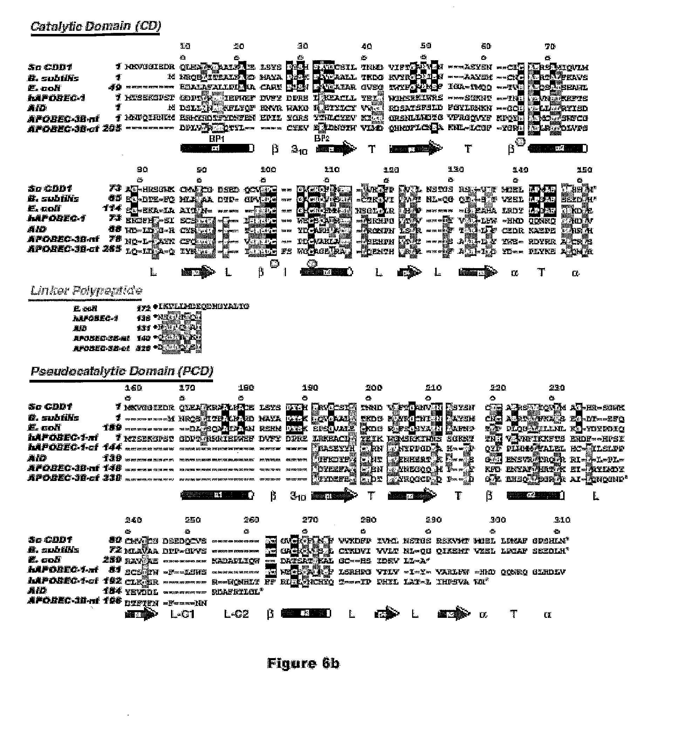

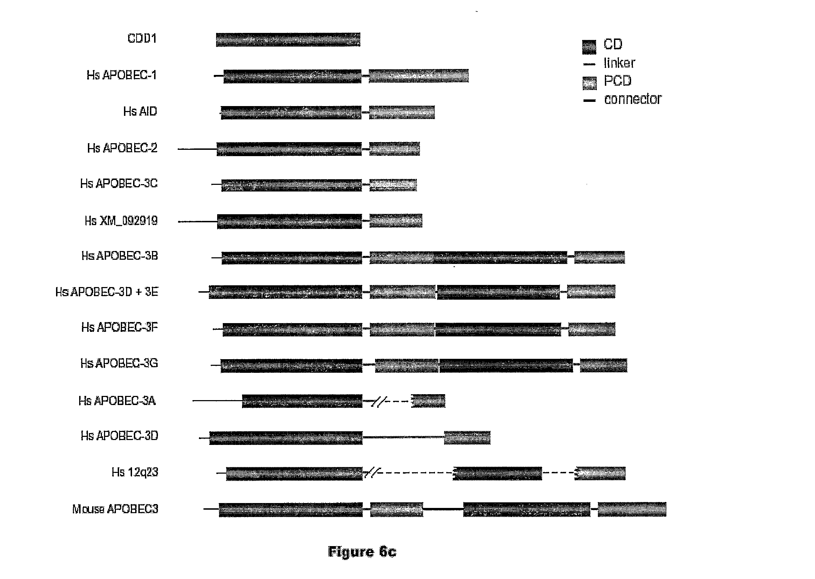

13. Figure 6 shows schematic depictions and structure-based alignments of APOBEC-1 in relation to its related proteins (ARPs). Panel A shows the gene duplication model for cytidine deaminases. CDD1 belongs to the tetrameric class of cytidine deaminases with a quaternary fold nearly identical to that of the tetrameric cytidine deaminase from *B. subtilis* (Johansson, E., (2002) Biochemistry. 41:2563-70). Such tetrameric enzymes exhibit the classical αββαβαββ topology of the Zinc Dependent Deaminase Domain (ZDD) observed first in the Catalytic Domain (CD) of the dimeric enzyme from *E. coli* (Betts, L. (1994) J Mol Biol. 235:635-56). According to the gene duplication model, an ancestral CDD1-like monomer (upper left ribbon) duplicated and fused to produce a bipartite monomer. Over time a C-terminal Pseudo-Catalytic Domain (PCD) arose that lost substrate and Zn²⁺ binding abilities (upper right ribbon). The interdomain CD-PCD junction is characterized by a linker that features conserved Gly residues necessary for editing. The putative function of the PCD is to stabilize the hydrophobic monomer core and to engage in auxiliary factor binding. Modem representatives of this fold include APOBEC-1 and AID. Other ARPs such as APOBEC-3B may have arisen through a second gene duplication to produce a pseudo-homodimer on a single polypeptide chain (lower ribbon); properties of the connector polypeptide are unknown. Signature sequences compiled from strict structure-based alignments (upper) and relaxed computational searches (lower) are shown below respective ribbon diagrams, where X represents any amino acid. Linker regions (lines) and the location of Zn²⁺ binding (spheres) are depicted. Although experimental evidence suggests APOBEC-3B has reduced Zn²⁺ binding and exists as a dimer (Jarmuz, A., (2002) Genomics 79:285-96), modeling studies suggest it binds Zn²⁺ as shown and may function as a monomer. Inset spheres represent the proper (222) CDD1-like quaternary structure symmetry whereas APOBEC-1-like enzymes exhibit pseudo-symmetry relating CD and PCD subunits. Panel B shows the structure based sequence alignment for ARPs. Sequences from human APOBEC-1, AID, and APOBEC-3B were aligned with the known cytidine deaminase structures from *E. coli, B. subtilis* and *S. cerevisiae*. Alignments were optimized to minimize gaps in major secondary structure elements depicted as red tubes (α-helices) and arrows (β-strands); loops, turns, and insertions are marked L and T and i, respectively. L-C 1 and L-C2 represent distinct loop structures in the dimeric versus tetrameric cytidine deaminases; ARP enzymes were modeled according to the dimeric conformation (L-C2). Sections of basic residues that overlap the bipartite NLS are marked BP-1 and BP-2. Panel C shows a schematic diagram of the domain structure observed in APOBEC-1 and related ARPs based upon computer-based sequence alignments using the ZDD signature sequence shown in the lower panel of A.

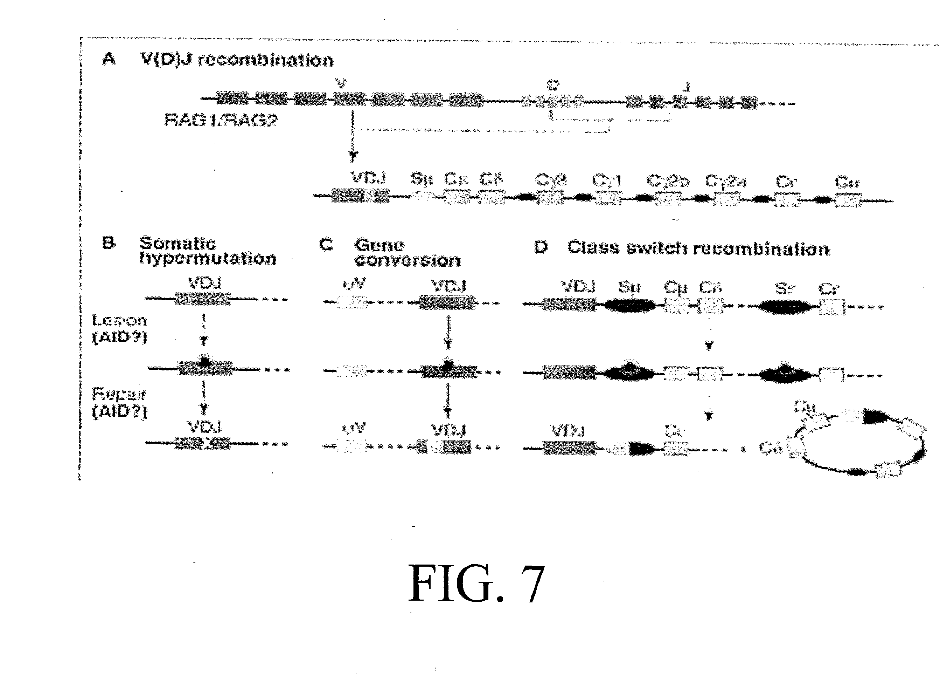

14. Figure 7 shows antibody diversity generated during B-cell development and maturation by multiple genetic mechanisms; namely Ig gene rearrangement, somatic hypermutation and gene conversion. Initially, immature B lymphocytes developing in fetal liver or adult bone marrow use RAG1 and RAG2 proteins to generate DNA double strand breaks whose ends are rejoined by non-homologous end joining. The rearranged immunoglobulin V (variable), D (diversity) and J (joining) gene segments at the Ig heavy chain locus encode a variable region that is expressed initially with the µ constant region (Cµ) to form a primary antibody repertoire composed of IgM antibodies (Figure 7a). In sheep, rabbit and chicken, additional pre-immune diversification is mediated by gene conversion (GC) in which stretches of nucleotide sequences from one of several pseudogene V elements are recombined into the VDJ exon to generate diversity. A secondary antibody repertoire is generated in B cells within germinal centers of secondary lymphoid organs following antigen activation and T-cell help (Figure 7B) (Fugmann (2002) Immunology 295:1244-5).

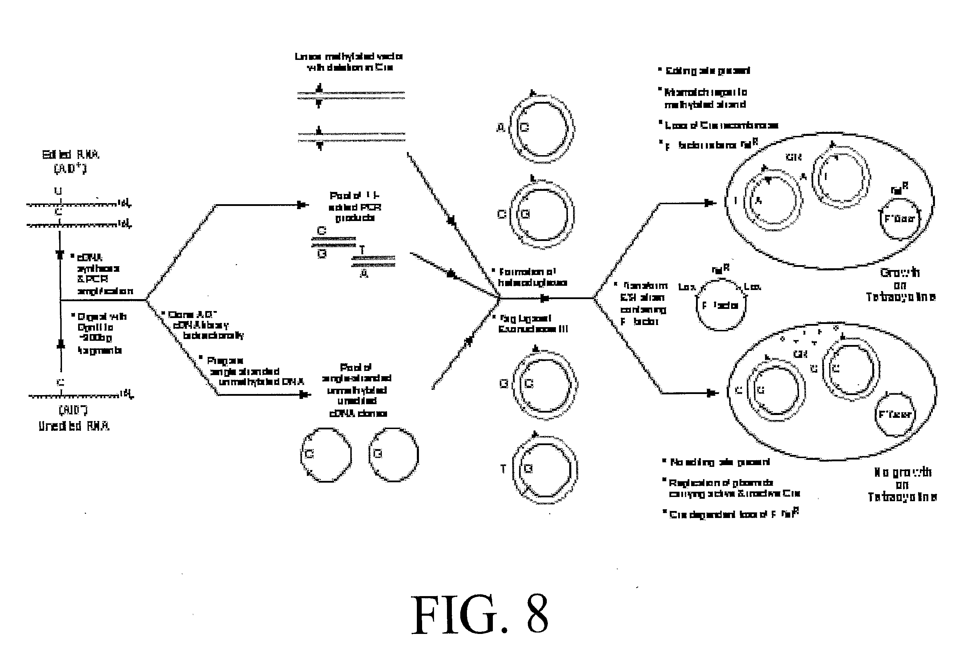

15. Figure 8 shows selection of AID edited mRNAs by *E. coli* mismatch repair and Cre recombinase (Faham (2001) Hum. Mol. Genet. 10:1657-64) AID editing target sites are identified as outlined in this figure. The system, developed for the identification of single nucleotide polymorphisms in DNA, is used to identify mRNA editing substrates as well as sites of DNA mutation. Double-stranded cDNA are synthesized and PCR amplified from mRNA isolated from wild type NIH3T3 cells and from transfected NIH3T3 cells that have expressed AID for 48-72 h (a time period in which CSR was observed on an artificial switch construct). The two separate double stranded cDNA pools are digested with DpnII to generate approximately 300 bp fragments with GATC overhangs. cDNAs from wild type NIH3T3 cells are cloned into BamHI digested (GATC overhang) Cre expression vector (pCre100), transformed into dam minus E.coli and unmethylated, single-stranded DNA isolated using helper phage M13K07. The pool of cDNA fragments prepared from RNA isolated from AID-transfected NIH3T3 cells are methylated using TaqI methylase (NEB) and then combined with BamHI linearized, methylated pCre200 (identical to pCre100 except for an inactivating 5 bp deletion within the Cre recombinase gene). The resultant methylated, Cre-deficient, edited cDNA pool is combined with the single-stranded, unmethylated, active-Cre+, unedited cDNA library, denatured and then reannealed to form heteroduplexes. Taq DNA ligase (NEB) is used to form closed circles of hemi-methylated heteroduplexes. Addition of exonuclease III converts DNA that has not been closed with Taq ligase to single stranded DNA, which is then removed. The heteroduplex mixture is transformed into an electrocompetent *E. coli* strain (Editing Site Identifier; ESI) engineered to carry on its episome (F' factor) a tetracycline resistance gene flanked by two lox sites. The heteroduplex mixture contains: (i) perfect cDNA homoduplexes from mRNAs that are not AID substrates from the two cell sources (not shown) and (ii) four different possible cDNA duplexes resulting from AID mRNA substrates in their unedited (homoduplex) and edited (heteroduplex) forms (shown). These appear in the figure as two homoduplexes with C:G and G:C base pairs at the editing site and two heteroduplexes with mismatched base pairs at the editing site corresponding to A:C and T:G. The selection mismatch repair and cre recombinant system of Figure 8 can be used to identify mutated DNA sequences. This system can be applied for evaluating mRNA editing sites or DNA mutation sites due to APOBEC-1, AID, CEM15 and any other ARP.

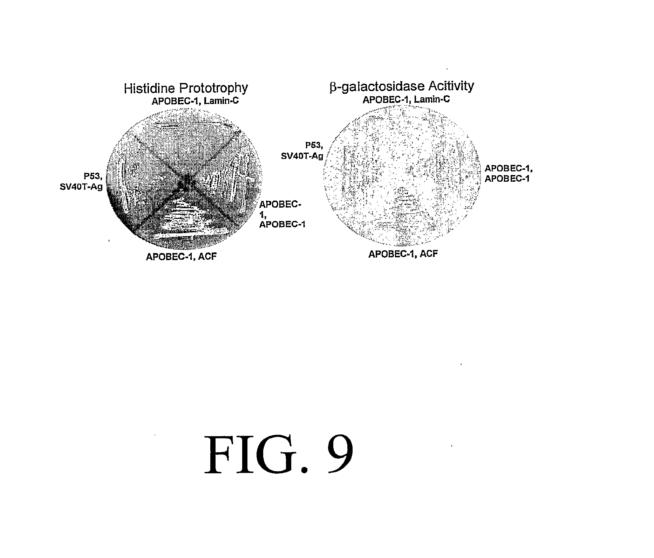

16. Figure 9 shows the selection scheme and verification of true positives from Example 7, using cDNAs encoding APOBEC-and ACF. Success with this system in selecting appropriate interactions is evident as robust growth under hisselection (left) and appearance of colonies on filter 'lifts' (right) for APOBEC-1 interaction as homodimers and heterodimers with ACF. The positive control (p53 binds to SV40T antigen) and negative control (lamin C does not bind to APOBEC-1) confirmed the stringency of the selection system.

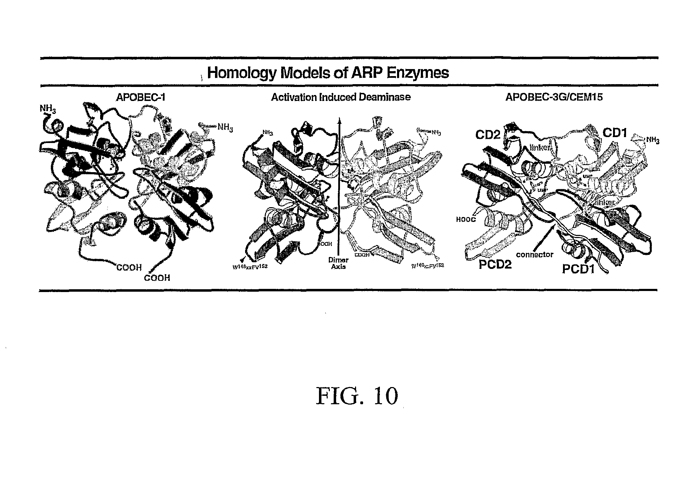

17. Figure 10 shows homology models of ARP enzymes. The linker appears in all ARPs and can provide an important flexibility element that sequesters the single-stranded substrate in an active site cleft where it is edited or mutated, respectively. Although E. coli exhibits a comparable linker in its three-dimensional structure, the linker is long∼19 amino acids and appears well-ordered in the structure. This indicates some degree of rigidity that can preclude large polymeric substrates such as RNA or DNA from entering into its active site. CEM15's general structure is expected to be analogous to APOBEC-1 and AID (above - right).

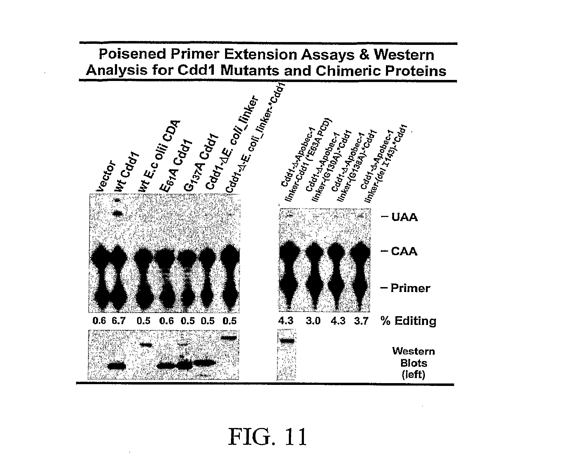

18. Figure 11 shows Poisened primer extension assays and western analysis for Cdd1 mutants and chimeric proteins. In the context of late log phase growth in yeast with galactose feeding, overexpressed Cdd1 is capable of C to U specific editing of reporter apoB mRNA at site C6666 at a level of 6.7%, which is ∼10x times greater than the negative control (empty vector - compare lanes 1 and 2, above). In contrast, the CDA from E. coli (equivalent to PDB entry 1AF2) is incapable of editing on the reporter substrate (lane 3). Similarly, the active site mutants E61A and G137A abolish detectable Cdd1 activity (lanes 4 and 5). Likewise, the addition of the E. coli linker sequence (lane 6) impairs editing function as well. In a series of chimeric constructs in which the Cdd1 tetramer was converted into a molecular dimer, the chimeric molecule appears functional, as long as an amino acid linker of 7-8 amino acids is used to join the respective Cdd1 subunits (See Right Panel lanes 1-4). However, when the longer E. coli linker is used to join Cdd1 monomers, there is no detectable activity on the reporter substrate, although the chimeric protein is expressed (See Western blot). Paradoxically, when conserved Gly residues of the APOBEC-1 linker (130 and 138) are mutated to Ala, the chimeric enzyme is still active (Lanes 3 and 4 of right panel).

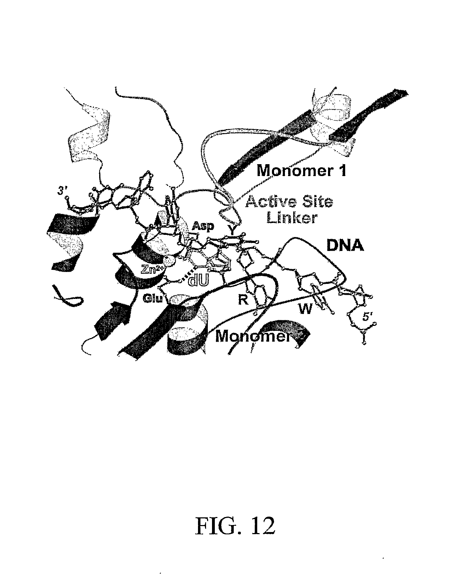

19. Figure 12 shows an ARP model that shows a restructuring of the active site linker that makes the entire region spanning from 130 to 142 (human APOBEC-1 numbering) flexible in a manner that moves to accommodate large polymeric substrates such as RNA or DNA.

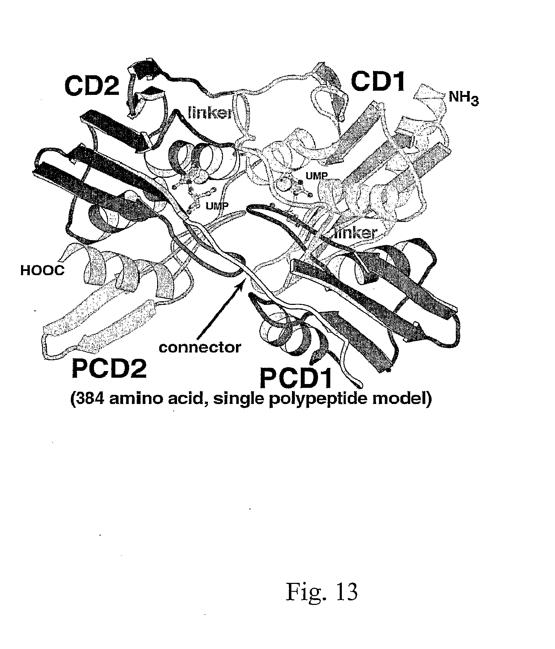

20. Figure 13 shows the model for CEM15. The CEM15 sequence was modeled manually using the computer graphics package O (Jones Acta Crystallogr A, (1991) 47 ( Pt 2): p. 110-9), thereby preserving the core ZDD fold; gaps and insertions were localized to loops and modeled according to one of the three known structures, or by use of main-chain conformational libraries. Amino acid side-chains were modeled using rotamer libraries (Jones Acta Crystallogr A, (1991) 47 ( Pt 2): p. 110-9). The resulting model demonstrates that the 384 amino acid sequence of CEM15 can be accommodated by a dimeric CDA *quaternary* fold (analogous to the *E. coli* CDA or APOBEC-1 with 2 x 236 amino acids).

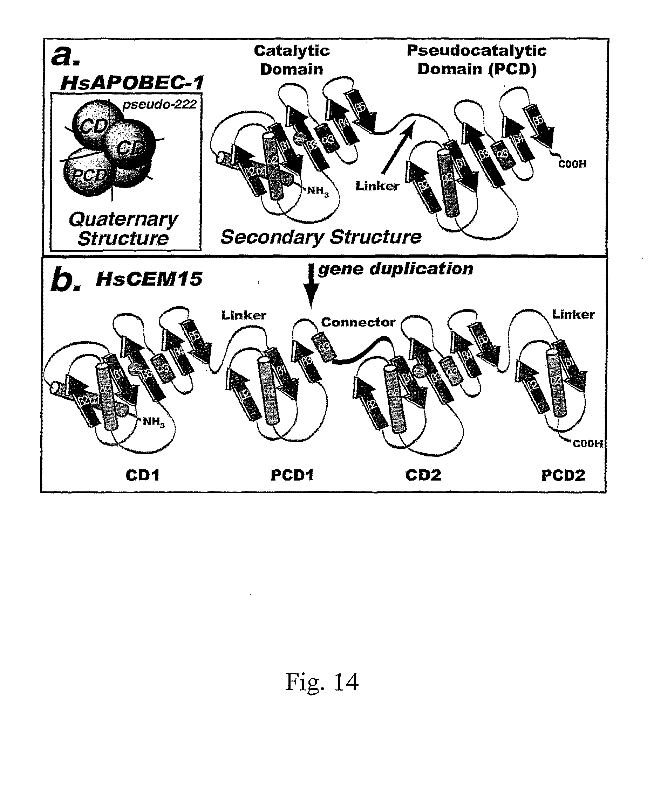

21. Figure 14 shows an APOBEC-1 structural model compared to a CEM15 structural model. CEM15 adopts a CD1-PCD1-CD2-PCD2 *tertiary* structure with pseudo-222 symmetry (Fig. 14a) on a single polypeptide chain (Fig. 14b).

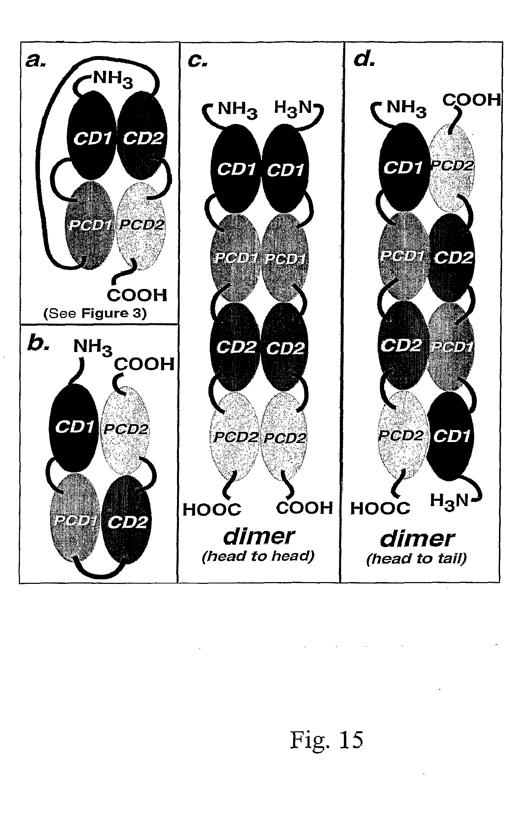

22. Figure 15 shows possible CEM15 oligomers. These mutants address whether the CEM15 functions as a monomer, or as a dimer that dictates substrate specificity. Dimeric CEM15 structures (Figs. 15c & 15d) show mutually exclusive intermolecular contacts. The salient feature of interaction 15c, is that each CD pairs with itself, and similarly for each PCD. In contrast, every domain in 15d falls in a unique environment (i.e. no CD or PCD pairs with itself). Therefore, to evaluate the need for either single or dual catalytic domain requirements for the anti-viral effect, express truncations are expressed. For example, if the dual CD-PCD domain structure were required to ablate viral infectivity, truncation products of the form CD1-PCD1 or CD2-PCD2 precludes folding of structures depicted in 15a, 15b and 15d, whereas model 15c can fold, showing that either CD1-PCD1 or CD2-PCD2 is sufficient to suppress viral infectivity. These results show that anti-HIV-1 therapeutics can disrupt Vif suppression of catalytic activity at either a single CD or both CD1 and CD2 simultaneously.

### IV. DETAILED DESCRIPTION

23. The invention provides a means of delivery of deaminases, which avoids the problems of unregulated protein expression and the risk that over-expression can induce aberrant mRNA editing or unwanted nonspecific DNA mutations associated with delivery and expression of these proteins via gene therapy. Such deaminases are useful in a variety of diseases, such as those where the lack of enzyme expression or mutations within the endogenous genes encoding these enzymes are responsible for the absence, or reduction of, appropriate levels of enzyme activity.

### A. APOBEC-1

24. One example of a Cytosine Deaminase Active on RNA (CDAR) is APOBEC-1 (apolipoprotein B mRNA editing catalytic subunit 1) (accession # NM_005889) encoded on human chromosome 12. (Grosjean and Benne (1998); Lau (1994) PNAS 91:8522-26; Teng (1993) Science 260:1816-19). APOBEC-1 edits apoB mRNA primarily at nucleotide 6666 (C₆₆₆₆) and to a lesser extent at C8702 (Powell (1987) Cell 50:831-40; Chen (1987) Science 238: 363-366; Smith (1993) Seminars in Cell Biology 4:267-78) in a zinc dependent fashion (Smith (1997) RNA 3:1105-1123). This editing creates an in-frame translation stop codon, UAA, from a glutamine codon, CAA at position C₆₆₆₆ (Grosjean and Benne (1998); Powell (1987) Cell 50:831-840; Chen (1987) Science 238:363-66). The biomedical significance of apoB mRNA editing is that it results in increased production and secretion of B48 containing very low density lipoproteins and, correspondingly, a decrease in the abundance of the atherogenic apoB100 containing low density lipoproteins in serum (Davidson (1988) JBC 262:13482-85; Baum (1990) JBC 265:19263-70; Wu (1990) JBC 265:12312-12316; Harris and Smith (1992) Biochem. Biophys. Res. Commun. 183:899-903; Inui (1994) J. Lipid Res. 35:1477-89;Funahashi (1995) J. Lipid Res. 36:414-428; Giannoni J. Lipid Res. 36:1664-75; Lau (1995) J. Lipid Res. 36: 2069-78; Phung (1996) Metabolism 45:1056-58; Van Mater (1998) Biochem. Biophys. Res. Commun. 252:334-39; von Wronski (1998) Metab. Clin.Exp. 7:869-73; Grosjean and Benne (1998); Powell (1987) Cell 50:831-840; Chen (1987) Science 238:363-66; Scott (1989) J. Mol. Med. 6:63-80; Greeve (1993) J. Lipid Res. 34:1367-83).
25. In APOBEC-1 gene knockout mice, apoB mRNA was unedited, demonstrating that no other CDAR is expressed which can use apoB mRNA as a substrate (Nakamuta (1996) JBC 271:25981-88;Morrison (1996) PNAS 271:25981-88; Hirano (1996) J. Biol. Chem. 271:9887-90; Yamanaka (1997) Genes Dev. 11:321-33; Yamanaka (1995) PNAS 92:9493-87; Sowden (1998) Nucl. Acids Res. 26:1644-1652). ApoB is translated from a 14 kb mRNA that is transcribed from a single copy gene located on human chromosome 2 (Scott (1989) J. Mol. Med. 6:65-80). ApoB protein is a non-exchangeable structural component of chylomicrons and of very low density (VLDL) and low density (LDL) lipoprotein particles.
26. RNA secondary structure does not appear to be required for apoB RNA editing. Instead, apoB mRNA editing requires an 11 nucleotide motif known as the mooring sequence. Placement of the mooring sequence 4-8 nucleotides 3' of a cytidine within reporter RNAs is frequently sufficient for that RNA to support editing (Smith (1993) Seminars in Cell Biol. 4:267-78; Sowden (1998) Nucl. Acids Res. 26:1644-1652; Backus and Smith (1992) Nucl. Acids Res. 22:6007-14; Backus and Smith (1991) Nucl. Acids Res. 19:6781-86; Backus and Smith (1994) Biochim. Biophys. Acta 1217:65-73; Backus (1994) Biochim. Biophys. Acta 1219:1-14; Sowden (1996) RNA 2:274-88). The mooring sequence is left intact in edited mRNA and therefore its occurrence downstream of a cytidine is predictive of an editing site.
27. APOBEC-1 relies on auxiliary proteins for RNA recognition (Grosjean and Benne (1998); Teng (1993) Science 260:1816-19; Sowden (1998) Nucl. Acids Res. 26:1644-52; Inui (1994) J. Lipid Res. 35:1477-89; Dance (2001) Nucl. Acids Res. 29:1772-80). APOBEC-1 only has weak RNA binding activity of low specificity (Anant (1995) JBC 270:14768-75; MacGinnitie (1995) JBC 270:14768-75). To edit apoB mRNA, APOBEC-1 requires, in addition to the mooring sequence described above, RNA binding proteins that bind apoB mRNA and to which APOBEC-1 can bind and orient itself to C6666. Under defined *in vitro* conditions, apoB RNA, recombinant APOBEC-1 and proteins known as ACF/ASP (APOBEC-1 Complementing Factor/APOBEC-1 Stimulating Protein) were all that was required for editing activity and are therefore considered as the minimal editing complex or editosome (Mehta (2000) Mol. Cell Biol. 20:1846-54; Lellek (2000) JBC 275:19848-56).
28. ACF was isolated and cloned using biochemical fractionation and yeast two hybrid genetic selection (Mehta (2000) Mol. Cell Biol. 20:1846-54; Lellek (2000) JBC 275:19848-56). Overexpression of 6His-tagged APOBEC-1 in mammalian cells enabled the intracellular assembled editosome to be affinity purified (Yang (1997) JBC 272:27700-06). These studies demonstrated that ACF associated with APOBEC-1 through 1M NaCl resistant interactions and that three other RNA binding proteins (100 kDa, 55 kDa and 44 kDa) with affinity for the mooring sequence co-purified with the editosome (Yang (1997) JBC 272:27700-06). P100 and p55 were both mooring sequence selective RNA binding proteins but p44 was a general RNA binding protein. Additional studies utilizing yeast two hybrid analyses using APOBEC-1 affinity and antibodies developed against the editosome and ACF have demonstrated proteins such as hnRNP ABBP 1 (Lau (1997) JBC 272:1452-55), the alternative splicing factor KSRP (Lellek (2000) JBC 275:19848-56) and αI3 serum proteinase inhibitor as positive modulators of editing activity (Schock (1996) PNAS 93:1097-1102) and hnRNP protein C (Greeve (1998) Biol. Chem. 379:1063-73) and GRY-RBP (Blanc (2001) JBC 276: 10272-83; Lau (2001) Biochem. Biophys. Res. Commun. 282:977-83) as negative modulators of apoB mRNA editing.
29. Structure-based homology modeling has provided insight into the fold of APOBEC-1 (Figure 6), and the modeling of APOBEC-1 has been corroborated by protein engineering, site-directed mutagenesis, and functional analyses. The current model for APOBEC-1 is a two domain structure comprising a catalytic domain (CD) (used interchangeably throughout with Cdd1) and a pseudo-catalytic domain (PCD) joined by a central linker, which folds over the active site (Figure 6). The linker sequence is conserved among ARPs, and sequence identity and length are essential for efficient RNA editing by APOBEC-1. The APOBEC-1 model also provides a rationale for losses in editing due to surface point mutations, such as F156L (Navaratnam Cell 81(2):187-95), located 25 Å from the active site. Such a change can influence auxiliary factor binding. Other mutations such as K33A/K34A abolish activity (Teng (1999) J Lipid Res, 40(4) 623-35).
30. Other putative members of the ARP family in humans were identified by genomic sequence analyses and include AID (Muramatsu (1999) JBC 274:18740-76; Muramatsu (2000) Cell 102:553-564); Revy (2000) Cell 102:565-76), APOBEC-2 (Liao (1999) Biochem. Biophys. Res. Commun. 260:398-404) and variants of phorbolins, which are also known as the APOBEC3 family (Anant (1998) Biol Chem. 379:1075-81; Jamuz, (2002) Genomics 79:285-96; Sheehy (2002) Nature 418:646-50; Madsen (1999) J. Invest. Dermatol. 113:162-69). These candidate CDARs have attracted interest because they share homology with the catalytic domain found in APOBEC-1 and the ADARs and they also have interesting physiological circumstances for their expression. One characteristic of the catalytic domain in ARPs and ADARs is the occurrence and spacing of a histidine and two cysteines (or three cysteines), required for the coordination of a zinc atom, also known as the zinc binding domain or ZBD (Grosjean and Benne; Mian (1998) J. Comput. Biol. 5:57-72). The ZBD of ADARs is distinguishable from that found in cytidine deaminases because the third cysteine in ADARs is located significantly further in primary sequence from the second conserved cysteine residue (Mian (1998) J. Comput. Biol. 5:57-72; Gerber (2001) TIBS 26:376-84). The ZBD of APOBEC-1 is located in the N-terminal half of the protein and modeling has suggested that a pseudo- (nonfunctional) ZBD domain is repeated in the C-terminus (Mian (1998) J. Comput. Biol. 5:57-72).
31. Table 1 shows APOBEC-1 and ARPS have been described previously (Anant, S., Am J Physiol Cell Physiol. 281:C1904-16.; Dance, G.S., (2001) Nucleic Acids Res. 29:1772-80.; Jarmuz, A., (2002) Genomics 79:285-96) and extended through amino acid similarity searches with the (1) hidden Markov modeling software SAM trained with CDD1, APOBEC-1, APOBEC-2, AID and Phorbolin 1, (2) PHI-BLAST, using the target patterns H(VA)-E-x-x-F-(x)19-(I/V)-(T/V)-(W/C)-x-x-S-W-(ST)-P-C-x-x-C and (HC)-x-E-x-x-F-x(19,30)-P-C-x(2,4)-C. The gene name and its chromosomal location are indicated and the Accession number of the encoded protein listed. Equivalent/former names are derived from GenBank (Anant, S., (1998) Biol Chem. 379:1075-81.; Sheehy, A.M., (2002) Nature 418:646-650.). The major tissues of expression are listed. More extensive listings, especially for neoplastic tissues, can be found in the LocusLink pages of Genbank for the individual ARPs which can be accessed from the Unigene Cluster entries. The identity of the APOBEC3 family genes and ESTs in the UniGene and LocusLink entries can be verified. For HsARP-6, HsARP-7, HsARP-8, HsARP-10 and HsARP-11 only EST data exists as evidence of a final protein product.

**TABLE 1**

| Gene/Chromosomal Location | Protein Accession # | Equivalent/Former Names/Variants (Acen #) | Expression | Proposed CDAR/ARP | Unigene Cluster |
|---|---|---|---|---|---|
| Yeast | | | | | |
| CDDI/Chr XII | NP_013346 | - | yeast | ScCDAR-1 | |
| Human | | | | | |
| APOBEC-1/12p13.1 | AAD00185 | - | small intestine, liver | HsCDAR-1 | Hs.560 |
| APOBEC-2/6p21 | NP_006780 | CAB44740 | cardiac & skeletal muscle | HsARP-1 | Hs227457 |
| | | ARCD-1 | | | |
| AID/12p13 | NP_065712 | - | B lymphocytes | HsARP-2 | Hs149342 |
| APOBEC-3A/22q131 | NP_663745 | Phorholin-1 (P31941) | keratinocytes | HsARP-3 | Hs348983 |
| APOBEC-3B/22q13.1 | Q9UHI7 | Phorbolin-3 | keratinocytes/ | HsARP-4 | Hs226307 |
| | | Phorbolin-1-related (U61084) Phorbolin-2(Q9UE74) APOBECIL ARCD-3 | colon | | (specific to U61084 not APOBEC-3B) |
| APOBEC-3C/22q13.1 | CAB45271 | Phorbolin-1 (AF165520) ARCD-2/ARCD-4 | spleen/testes/heart/thymus prostate/ovary/utens/PBLs | HsARP-5 | Hs.8583 |
| APOBEC-3D/22q13.1 | BF841711 (EST only) | - | head & neck eancers | HsARP-6 | |
| APOBEC-3E/22q13.1 | PSEUDOGENE | ARCD-6 | - | - | |
| APOBEC-3D13E/22q13.1 | NM_145298 | - | uterus | HsARP-7 | |
| APOBEC-3F/22q13.1 | BG_758984 | ARCD-5 | B lymphocytes | HsARP-8 | |
| | (EST only) | | | | |
| APOBEC-3G/22q13.1 | NP_068594 MDS019(AAH24268) HsCEM15 | Phorbolin-like-protein spleen/testes/heart/thy PBLs/colon/stomach/kidney mus uterus/panerense/placenta/prostale | | HsARP-9 | Hs.250619 |
| 22q13.1 | XP_092919 | - | - | HsARP-10 | |
| XP_092919 | | | | | |
| 12q23 | XP_115170 | - | - | HsARP-11 | |
| Mouse | | | | | |
| MmAPOBEC-1/6F2 | NP_112436 | - | small intestine/liver/spleen | MmCDAR-1 | Mm3333 |
| MmAPOBEC-2/17 | NP_033824 | - | B lymphocytes/kidney cardiac & skeletal muscle luain/skin | MmARP-1 | Mm27822 |
| MmAID/6F2 | NP_033775 | | B lymphocytes | MmARP-2 | Mm32398 |
| CEM15/15 | NP_084531 | XP_122858 | mammary tumour | MmARP-3 | Mm89702 |

32. These basic residues are a feature of all ARP family members, including Cdd1. The latter basic residues are close to the active site, and can be responsible for RNA binding. The quality of the APOBEC-1 model is derived from superposition of three high resolution CDA crystal structures (Betts (1994) J Mol Biol 235(2):635-56; Johansson (2000) Biochemistry 41(8):2563-70) that exhibit a nearly identical αβ₂αβαβ₂ fold despite modest sequence identity (∼24%); fold conservation also exists at the oligomeric level, since each enzyme exhibits ∼222 symmetry (Figures 6 and 12).
33. Structural homology is derived from the fact that dimeric CDAs arose from gene duplication of a CD precursor (Betts (1994) J Mol Biol 235(2):635-56; Johansson (2000) Biochemistry 41(8): p. 2563-70) producing a PCD, which although catalytically inactive, forms an inextricable part of the core protein fold. Pairwise superpositions of 75 backbone atoms from the yeast CDD1 crystal structure with comparable atoms from those CDA structures of *E*. *coli* and *B. subtilis* results in RMSD's of 1.42 Å and 0.76 Å, respectively, which exceeds the structural homology predicted by simple sequence alignments of proteins with unknown function (Chothia (1986) Embo J. 5(4)823-6; Lesk, J Mol Biol, 136(3):225-70.) Notably yeast CDD1, an enzyme used in pyrimidine salvage, edits ectopically expressed apoB mRNA in yeast. (Dance Nucleic Acids Res 29(8):1772-80). Hence, it is conceivable that the CDA motif of nucleoside metabolism has been co-opted to function on larger RNA substrates.
34. Threading of APOBEC-1 primary sequence through the known crystal structure of *E. coli* cytidine deaminase dimers indicated that APOBEC-1 structure is consistent with a head-to-tail homodimer with the active ZBD domain of one monomer in apposition with the pseudo-ZBD domain of the other monomer (Navaratnam (1995) Cell 81:187-95). In this model, one of the active deaminase domains is predicted to interact non-catalytically with RNA while the other active domain interacts with the cytidine to be edited (Navaratnam (1995) Cell 81:187-95). Importantly, dimerization has been shown to be important for editing activity (Lau (1994) PNAS 91:8522-26; Navaratnam (1995) Cell 81:187-95; Oka (1997) JBC 272:1456-60). A leucine-rich region (LRR) in the C-terminus of APOBEC-1 is a typical characteristic of cytidine deaminase that function as dimers. The LRR is essential for APOBEC-1 homodimer formation, apoB mRNA editing, APOBEC-1 interaction with ACF, and APOBEC-1's subcellular distribution (Lau (1994) PNAS 91:8522-26; MacGinnitie (1995) JBC 270:14768-75; Navaratnam (1995) Cell 81:187-95; Oka (1997) JBC 272:1456-60).

### B. AID

35. AID (GenBank accession # BC006296) is encoded on human chromosome 12 (Muramatsu (1999) JBC 274:18740-76; Muramatsu (2000) Cell 102:553-64; Revy (2000) Cell 102:565-76). AID has a zinc-dependent cytidine deaminase domain (ZDD) with characteristic sulfhydryl groups for zinc coordination, and glutamic acid for proton shuttling during hydrolytic deamination as well as a leucine-rich C-terminal domain for protein-protein interactions. Furthermore, AID has a 34% amino acid identity to APOBEC-1. This together with AID's *in vitro* cytidine deaminase activity (Muramatsu J. Biol. Chem. 274(26):18470-18476 (1999)) and the ability of AID catalytic domain mutations to inhibit CSR and SHM (Papavasiliou & Schatz, J. Exp. Med. 195(9):1193-1198 (2002)) shows that AID functions *in vivo* as a cytidine deaminase. Its location on human chromosome 12p13 also suggests it may be related to APOBEC-1 by a gene duplication event (Madsen, P., (1999) J Invest Dermatol. 113:162-9.57). This chromosomal region has been implicated in the autosomal recessive form of Hyper-IgM syndrome (HIGM2) (Lee, R.M. (1998) Gastroenterology. 115:1096-103). Most patients with this disorder have homozygous point mutations or deletions in three of the five coding exons, leading to missense or nonsense mutations (Dance, G.S., (2001) Nucleic Acids Res. 29:1772-80; Revy, P., (2000) Cell. 102:565-75). Significantly, some patients had missense mutations for key amino acids within AID's ZBD.
36. AID's homology with APOBEC-1 also suggests that it functions as an mRNA editing enzyme. AID's requirement in human B lymphocyte function is likely due to its role as the catalytic component of an enzyme complex that alters (edits) the sequence of an essential mRNA. AID can deaminate (edit) cytidine to form uridine of mRNA(s). The novel protein variant(s) encoded by edited mRNA(s) (referred to as AID-Editing-Target or AET) is proposed to promote class switch recombination (CSR) and somatic hypermutation (SHM) of Ig genes. Alternatively, the effect of mRNA editing may be to inactivate a protein(s) that is an inhibitor of CSR and SHM.
37. AID homologous knockout mice demonstrated that AID expression was the rate limiting step for class switch recombination (CSR) and required for an appropriate level of somatic hypermutation (SHM) (Minegishi, Y., (2000) Clin Immunol. 97:203-10). The expression of AID controls antibody diversity through multiple gene rearrangements involving mutation of DNA sequence and recombination. The initial expression of antibodies requires immunoglobulin (Ig) gene rearrangement that is AID-independent (Muramatsu, M., (2000) Cell 102:553-63). This occurs in immature B lymphocytes developing in fetal liver or adult bone marrow and requires DNA double strand breaks at the Ig heavy chain locus whose ends are rejoined by non-homologous end joining. The rearranged immunoglobulin V (variable), D (diversity) and J (joining) gene segments encode a variable region that is expressed initially with the mu (µ) constant region (Cµ) to form a primary antibody repertoire composed of IgM antibodies. In humans and many mammals, AID-dependent gene alterations occur in B lymphocytes that are growing in germinal centers of secondary lymphoid organs following antigen activation. This involves multiple mutations of the variable region through SHM as well as removing the Cµ and replacing it with one of several other constant regions (Cα, CΔ, Cε or Cγ) through CSR. In sheep, rabbits and chickens, pre-immune Ig gene diversification is mediated by an AID-dependent process known as gene conversion (GC) in which stretches of nucleotide sequences from one of several pseudogene V elements are recombined into the VDJ exon to generate diversity (Fugmann, S.D. (2002) Science 295:1244-5.; Honjo, T., (2002) Annu Rev Immunol. 20:165-96.)
38. Overexpression of AID in mouse fibroblasts and Ramos B cells induced CSR on an Ig reporter gene and stimulated the rate of SHM respectively (Muramatsu, M. (2000) Cell. 102:553-63; Okazaki, I.M. (2002) Nature. 416:340-45). Given AID's similarity to APOBEC-1, these genomic alterations have been proposed to be due to AID-dependent mRNA editing (Lee, R.M. (1998) Gastroenterology 115:1096-103). Editing could promote CSH and SHM through the expression of a novel protein or by reducing the expression/function of an inhibitory protein through alternative exon splicing or codon sense changes.
39. AID cannot substitute for APOBEC-1 in the editing of apoB mRNA (Lee, R.M. (1998) Gastroenterology. 115:1096-103) and, although this negative result may have been expected (given that most editing enzymes have substrate specificity (Grosjean and Benne (1998)), it did suggest that AID may have another activity. Consistent with the findings that AID is an mRNA editing enzyme is the finding that *de novo* protein synthesis subsequent to AID activity was necessary for CSR. Therefore, a novel protein made from edited mRNA was essential for CSR.
40. A competing hypothesis for AID's role in CSR and SHM is that it deaminates deoxycytidine in DNA (Rada, C. (2002) Proc. Natl. Acad. Sci USA. 99:7003-7008; Petersen-Mahrt, S.K., (2002) Nature. 418:99-104). The mutations observed in SHM (and those that arise proximal to the junctions of CSR) are C-T transitions (Yoshikawa, K., (2002) Science 296:2033-2036). Like APOBEC-1, AID has cytidine and deoxycytidine deaminase activity (Madsen, P. (1999) J Invest Dermatol. 113:162-957) and its ZDD is homologous to that of *E*. *coli* deoxycytidine deaminase (Figure 5). AID overexpression in NIH 3T3 fibroblasts resulted in the deamination of deoxycytidine in DNA encoding a green fluorescent protein (GFP) (Petersen-Mahrt, S.K. (2002) Nature 418:99-104) and also in antibiotic resistance and metabolic genes when AID expression in bacteria was placed under selection for a 'mutator' phenotype (Rada, C. (2002) Proc. Natl. Acad. Sci USA. 99:7003-7008). A variety of mutations were observed on GFP DNA including deletions and duplications, however, a preference for transitions at G/C base pairs clustered within regions predicted to have DNA secondary structure was observed. Similar mutations were observed in the bacteria overexpressing AID and their frequency was markedly enhanced when evaluated in an ung-1 background (lacking functional uracil-DNA glycosylase, an enzyme involved in repairing C to T mutations). These findings together with the observation that the mutation frequency of the GFP gene was 4.5 x 10⁻⁴/bp per cell generation, which was comparable to the 10⁻³ to 10⁻⁴ frequency observed on Ig genes in B cells, show that AID can act on DNA. The target hotspot for AID is characterized by the motif RGYW (SEQ ID NO: 9) (R is A or G, Y is C or T and W is A or T) (Honjo Annu Rev Immunol 20:165-96, 2002; Martin Nat Rev Immunol, 2(8):605-14, 2002).
41. Mutation hotspots in bacteria reporter genes were identified for APOBEC-1 and CEM15 although they have distinct substrate specificities (Harris Mol Cell 10(5):1247-53, 1996). Actively transcribed DNA was identified as the preferred AID substrate (Chaudhuri, Nature 422(6933):726-30, 2003), and specifically that dC is deaminated to dU in the strand of DNA that is displaced by transcription of RNA (the non-templating strand); corroborating other studies in which AID selectively deaminated dC in ssDNA or mutated dsDNA reporters within a nine base pair mismatch (the size of a transcription bubble) (Bransteitter, Proc Natl Acad Sci (2003); Ramiro Nat Immunol. 100(7):4102-7). AID appears to act processively on DNA, binding initially to SEQ ID NO: 9 and mutating dC to dU and then modifying multiple dC residues from that point along the same strand of DNA. AID's ability to act on DNA would not negate the possibility that it also acts on RNA. Whether AID is involved in DNA and/or RNA modification, its function clearly results in the diversification of expressed genomic sequences.These findings indicate that AID, if unregulated, can induce DNA mutations leading to disease such as cancer.
42. AID is constitutively expressed in human B cell malignancies such as diffuse large B cell lymphomas (DLBCL) and some chronic lymphocityc leukemias (CLL), follicular and MALT lymphomas; expression of aberrantly spliced AID mRNAs capable of encoding truncated AID isoforms is also frequently observed. In subsets of DLBCL and CLL, AID expression is uncoupled from somatic hypermutation activity, a feature that correlates with more aggressive forms of these diseases. It appears that AID function is aberrant in B cell cancers. In fact, oncogene mutations with patterns resembling SHM have been found at high frequency in B cell lymphomas. It appears that loss of targeting specificity of the SHM process is involved in the transformation and/or progression of B lymphoid malignancies. Constitutive AID expression in transgenic mice has been shown to cause T cell lymphomas and pulmonary adenomas, formally demonstrating AID's oncogenic potential. It appears that the oncogenic effect of AID is attributable to loss of regulation over its DNA mutator activity, as a consequence of over-expression, of expression of AID isoforms with altered function, or of defects in cofactors involved in determining specificity of SHM targeting, resulting in genome-wide mutagenesis. This represents a "mutator"-like phenotype, mechanistically distinct from that observed in DNA mismatch repair-deficient neoplasias, but with analogous functional consequences: rapid accumulation of multiple oncogenic hits, resulting in accelerated tumor progression. Also, APOBEC-1 and CEM15 expression are elevated in some patient's colorectal and breast cancers, respectively.
43. The prototypical example of the role of mutator phenotypes in cancer is mismatch-repair deficiency in hereditary non-polyposis colon cancer (HNPCC) (Bronner, Nature 369:258-61; Fishel, Cell 75:1027-38; Nicolaides, Nature 371:75-80). Evidence for a widespread role of mutator phenotypes in sporadic cancers has also accumulated, suggesting that hypermutagenesis represents an essential step in neoplastic development (Loeb, Cancer Res 51:3075; Loeb, Proc Natl Acad Sci, 100:776-781; Loeb, Cancer Res 61:3230-3239). Importantly, unlike other known mutator phenotypes - due to defective repair of spontaneous DNA damage - deregulated SHM activity actively causes genetic changes. In both cases, however, the outcome is the progressive, accelerated accumulation of oncogenic mutations.

### C. APOBEC-2

44. Human APOBEC-2 (Genbank Accession # XM004087) is encoded on chromosome 6 and is expressed uniquely in cardiac and skeletal muscle (Liao, Biochem Biophys. Res. Commun. 260:398-404). It shares homology with APOBEC-1's catalytic domain, has a leucine/isoleucine-rich C-terminus and a tandem structural homology of the ZBD in its C-terminus. APOBEC-2 deaminated free nucleotides *in vitro* but did not have editing activity on apoB mRNA.

### D. CEM15/APOBEC-3

45. Human phorbolin 1, phorbolin 1-related protein, phorbolin-2 and -3 share characteristics with C to U editing enzymes. Several proteins with homology to APOBEC-1 named Phorbolins 1, 2, 3, and Phorbolin-1 related protein were identified in skin from patients suffering from psoriasis and were shown to be induced (in the case of Phorbolins 1 and 2) in skin treated with phorbol 12-myristate-1-acetate (Muramatsu, M. (1999) J Biol Chem. 274:18470-6). The genes for these proteins were subsequently renamed as members of the APOBEC-3 or ARCD family locus (Table 1) (Madsen, P. (1999) J Invest Dermatol. 113:162-9). Bioinformatic studies revealed the presence of two additional APOBEC-1 related proteins in the human genome. One is an expressed gene (XM_092919) located just 2 kb away from APOBEC-3G, and is thus likely to be an eighth member of the family. The other is at position 12q23, and has similarity to APOBEC-3G.
46. APOBEC-3 variants show homology to cytidine deaminases (Figure 6c). As anticipated from the SBSA, some of these proteins bind zinc and have RNA binding capacities similar to APOBEC-1 (Madsen, P. (1999) J Invest Dermatol. 113:162-9). However, analysis of APOBEC-3A, -3B and -3G revealed them unable to edit apoB mRNA (Madsen, P. (1999) J Invest Dermatol. 113:162-9; Muramatsu, M. (1999) J Biol Chem. 274:18470-6). It has been shown that the frequency of deleterious mutations in HIV and impaired infectivity correlated with the expression of CEM15 (APOBEC-3G) (Anant, S. (2002) Biochim Biophys Acta. 1575:54-62). HIV expressing functional Vif (viral infectivity factor) protein was able to overcome the effects of CEM 15 due to the ability of Vif to bind (directly or indirectly) to CEM15 and inactivate it. In contrast, it is unlikely that APOBEC-3B functions as an APOBEC-1 like editase because it is missing fundamental sequence elements that are required for mRNA editing by both APOBEC-1 and CDD1 (Anant, S. (2001) Am J Physiol Cell Physiol. 281:C1904-16) and it has impaired ability to coordinate Zn²⁺ and deaminate cytidine (Madsen, P. J Invest Dermatol. 113:162-9,1999). APOBEC-3E has been proposed to be a pseudogene (Madsen, P. J Invest Dermatol. 113:162-9, 1999), yet the EST database suggests that APOBEC-3D and APOBEC-3E are alternatively spliced to form a single CD-PCD-CD-PCD encoding transcript. The limited tissue expression, and association with pre-cancerous and cancerous cells (see Table 1), and in the case of APOBEC-3G, antagonism of the HIV viral protein Vif suggests specific roles for the APOBEC-3 family in growth/cell cycle regulation or antiviral control.
47. CEM15 antiviral activity is derived from effects on viral RNA or reverse transcripts. CEM15 deaminates dC to dU as the first strand of DNA is being made by reverse transcriptase or soon after its completion, and this results in dG to dA changes at the corresponding positions during second strand DNA synthesis. The infectivity assay in the context of Vif minus pseudotyped viruses and 293 T cells either lacking or expressing CEM15 is found in Example 10. An assay was developed using VSV G-protein pseudotyped lentiviral particles that confirmed the inhibitory effect of CEM15 on the infectivity of Vif+ and Vif- particles and is amenable to the rapid demarcation of the regions of HIV-1 DNA (or RNA) that is the target for CEM15 catalytic activity.
48. Human HIV-1 virus contains a 10-kb single-stranded, positive-sense RNA genome that encodes three major classes of gene products that include: (*i*) structural proteins such as Gag, Pol and Env; (*ii*) essential *trans*-acting proteins (TAT, Rev); and (*iii*) "auxiliary" proteins that are not required for efficient virus replication in at least some cell culture systems (Vpr, Vif, Vpu, Nef). Among these proteins, Vif is required for efficient virus replication *in vivo*, as well as in certain host cell types *in vitro* (Fisher, Science 237(4817):888-93, 1987; Strebel, Nature 328(6132):728-30, 1987) because of its ability to overcome the action of a cellular antiviral system (Madani, J Virol 72(12):10251-5, 1998; Simon, Nat Med 4(12):1397-400, 1998).
49. The *in vitro* replicative phenotype of *vif*-deleted molecular clones of HIV-1 is strikingly different in *vif*-permissive cells (e.g. 293T, SUPT1 and CEM-SS T cell lines), as compared to *vif*-non-permissive cells (e.g. primary T cells, macrophages, or CEM, H9 and HUT78 T cell lines). In the former cells, *vif*-deleted HIV-1 clones replicate with an efficiency that is essentially identical to that of wild-type virus, whereas in the latter cells, replication of *vif*-negative HIV-1 mutants is arrested due to a failure to accumulate reverse transcripts and inability to generate infectious proviral integrants in the host cell (Sova, J Virol 67(10):6322-6, 1993; von Schwedler, J Virol 67(8):4945-55, 1993; Simon, J Virol 70(8):5297-305, 1996; Courcoul J Virol 69(4):2068-74, 1995). These defects are due to the expression of the host protein CEM15 (Sheehy, A.M., (2002) Nature 418:646-650) in non-permissive cells for *vif* minus viruses. CEM15 antiviral activity is derived from effects on viral RNA or reverse transcripts (Sheehy, A.M., (2002) Nature 418:646-650). CEM15 deaminates dC to dU as the first strand of DNA is being made by reverse transcriptase or soon after its completion, and this results in dG to dA changes at the corresponding positions during second strand DNA synthesis (Harris, Cell 113:803-809, 2003).
50. Vif is known to have binding affinity for both viral RNA genomes and a variety of viral and cellular proteins (Simon, (1996) J. Virol. 70 (8):5297-5305; Khan, (2001) J. Virol. 75(16):7252-7265; Henzler, (2001) J. Gen Virol. 82: p. 561-573). Vif also can forms homodimers and homotetramers through its proline rich domain (Yang, (2002) J. Biol Chem. 278(8):6596-6602). The infectivity assay in the context of Vif minus pseudotyped viruses and 293 T cells either lacking or expressing CEM15 is found in Example 1. An assay was developed using VSV G-protein pseudotyped lentiviral particles that confirmed the inhibitory effect of CEM15 on the infectivity of vif+ and vif- HIV-1 particles and is amenable to the rapid demarcation of the regions of HIV-1 DNA (or RNA) that is the target for CEM15 catalytic activity.
51. Primary sequence alignments (Figure 5) and the structural constraints relating CDAs to APOBEC-1 suggest that CEM15 evolved from an APOBEC-1like precursor by gene duplication. The resulting CEM15 structure exhibits two active sites per polypeptide chain with the topology CD1-PCD1-connector-CD2-PCD2. Knowledge of the structural homology among CDAs and ARPs is sufficient to understand how features of CEM15 contribute to its anti-viral activity.
52. The premise of molecular modeling is that primary sequence analysis alone is insufficient to evaluate effectively the HIV-1 anti-infectivity activity of CEM15. The use of homology to model CEM15 is based on three known CDA crystal structures (Betts J Mol Biol, (1994) 235(2): p. 635-56; Johansson, E. Biochemistry, (2002) 41(8): p. 2563-70) and knowledge gained from similar work with APOBEC-1. CEM15 modeling has been accomplished by threading its amino acid sequence onto a composite three-dimensional template derived by superposition (Winn J Synchrotron Radiat, 2003. 10(Pt 1): p. 23-5; Kabsch, W Acta. Crystallogr. (1976) A32: p. 922-923; Potterton Acta Crystallogr D Biol Crystallogr, (2002) 58(Pt 11): p. 1955-7) of known crystal structures, representing dimeric and tetrameric quaternary folds. The CEM15 sequence was modeled manually using the computer graphics package O (Jones Acta Crystallogr A, (1991) 47 ( Pt 2): p. 110-9), thereby preserving the core ZDD fold; gaps and insertions were localized to loops and modeled according to one of the three known structures, or by use of main-chain conformational libraries. Amino acid side-chains were modeled using rotamer libraries (Jones Acta Crystallogr A, (1991) 47 ( Pt 2): p. 110-9). The resulting model (Fig. 13) demonstrates that the 384 amino acid sequence of CEM15 can be accommodated by a dimeric CDA *quaternary* fold (analogous to the *E. coli* CDA or APOBEC-1 with 2 x 236 amino acids). Albeit CEM15 adopts a CD1-PCD1-CD2-PCD2 *tertiary* structure with pseudo-222 symmetry (Fig. 14a) on a single polypeptide chain (Fig. 14b). The resulting CEM15 model provides a rational basis for the design of four classes of mutants: (*ia*) active site zinc (cyan sphere, Fig. 13) ligand changes His65Ala (257), Cys97Ala (288), and Cys100A1a (291), (CD2 residues are noted parenthetically) and (*ib*) active site proton shuttle Glu57Gln (259). Notably, comparable type (*i*) mutations in other CDAs abolish activity (Carlow, D.C.,. Biochemistry, (1995) 34(13): p. 4220-4; Navaratnam, J Mol Biol, (1998) 275(4): p. 695-714; Kuyper, L.F J. Crystal Growth, (1996) 168: p. 135-169); (*ii*) Substitution of the active site linker (Figs.14a & 13) with a comparably sized linker sequence from *E. coli* abolishes ACF-dependent mRNA editing activity by APOBEC-1 in HepG2 cells. The linkers in the first and second active sites of CEM15 are conserved amongst ARPs. However, an insert exists prior to the first linker. The CEM15 model indicates that mutation of either linker would ablate activity whereas modification of the insert should not; (*iii*) mutation of surface residues, e.g. F164 (F350) in the PCD(s) is predicted to disrupt auxiliary factor binding (but not mononucleoside deaminase activity), equivalent to the inactivating F156L mutation in APOBEC-1. None of these mutations is expected to significantly disrupt the CEM15 polypeptide fold, but rather, will help localize regions of the structure necessary for anti-viral activity.
53. The number of possible CEM15 quaternary structures is limited; in fact evidence for a dimeric structure has been cited as 'unpublished' (Jarmuz, Genomics, (2002) 79(3):285-96).Therefore, a fourth class of mutants (truncations) are recognized that can be used to evaluate the requirement of single or dual CD domains for CEM15 activity. These mutants address whether CEM15 functions as a monomer, or a dimer that dictates substrate specificity. Dimeric CEM15 structures (Figs. 15c & 15d) show mutually exclusive intermolecular contacts. The salient feature of interaction 15c, is that each CD pairs with itself, and similarly for each PCD. In contrast, every domain in 15d falls in a unique environment (i.e. no CD or PCD pairs with itself). Therefore, to evaluate the need for either single or dual catalytic domain requirements for the anti-viral effect, express truncations are expressed. For example, if the dual CD-PCD domain structure were required to ablate viral infectivity, truncation products of the form CD1-PCD1 or CD2-PCD2 precludes folding of structures depicted in 15a, 15b and 15d, whereas model 15c can fold, showing that either CD1-PCD1 or CD2-PCD2 is sufficient to suppress viral infectivity. These results show that anti-HIV-1 therapeutics can disrupt Vif suppression of catalytic activity at either a single CD or both CD1 and CD2 simultaneously.

### E. Definitions

54. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.
55. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed.
56. In this specification and in the claims which follow, reference will be made to a number of terms which shall be defined to have the following meanings:
57. "Optional" or "optionally" means that the subsequently described event or circumstance may or may not occur, and that the description includes instances where said event or circumstance occurs and instances where it does not.
58. By "subject" is meant an individual. Preferably, the subject is a mammal such as a primate, and, more preferably, a human. The term "subject" can include domesticated animals, such as cats, dogs, etc., livestock (e.g., cattle, horses, pigs, sheep, goats, etc.), and laboratory animals (e.g., mouse, rabbit, rat, guinea pig, etc.).
59. By "contacting" is meant an instance of exposure of at least one substance to another substance. For example, contacting can include contacting a substance, such as a cell, or cell to a chimeric protein or analog described herein. A cell can be contacted with the chimeric protein or analog, for example, by adding the protein or analog to the culture medium (by continuous infusion, by bolus delivery, or by changing the medium to a medium that contains the agent) or by adding the agent to the extracellular fluid *in vivo* (by local delivery, systemic delivery, intravenous injection, bolus delivery, or continuous infusion). The duration of contact with a cell or group of cells is determined by the time the protein or analog is present at physiologically effective levels or at presumed physiologically effective levels in the medium or extracellular fluid bathing the cell. In the present invention, for example, a virally infected cell (e.g., an HIV infected cell) or a cell at risk for viral infection (e.g., before, at about the same time, or shortly after HIV infection of the cell) is contacted with a chimeric protein or analog.
60. "Treatment" or "treating" means to administer a composition to a subj ect with an undesired condition or at risk for the condition. The condition can be any pathogenic disease, autoimmune disease, cancer or inflammatory condition. The effect of the administration of the composition to the subject can have the effect of but is not limited to reducing the symptoms of the condition, a reduction in the severity of the condition, or the complete ablation of the condition.
61. By "effective amount" is meant a therapeutic amount needed to achieve the desired result or results, e.g., editing nucleic acids, interrupting CEM15-Vif binding, reducing viral infectivity, inducing class switch recombination, inducing somatic hypermutation, enhancing or blunting physiological functions, altering the qualitative or quantitative nature of the proteins expressed by cell or tissues, and eliminating or reducing disease causing molecules and/or the mRNA or DNA that encodes them, etc.
62. Herein, "inhibition" or "inhibits" means to reduce activity as compared to a control (e.g., activity in the absence of such inhibition). It is understood that inhibition can mean a slight reduction in activity to the complete ablation of all activity. An "inhibitor" can be anything that reduces activity. For example, an inhibition of CEM15-Vif binding by a disclosed composition can be determined by assaying the amount of CEM15-Vif binding in the presence of the composition to the amount of CEM15-Vif binding in the absence of the composition. In this example, if the amount of CEM15-Vif binding is reduced in the presence of the composition as compared to the amount of CEM15-Vif binding in the absence of the composition, the composition can be said to inhibit the CEM15-Vif binding. The AID molecule can also be inhibited.
63. Many methods disclosed herein refer to "systems." It is understood that systems can, for example, be cells or, for example, columns or batch processing containers, or, for example, culture plates, or for example the combination of unique bacterial or mammalian cells together with recombinant molecules expressed therein such as in a genetic screening system used for the purpose of enriching and identifying macromolecules with sequences of specific interest. A system is a set of components, any set of components that allows for the steps of the method to performed. Typically a system will comprise one or more components, such as a protein(s) or reagent(s). One type of system disclosed would be a cell that comprises both Vif and CEM15, for example. Another type of system would be one that comprises a cell and an infective unit (e.g., an HIV unit). A third type of system might be a chromatography column that has CEM15, AID, or other deaminase or putative deaminase, bound to the column. A fourth type of system might be a cell that comprises eitherAID or CEM15.
64. By "virally infected mammalian cell system" is meant an *in vitro* or *in vivo* system infected by a virus. Such a system can include mammalian cellular components; mammalian cells, tissues, or organs; and whole animal systems.
65. It is understood that the disclosed compositions can be labeled. Labeling can include covalent attachment of one or more labels, directly or through a spacer (e.g., an amide group), to non-interfering position(s) on the molecule being labeled, which can be determined empirically or through structure and structure-activity data and/or molecular modeling. Derivitization (e.g., labeling) of the compositions should not substantially interfere with the desired biological or pharmacological activity of the composition.
66. It is understood that the term "deaminase" refers to an enzyme in the ARP of ADAR family. Such an enzyme has the ability to remove an amine group from a cytidine/deoxycytidine or adenosie residue (respectively) through a hydrolytic elimination reaction, whether these substrates exist as free nucleosides/nucleotides or as part of the sequence of nucleotides with RNA or DNA. APOBEC-1, CEM15, and AID are discussed as the specific deaminases of interest and their expression as chimeric proteins and delivery into cells and tissues as TAT-deaminases are described, but also contemplated are other members of the ARP family. All deaminases can be used for expression, purification and intracellular delivery. A lack of expression or a deficiency in the expression of these ARPs in cells and tissues resulting in disease or suboptimal function, or when an elevated level of deaminase enzyme and activity can be beneficial, these ARPs can be used with the methods described herein.

### F. Compositions

67. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that, while specific reference of each various individual and collective combinations and permutation of these compounds may not be explicitly disclosed, each is specifically contemplated and described herein. For example, if a particular CEM15, Vif, CMPK, AID, or TAT is disclosed and discussed and a number of modifications that can be made to a number of molecules including the CEM15, Vif, CMPK, AID, or TAT are discussed, specifically contemplated is each and every combination and permutation of CEM15, Vif, CMPK, AID, or TAT and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.
68. Disclosed are chimeric proteins comprising a protein transduction domain; and a deaminase domain, wherein the deaminase edits viral RNA. Also disclosed are chimeric proteins comprising a protein transduction domain and a deaminase domain; wherein the deaminase can deaminate cytidine to form uridine in an RNA molecule, or deaminate cytidine to form thymidine in a DNA molecule.
69. The present invention also relates to a chimeric protein that is capable of being used to transduce B cells, either *in vitro* or *in vivo,* for purposes of inducing antibody production in B cells and thereby treat CSR and/or SHM conditions as well as B cell lymphomas.
70. By "chimeric protein" is meant any single polypeptide unit that comprises two distinct polypeptide domains joined by a peptide bond, optionally by means of an amino acid linker, or a non-peptide bond, wherein the two domains are not naturally occurring within the same polypeptide unit. Typically, such chimeric proteins are made by expression of a cDNA construct but could be made by protein synthesis methods known in the art. The chimeric proteins of the present invention contain, as a first polypeptide domain, a protein transduction domain (e.g., poly-arginine, poly-lysine peptide, third alpha helix of Antennapedia homeodomain protein, HSV-1 virion protein (VP) 22, HIV-1 Vpr, or HIV TAT protein) and, as a second polypeptide domain, a deaminase domain (e.g., an RNA or DNA deaminase such as adenosine to inosine deaminase or a cytidine to uridine deaminase). Such a chimeric protein can comprise a fragment or derivative of a naturally occurring protein transduction domain or a fragment or derivative of a naturally occurring deaminase. The chimeric protein of the invention optionally contains a mimetic of the naturally occurring protein transduction domain or a mimetic of the naturally occurring deaminase. The distinct polypeptide domains can be in reverse orientation to those examples given herein, or in any order within the chimeric protein.
71. "Deaminases" include deoxycytidine deaminase, cytidine deaminase, adenosine deaminase, RNA deaminase, DNA deaminase, and other deaminases. In one embodiment the deaminase is not APOBEC-1 (see international patent application designated PCT/US02/05824, which is incorporated herein by reference in its entirety for APOBEC-1, chimeric proteins related thereto, and uses thereof) (Gen Bank Accession # NP_001635), REE (see U.S. Pat. No. 5,747,319, which is incorporated herein by reference in its entirety for REE and uses thereof), or REE-2 (see U.S. Pat. No. 5,804,185, which is incorporated herein by reference in its entirety for REE-2 and uses thereof). Deaminases as described herein can include the following structural features: three or more CD repeats, two or more functional CDrepeats, one or more zinc binding domains (ZBDs), binding site(s) for mooring sequences, or protein-protein interaction (binding sites) for auxiliary RNA binding proteins or protein-protein interaction sites for DNA binding proteins or protein-protein interaction sites for proteins that interact with the deaminase to stimulate or suppress their activities either on cytidines in RNA or deoxycytidines in DNA or free ribose or deoxyribose nucleosides or nucleotides. Deaminases optionally edit viral RNA, host cell mRNA, viral DNA, host cell DNA or any combination thereof. One deaminase described herein is CEM15. CEM15 is identical to Phorbolin or APOBEC-3G (see, for example, Accession #NP_068594.) The terms APOBEC-3G and CEM15 are used interchangeably throughout. CEM15 reduces HIV infectivity as a DNA mutating (editing) enzyme. CEM15 mRNA substrates transcribed from either HIV-1 viral genomes or host cell genomes can be edited by CEM15 as well. Another deaminase described herein is AID. AID induces CSR, SHM, and gene conversion by mutating DNA and/or editing RNA.
72. Also disclosed are chimeric proteins comprising a protein transduction domain and a deaminase domain, wherein the deaminase edits viral RNA, and wherein the protein transduction domain is selected from the group consisting of poly-arginine, poly-lysine peptide, third alpha helix of Antennapedia homeodomain protein, HSV-1 virion protein (VP) 22, HIV-1 Vpr, and HIV TAT protein. Also disclosed are chimeric proteins comprising a protein transduction domain; and a deaminase domain, wherein the deaminase edits viral RNA, and wherein the protein transduction domain is an HIV TAT domain. Also disclosed are chimeric proteins, wherein the TAT domain comprises SEQ ID NO: 43 or a variant thereof.
73. By way of example, protein transduction domains from several known proteins can be employed, including without limitation, HIV-1 TAT protein, *Drosophila* homeotic transcription factor (ANTP), HSV-1 VP22 transcription factor, membrane-permeable sequences of the SN50 peptide, the Grb2 SH2 domain, and integrin β₃, β₁, and α_{IIb} cytoplasmic domains (Schwarze, TiPS 21:45-48 (2000), which is hereby incorporated by reference in its entirety), and others as described below.
74. A preferred protein transduction domain is the protein transduction domain of the human immunodeficiency virus (HIV) TAT protein. An exemplary HIV TAT protein transduction domain has an amino acid sequence of SEQ ID NO: 43 as follows: 
Arg Lys Lys Arg Arg Gln Arg Arg Arg
                5
75. This protein transduction domain has also been noted to be a nuclear translocation domain CB1Y (Sequence Compendium2000, Kuiken (eds.), Theoretical Biology and Biophysics Group, Los Alamos National Laboratory, which is hereby incorporated by reference in its entirety. One DNA molecule which encodes the HIV TAT protein transduction domain has a nucleotide sequence of SEQ ID NO: 44 as follows:
   agaaaaaaaa gaagacaaag aagaaga
76. Variations of these TAT sequences can also be employed. Such sequence variants have been reported in HIV Sequence Compendium 2000, Kuiken (eds.), Theoretical Biology and Biophysics Group, Los Alamos National Laboratory, which is hereby incorporated by reference in its entirety. The chimeric proteins comprising these variants described herein are useful with CEM15 or AID. In the context of the chimeric TAT-deaminase, one or more glycine residues can be added between TAT and the deaminase to improve flexibility between the TAT and deaminase domain, thereby enabling improved function of each domain.
77. Regarding AID, an example of a chimeric protein of the present invention which is suitable for use in humans is designated TAT-AID-HA-6His. The ' 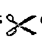 ' designates the site where a proteolytic cleavage motif may be inserted in future versions of this protein. Regions 6His, TAT and HA are not drawn to scale and correspond to the protein transduction motif, hemagglutinin epitope tag (for detection) and six Histidine motif (for purification). The construct can optionally include a CMPK domain or other suitable peptide domains as described for TAT-CEM15-CMPK. Similarly, the HA and 6His tags can be alternatively substituted with other appropriate detection of affinity purification tags as described above for TAT-CEM15-CMPK. Variations on the relative orientation of domains at the N- or C-terminus of the chimera are the same for the AID chimera as for the CEM15 chimera, as described above. 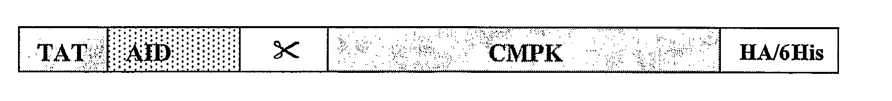
78. This chimeric protein (human) includes: an N-terminal HIV TAT protein transduction domain, a polypeptide fragment of human AID, a hemagglutinin domain, and a C-terminal His tag. The amino acid sequence (SEQ ID NO: 39) and encoding nucleotide sequence (SEQ ID NO: 40) of this exemplary chimeric protein (human) is set forth below. 
MRKKR RQRRR VDSLL MNRRK FLYQF KNVRW AKGRR ETYLC YVVKR RDSAT
SFSLD FGYLR NKNGC HVELL FLRYI SDWDL DPGRC YRVTW FTSWS PCYDC 100
ARHVA DFLRG NPNLS LRIFT ARLYF CEDRK AEPEG LRRLH RAGVQ IAIMT
 
FKDYF YCWNT FVENH ERTFK AWEGL HENSV RLSRQ LRRIL LPLYE VDDLR 200
DAFRT LGLHA AMADT FLEHM CRLDI DSEPT IARNT GIICT IGPAS RSVDK
LKEMI KSGMN VARLN FSHGT HEYHE GTIKN VREAT ESFAS DPITY RPVAI 300
ALDTK GPEIR TGLIK GSGTA EVELK KGAAL KVTLD NAFME NCDEN VLWVD
YKNLI KVIDV GSKIY VDDGL ISLLV KEKGK DFVMT EVENG GMLGS KKGVN 400
LPGAA VDLPA VSEKD IQDLK FGVEQ NVDMV FASFI RKAAD VHAVR KVLGE
KGKHI KIISK IENHE GVRRF DEIME ASDGI MVARG DLGIE IPAEK VFLAQ 500
KMMIG RCNRA GKPII CATQM LESMI KKPRP TRAEG SDVAN AVLDG ADCIM
LSGET AKGDY PLEAV RMQHA IAREA EAAMF HRQQF EEILR HSVHH REPAD 600
AMAAG AVEAS FKCLA AALIV MTESG RSAHL VSRYR PRAPI IAVTR NDQTA
RQAHL YRGVF PVLCK QPAHD AWAED VDLRV NLGMN VGKAR GFFKT GDLVI 700
VLTGW RPGSG YTNTM RVVPV PLEYP YDVPD YAHHH HHH (SEQ ID NO: 39)

atgag aaaaa aaaga agaca aagaa gaaga gtgga cagcc tcttg atgaa
ccgga ggaag tttct ttacc aattc aaaaa tgtcc gctgg gctaa gggtc 100
ggcgt gagac ctacc tgtgc tacgt agtga agagg cgtga cagtg ctaca
tcctt ttcac tggac tttgg ttatc ttcgc aataa gaacg gctgc cacgt 200
ggaat tgctc ttcct ccgct acatc tcgga ctggg accta gaccc tggcc
gctgc taccg cgtca cctgg ttcac ctcct ggagc ccctg ctacg actgt 300
gcccg acatg tggcc gactt tctgc gaggg aaccc caacc tcagt ctgag
gatct tcacc gcgcg cctct acttc tgtga ggacc gcaag gctga gcccg 400
agggg ctgcg gcggc tgcac cgcgc cgggg tgcaa atagc catca tgacc
ttcaa agatt atttt tactg ctgga atact tttgt agaaa accat gaaag 500
aactt tcaaa gcctg ggaag ggctg catga aaatt cagtt cgtct ctcca
gacag cttcg acgaa tcctt ttgcc cctgt atgag gttga tgact tacga 600
gacgc atttc gtact ttggg acttc acgct gccat ggcag acacc tttct
ggagc acatg tgccg cctgg acatc gactc cgagc caacc attgc cagaa 700
acacc ggcat catct gcacc atcgg cccag cctcc cgctc tgtgg acaag
ctgaa ggaaa tgatt aaatc tggaa tgaat gttgc ccgcc tcaac ttctc 800
gcacg gcacc cacga gtatc atgag ggcac aatta agaac gtgcg agagg
ccaca gagag ctttg cctct gaccc gatca cctac agacc tgtgg ctatt 900
gcact ggaca ccaag ggacc tgaaa tccga actgg actca tcaag ggaag
tggca cagca gaggt ggagc tcaag aaggg cgcag ctctc aaagt gacgc 1000
tggac aatgc cttca tggag aactg cgatg agaat gtgct gtggg tggac
tacaa gaacc tcatc aaagt tatag atgtg ggcag caaaa tctat gtgga 1100
tgacg gtctc atttc cttgc tggtt aagga gaaa ggcaag gactt tgtca
tgact gaggt tgaga acggt ggcat gcttg gtagt aagaa gggag tgaac 1200
ctccc aggtg ctgcg gtcga cctgc ctgca gtctc agaga aggac attca
ggacc tgaaa tttgg cgtgg agcag aatgt ggaca tggtg ttcgc ttcct 1300
tcatc cgcaa agctg ctgat gtcca tgctg tcagg aaggt gctag gggaa
aaggg aaagc acatc aagat tatca gcaag attga gaatc acgag ggtgt 1400
gcgca ggttt gatga gatca tggag gccag cgatg gcatt atggt ggccc
gtggt gacct gggta ttgag atccc tgctg aaaaa gtctt cctcg cacag 1500
aagat gatga ttggg cgctg caaca gggct ggcaa accca tcatt tgtgc
cactc agatg ttgga aagca tgatc aagaa acctc gcccg acccg cgctg 1600
agggc agtga tgttg ccaat gcagt tctgg atgga gcaga ctgca tcatg
ctgtc tgggg agacc gccaa gggag actac ccact ggagg ctgtg cgcat 1700
gcagc acgct attgc tcgtg aggct gaggc cgcaa tgttc catcg tcagc
agttt gaaga aatct tacgc cacag tgtac accac aggga gcctg ctgat 1800
gccat ggcag caggc gcggt ggagg cctcc tttaa gtgct tagca gcagc
tctga tagtt atgac cgagt ctggc aggtc tgcac acctg gtgtc ccggt 1900
accgc ccgcg ggctc ccatc atcgc cgtca cccgc aatga ccaaa cagca
cgcca ggcac acctg taccg cggcg tcttc cccgt gctgt gcaag cagcc 2000
ggccc acgat gcctg gcag aggat gtgga tctcc gtgtg aacct gggca
tgaat gtcgg caaag cccgt ggatt cttca agacc gggga cctgg tgatc 2100
gtgct gacgg gctgg cgccc cggct ccggc tacac caaca ccatg cgggt
ggtgc ccgtg ccact cgagt acccc tacga cgtgc ccgac tacgc ccacc 2200
accac cacca ccact ga (SEQ ID NO: 40)
79. In regard to CEM15, an exemplary chimeric protein of the present invention which is suitable for use in humans, designated TAT-CEM15-HA-6His. 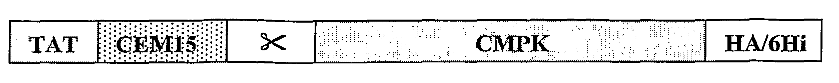
80. The 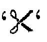 designates the site where a proteolytic cleavage motif may be inserted in future versions of this protein, such as but not limited to thrombin or Tev proteinase recognition or cleavage sites. Domains 6His, TAT and HA are not drawn to scale and correspond to the protein transduction motif, haemagglutinin epitope tag (for detection) and six Histidine motif (for purification). The location of these domains relative to one another is meant as an example as described above, but can also be varied. The association of the CMPK (chicken muscle pyruvate kinase) peptide serves to improve yield and solubility of the expressed protein when expressed in bacteria. CMPK is meant as an example but can be substituted with a variety of other proteins that serve a similar purpose, such as (but not limited to) GST (glutathione-S-transferase), GFP (green fluorescent protein) or maltose binding protein or protein A sequence (TAP). TAT-deaminase liberated from the associated peptide by proteolytic cleavage generates the therapeutic protein. The 6His tag is employed in the initial purification of the chimera, and the adsorption of the associated peptide following cleavage in the process yields purified TAT-deaminase. Any suitable affinity purification or detection tag such as GST, TAP, maltose binding protein or epitope are considered subtitutes for 6His or HA tags.
81. This chimeric protein (human) includes: an N-terminal HIV TAT protein transduction domain, a polypeptide fragment of human CEM15 (or alternatively a fragment of human AID or any other of the ARPs), a hemagglutinin domain, a C-terminal His tag, and optionally, a CMPK domain. The amino acid sequence (SEQ ID NO: 1) and encoding nucleotide sequence (SEQ ID NO: 2) of the CEM15 protein (human) is set forth below. The chimeric CEM15 protein can be the same as the chimeric AID protein described above, wherein the CEM15 portion of the chimeric CEM15 protein can be substituted for the AID portion of the AID chimeric protein found in SEQ ID NO: 39. 
MKPHF RNTVE RMYRD TFSYN FYNRP ILSRR NTVWL CYEVK TKGPS RPPLD
AKIFR GQVYS ELKYH PEMRF FHWFS KWRKL HRDQE YEVTW YISWS PCTKC 100
TRDMA TFLAE DPKVT LTIFV ARLYY FWDPD YQEAL RSLCQ KRDGP RATMK
IMNYD EFQHC WSKFV YSQRE LFEPW NNLPK YYILL HIMLG EILRH SMDPP 200
TFTFN FNNEP WVRGR HETYL CYEVE RMHND TWVLL NQRRG FLCNQ APHKH
GFLEG RHAEL CFLDV IPFWK LDLDQ DYRVT CFTSW SPCFS CAQEM AKFIS 300
KNKHV SLCIF TARIY DDQGR CQEGL RTLAE AGAKI SIMTY SEFKH CWDTF
VDHQG CPFQP WDGLD EHSQD LSGRL RAILQ NQEN (SEQ ID NO: 1)

atgaa gcctc acttc agaaa cacag tggag cgaat gtatc gagac acatt
ctcct acaac tttta taata gaccc atcct ttctc gtcgg aatac cgtct 100
ggctg tgcta cgaag tgaaa acaaa gggtc cctca aggcc ccctt tggac
gcaaa gatct ttcga ggcca ggtgt attcc gaact taagt accac ccaga 200
gatga gattc ttcca ctggt tcagc aagtg gagga agctg catcg tgacc
aggag tatga ggtca cctgg tacat atcct ggagc ccctg cacaa agtgt 300
acaag ggata tggcc acgtt cctgg ccgag gaccc gaagg ttacc ctgac
catct tcgtt gcccg cctct actac ttctg gaccc cagat tacca ggagg 400
cgctt cgcag cctgt gtcag aaaag agacg gtccg cgtgc cacca tgaag
atcat gaatt atgac gaatt tcagc actgt tggag caagt tcgtg tacag 500
ccaaa gagag ctatt tgagc cttgg aataa tctgc ctaaa tatta tatat
tactg cacat catgc tgggg gagat tctca gacac tcgat ggatc caccc 600
acatt cactt tcaac tttaa caatg aacct tgggt cagag gacgg catga
gactt acctg tgtta tgagg tggag cgcat gcaca atgac acctg ggtcc 700
tgctg aacca gcgca ggggc tttct atgca accag gctcc acata aacac
ggttt ccttg aaggc cgcca tgcag agctg tgctt cctgg acgtg attcc 800
ctttt ggaag ctgga cctgg accag gacta caggg ttacc tgctt cacct
cctgg agccc ctgct tcagc tgtgc ccagg aaatg gctaa attca tttca 900
aaaaa caaac acgtg agcct gtgca tcttc actgc ccgca tctat gatga
tcaag gaaga tgtca ggagg ggctg cgcac cctgg ccgag gctgg ggcca 1000
aaatt tcaat aatga catac agtga attta agcac tgctg ggaca ccttt
gtgga ccacc aggga tgtcc cttcc agccc tggga tggac tagat gagca 1100
cagcc aagac ctgag tggga ggctg cgggc cattc tccag aatca ggaaa
actga (SEQ ID NO: 2)
82. A further aspect of the present invention relates to chimeric proteins formed following the identification of mRNA(s) that are edited by AID, CEM15, or any other ARP. Thus, proteins translated from the edited mRNAs engineered with or without CMPK as shown in the diagram for suitable expression, purification, and TAT-mediated delivery (as described above) are designed as chimeras as shown below. 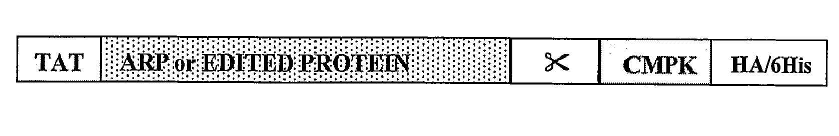
83. The construct can optionally include a CMPK domain or other suitable peptide domains as described for TAT-AID-CMPK-HA/6His. Similarly, the HA and 6His tags can be alternatively substituted with other appropriate detection or affinity purification tags as described above. Variations on the relative orientation of domains at the N- or C- terminus of the chimera are considered herein as described for TAT-AID-CMPK-HA/6His.
84. The second polypeptide can be a full length human or other mammalian AID protein or a polypeptide fragment thereof that maintains its utility as a deaminase. Human AID has an amino acid sequence (SEQ ID NO: 3) as follows: 

 100


 This protein is encoded by a DNA molecule having a nucleotide sequence (SEQ ID NO: 4) as follows: 
atggacagcc tcttgatgaa ccggaggaag tttctttacc aattcaaaaa
tgtccgctgg gctaagggtc ggcgtgagac ctacctgtgc tacgtagtga 100
agaggcgtga cagtgctaca tccttttcac tggactttgg ttatcttcgc
aataagaacg gctgccacgt ggaattgctc ttcctccgct acatctcgga 200
ctgggaccta gaccctggcc gctgctaccg cgtcacctgg ttcacctcct
ggagcccctg ctacgactgt gcccgacatg tggccgactt tctgcgaggg 300
aaccccaacc tcagtctgag gatcttcacc gcgcgcctct acttctgtga
ggaccgcaag gctgagcccg aggggctgcg gcggctgcac cgcgccgggg 400
tgcaaatagc catcatgacc ttcaaagatt atttttactg ctggaatact
tttgtagaaa accatgaaag aactttcaaa gcctgggaag ggctgcatga 500
aaattcagtt cgtctctcca gacagcttcg gcgcatcctt ttgcccctgt
atgaggttga tgacttacga gacgcatttc gtactttggg actttga 597
85. The above-listed nucleotide and amino acid sequences have been reported as Genbank Accession Nos. BC006296 and AAH06296, each of which is hereby incorporated by reference in its entirety.
86. Other cellular uptake polypeptides and their use have been described in the literature, including without limitation, *Drosophila* homeotic transcription factor (ANTP), HSV-1 VP22 transcription factor, membrane-permeable sequences of the SN50 peptide, the Grb2 SH2 domain, and integrin β₃, β₁, and α_{IIb}, cytoplasmic domains (Schwarze, TiPS 21:45-48 (2000), which is hereby incorporated by reference in its entirety). Such polypeptides can be used in the chimeric proteins of the invention.
87. By "deaminating function" is meant a deamination of a nucleotide (e.g., cytidine, deoxycytidine, adenosine, or deoxyadenosine). Deaminating function is detected by measuring the amount of deaminated nucleotide, according to the methods taught herein.
88. Also disclosed are chimeric proteins comprising a protein transduction domain, and a deaminase domain, wherein the deaminase edits viral RNA, and wherein the deaminase domain comprises CEM15. Also disclosed are chimeric proteins, wherein the CEM15 domain comprises SEQ ID NO: 1.
89. Also disclosed are chimeric proteins comprising a protein transduction domain; and a deaminase domain, wherein the deaminase edits mRNA or DNA, and wherein the deaminase domain comprises AID. Also disclosed are chimeric proteins, wherein the AID domain comprises SEQ ID NO: 3.
90. The chimeric proteins of the present invention can include full length domains (e.g., full length CEM15, AID, or full length TAT protein) or fragments or derivatives of either or both domains. A "fragment" is a polypeptide that is less than the full length of a particular protein or functional domain.
91. By "derivative" or "variant" is meant a polypeptide having a particular sequence that differs at one or more positions from a reference sequence. The fragments or derivatives of a full length protein preferably retain at least one function of the full length protein. For example, a fragment or derivative of a deaminase includes a fragment of a deaminase or a derivative deaminase (e.g., APOBEC-1, AID, CEM15, or any other ARP) that retains at least one binding or deaminating function of the full length protein. By way of example, the fragment or derivative can include a Zinc-Dependent Cytidine Deaminase domain or can include 20, 30, 40, 50, 60, 70 80, 90% similarity with the full length deaminase. The fragment or derivative can include conservative or non-conservative amino acid substitutions. The fragment or derivative can include a linker sequence joining a catalytic domain (CD) to a pseudo-catalytic domain (PCD) and can have the domain structure CD-PCD-CD-PCD or any repeats thereof. The fragment or derivative can comprise a CD. Other fragments or derivatives are identified by structure-based sequence alignment (SBSA) as shown herein. See Figure 6B that reveals the consensus structural domain attributes of APOBEC-1 and ARPs (Figure 6C). The fragment or derivative optionally can form a homodimer or a homotetramer. Also disclosed are chimeric proteins, wherein the deaminase domain is a fragment or derivative of CEM15 or AID having deaminase function.
92. Also disclosed are chimeric proteins, wherein the CEM15 fragment or derivative has at least 20, 30, 40, 50, 60, 70, 80, or 90 % amino acid similarity with CEM15.
93. Also disclosed are chimeric proteins, wherein the AID fragment or derivative has at least 20, 30, 40, 50, 60, 70, 80, or 90 % amino acid similarity with AID.
94. Also disclosed are chimeric proteins comprising a protein transduction domain and a deaminase domain and further comprising an epitope tag. By "epitope tag" is meant any tag useful in detecting the chimeric protein in biologic fluids or tissues. Examples include hemagglutinin and V5 (as well as other tags discussed above). The polypeptide that includes an epitope tag can be any epitope tag that is recognized with antibodies raised against the epitope tag. An exemplary epitope tag is a hemagglutinin (HA) domain. The HA domain is present only when it is desirable to examine, i.e., *in vitro*, localization of the first chimeric protein within cells that have translocated it. One suitable HA domain has an amino acid sequence of SEQ ID NO: 46. This HA sequence is encoded by a DNA molecule having a nucleotide sequence of SEQ ID NO: 47.
95. Also disclosed are chimeric proteins comprising a protein transduction domain and a deaminase domain and further comprising a purification tag. By "purification tag" is meant a tag that is useful in affinity purification of the chimeric protein. Such tags include for example, a GST tag (or other tags as discussed above), which includes 2, 3, 4, 5, 6, or more adjacent histidine residues, or a glutathione-S transferase tag. The polypeptide that includes a plurality of histidine residues preferably contains a sufficient number of histidine residues so as to allow the chimeric protein containing such histidine residues to be bound by an antibody which recognizes the plurality of histidine residues. One type of DNA molecule encoding Hₙ is (cac)ₙ, where n is greater than 1, but preferably greater than about 5. This His region can be used during immuno-purification, which is described in greater detail below.
96. Also disclosed are chimeric proteins comprising a protein transduction domain and a deaminase domain and further comprising a polypeptide domain that enhances solubility of the chimeric protein or promotes cytoplasmic or nuclear localization of the chimeric protein. By "enhances solubility" is meant that the solubility of the chimeric protein is enhanced as compared to the solubility in the absence of the enhancing agent. The solubility can be enhanced in bacterial, yeast or baccolovirus expression systems. By "promoting cytoplasmic or nuclear localization" is meant that the promoting polypeptide domain facilitates targeting of the chimeric protein to the nucleus (via nuclear localization signals or NLS) or to the cytoplasm (via nuclear export signals, NES, or cytoplasmic retention signals (CSRs)) by either moving the protein to the desired cellular compartment or by retaining the protein in the desired compartment. The promoting polypeptide can also affect the distribution of the chimeric protein between the cytoplasm and nucleus via a bulk protein effect such as the effect of CMPK on APOBEC-1 in the context of a chimeric protein.
97. The chimeric protein of the present invention can also include one or more other polypeptide sequences, including without limitation: (i) a polypeptide that includes a cytoplasmic localization protein or a fragment thereof which, upon cellular uptake of the first chimeric protein, localizes the first chimeric protein to the cytoplasm; (ii) a polypeptide that includes a plurality of adjacent histidine residues; and (iii) a polypeptide that includes an epitope tag.
98. The polypeptide that includes a cytoplasmic localization protein or a fragment thereof can be any protein, or fragment thereof, which can effectively retain the first chimeric protein within the cytoplasm of a cell into which the first chimeric protein has been translocated. One such protein is chicken muscle pyruvate kinase ("CMPK"), which has an amino acid sequence of SEQ ID No: 41 as follows: 
Met Ser Lys His His Asp Ala Gly Thr Ala Phe Ile Gln Thr
Gln Gln Leu His Ala Ala Met Ala Asp Thr Phe Leu Glu His
Met Cys Arg Leu Asp Ile Asp Ser Glu Pro Thr Ile Ala Arg
Asn Thr Gly Ile Ile Cys Thr Ile Gly Pro Ala Ser Arg Ser
Val Asp Lys Leu Lys Glu Met Ile Lys Ser Gly Met Asn Val
Ala Arg Leu Asn Phe Ser His Gly Thr His Glu Tyr His Glu
Gly Thr Ile Lys Asn Val Arg Glu Ala Thr Glu Ser Phe Ala
Ser Asp Pro Ile Thr Tyr Arg Pro Val Ala Ile Ala Leu Asp
Thr Lys Gly Pro Glu Ile Arg Thr Gly Leu Ile Lys Gly Ser
Gly Thr Ala Glu Val Glu Leu Lys Lys Gly Ala Ala Leu Lys
Val Thr Leu Asp Asn Ala Phe Met Glu Asn Cys Asp Glu Asn
Val Leu Trp Val Asp Tyr Lys Asn Leu Ile Lys Val Ile Asp
Val Gly Ser Lys Ile Tyr Val Asp Asp Gly Leu Ile Ser Leu
Leu Val Lys Glu Lys Gly Lys Asp Phe Val Met Thr Glu Val
Glu Asn Gly Gly Met Leu Gly Ser Lys Lys Gly Val Asn Leu
Pro Gly Ala Ala Val Asp Leu Pro Ala Val Ser Glu Lys Asp
Ile Gln Asp Leu Lys Phe Gly Val Glu Gln Asn Val Asp Met
Val Phe Ala Ser Phe Ile Arg Lys Ala Ala Asp Val His Ala
Val Arg Lys Val Leu Gly Glu Lys Gly Lys His Ile Lys Ile
Ile Ser Lys Ile Glu Asn His Glu Gly Val Arg Arg Phe Asp
Glu Ile Met Glu Ala Ser Asp Gly Ile Met Val Ala Arg Gly
Asp Leu Gly Ile Glu Ile Pro Ala Glu Lys Val Phe Leu Ala
Gln Lys Met Met Ile Gly Arg Cys Asn Arg Ala Gly Lys Pro
Ile Ile Cys Ala Thr Gln Met Leu Glu Ser Met Ile Lys Lys
Pro Arg Pro Thr Arg Ala Glu Gly Ser Asp Val Ala Asn Ala
Val Leu Asp Gly Ala Asp Cys Ile Met Leu Ser Gly Glu Thr
Ala Lys Gly Asp Tyr Pro Leu Glu Ala Val Arg Met Gln His
Ala Ile Ala Arg Glu Ala Glu Ala Ala Met Phe His Arg Gln
Gln Phe Glu Glu Ile Leu Arg His Ser Val His His Arg Glu
Pro Ala Asp Ala Met Ala Ala Gly Ala Val Glu Ala Ser Phe
Lys Cys Leu Ala Ala Ala Leu Ile Val Met Thr Glu Ser Gly
Arg Ser Ala His Leu Val Ser Arg Tyr Arg Pro Arg Ala Pro
Ile Ile Ala Val Thr Arg Asn Asp Gln Thr Ala Arg Gln Ala
His Leu Tyr Arg Gly Val Phe Pro Val Leu Cys Lys Gln Pro
Ala His Asp Ala Trp Ala Glu Asp Val Asp Leu Arg Val Asn
Leu Gly Met Asn Val Gly Lys Ala Arg Gly Phe Phe Lys Thr
Gly Asp Leu Val Ile Val Leu Thr Gly Trp Arg Pro Gly Ser
Gly Tyr Thr Asn Thr Met Arg Val Val Pro Val Pro
99. A DNA molecule encoding the full length CMPK has a nucleotide sequence according to SEQ ID No: 42 as follows: 
atgtcgaagc accacgatgc agggaccgct ttcatccaga cccagcagct gcacgctgcc 60
atggcagaca cctttctgga gcacatgtgc cgcctggaca tcgactccga gccaaccatt 120
gccagaaaca ccggcatcat ctgcaccatc ggcccagcct cccgctctgt ggacaagctg 180
aaggaaatga ttaaatctgg aatgaatgtt gcccgcctca acttctcgca cggcacccac 240
gagtatcatg agggcacaat taagaacgtg cgagaggcca cagagagctt tgcctctgac 300
ccgatcacct acagacctgt ggctattgca ctggacacca agggacctga aatccgaact 360
ggactcatca agggaagtgg cacagcagag gtggagctca agaagggcgc agctctcaaa 420
gtgacgctgg acaatgcctt catggagaac tgcgatgaga atgtgctgtg ggtggactac 480
aagaacctca tcaaagttat agatgtgggc agcaaaatct atgtggatga cggtctcatt 540
tccttgctgg ttaaggagaa aggcaaggac tttgtcatga ctgaggttga gaacggtggc 600
atgcttggta gtaagaaggg agtgaacctc ccaggtgctg cggtcgacct gcctgcagtc 660
tcagagaagg acattcagga cctgaaattt ggcgtggagc agaatgtgga catggtgttc 720
gcttccttca tccgcaaagc tgctgatgtc catgctgtca ggaaggtgct aggggaaaag 780
ggaaagcaca tcaagattat cagcaagatt gagaatcacg agggtgtgcg caggtttgat 840
gagatcatgg aggccagcga tggcattatg gtggcccgtg gtgacctggg tattgagatc 900
cctgctgaaa aagtcttcct cgcacagaag atgatgattg ggcgctgcaa cagggctggc 960
aaacccatca tttgtgccac tcagatgttg gaaagcatga tcaagaaacc tcgcccgacc 1020
cgcgctgagg gcagtgatgt tgccaatgca gttctggatg gagcagactg catcatgctg 1080
tctggggaga ccgccaaggg agactaccca ctggaggctg tgcgcatgca gcacgctatt 1140
gctcgtgagg ctgaggccgc aatgttccat cgtcagcagt ttgaagaaat cttacgccac 1200
agtgtacacc acagggagcc tgctgatgcc atggcagcag gcgcggtgga ggcctccttt 1260
aagtgcttag cagcagctct gatagttatg accgagtctg gcaggtctgc acacctggtg 1320
tcccggtacc gcccgcgggc tcccatcatc gccgtcaccc gcaatgacca aacagcacgc 1380
caggcacacc tgtaccgcgg cgtcttcccc gtgctgtgca agcagccggc ccacgatgcc 1440
tgggcagagg atgtggatct ccgtgtgaac ctgggcatga atgtcggcaa agcccgtgga 1500
ttcttcaaga ccggggacct ggtgatcgtg ctgacgggct ggcgccccgg ctccggctac 1560
accaacacca tgcgggtggt gcccgtgcca tga 1593
100. The amino acid sequence and nucleotide sequence for the full length CMPK is reported at Genbank Accession Nos. AAA49021 and JO0903, respectively, each of which is hereby incorporated by reference in its entirety.
101. Fragments of CMPK which afford cytoplasmic retention of the first chimeric protein include, without limitation, polypeptides containing at a minimum residues 1-479 of SEQ ID NO: 41.
102. Also disclosed are chimeric proteins comprising a protein transduction domain and a deaminase domain and further comprising a protein cleavage site. By "protein cleavage site" is meant a proteolytic site or any variant thereof.
103. Disclosed are chimeric proteins comprising a protein transducing domain and a deaminase domain that edits DNA. Also disclosed are chimeric proteins, wherein the deaminase domain edits viral DNA.
104. Also disclosed are chimeric proteins comprising a protein transducing domain and a deaminase domain that edits DNA, wherein the deaminase is a cytidine deaminase.
105. Disclosed is a chimeric protein comprising a protein transducing domain; and a deaminase domain, wherein the deaminase is not APOBEC-1. Also disclosed are chimeric proteins, wherein the deaminase has less than 20, 30, 40, 50, 60, 70, 80, 90 % amino acid similarity with APOBEC-1. An amino acid sequence of APOBEC-1 is provided as SEQ ID NO: 5.
106. Also disclosed are chimeric proteins, wherein the deaminase has more than 20, 30, 40, 50, 60, 70, 80, or 90 amino acid similarity with CEM15 (SEQ ID NO:1).
107. Also disclosed are chimeric proteins, wherein the deaminase has more than 20, 30, 40, 50, 60, 70, 80, or 90 amino acid similarity with AID (SEQ ID NO: 3).
108. Disclosed are chimeric proteins comprising a protein transducing domain, and a deaminase, wherein the deaminase does not edit ApoB1 mRNA.
109. Disclosed are chimeric proteins comprising a protein transducing domain and a deaminase domain, wherein the deaminase comprises more than two CD repeats. Also disclosed are chimeric proteins, wherein more than one of the CD repeats has a deaminating function.
110. By an "anchor oligonucleotide" is meant an oligonucleotide that binds the deaminase to the nucleotide sequence in the specific site necessary for deamination to occur.
111. Disclosed are chimeric proteins comprising a protein transducing domain, a deaminase domain, and an anchor oligonucleotide.
112. Disclosed are CEM15 mimetics, wherein the mimetic binds viral infectivity factor (e.g., Vif). Disclosed are chimeric proteins or peptides comprising a protein transducing domain and the CEM15 mimetic.
113. Disclosed are auxiliary protein and Vif mimetics, wherein the mimetic binds CEM15 and regulates or determines the (i) subcellular localization of CEM15 or (ii) its substrate specificity in terms of specific RNA or DNA sequence in which CEM15 selects cytidines or deoxycytidines to deaminate or (iii) its function in terms of the level or efficiency of the deamination reaction. Disclosed are chimeric proteins or peptides comprising a protein transducing domain and the auxiliary protein or Vif mimetic.
114. Also disclosed are AID mimetics, wherein the mimetic binds to an auxiliary protein that either regulates or determines the (i) subcellular localization of AID or (ii) its substrate specificity in terms of specific RNA or DNA sequence in which AID selects cytidines or deoxycytidines to deaminate or (iii) its function in terms of the level or efficiency of the deamination reaction. Mimetics of the auxiliary protein or of AID itself that alter any or all of the three functions described above are also contemplated herein. Disclosed are chimeric proteins or peptides comprising a protein transducing domain and the auxiliary protein mimetic.
115. Also disclosed are ARP mimetics, wherein the mimetic binds to an auxiliary protein that either regulates or determines the (i) subcellular localization of the ARP or (ii) its substrate specificity in terms of specific RNA or DNA sequence in which the ARP selects cytidines or deoxycytidines to deaminate or (iii) its function in terms of the level or efficiency of the deamination reaction. Mimetics of the auxiliary protein or of the ARP itself that alter any or all of the three functions described above are also contemplated herein. Disclosed are chimeric proteins or peptides comprising a protein transducing domain and the auxiliary protein mimetic.
116. Disclosed are isolated nucleotide sequences that encode the chimeric protein of the invention. For example, the invention provides a nucleotide sequence that encodes a chimeric protein comprising a protein transduction domain and a deaminase domain, wherein the deaminase edits RNA or DNA. Also disclosed are vectors comprising the nucleotide sequence that encodes a chimeric protein comprising a protein transduction domain and a deaminase domain. Also disclosed are recombinant host cells comprising the vector comprising the nucleotide sequence that encodes a chimeric protein comprising a protein transduction domain and a deaminase domain, wherein the deaminase edits viral RNA, or cellular RNA or DNA. Also provided are expression vectors, wherein the expression vector is operable in prokaryotic or eukaryotic cells. Further provided are nucleic acid sequences that selectively hybridize under stringent conditions with the nucleic acids that encode the chimeric proteins of the invention.
117. In one embodiment, the invention provides a composition comprising the chimeric protein and an auxiliary protein that is required to produce an editosome on RNA or a mutasome on DNA.

### 1. Sequence similarities

118. It is understood that, as discussed herein, the use of the terms "homology" and "identity" are used interchangeably with "similarity" with regard to amino acid or nucleic acid sequences. Homology is further used to refer to similarities in secondary and tertiary structures. In general, it is understood that one way to define any known variants and derivatives or those that might arise, of the disclosed genes and proteins herein, is through defining the variants and derivatives in terms of similarity to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent similarity to the stated sequence or the native sequence. For example, SEQ ID NOs: 2, 4, 42, and 44 set forth particular nucleic acid sequences that encode a CEM 15, AID, CMPK, and a TAT protein, respectively, and SEQ ID NOs: 1, 3, 41, and 43 set forth particular sequences of the proteins encoded by those nucleic acids. Specifically disclosed are variants of these and other genes and proteins herein disclosed which have at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent similarity to the stated sequence. Those of skill in the art readily understand how to determine the similarity of two proteins or nucleic acids, such as genes. For example, the similarity can be calculated after aligning the two sequences so that the similarity is at its highest level.
119. Another way of calculating similarity can be performed by published algorithms. Optimal alignment of sequences for comparison may be conducted by the algorithm of Smith and Waterman Adv. Appl. Math. 2: 482 (1981), by the alignment algorithm of Needleman and Wunsch, J. Mol Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.
120. The same types of similarity can be obtained for nucleic acids by for example the algorithms disclosed in Zuker, M. Science 244:48-52, 1989, Jaeger, Proc. Natl. Acad. Sci. USA 86:7706-7710, 1989, Jaeger, Methods Enzymol. 183:281-306, 1989, which are herein incorporated by reference for at least material related to nucleic acid alignment. It is understood that any of the methods typically can be used and that in certain instances the results of these various methods may differ, but the skilled artisan understands if identity is found with at least one of these methods, the sequences would be said to have the stated identity, and be disclosed herein.
121. For example, as used herein, a sequence recited as having a particular percent similarity to another sequence refers to sequences that have the recited homology as calculated by any one or more of the calculation methods described above. For example, a first sequence has 80 percent similarity, as defined herein, to a second sequence if the first sequence is calculated to have 80 percent similarity to the second sequence using the Zuker calculation method even if the first sequence does not have 80 percent similarity to the second sequence as calculated by any of the other calculation methods. As another example, a first sequence has 80 percent similarity, as defined herein, to a second sequence if the first sequence is calculated to have 80 percent similarity to the second sequence using both the Zuker calculation method and the Pearson and Lipman calculation method even if the first sequence does not have 80 percent similarity to the second sequence as calculated by the Smith and Waterman calculation method, the Needleman and Wunsch calculation method, the Jaeger calculation methods, or any of the other calculation methods. As yet another example, a first sequence has 80 percent similarity, as defined herein, to a second sequence if the first sequence is calculated to have 80 percent similarity to the second sequence using each of calculation methods (although, in practice, the different calculation methods will often result in different calculated similarity percentages).
122. Other structural similarities, aside from sequence similarity are also disclosed. For example, homology, as noted by similar secondary and tertiary structure can be analyzed, as taught herein. Homologous proteins may have minimal sequence similarity but have a homologous catalytic domain. Thus, deaminases as used herein may be structurally similar based on the structure of the catalytic domain or other domain but have lower than 70% sequence similarity.

### 2. Hybridization/selective hybridization

123. The term "hybridization" typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.
124. Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 5-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. (Sambrook, Molecular Cloning: A Laboratory Manual, 2nd Ed., Cold Spring Harbor Laboratory, Cold Spring Harbor, New York, 1989; Kunkel, Methods Enzymol. 1987:154:367, 1987 which is herein incorporated by reference for material at least related to hybridization of nucleic acids). A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.
125. Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.
126. Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.
127. Just as with similarity, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.
128. It is understood that those of skill in the art understand that if a composition or method meets any one of these criteria for determining hybridization either collectively or singly it is a composition or method that is disclosed herein.

### 3. Compositions identified by screening with disclosed compositions / combinatorial chemistry

### a) Combinatorial chemistry and protein mimetics

129. Given the information herein molecules that function like the disclosed molecules can be identified and used as discussed herein. For example, the knowledge that CEM15 interacts (directly or indirectly) with Vif and that delivery of CEM 15 and related molecules via the disclosed delivery constructs indicates targets for identifying molecules that will affect HIV infectivity. Disclosed are compositions and methods of making these compositions that bind Vif, such that CEM15 binding is competitively inhibited. Regarding AID, the knowledge that AID influences class switch recombination and somatic hypermutation, and that delivery of AID and related molecules via the disclosed delivery constructs indicates targets for identifying molecules that will affect SHM and CSR. As discussed herein, this knowledge can be used along with, for example, combinatorial chemistry techniques, to identify molecules that function as desired, by for example, inhibiting CEM15 and Vif binding, or enhancing or reducing AID activity, or mimic other deaminases.
130. The disclosed compositions, such as deaminases (e.g., ARPs such as CEM15 and AID), Vif, or TAT can be used as targets for any combinatorial technique to identify molecules or macromolecular molecules that interact with the disclosed compositions in a desired way or mimic their function. The nucleic acids, peptides, and related molecules disclosed herein can be used as targets for the combinatorial approaches. Also disclosed are the compositions that are identified through combinatorial techniques or screening techniques in which the compositions disclosed in SEQ ID NOS: 1, 3, 7, 43, or portions thereof, are used as the target in a combinatorial or screening protocol.
131. It is understood that when using the disclosed compositions in combinatorial techniques or screening methods, molecules, such as macromolecular molecules, will be identified that have particular desired properties such as inhibition or stimulation or the target molecule's function. The molecules identified and isolated when using the disclosed compositions, such as, CEM15, Vif, CMPK, AID, or TAT, are also disclosed. Thus, the products produced using the combinatorial or screening approaches that involve the disclosed compositions, such as, CEM15, Vif, CMPK, AID or TAT, are also disclosed.
132. Combinatorial chemistry includes but is not limited to all methods for isolating small molecules or macromolecules that are capable of binding either a small molecule or another macromolecule, typically in an iterative process. Proteins, oligonucleotides, and sugars are examples of macromolecules. For example, oligonucleotide molecules with a given function, catalytic or ligand-binding, can be isolated from a complex mixture of random oligonucleotides in what has been referred to as "in vitro genetics" (Szostak, TIBS 19:89, 1992). One synthesizes a large pool of molecules bearing random and defined sequences and subjects that complex mixture, for example, approximately 10¹⁵ individual sequences in 100 µg of a 100 nucleotide RNA, to some selection and enrichment process. Through repeated cycles of affinity chromatography and PCR amplification of the molecules bound to the ligand on the column, Ellington and Szostak (1990) estimated that 1 in 10¹⁰ RNA molecules folded in such a way as to bind a small molecule dyes. DNA molecules with such ligand-binding behavior have been isolated as well (Ellington and Szostak, 1992; Bock, 1992). Techniques aimed at similar goals exist for small organic molecules, proteins, antibodies and other macromolecules known to those of skill in the art. Screening sets of molecules for a desired activity whether based on small organic libraries, oligonucleotides, or antibodies is broadly referred to as combinatorial chemistry. Combinatorial techniques are particularly suited for defining binding interactions between molecules and for isolating molecules that have a specific binding activity, often called aptamers when the macromolecules are nucleic acids.
133. There are a number of methods for isolating proteins that either have *de novo* activity or a modified activity. For example, phage display libraries have been used to isolate numerous peptides that interact with a specific target. (See for example, United States Patent No. 6,031,071; 5,824,520; 5,596,079; and 5,565,332 which are herein incorporated by reference in their entirety for their material related to phage display and methods relate to combinatorial chemistry)
134. A preferred method for isolating proteins that have a given function is described by Roberts and Szostak (Roberts R.W. and Szostak J.W. Proc. Natl. Acad. Sci. USA, 94(23)12997-302 (1997). This combinatorial chemistry method couples the functional power of proteins and the genetic power of nucleic acids. An RNA molecule is generated in which a puromycin molecule is covalently attached to the 3'-end of the RNA molecule. An *in vitro* translation of this modified RNA molecule causes the correct protein, encoded by the RNA to be translated. In addition, because of the attachment of the puromycin, a peptdyl acceptor which cannot be extended, the growing peptide chain is attached to the puromycin which is attached to the RNA. Thus, the protein molecule is attached to the genetic material that encodes it. Normal *in vitro* selection procedures can now be done to isolate functional peptides. Once the selection procedure for peptide function is complete traditional nucleic acid manipulation procedures are performed to amplify the nucleic acid that codes for the selected functional peptides. After amplification of the genetic material, new RNA is transcribed with puromycin at the 3'-end, new peptide is translated and another functional round of selection is performed. Thus, protein selection can be performed in an iterative manner just like nucleic acid selection techniques. The peptide which is translated is controlled by the sequence of the RNA attached to the puromycin. This sequence can be anything from a random sequence engineered for optimum translation (i.e. no stop codons etc.) or it can be a degenerate sequence of a known RNA molecule to look for improved or altered function of a known peptide. The conditions for nucleic acid amplification and in vitro translation are well known to those of ordinary skill in the art and are preferably performed as in Roberts and Szostak (Roberts R.W. and Szostak J.W. Proc. Natl. Acad. Sci. USA, 94(23)12997-302 (1997)).
135. Another preferred method for combinatorial methods designed to isolate peptides is described in Cohen (Cohen B.A., Proc. Natl. Acad. Sci. USA 95(24):14272-7 (1998)). This method utilizes and modifies two-hybrid technology. Yeast two-hybrid systems are useful for the detection and analysis of protein:protein interactions. The two-hybrid system, initially described in the yeast *Saccharomyces cerevisiae*, is a powerful molecular genetic technique for identifying new regulatory molecules, specific to the protein of interest (Fields and Song, Nature 340:245-6 (1989)). Cohen modified this technology so that novel interactions between synthetic or engineered peptide sequences could be identified which bind a molecule of choice. The benefit of this type of technology is that the selection is done in an intracellular environment. The method utilizes a library of peptide molecules that attached to an acidic activation domain. A peptide of choice, for example a portion of Vif is attached to a DNA binding domain of a transcriptional activation protein, such as Gal 4. By performing the Two-hybrid technique on this type of system, molecules that bind the extracellular portion of Vif can be identified.
136. Using methodology well known to those of skill in the art, in combination with various combinatorial libraries, one can isolate and characterize those small molecules or macromolecules, which bind to or interact with the desired target. The relative binding affinity of these compounds can be compared and optimum compounds identified using competitive binding studies, which are well known to those of skill in the art.
137. Techniques for making combinatorial libraries and screening combinatorial libraries to isolate molecules which bind a desired target are well known to those of skill in the art. Representative techniques and methods can be found in but are not limited to United States patents 5,084,824, 5,288,514, 5,449,754, 5,506,337, 5,539,083, 5,545,568, 5,556,762, 5,565,324, 5,565,332, 5,573,905, 5,618,825, 5,619,680, 5,627,210, 5,646,285, 5,663,046, 5,670,326, 5,677,195, 5,683,899, 5,688,696, 5,688,997, 5,698,685, 5,712,146, 5,721,099, 5,723,598, 5,741,713, 5,792,431, 5,807,683, 5,807,754, 5,821,130, 5,831,014, 5,834,195, 5,834,318, 5,834,588, 5,840,500, 5,847,150, 5,856,107, 5,856,496, 5,859,190, 5,864,010, 5,874,443, 5,877,214, 5,880,972, 5,886,126, 5,886,127, 5,891,737, 5,916,899, 5,919,955, 5,925,527, 5,939,268, 5,942,387, 5,945,070, 5,948,696, 5,958,702, 5,958,792, 5,962,337, 5,965,719, 5,972,719, 5,976,894, 5,980,704, 5,985,356, 5,999,086, 6,001,579, 6,004,617, 6,008,321, 6,017,768, 6,025,371, 6,030,917, 6,040,193, 6,045,671, 6,045,755, 6,060,596, and 6,061,636.
138. Combinatorial libraries can be made from a wide array of molecules using a number of different synthetic techniques. For example, libraries containing fused 2,4-pyrimidinediones (United States patent 6,025,371) dihydrobenzopyrans (United States Patent 6,017,768and 5,821,130), amide alcohols (United States Patent 5,976,894), hydroxy-amino acid amides (United States Patent 5,972,719) carbohydrates (United States patent 5,965,719), 1,4-benzodiazepin-2,5-diones (United States patent 5,962,337), cyclics (United States patent 5,958,792), biaryl amino acid amides (United States patent 5,948,696), thiophenes (United States patent 5,942,387), tricyclic tetrahydroquinolines (United States patent 5,925,527), benzofurans (United States patent 5,919,955), isoquinolines (United States patent 5,916,899), hydantoin and thiohydantoin (United States patent 5,859,190), indoles (United States patent 5,856,496), imidazol-pyrido-indole and imidazol-pyrido-benzothiophenes (United States patent 5,856,107) substituted 2-methylene-2, 3-dihydrothiazoles (United States patent 5,847,150), quinolines (United States patent 5,840,500), PNA (United States patent 5,831,014), containing tags (United States patent 5,721,099), polyketides (United States patent 5,712,146), morpholino-subunits (United States patent 5,698,685 and 5,506,337), sulfamides (United States patent 5,618,825), and benzodiazepines (United States patent 5,288,514).
139. As used herein combinatorial methods and libraries included traditional screening methods and libraries as well as methods and libraries used in interative processes.

### b) Computer assisted design

140. The disclosed compositions can be used as targets for any molecular modeling technique to identify either the structure of the disclosed compositions or to identify potential or actual molecules, such as small molecules, which interact in a desired way with the disclosed compositions. The nucleic acids, peptides, proteins and related molecules disclosed herein can be used as targets in any molecular modeling program or approach.
141. It is understood that when using the disclosed compositions in modeling techniques, molecules, such as macromolecular molecules, will be identified that have particular desired properties such as inhibition or stimulation or the target molecule's function. The molecules identified and isolated when using the disclosed compositions, such as, CEM15, AID, Vif, CMPK, or TAT, are also disclosed. Thus, the products produced using the molecular modeling approaches that involve the disclosed compositions, such as, CEM15, AID, Vif, CMPK, or TAT, are also considered herein disclosed.
142. Thus, one way to isolate molecules that bind a molecule of choice is through rational design. This is achieved through structural information and computer modeling. Computer modeling technology allows visualization of the three-dimensional atomic structure of a selected molecule and the rational design of new compounds that will interact with the molecule. The three-dimensional construct typically depends on data from x-ray crystallographic analyses or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.
143. Examples of molecular modeling systems are the CHARMm and QUANTA programs, Polygen Corporation, Waltham, MA. CHARMm performs the energy minimization and molecular dynamics functions. QUANTA performs the construction, graphic modeling and analysis of molecular structure. QUANTA allows interactive construction, modification, visualization, and analysis of the behavior of molecules with each other.
144. A number of articles review computer modeling of drugs interactive with specific proteins, such as Rotivinen (1988) Acta Pharmaceutica Fennica 97, 159-166; Ripka, New Scientist 54-57 (June 16, 1988); McKinaly and Rossmann, 1989 Annu. Rev. Pharmacol._Toxiciol. 29, 111-122; Perry and Davies, QSAR: Quantitative Structure-Activity Relationships in Drug Design pp. 189-193 (Alan R. Liss, Inc. 1989); Lewis and Dean, 1989 Proc. R. Soc. Lond. 236, 125-140 and 141-162; and, with respect to a model enzyme for nucleic acid components, Askew, 1989 J. Am. Chem. Soc. 111, 1082-1090. Other computer programs that screen and graphically depict chemicals are available from companies such as BioDesign, Inc., Pasadena, CA., Allelix, Inc, Mississauga, Ontario, Canada, and Hypercube, Inc., Cambridge, Ontario. Although these are primarily designed for application to drugs specific to particular proteins, they can be adapted to design of molecules specifically interacting with specific regions of DNA or RNA, once that region is identified.
145. Although described above with reference to design and generation of compounds which could alter binding, one could also screen libraries of known compounds, including natural products or synthetic chemicals, and biologically active materials, including proteins, for compounds which alter substrate binding or enzymatic activity.
146. A compound that is identified or designed as a result of any of the disclosed methods can be obtained (or synthesized) and tested for its biological activity, e.g., competitive inhibition of CEM15-Vif binding or inhibition of HIV infectivity, or in the case of AID, the ability to deaminate cytidine to form uridine in an mRNA molecule or deaminate cytidine to form deoxycytidine in a DNA molecule.
147. Also disclosed are compositions produced by any of the processes as disclosed herein, as well as compositions capable of being identified by the processes disclosed herein.
148. Disclosed are cells that comprise an exogenous inhibitor of a CEM15-Vif interaction.
149. Also disclosed are cells that comprise an exogenous inhibitor of AID.
150. It is understood that the disclosed methods can be performed with libraries of molecules as well as a single molecule. Typically, if a library of molecules is being used, a step of separating the molecules within the library that, for example, bind to Vif competitively with CEM15, or to bind competitively with AID, from those that do not bind. This step of separation can be performed in a number of ways, including for example, through various chromatography means, including column chromatography, as well as using high through put mechanism, such as affinity sorting fluorescence analysis or fluorescence activated cell sorting (FACS) by flow cytometry.

### 4. Peptides

### a) Protein variants

151. As discussed herein there are numerous variants of the TAT protein, CEM15 protein, AID protein, and Vif protein that are known and herein contemplated. In addition, to the known functional CEM15, Vif, CMPK, AID, or TAT strain variants there are derivatives of the CEM15, Vif, CMPK, AID or TAT proteins which also function in the disclosed methods and compositions. Protein variants and derivatives are well understood to those of skill in the art and it can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables 2 and 3 and are referred to as conservative substitutions.
152.

**TABLE 2:Amino Acid Abbreviations**

| **Amino Acid** | **Abbreviations** |
|---|---|
| Alanine | Ala A |
| Allosoleucine | AIle |
| Arginine | Arg R |
| Asparagines | Asn N |
| Aspartic acid | Asp D |
| Cysteine | Cys C |
| Glutamic acid | Glu E |
| Glutamine | Gln Q |
| Glycine | Gly G |
| Histidine | His H |
| Isolelucine | Ile I |
| Leucine | Leu L |
| Lysine | Lys K |
| Phenylalanine | Phe F |
| Proline | Pro P |
| Pyroglutamic acid | Pglu |
| Serine | Ser S |
| Threonine | Thr T |
| Tyrosine | Tyr Y |
| Tryptophan | Trp W |
| Valine | Val V |

**TABLE 3:Amino Acid Substitutions**

| Original Residue Exemplary Conservative Substitutions |
|---|
| Ala; Ser |
| Arg;Lys; Gln |
| Asn; Gln; His |
| Asp; Glu |
| Cys; Ser |
| Gln; Asn, Lys |
| Glu; Asp |
| Gly; Pro |
| His; Asn; Gln |
| Ile; Leu; Val |
| Leu; Ile; Val |
| Lys; Arg; Gln; |
| Met; Leu; Ile |
| Phe; Met; Leu; Tyr |
| Ser; Thr |
| Thr; Ser |
| Trp; Tyr |
| Tyr; Trp; Phe |
| Val; Ile; Leu |

153. Substantial changes in function or immunological identity are made by selecting substitutions that are less conservative than those in Table 3, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.
154. For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.
155. Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.
156. Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.
157. It is understood that one way to define the variants and derivatives of the disclosed proteins herein is through defining the variants and derivatives in terms of homology/identity to specific known sequences. For example, SEQ ID NO: 43 sets forth a particular sequence of a TAT protein, SEQ ID NO: 1 sets forth a particular sequence of a CEM15 protein, SEQ ID NO: 3 sets forth a particular sequence of an AID protein, and SEQ ID NO: 41 seats forth a particular sequence for a CMPK protein. Specifically disclosed are variants of these and other proteins herein disclosed which have at least, 70% or 75% or 80% or 85% or 90% or 95% similarity to the stated sequence. Those of skill in the art readily understand how to determine the similarity of two proteins. For example, the similarity can be calculated after aligning the two sequences so that the similarity is at its highest level or by a variety of methods described above.
158. Another way of calculating similarity can be performed by published algorithms. Optimal alignment of sequences for comparison may be conducted by the local algorithm of Smith and Waterman Adv. Appl. Math. 2: 482 (1981), by the alignment algorithm of Needleman and Wunsch, J. Mol Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.
159. The same types of similarity can be obtained for nucleic acids by for example the algorithms disclosed in Zuker, M. Science 244:48-52, 1989, Jaeger Proc. Natl. Acad. Sci. USA 86:7706-7710, 1989, Jaeger Methods Enzymol. 183:281-306, 1989, which are herein incorporated by reference for at least material related to nucleic acid alignment.
160. It is understood that the description of conservative mutations and similarity can be combined together in any combination, such as embodiments that have at least 70% similarity to a particular sequence wherein the variants are conservative mutations.
161. As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. For example, one of the many nucleic acid sequences that can encode the protein sequence set forth in SEQ ID NOs: 1, 3, 7 and 43 is set forth in SEQ ID NO: 2, 4, 8 and 44, respectively. Provided herein are all degenerate variants of the nucleic acid sequences and all amino acids sequences with conservative amino acid substitutions.
162. It is understood that there are numerous amino acid and peptide analogs which can be incorporated into the disclosed compositions. For example, there are numerous D amino acids or amino acids which have a different functional substituent then the amino acids shown in Table 2 and Table 3. The opposite stereo isomers of naturally occurring peptides are disclosed, as well as the stereo isomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site specific way (Thorson Methods in Molec. Biol. 77:43-73 (1991), Zoller, Current Opinion in Biotechnology, 3:348-354 (1992); Ibba, Biotechnology & Genetic Engineering Reviews 13:197-216 (1995), Cahill TIBS, 14(10):400-403 (1989); Benner, TIB Tech, 12:158-163 (1994); Ibba and Hennecke, Bio/technology, 12:678-682 (1994) all of which are herein incorporated by reference at least for material related to amino acid analogs).
163. Molecules can be produced that resemble peptides, but which are not connected via a natural peptide linkage. For example, linkages for amino acids or amino acid analogs can include CH2NH--, --CH2S--, --CH2--CH2 --, --CH=CH-- (cis and trans), --COCH2 --, --CH(OH)CH2--, and --CHH2SO-(These and others can be found in Spatola, A. F. in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci (1980) pp. 463-468; Hudson, D. Int J Pept Prot Res 14:177-185 (1979) (--CH2NH--, CH2CH2--); Spatola, Life Sci 38:1243-1249 (1986) (--CH H2--S); Hann J. Chem. Soc Perkin Trans. I 307-314 (1982) (--CH--CH--, cis and trans); Almquist, J. Med. Chem. 23:1392-1398 (1980) (--COCH2--); Jennings-White, Tetrahedron Lett 23:2533 (1982) (--COCH2--); Szelke, European Appln, EP 45665 CA (1982): 97:39405 (1982) (--CH(OH)CH2--); Holladay, Tetrahedron. Lett 24:4401-4404 (1983) (-C(OH)CH2--); and Hruby Life Sci 31:189-199 (1982) (--CH2--S--); each of which is incorporated herein by reference. A particularly preferred non-peptide linkage is--CH2NH--. It is understood that peptide analogs can have more than one atom between the bond atoms, such as b-alanine, g-aminobutyric acid, and the like.
164. Amino acid analogs and analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, and others.
165. D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations. (Rizo and Gierasch Ann. Rev. Biochem. 61:387 (1992), incorporated herein by reference).

### 5. Functional Nucleic Acids

166. Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. The functional nucleic acid molecules can act as affectors, inhibitors, modulators, and stimulators of a specific activity possessed by a target molecule, or the functional nucleic acid molecules can possess a de novo activity independent of any other molecules.
167. Functional nucleic acid molecules can interact with any macromolecule, such as DNA, RNA, polypeptides, or carbohydrate chains. Thus, functional nucleic acids can interact with, for example, the mRNA of CEM15, AID, Vif, or TAT, or any other disclosed molecule, or the genomic DNA of CEM15, AID, Vif, or TAT, or any other disclosed molecule or they can interact with the polypeptide CEM15, AID, Vif, or TAT, or any other disclosed molecule. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.
168. Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be in vitro selection experiments and DNA modification studies using DMS and DEPC. It is preferred that antisense molecules bind the target molecule with a dissociation constant (kD) less than 10-6. It is more preferred that antisense molecules bind with a kD less than 10-8. It is also more preferred that the antisense molecules bind the target moelcule with a kD less than 10-10. It is also preferred that the antisense molecules bind the target molecule with a kD less than 10-12. A representative sample of methods and techniques which aid in the design and use of antisense molecules can be found in the following non-limiting list of United States patents: 5,135,917, 5,294,533, 5,627,158, 5,641,754, 5,691,317, 5,780,607, 5,786,138, 5,849,903, 5,856,103, 5,919,772, 5,955,590, 5,990,088, 5,994,320, 5,998,602, 6,005,095, 6,007,995, 6,013,522, 6,017,898, 6,018,042, 6,025,198, 6,033,910, 6,040,296, 6,046,004, 6,046,319, and 6,057,437.
169. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP (United States patent 5,631,146) and theophiline (United States patent 5,580,737), as well as large molecules, such as reverse transcriptase (United States patent 5,786,462) and thrombin (United States patent 5,543,293). Aptamers can bind very tightly with kDs from the target molecule of less than 10-12 M. It is preferred that the aptamers bind the target molecule with a kD less than 10-6. It is more preferred that the aptamers bind the target molecule with a kD less than 10-8. It is also more preferred that the aptamers bind the target molecule with a kD less than 10-10. It is also preferred that the aptamers bind the target molecule with a kD less than 10-12. Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a kD with the target molecule at least 10 fold lower than the kD with a background binding molecule. It is more preferred that the aptamer have a kD with the target molecule at least 100 fold lower than the kD with a background binding molecule. It is more preferred that the aptamer have a kD with the target molecule at least 1000 fold lower than the kD with a background binding molecule. It is preferred that the aptamer have a kD with the target molecule at least 10000 fold lower than the kD with a background binding molecule. It is preferred when doing the comparison for a polypeptide for example, that the background molecule be a different polypeptide. For example, when determining the specificity of CEM15, AID, Vif, or TAT, or any other disclosed molecule aptamers, the background protein could be serum albumin. Representative examples of how to make and use aptamers to bind a variety of different target molecules can be found in the following non-limiting list of United States patents: 5,476,766, 5,503,978, 5,631,146, 5,731,424, 5,780,228, 5,792,613, 5,795,721, 5,846,713, 5,858,660 , 5,861,254, 5,864,026, 5,869,641, 5,958,691, 6,001,988, 6,011,020, 6,013,443, 6,020,130, 6,028,186, 6,030,776, and 6,051,698.
170. Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (for example, but not limited to the following United States patents: 5,334,711, 5,436,330, 5,616,466, 5,633,133, 5,646,020, 5,652,094, 5,712,384, 5,770,715, 5,856,463, 5,861,288, 5,891,683, 5,891,684, 5,985,621, 5,989,908, 5,998,193, 5,998,203, WO 9858058 by Ludwig and Sproat, WO 9858057 by Ludwig and Sproat, and WO 9718312 by Ludwig and Sproat) hairpin ribozymes (for example, but not limited to the following United States patents: 5,631,115, 5,646,031, 5,683,902, 5,712,384, 5,856,188, 5,866,701, 5,869,339, and 6,022,962), and tetrahymena ribozymes (for example, but not limited to the following United States patents: 5,595,873 and 5,652,107). There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence. Representative examples of how to make and use ribozymes to catalyze a variety of different reactions can be found in the following non-limiting list of United States patents: 5,646,042, 5,693,535, 5,731,295, 5,811,300, 5,837,855, 5,869,253, 5,877,021, 5,877,022, 5,972,699, 5,972,704, 5,989,906, and 6,017,756.
171. Triplex forming functional nucleic acid molecules are molecules that can interact with either double-stranded or single-stranded nucleic acid. When triplex molecules interact with a target region, a structure called a triplex is formed, in which there are three strands of DNA forming a complex dependant on both Watson-Crick and Hoogsteen base-pairing. Triplex molecules are preferred because they can bind target regions with high affinity and specificity. It is preferred that the triplex forming molecules bind the target molecule with a kD less than 10-6. It is more preferred that the triplex forming molecules bind with a kD less than 10-8. It is also more preferred that the triplex forming molecules bind the target moelcule with a kD less than 10-10. It is also preferred that the triplex forming molecules bind the target molecule with a kD less than 10-12. Representative examples of how to make and use triplex forming molecules to bind a variety of different target molecules can be found in the following non-limiting list of United States patents: 5,176,996, 5,645,985, 5,650,316, 5,683,874, 5,693,773, 5,834,185, 5,869,246, 5,874,566, and 5,962,426.
172. External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. (WO 92/03566 by Yale, and Forster and Altman, Science 238:407-409 (1990)).
173. Similarly, eukaryotic EGS/RNAse P-directed cleavage of RNA can be utilized to cleave desired targets within eukaryotic cells. (Yuan, Proc. Natl. Acad. Sci. USA 89:8006-8010 (1992); WO 93/22434 by Yale; WO 95/24489 by Yale; Yuan and Altman, EMBO J 14:159-168 (1995), and Carrara, Proc. Natl. Acad. Sci. (USA) 92:2627-2631 (1995)). Representative examples of how to make and use EGS molecules to facilitate cleavage of a variety of different target molecules can be found in the following non-limiting list of United States patents: 5,168,053, 5,624,824, 5,683,873, 5,728,521, 5,869,248, and 5,877,162.

### 6. Delivery of the compositions to cells

174. The disclosed chimeric proteins and compositions can be delivered to the target cells in a variety of ways. TAT-deaminase can be added directly to cells in culture or injected into the body, whereupon the TAT-deaminase transduces through the cell membrane and into the cell's interior. Alteratively, the compositions can be delivered through electroporation, or through lipofection, or through calcium phosphate precipitation. The delivery mechanism chosen will depend in part on the type of cell targeted and whether the delivery is occurring for example *in vivo* or *in vitro.*
175. Thus, the compositions can comprise, for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham, Am. J. Resp. Cell. Mol. Biol. 1:95-100 (1989); Felgner, Proc. Natl. Acad. Sci USA 84:7413-7417 (1987); U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

### 7. Nucleic acids

176. There are a variety of molecules disclosed herein that are nucleic acid based, including for example the nucleic acids that encode the chimeric proteins or domains thereof, for example CEM15 and TAT, or AID and TAT, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantageous that the antisense molecule be made up of nucleotide analogs that reduce the degradation of the antisense molecule in the cellular environment.

### a) Nucleotides and related molecules

177. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenine-9-yl (A), cytosine-1-yl (C), guanine-9-yl (G), uracil-1-yl (U), and thymine-1-yl (T). The sugar moiety of a nucleotide is a ribose or a deoxyribose. The phosphate moiety of a nucleotide is pentavalent phosphate. A non-limiting example of a nucleotide would be 3'-AMP (3'-adenosine monophosphate) or 5'-GMP (5'-guanosine monophosphate).
178. A nucleotide analog is a nucleotide that contains some type of modification to either the base, sugar, or phosphate moieties. Modifications to the base moiety would include natural and synthetic modifications of A, C, G, and T/U as well as different purine or pyrimidine bases, such as uracil-5-yl (.psi.), hypoxanthine-9-yl (I), and 2-aminoadenine-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Often time base modifications can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range of base modifications. Each of these patents is herein incorporated by reference.
179. Nucleotide analogs can also include modifications of the sugar moiety. Modifications to the sugar moiety would include natural modifications of the ribose and deoxy ribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀, alkyl or C₂ to C₁₀ alkenyl and alkynyl. 2' sugar modifications also include but are not limited to -O[(CH₂)ₙ O]ₘ CH₃, -O(CH₂)ₙ OCH₃, -O(CH₂)ₙ NH₂, -O(CH₂)ₙ CH₃, -O(CH₂)ₙ -ONH₂, and -O(CH₂)ₙON[(CH₂)ₙ CH₃)]₂, where n and m are from 1 to about 10.
180. Other modifications at the 2' position include but are not limited to: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂ N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference in its entirety.
181. Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkage between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference.
182. It is understood that nucleotide analogs need only contain a single modification but may also contain multiple modifications within one of the moieties or between different moieties.
183. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.
184. Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference.
185. It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA molecules, each of which is herein incorporated by reference. (See also Nielsen, Science, 1991, 254, 1497-1500).
186. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety (Letsinger, Proc. Natl. Acad. Sci. USA, 1989, 86, 6553-6556), cholic acid (Manoharan, Bioorg. Med. Chem. Let., 1994, 4, 1053-1060), a thioether, e.g., hexyl-S-tritylthiol (Manoharan, Ann. N.Y. Acad. Sci., 1992, 660, 306-309; Manoharan, Bioorg. Med. Chem. Let., 1993, 3, 2765-2770), a thiocholesterol (Oberhauser, Nucl. Acids Res., 1992, 20, 533-538), an aliphatic chain, e.g., dodecandiol or undecyl residues (Saison-Behmoaras, EMBO J., 1991, 10, 1111-1118; Kabanov, FEBS Lett., 1990, 259, 327-330; Svinarchuk, Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan, Tetrahedron Lett., 1995, 36, 3651-3654; Shea., Nucl. Acids Res., 1990, 18, 3777-3783), a polyamine or a polyethylene glycol chain (Manoharan, Nucleosides & Nucleotides, 1995, 14, 969-973), or adamantane acetic acid (Manoharan, Tetrahedron Lett., 1995, 36, 3651-3654), a palmityl moiety (Mishra, Biochim. Biophys. Acta, 1995, 1264, 229-237), or an octadecylamine or hexylamino-carbonyl-oxycholesterol moiety (Crooke, J. Pharmacol. Exp. Ther., 1996, 277, 923-937. Numerous United States patents teach the preparation of such conjugates and include, but are not limited to U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.
187. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.
188. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### b) Sequences

189. There are a variety of sequences for the PTD domain, the deaminase domain, and other domains of the chimeric proteins. It is understood that the description related to these sequences is applicable to any sequence related thereto unless specifically indicated otherwise. Those of skill in the art understand how to resolve sequence discrepancies and differences and to adjust the compositions and methods relating to a particular sequence to other related sequences. Primers and/or probes can be designed for any sequence given the information disclosed herein and known in the art.

### 8. Antibodies

### a) Antibodies Generally

190. The invention further provides antibodies to the chimeric proteins or any portion thereof. As used herein, the term "antibody" encompasses, but is not limited to, whole immunoglobulin (i.e., an intact antibody) of any class. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V(H)) followed by a number of constant domains. Each light chain has a variable domain at one end (V(L)) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. The light chains of antibodies from any vertebrate species can be assigned to one of two clearly distinct types, called kappa (k) and lambda (l), based on the amino acid sequences of their constant domains. Depending on the amino acid sequence of the constant domain of their heavy chains, immunoglobulins can be assigned to different classes. There are five major classes of human immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. One skilled in the art would recognize the comparable classes for mouse. The heavy chain constant domains that correspond to the different classes of immunoglobulins are called alpha, delta, epsilon, gamma, and mu, respectively.
191. The term "variable" is used herein to describe certain portions of the variable domains that differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a b-sheet configuration, connected by three CDRs, which form loops connecting, and in some cases forming part of, the b-sheet structure. The CDRs in each chain are held together in close proximity by the FR regions and, with the CDRs from the other chain, contribute to the formation of the antigen binding site of antibodies (see Kabat E. A. et al., "Sequences of Proteins of Immunological Interest," National Institutes of Health, Bethesda, Md. (1987)). The constant domains are not involved directly in binding an antibody to an antigen, but exhibit various effector functions, such as participation of the antibody in antibody-dependent cellular toxicity.
192. As used herein, the term "antibody or fragments thereof" encompasses chimeric antibodies and hybrid antibodies, with dual or multiple antigen or epitope specificities, and fragments, such as scFv, sFv, F(ab')2, Fab', Fab and the like, including hybrid fragments. Thus, fragments of the antibodies that retain the ability to bind their specific antigens are provided. For example, fragments of antibodies which maintain Vif binding activity are included within the meaning of the term "antibody or fragment thereof." Such antibodies and fragments can be made by techniques known in the art and can be screened for specificity and activity according to the methods set forth in the Examples and in general methods for producing antibodies and screening antibodies for specificity and activity (See Harlow and Lane, Antibodies, A Laboratory Manual. Cold Spring Harbor Publications, New York, (1988)).
193. Also included within the meaning of "antibody or fragments thereof" are conjugates of antibody fragments and antigen binding proteins (single chain antibodies) as described, for example, in U.S. Pat. No. 4,704,692, the contents of which are hereby incorporated by reference.
194. Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region (J(H)) gene in chimeric and germ-line mutant mice results in complete inhibition of endogenous antibody production. Transfer of the human germ-line immunoglobulin gene array in such germ-line mutant mice will result in the production of human antibodies upon antigen challenge (see, e.g., Jakobovits, Proc. Nat1. Acad. Sci. USA, 90:2551-255 (1993); Jakobovits, Nature, 362:255-258 (1993); Bruggemann, Year in Immuno., 7:33 (1993)). Human antibodies can also be produced in phage display libraries (Hoogenboom, J. Mol. Biol., 227:381 (1991); Marks, J. Mol. Biol., 222:581 (1991)). The techniques of Cole and Boerner are also available for the preparation of human monoclonal antibodies (Cole, Monoclonal Antibodies and Cancer Therapy, Alan R. Liss, p. 77 (1985); Boerner, J. Immunol., 147(1):86-95 (1991)).
195. The present invention further provides a hybidoma cell that produces the monoclonal antibody of the invention. The term "monoclonal antibody" as used herein refers to an antibody obtained from a substantially homogeneous population of antibodies, i.e., the individual antibodies comprising the population are identical except for possible naturally occurring mutations that may be present in minor amounts. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired activity (See, U.S. Pat. No. 4,816,567 and Morrison, Proc. Natl. Acad. Sci. USA, 81:6851-6855 (1984)).
196. Generally, either peripheral blood lymphocytes ("PBLs") are used in methods of producing monoclonal antibodies if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, "Monoclonal Antibodies: Principles and Practice" Academic Press, (1986) pp. 59-103). Immortalized cell lines are usually transformed mammalian cells, including myeloma cells of rodent, bovine, equine, and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells. Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif. and the American Type Culture Collection, Rockville, Md. Human myeloma and mouse-human heteromyeloma cell lines also have been described for the production of human monoclonal antibodies (Kozbor, J. Immunol., 133:3001 (1984); Brodeur, "Monoclonal Antibody Production Techniques and Applications" Marcel Dekker, Inc., New York, (1987) pp. 51-63). The culture medium in which the hybridoma cells are cultured can then be assayed for the presence of monoclonal antibodies directed against Vif. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art, and are described further in the Examples below or in Harlow and Lane "Antibodies, A Laboratory Manual" Cold Spring Harbor Publications, New York, (1988).
197. After the desired hybridoma cells are identified, the clones may be subcloned by limiting dilution or FACS sorting procedures and grown by standard methods. Suitable culture media for this purpose include, for example, Dulbecco's Modified Eagle's Medium and RPMI-1640 medium. Alternatively, the hybridoma cells may be grown in vivo as ascites in a mammal. The monoclonal antibodies secreted by the subclones may be isolated or purified from the culture medium or ascites fluid by conventional immunoglobulin purification procedures such as, for example, protein A-Sepharose, protein G, hydroxylapatite chromatography, gel electrophoresis, dialysis, or affinity chromatography.
198. The monoclonal antibodies may also be made by recombinant DNA methods, such as those described in U.S. Pat. No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, plasmacytoma cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences (U.S. Pat. No. 4,816,567) or by covalently joining to the immunoglobulin coding sequence all or part of the coding sequence for a non-immunoglobulin polypeptide. Optionally, such a non-immunoglobulin polypeptide is substituted for the constant domains of an antibody of the invention or substituted for the variable domains of one antigen-combining site of an antibody of the invention to create a chimeric bivalent antibody comprising one antigen-combining site having specificity for Vif and another antigen-combining site having specificity for a different antigen.
199. *In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994, U.S. Pat. No. 4,342,566, and Harlow and Lane, Antibodies, A Laboratory Manual, Cold Spring Harbor Publications, New York, (1988). Papain digestion of antibodies typically produces two identical antigen binding fragments, called Fab fragments, each with a single antigen binding site, and a residual Fc fragment. Pepsin treatment yields a fragment, called the F(ab')2 fragment, that has two antigen combining sites and is still capable of cross-linking antigen.
200. The Fab fragments produced in the antibody digestion also contain the constant domains of the light chain and the first constant domain of the heavy chain. Fab' fragments differ from Fab fragments by the addition of a few residues at the carboxy terminus of the heavy chain domain including one or more cysteines from the antibody hinge region. The F(ab')2 fragment is a bivalent fragment comprising two Fab' fragments linked by a disulfide bridge at the hinge region. Fab'-SH is the designation herein for Fab' in which the cysteine residue(s) of the constant domains bear a free thiol group. Antibody fragments originally were produced as pairs of Fab' fragments which have hinge cysteines between them. Other chemical couplings of antibody fragments are also known.
201. An isolated immunogenically specific paratope or fragment of the antibody is also provided. A specific immunogenic epitope of the antibody can be isolated from the whole antibody by chemical or mechanical disruption of the molecule. The purified fragments thus obtained are tested to determine their immunogenicity and specificity by the methods taught herein. Immunoreactive paratopes of the antibody, optionally, are synthesized directly. An immunoreactive fragment is defined as an amino acid sequence of at least about two to five consecutive amino acids derived from the antibody amino acid sequence.
202. One method of producing proteins comprising the antibodies or chimeric proteins of the present invention is to link two or more peptides or polypeptides together by protein chemistry techniques described herein.
203. A variety of immunoassay formats may be used to select antibodies that selectively bind with a particular protein, variant, or fragment. For example, solid-phase ELISA immunoassays are routinely used to select antibodies selectively immunoreactive with a protein, protein variant, or fragment thereof. See Harlow and Lane, Antibodies, A Laboratory Manual. Cold Spring Harbor Publications, New York, (1988), for a description of immunoassay formats and conditions that could be used to determine selective binding. The binding affinity of a monoclonal antibody can, for example, be determined by the Scatchard analysis of Munson, Anal. Biochern., 107:220 (1980).
204. Also provided is an antibody reagent kit comprising containers of the monoclonal antibody or fragment thereof of the invention and one or more reagents for detecting binding of the antibody or fragment thereof to the Vif. The reagents can include, for example, fluorescent tags, enzymatic tags, or other tags. The reagents can also include secondary or tertiary antibodies or reagents for enzymatic reactions, wherein the enzymatic reactions produce a product that can be visualized.
205. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

### b) Human antibodies

206. The human antibodies of the invention can be prepared using any technique. Examples of techniques for human monoclonal antibody production include those described by Cole (Monoclonal Antibodies and Cancer Therapy, Alan R. Liss, p. 77, 1985) and by Boerner (J. Immunol., 147(1):86-95, 1991). Human antibodies of the invention (and fragments thereof) can also be produced using phage display libraries (Hoogenboom, J. Mol. Biol., 227:381, 1991; Marks, J. Mol. Biol., 222:581, 1991).
207. The human antibodies of the invention can also be obtained from transgenic animals. For example, transgenic, mutant mice that are capable of producing a full repertoire of human antibodies, in response to immunization, have been described (see, e.g., Jakobovits, Proc. Natl. Acad. Sci. USA, 90:2551-255 (1993); Jakobovits, Nature, 362:255-258 (1993); Bruggermann, Year in Immunol. 7:33 (1993)). Specifically, the homozygous deletion of the antibody heavy chain joining region (J*(H)*) gene in these chimeric and germ-line mutant mice results in complete inhibition of endogenous antibody production, and the successful transfer of the human germ-line antibody gene array into such germ-line mutant mice results in the production of human antibodies upon antigen challenge. Antibodies having the desired activity are selected using Env-CD4-co-receptor complexes as described herein.

### c) Humanized antibodies

208. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fc, Fv, Fab, Fab', or other antigen-binding portion of an antibody) which contains a portion of an antigen binding site from a non-human (donor) antibody integrated into the framework of a human (recipient) antibody.
209. To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones, Nature, 321:522-525 (1986), Reichmann, Nature, 332:323-327 (1988), and Presta, Curr. Opin. Struct. Biol., 2:593-596 (1992)).
210. Methods for humanizing non-human antibodies are well known in the art. For example, humanized antibodies can be generated according to the methods of Winter and co-workers (Jones, Nature, 321:522-525 (1986), Riechmann, Nature, 332:323-327 (1988), Verhoeyen, Science, 239:1534-1536 (1988)), by substituting rodent CDRs or CDR sequences for the corresponding sequences of a human antibody. Methods that can be used to produce humanized antibodies are also described in U.S. Patent No. 4,816,567 (Cabilly), U.S. Patent No. 5,565,332 (Hoogenboom), U.S. Patent No. 5,721,367 (Kay), U.S. Patent No. 5,837,243 (Deo), U.S. Patent No. 5, 939,598 (Kucherlapati), U.S. Patent No. 6,130,364 (Jakobovits), and U.S. Patent No. 6,180,377 (Morgan).

### d) Administration of antibodies

211. Antibodies of the invention are preferably administered to a subject in a pharmaceutically acceptable carrier. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of antibody being administered.
212. The antibodies can be administered to the subject, patient, or cell by injection (e.g., intravenous, intraperitoneal, subcutaneous, intramuscular), or by other methods such as infusion that ensure its delivery to the bloodstream in an effective form. Local or intravenous injection is preferred. Furthermore, *ex vivo* administration can be used wherein cells or tissues are isolated, treated, and returned to the subject to be treated.
213. Effective dosages and schedules for administering the antibodies may be determined empirically, and making such determinations is within the skill in the art. Those skilled in the art will understand that the dosage of antibodies that must be administered will vary depending on, for example, the subject that will receive the antibody, the route of administration, the particular type of antibody used and other drugs being administered. Guidance in selecting appropriate doses for antibodies is found in the literature on therapeutic uses of antibodies, e.g., Handbook of Monoclonal Antibodies, Ferrone, eds., Noges Publications, Park Ridge, N.J., (1985) ch. 22 and pp. 303-357; Smith, Antibodies in Human Diagnosis and Therapy, Haber, eds., Raven Press, New York (1977) pp. 365-389. A typical daily dosage of the antibody used alone might range from about 1 µg/kg to up to 100 mg/kg of body weight or more per day, depending on the factors mentioned above.
214. Following administration of an antibody for treating, inhibiting, or preventing an HIV infection, the efficacy of the therapeutic antibody can be assessed in various ways well known to the skilled practitioner. For instance, one of ordinary skill in the art will understand that an antibody of the invention is efficacious in treating or inhibiting an HIV infection in a subject by observing that the antibody reduces viral load or prevents a further increase in viral load. Viral loads can be measured by methods that are known in the art, for example, using polymerase chain reaction assays to detect the presence of HIV nucleic acid or antibody assays to detect the presence of HIV protein in a sample (e.g., but not limited to, blood) from a subject or patient, or by measuring the level of circulating anti-HIV antibody levels in the patient. Efficacy of the antibody treatment may also be determined by measuring the number of CD4⁺ T cells in the HIV-infected subject. An antibody treatment that inhibits an initial or further decrease in CD4⁺ T cells in an HIV-positive subject or patient, or that results in an increase in the number of CD4⁺ T cells in the HIV-positive subject, is an efficacious antibody treatment.
215. Antibodies disclosed herein can also be used to detect various compounds of the invention. Such antibodies can be used for research and clinical purposes.

### 9. Pharmaceutical carriers/Delivery of pharmaceutical products

216. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.
217. The compositions may be administered orally, parenterally (e.g., intravenously), by intramuscular injection, by intraperitoneal injection, transdermally, extracorporeally, topically or the like, although topical intranasal administration or administration by inhalant is typically preferred. As used herein, "topical intranasal administration" means delivery of the compositions into the nose and nasal passages through one or both of the nares and can comprise delivery by a spraying mechanism or droplet mechanism, or through aerosolization of the nucleic acid or vector. The latter may be effective when a large number of animals is to be treated simultaneously. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.
218. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.
219. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, Bioconjugate Chem., 2:447-451, (1991); Bagshawe, K.D., Br. J. Cancer, 60:275-281, (1989); Bagshawe, Br. J. Cancer, 58:700-703, (1988); Senter, Bioconjugate Chem., 4:3-9, (1993); Battelli, Cancer Immunol. Immunother., 35:421-425, (1992); Pietersz and McKenzie, Immunolog. Reviews, 129:57-80, (1992); and Roffler, Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo*. The following references are examples of the use of this technology to target specific proteins to tumor tissue (Hughes, Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).
220. Liposomes are vesicles comprised of one or more concentrically ordered lipid bilayers which encapsulate an aqueous phase. They are normally not leaky, but can become leaky if a hole or pore occurs in the membrane, if the membrane is dissolved or degrades, or if the membrane temperature is increased to the phase transition temperature. Current methods of drug delivery via liposomes require that the liposome carrier ultimately become permeable and release the encapsulated drug at the target site. This can be accomplished, for example, in a passive manner wherein the liposome bilayer degrades over time through the action of various agents in the body. Every liposome composition will have a characteristic half-life in the circulation or at other sites in the body and, thus, by controlling the half-life of the liposome composition, the rate at which the bilayer degrades can be somewhat regulated.
221. In contrast to passive drug release, active drug release involves using an agent to induce a permeability change in the liposome vesicle. Liposome membranes can be constructed so that they become destabilized when the environment becomes acidic near the liposome membrane (see, e.g., Proc. Natl. Acad. Sci. USA 84:7851 (1987); Biochemistry 28:908 (1989), which is hereby incorporated by reference in its entirety). When liposomes are endocytosed by a target cell, for example, they can be routed to acidic endosomes which will destabilize the liposome and result in drug release.
222. Alternatively, the liposome membrane can be chemically modified such that an enzyme is placed as a coating on the membrane which slowly destabilizes the liposome. Since control of drug release depends on the concentration of enzyme initially placed in the membrane, there is no real effective way to modulate or alter drug release to achieve "on demand" drug delivery. The same problem exists for pH-sensitive liposomes in that as soon as the liposome vesicle comes into contact with a target cell, it will be engulfed and a drop in pH will lead to drug release. This liposome delivery system can also be made to target B cells by incorporating into the liposome structure a ligand having an affinity for B cell-specific receptors.
223. Compositions including the liposomes in a pharmaceutically acceptable carrier are also contemplated.
224. Transdermal delivery devices have been employed for delivery of low molecular weight proteins by using lipid-based compositions (i.e., in the form of a patch) in combination with sonophoresis. However, as reported in U.S. Patent No. 6,041,253 to Ellinwood, Jr. et al., which is hereby incorporated by reference in its entirety, transdermal delivery can be further enhanced by the application of an electric field, for example, by ionophoresis or electroporation. Using low frequency ultrasound which induces cavitation of the lipid layers of the stratum corneum, higher transdermal fluxes, rapid control of transdermal fluxes, and drug delivery at lower ultrasound intensities can be achieved. Still further enhancement can be obtained using a combination of chemical enhancers and/or magnetic field along with the electric field and ultrasound.
225. Implantable or injectable protein depot compositions can also be employed, providing long-term delivery of, e.g., the first and second chimeric proteins. For example, U.S. Patent No. 6,331,311 to Brodbeck, which is hereby incorporated by reference in its entirety, reports an injectable depot gel composition which includes a biocompatible polymer, a solvent that dissolves the polymer and forms a viscous gel, and an emulsifying agent in the form of a dispersed droplet phase in the viscous gel. Upon injection, such a gel composition can provide a relatively continuous rate of dispersion of the agent to be delivered, thereby avoiding an initial burst of the agent to be delivered.
226. Yet another approach for targeting B cells with the chimeric protein or the composition of the present invention is to remove B cells from a subject and then expose the B cells to the chimeric protein or composition under conditions effective to cause B cells to transduce the chimeric protein. Thereafter, the transduced B cells can be returned or administered to the subject in need thereof.
227. Either administration of the chimeric protein or administration of *in vitro* transduced B cells can be utilized to correct a condition associated with improper AID function in B cells, affording a patient with sufficient B cell titers to treat CSR, SHM, or B cell lymphoma in accordance with the presently claimed invention.
228. Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow.

### a) Pharmaceutically Acceptable Carriers

229. By "delivery of the chimeric protein into a cell" is meant contacting the cell with the chimeric protein under conditions effective for cellular uptake of the chimeric protein. Such delivery occurs in the absence of genetically modifying the cell. Thus, administration of the chimeric protein of the invention provides a transient, dose-dependent delivery of the deaminase, thereby avoiding promiscuous editing and minimizing other potential undesirable side affects resulting from sustained enhanced RNA editing or DNA mutating activity. This provides a significant advantage over gene therapy as the delivery can be controlled in a dose-dependent fashion, is adaptable to variations in the subject's needs, protein administration is reversible, and is generally more acceptable to a subject.
230. Disclosed is a composition comprising the chimeric protein and a pharmaceutical carrier. Such compositions can be used therapeutically in combination with a pharmaceutically acceptable carrier.
231. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.
232. Pharmaceutical compositions may include carriers, thickeners, diluents, buffers, preservatives, surface active agents and the like in addition to the molecule of choice. Pharmaceutical compositions may also include one or more active ingredients such as antimicrobial agents, anti-inflammatory agents, anesthetics, and the like.
233. The pharmaceutical composition may be administered in a number of ways depending on whether local or systemic treatment is desired, and on the area to be treated. Administration may be topically (including opthamalically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.
234. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.
235. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.
236. Compositions for oral administration include powders or granules, suspensions or solutions in water or non-aqueous media, capsules, sachets, or tablets. Thickeners, flavorings, diluents, emulsifiers, dispersing aids or binders may be desirable.
237. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

238. The dosage ranges for the administration of the compositions are those large enough to produce the desired effect in which the symptoms disorder are affected. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any contraindications. Dosage can vary, and can be administered in one or more close administrations daily, for one or several days. While individual needs vary, determination of optimal ranges of effective amounts of each of the first and second chimeric proteins is within the skill of the art. Typical dosages comprise about 0.01 to about 100 mg/kg·body wt. The preferred dosages comprise about 0.1 to about 100 mg/kg·body wt. The most preferred dosages comprise about 1 to about 100 mg/kg·body wt.
239. Other chimeric proteins or mimetics which do not have a specific pharmaceutical function, but which may be used for tracking changes within cellular chromosomes or for the delivery of diagnostic tools for example can be delivered in ways similar to those described for the pharmaceutical products.
240. The chimeric proteins can also be used for example as tools to isolate and test new drug candidates for a variety of diseases.

### 10. Chips and micro arrays

241. Disclosed are chips where at least one address is the sequences or part of the sequences set forth in any of the nucleic acid sequences disclosed herein. Also disclosed are chips where at least one address is the sequences or portion of sequences set forth in any of the peptide sequences disclosed herein.
242. Also disclosed are chips where at least one address is a variant of the sequences or part of the sequences set forth in any of the nucleic acid sequences disclosed herein. Also disclosed are chips where at least one address is a variant of the sequences or portion of sequences set forth in any of the peptide sequences disclosed herein.

### 11. Computer readable media

243. It is understood that the disclosed nucleic acids and proteins can be represented as a sequence consisting of the nucleotides or amino acids. There are a variety of ways to display these sequences, for example the nucleotide guanosine can be represented by G or g. Likewise the amino acid valine can be represented by Val or V. Those of skill in the art understand how to display and express any nucleic acid or protein sequence in any of the variety of ways that exist, each of which is considered herein disclosed. Specifically contemplated herein is the display of these sequences on computer readable mediums, such as, commercially available floppy disks, tapes, chips, hard drives, compact disks, and video disks, or other computer readable mediums. Also disclosed are the binary code representations of the disclosed sequences. Those of skill in the art understand what computer readable mediums. Thus, computer readable mediums on which the nucleic acids or protein sequences are recorded, stored, or saved.
244. Disclosed are computer readable mediums comprising the sequences and information regarding the sequences set forth herein.

### 12. Kits

245. Disclosed herein are kits that are drawn to reagents (e.g., chimeric proteins or mimetics) that can be used in practicing the methods disclosed herein. The kits can include any reagent or combination of reagent discussed herein or that would be understood to be required or beneficial in the practice of the disclosed methods.

### 13. Compositions with similar functions

246. It is understood that the compositions disclosed herein have certain functions, for example, RNA editing and/or DNA mutation (editing), blocking Vif binding of endogenous CEM15, or binding Vif. In the case of AID, the function of the composition includes deaminating cytidine to form uridine in an mRNA molecule or deaminating deoxycytidine to form deoxyuridine in a DNA molecule, inducing immunoglobulin production, inducing CSR and/or SHM, inducing an immune response, treating hyper-IgM syndrome, and treating B-lymphocyte lymphoma. Disclosed herein are certain structural requirements for performing the disclosed functions, and it is understood that there are a variety of structures which can perform the same function which are related to the disclosed structures, and that these structures will ultimately achieve the same result, for example, inhibition of the Vif-CEM15 interaction, or one of the above named AID functions, or any ARP function, as previously described.

### G. Methods of using the compositions

247. Disclosed are methods for reducing interactions between CEM15 and Vif comprising incubating an inhibitor of the interaction between CEM15 and Vif. Also disclosed are methods for inhibiting HIV infectivity comprising administering an inhibitor of the interaction between CEM15 and Vif.
248. Disclosed are methods of treating a subject comprising administering to the subject an inhibitor of viral infectivity (e.g., HIV infectivity), wherein the inhibitor reduces the interaction between a deaminase (e.g., CEM15) and a viral infectivity factor (Vif), and wherein the subject is in need of such treatment. Disclosed are methods of identifying an inhibitor of an interaction between the deaminase and the viral infectivity factor, Vif comprising incubating a library of molecules with the deaminase to form a mixture, and identifying the molecules that disrupt the interaction between the deaminase and the viral infectivity factor. An isolating step can comprise incubating the mixture with molecule comprising Vif or a fragment or derivative thereof.
249. Disclosed are methods of identifying an inhibitor of an interaction between a deaminase and a viral infectivity factor (e.g., CEM15 and Vif, respectively) comprising incubating a library of molecules with the viral infectivity factor to form a mixture, and identifying the molecules that disrupt the interaction between the deaminase and the viral infectivity factor. The interaction disrupted can comprise an interaction between the viral infectivity factor and an amino acid of deaminase. An isolation step can comprises incubating the mixture with a molecule comprising a CEM15 or fragment or derivative thereof.
250. By "interrupting viral infectivity" is meant stopping or reducing the production of infective viral genomes. HIV infectivity, for example, is known to depend on a variety of proteins leading to the synthesis of double stranded DNA from single stranded HIV RNA genome and the integration of HIV DNA into the host cell's chromosomal DNA from where it is expressed to form viral genomes and viral proteins necessary for virion production. Viral infectivity factor or Vif, is a viral protein that enters the host cell as part of the infectious virion and assists the virus in establishing itself as an integrated DNA sequence. Recently, the requirement for Vif has been proposed to be its ability to bind to a cellular protein, CEM15, and inactivate cellular processes that would otherwise reduce viral infectivity (Sheehy, A.M., (2002) Nature 418:656-650). As an example, TAT-CEM15 mimetic peptide delivery into cells provides excess CEM15 interaction sites for Vif to bind to, beyond the capacity of virion Vif to adsorb, thus effectively freeing the cellular CEM15 deaminase from inhibition and enabling it to act on (mutate) HIV-1 to suppress its infectivity.
251. Disclosed are methods of interrupting viral infectivity (e.g., retroviral infectivity like HIV infectivity) comprising contacting an infected cell or a cell prior to infection with the chimeric protein comprising a protein transduction domain and a deaminase domain, under conditions that allow delivery of the chimeric protein into the cell, wherein the chimeric protein binds with a viral infectivity factor (Vif) to interrupt viral infectivity. Interruption of viral infectivity may occur at the different level, including, for example, at the level of RNA on the incoming virus, on first or second strand cDNA, after dsDNA integration and/or on transcripts from the viral integrin.
252. Disclosed are methods of treating a subject with a viral infection (e.g., HIV infection) or at risk for an infection comprising administering to the subject an effective amount of a chimeric protein comprising a protein transduction domain and a deaminase domain. Preferably, the administration step is dose-dependent and transient. As used throughout, administration of a protein or agent described herein can be combined with various others therapies. For example, a subject with HIV may be treated concomitantly with protease inhibitors and other agents.
253. Also disclosed are methods that include mixing a pharmaceutical carrier with the inhibitor as disclosed herein and produced by any of the disclosed methods.
254. Disclosed are methods of inhibiting infectivity (e.g., HIV infectivity) comprising administering an agent that prevents or reduces infectivity, wherein the system supports infectivity via a deaminase interaction; assaying the effect of the agent on the amount of infectivity in the system; and selecting an agent that causes a decrease in the amount of infectivity present in the system because of an inhibition of the deaminase interaction relative to the system without the addition of the composition.
255. Also disclosed are methods of inhibiting HIV infectivity comprising administering a composition that reduces an interaction between CEM 15 and Vif.
256. Also disclosed are methods of inducing production of immunoglobulins of the various classes and their subtypes comprising contacting a B lymphoblast with a chimeric AID protein, under conditions effective to cause cellular uptake of the chimeric protein, and thereby induce antibody production in the B lymphoblast. The B lymphoblast can be *in vitro* or *in vivo.* Antibody production can include IgG, IgE, or IgA production.
257. Also disclosed are methods of inducing class switch recombination in a B lymphocyte cell comprising contacting a B lymphocyte cell with a chimeric AID protein, under conditions effective to cause cellular uptake of the chimeric protein, and thereby induce class switch recombination during antibody production in the B lymphocyte cell. The B lymphoblast can be *in vitro* or *in vivo.* The B lymphocyte cell, prior to contacting, can exhibit normal or deficient levels of CSR during antibody production.
258. Also disclosed are methods of inducing somatic hypermutation in a B lymphocyte cell comprising contacting a B lymphocyte cell with a chimeric AID protein, under conditions effective to cause cellular uptake of the chimeric protein, and thereby induce somatic hypermutation during antibody production in the B lymphocyte cell. The contacting step can be *in vitro* or *in vivo.* The B lymphocyte cell, prior to contacting, can exhibit normal or deficient levels of SHM during antibody production.
259. Also disclosed are methods of inducing an immune response to an antigen in a subject comprising contacting a B lymphocyte cell with a chimeric protein under conditions effective to cause cellular uptake of the chimeric protein, and thereby induce antibody production in the B lymphocyte cell to afford a stronger immune response to an antigen in the subject. The B lymphoblast can be *in vitro* or *in vivo.* Antibody production can include IgG, IgE, or IgA production. In one example, the contacting is carried out *in vitro,* and the method further comprises introducing a B lymphocyte cell into a subject. Such methods are useful when employed concomitantly with vaccines.
260. Disclosed are methods of treating a subject for hyper-IgM syndrome comprising administering to a subject with hyper-IgM syndrome an effective amount of a chimeric protein, wherein the chimeric protein is taken up by B lymphocyte cell and induces antibody production sufficient to treat the hyper-IgM syndrome. Antibody production can include IgG, IgE, or IgA production.
261. Also disclosed are methods of treating a subject for hyper-IgM syndrome comprising administering to a subject with hyper-IgM syndrome a population of B lymphocyte cells, wherein the B lymphocyte is contacted with a therapeutic amount of the chimeric protein of the invention, wherein the administered B lymphocyte cells exhibit antibody production sufficient to treat the hyper-IgM syndrome. Antibody production can include IgG, IgE, or IgA production.
262. Disclosed are methods for treating a subject for B cell lymphoma comprising administering to a subject exhibiting B lymphocyte cell lymphoma an effective amount of a chimeric protein, wherein the chimeric protein is taken up by cancerous B lymphocyte cells, and inhibits or blunts cell growth thereof, thereby treating the lymphoma.
263. By "an agent that enhances the efficiency of editing" is meant a genetic, pharmacologic, or metabolic agent or condition that increases the RNA or DNA editing or mutating function of the chimeric protein, as compared to the amount of editing that occurs in the absence of the agent. Some of the conditions and agents that modulate editing activity include: (i) changes in the diet, (ii) hormonal changes (e.g., levels of insulin or thyroid hormone), (iv) osmolarity (e.g., hyper or hypo osmolarity), (v) ethanol, (vi) inhibitors of RNA or protein synthesis and (vii) conditions that promote liver proliferation. Thus, the methods of the invention can further comprise administering to the subject an agent that enhances the efficiency of mRNA editing function of the chimeric protein.
264. Also disclosed are methods of treating a subject for neoplasia, comprising administering to a subject exhibiting neoplasia an effective amount of an inhibitor of a cytidine deaminase, wherein the inhibitor reduces neoplasia. In one example, the cytidine deaminase can be AID, CEM15, or APOBEC-1.
265. Disclosed are methods of treating a condition in a subject comprising administering to the subject a chimeric protein comprising a protein transduction domain and a deaminase domain. It is understood that the effect of the administration of the composition to the subject can have the effect of but is not limited to reducing the symptoms of the condition, a reduction in the severity of the condition, or the complete ablation of the condition.
266. Also disclosed are methods of treating a condition, wherein the condition is a cancer. The cancer can be selected from the group consisting of lymphomas (Hodgkins and non-Hodgkins), B cell lymphoma, T cell lymphoma, myeloid leukemia, leukemias, mycosis fungoides, carcinomas, carcinomas of solid tissues, squamous cell carcinomas, adenocarcinomas, sarcomas, gliomas, blastomas, neuroblastomas, plasmacytomas, histiocytomas, melanomas, adenomas, hypoxic tumours, myelomas, AIDS-related lymphomas or sarcomas, metastatic cancers, bladder cancer, brain cancer, nervous system cancer, squamous cell carcinoma of head and neck, neuroblastoma/glioblastoma, ovarian cancer, skin cancer, liver cancer, melanoma, squamous cell carcinomas of the mouth, throat, larynx, and lung, colon cancer, cervical cancer, cervical carcinoma, breast cancer, epithelial cancer, renal cancer, genitourinary cancer, pulmonary cancer, esophageal carcinoma, head and neck carcinoma, hematopoietic cancers, testicular cancer, colo-rectal cancers, prostatic cancer, or pancreatic cancer.
267. Also disclosed are methods, wherein the condition is a, infectious disease (e.g., a viral disease). Also disclosed are methods, wherein the viral infection can be selected from the list of viruses consisting of Herpes simplex virus type-1, Herpes simplex virus type-2, Cytomegalovirus, Epstein-Barr virus, Varicella-zoster virus, Human herpesvirus 6, Human herpesvirus 7, Human herpesvirus 8, Variola virus, Vesicular stomatitis virus, Hepatitis A virus, Hepatitis B virus, Hepatitis C virus, Hepatitis D virus, Hepatitis E virus, Rhinovirus, Coronavirus, Influenza virus A, Influenza virus B, Measles virus, Polyomavirus, Human Papilomavirus, Respiratory syncytial virus, Adenovirus, Coxsackie virus, Dengue virus, Mumps virus, Poliovirus, Rabies virus, Rous sarcoma virus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Simian Immunodeficiency cirus, Human T-cell Leukemia virus type-1, Hantavirus, Rubella virus, Simian Immunodeficiency virus, Human Immunodeficiency virus type-1, and Human Immunodeficiency virus type-2.
268. Also disclosed are methods, wherein the disease is a bacterial infection. The bacterial infection can include *M tuberculosis, M. bovis, M. bovis* strain BCG, BCG substrains, *M. avium, M intracellulare, M africanum, M. kansasii, M marinum, M. ulcerans, M. avium* subspecies *paratuberculosis, Nocardia asteroides*, other *Nocardia* species, *Legionella pneumophila*, other *Legionella* species, *Salmonella typhi*, other *Salmonella* species, *Shigella* species, *Yersinia pestis, Pasteurella haemolytica, Pasteurella multocida*, other *Pasteurella species, Actinobacillus pleuropneumoniae, Listeria monocytogenes, Listeria ivanovii, Brucella abortus*, other *Brucella* species, *Cowdria ruminantium, Chlamydia pneumoniae, Chlamydia trachomatis, Chlamydia psittaci, Coxiella burnetti,* other *Rickettsial* species, *Ehrlichia* species, *Staphylococcus aureus, Staphylococcus epidermidis, Streptococcus pyogenes, Streptococcus agalactiae, Bacillus anthracis, Escherichia coli, Vibrio cholerae, Campylobacter* species, *Neiserria meningitidis, Neiserria gonorrhea, Pseudomonas aeruginosa,* other *Pseudomonas* species, *Haemophilus influenzae, Haemophilus ducreyi,* other *Hemophilus species, Clostridium tetani,* other *Clostridium* species, *Yersinia enterolitica, and* other *Yersinia species*.
269. Also disclosed are methods, wherein the disease to be treated is a parasitic infection. The parasitic infection can include *Toxoplasma gondii, Plasmodium falciparum, Plasmodium vivax, Plasmodium malariae,* other *Plasmodium* species., *Trypanosoma brucei, Trypanosoma cruzi, Leishmania major,* other *Leishmania* species., *Schistosoma mansoni,* other *Schistosoma* species., and *Entamoeba histolytica.*
270. Also disclosed are methods, wherein the disease is a fungal infection. The fungal infection can include *Candida albicans, Cryptococcus neoformans, Histoplama capsulatum, Aspergillus fumigatus, Coccidiodes immitis, Paracoccidiodes brasiliensis, Blastomyces dermitidis, Pneomocystis carnii, Penicillium marneffi, and Alternaria alternata*.

### 1. Methods of using the compositions as research tools

271. The disclosed compositions can be used in a variety of ways as research tools. For example, the disclosed compositions, such as the TAT-CEM15, or the TAT-AID chimeric protein, can be used to study the interactions between Vif and CEM15 in virions or T-cells, or AID and B-cells, respectively, by, for example, acting as inhibitors of binding or enhancers of production, respectively.
272. The compositions can be used for example as targets in combinatorial chemistry protocols or other screening protocols to isolate molecules that possess desired functional properties related to inhibition of the CEM15-Vif interaction.
273. The compositions can also be used for example as targets in combinatorial chemistry protocols or other screening protocols to isolate molecules that possess desired functional properties related to AID.
274. The disclosed compositions can also be used diagnostic tools related to diseases that are related to RNA or DNA editing, such as HIV, B-cell lymphoma, CSR or SHM disorders.
275. The disclosed compositions can be used as discussed herein as either reagents in microarrays or as reagents to probe or analyze existing microarrays. The disclosed compositions can be used in any known method for isolating or identifying single nucleotide polymorphisms. The compositions can also be used in any method for determining allelic analysis. The compositions can also be used in any known method of screening assays, related to chip/micro arrays. The compositions can also be used in any known way of using the computer readable embodiments of the disclosed compositions, for example, to study relatedness or to perform molecular modeling analysis related to the disclosed compositions.
276. Disclosed are methods of screening for a viral RNA deaminase mimetic comprising adding the agent to be screened to a virally infected mammalian system and detecting levels of edited viral RNA and/or mutated (edited) viral DNA, elevated levels of edited viral RNA or mutated (edited) viral DNA indicating a viral RNA deaminase mimetic or a viral DNA deaminase mimetic. Optionally, the method can further comprise detecting binding of the agent to be screened to a viral integration factor.
277. Also disclosed are methods of screening for cellular RNA and DNA deaminases comprising adding the agent to be screened to a virally infected mammalian system; and detecting levels of edited cellular RNA and/or mutated (edited) cellular DNA, elevated levels of edited cellular RNA or mutated (edited) cellular DNA indicating a cellular RNA or DNA deaminase mimetic.
278. Disclosed are methods of identifying inhibitors of deaminase interactions, such as CEM15-Vif interactions, or AID-B cell interaction, comprising, (a) administering a composition to a system, wherein the system supports the interaction, (b) assaying the effect of the composition on the amount of the interacting complex (e.g., CEM15-Vif or AID-B-cell) in the system, and (c) selecting a agent that causes a decrease in the amount of interacting complex present in the system relative to the system without the addition of the composition.
279. Also disclosed are methods of identifying inhibitors of viral infectivity (e.g., HIV infectivity) comprising, (a) administering an agent to a system, wherein the system supports infectivity via a deaminase interaction (e.g., CEM15-Vif), (b) assaying the effect of the agent on the amount of infectivity in the system, and (c) selecting an agent that causes a decrease in the amount of infectivity present in the system because of an inhibition of the interaction relative to the system without the addition of the agent.
280. Disclosed are methods of identifying an inhibitor of an interaction between CEM15 and Vif comprising (a) administering a composition to a system, wherein the system comprises CEM15, (b) assaying the effect of the composition on a CEM15-Vif interaction, and (c) selecting a composition which inhibits a CEM15-Vif interaction.
281. Also disclosed are methods of screening for inhibitors of AID, comprising adding the agent to be screened to cells expressing AID; and detecting levels of AID and/or RNA or DNA mutation rates and/or antibody production rates; reduced levels of AID and/or RNA or DNA mutation rates and/or antibody production rates indicating an AID inhibitor.
282. The virus can be a retrovirus (e.g., HIV). The virus can be an RNA virus. Also disclosed are methods, wherein the RNA virus can be selected from the list of viruses consisting of Vesicular stomatitis virus, Hepatitis A virus, Hepatitis C virus, Rhinovirus, Coronavirus, Influenza virus A, Influenza virus B, Measles virus, Respiratory syncytial virus, Adenovirus, Coxsackie virus, Dengue virus, Mumps virus, Poliovirus, Rabies virus, Rous sarcoma virus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Hantavirus, and Rubella virus.
283. Also disclosed are methods, wherein the viral RNA deaminase mimetic is a CEM15 mimetic.
284. Disclosed are methods of screening for a viral DNA deaminase mimetic comprising adding the agent to be screened to a virally infected mammalian system; and detecting levels of edited viral DNA, elevated levels of edited viral RNA indicating a viral RNA deaminase mimetic. Optionally, the method can further comprise detecting binding of the agent to be screened to a viral integration factor.
285. Also disclosed are methods, wherein the viral DNA deaminase mimetic is a CEM15 mimetic. Also disclosed are methods, wherein the virus is a DNA virus. The DNA virus can be selected from the list of viruses consisting of Herpes simplex virus type-1, Herpes simplex virus type-2, Cytomegalovirus, Epstein-Barr virus, Varicella-zoster virus, Human herpesvirus 6, Human herpesvirus 7, Human herpesvirus 8, Variola virus, Hepatitis B virus, Hepatitis D virus, Polyomavirus, and Human Papilomavirus.
286. Also disclosed are methods of screening for AID mimetics, antagonists, or agonists, comprising adding the agent to be screened to a solution comprising B-cells; and detecting levels of edited cellular RNA and/or mutated (edited) cellular DNA, elevated levels of edited cellular RNA or mutated (edited) cellular DNA indicating a cellular RNA or DNA deaminase mimetic.
287. The present invention also discloses methods of using computer readable media to analyze a comparison sequence.

### H. Methods of making the compositions

288. The compositions disclosed herein and the compositions necessary to perform the disclosed methods can be made using any method known to those of skill in the art for that particular reagent or compound unless otherwise specifically noted.
289. Disclosed are methods of manufacturing a composition for inhibiting the interaction between a deaminase (e.g., CEM15) and a viral infectivity factor (Vif) comprising synthesizing the inhibitors as disclosed herein.
290. Disclosed are methods of making a composition capable of inhibiting infectivity (e.g., HIV infectivity) comprising admixing a compound with a pharmaceutically acceptable carrier, wherein the compound is identified by administering the compound to a system, wherein the system supports infectivity via a deaminase interaction, assaying the effect of the compound on the amount of infectivity in the system, and selecting a compound which causes a decrease in the amount of infectivity in the system because of an inhibition of the deaminase interaction, relative to the system without the addition of the compound.
291. Disclosed are methods of manufacturing an inhibitor to viral budding comprising (a) administering a composition to a system, wherein the system supports viral infectivity via a deaminase interaction, (b) assaying the effect of the composition on the amount of infectivity in the system, (c) selecting a composition which cause a decrease in the amount of infectivity present in the system because of an inhibition of the deaminase interaction, relative to the system with the addition of the composition, and (d) synthesizing the composition. Also disclosed are methods further comprising the step of admixing the composition with a pharmaceutical carrier.

### 1. Nucleic acid synthesis

292. For example, the nucleic acids, such as, the oligonucleotides to be used as primers can be made using standard chemical synthesis methods or can be produced using enzymatic methods or any other known method. Such methods can range from standard enzymatic digestion followed by nucleotide fragment isolation (see for example, Sambrook Molecular Cloning: A Laboratory Manual, 2nd Edition (Cold Spring Harbor Laboratory Press, Cold Spring Harbor, N.Y., 1989, Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, MA or ABI Model 380B). Synthetic methods useful for making oligonucleotides are also described by Ikuta, Ann. Rev. Biochem. 53:323-356 (1984), (phosphotriester and phosphite-triester methods), and Narang, Methods Enzymol., 65:610-620 (1980), (phosphotriester method). Protein nucleic acid molecules can be made using known methods such as those described by Nielsen, Bioconjug. Chem. 5:3-7 (1994).

### 2. Peptide synthesis

293. One method of producing the disclosed proteins, such as combinations of SEQ ID NOs: 1 and 43, is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (t*ert* -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY (which is herein incorporated by reference at least for material related to peptide synthesis). Alternatively, the peptide or polypeptide is independently synthesized *in vivo* as described herein. Once isolated, these independent peptides or polypeptides may be linked to form a peptide or fragment thereof via similar peptide condensation reactions.
294. For example, enzymatic ligation of cloned or synthetic peptide segments allow relatively short peptide fragments to be joined to produce larger peptide fragments, polypeptides or whole protein domains (Abrahmsen L, Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson, Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M (1992) FEBS Lett. 307:97-101; Clark-Lewis I, J.Biol.Chem., 269:16075 (1994); Clark-Lewis I., Biochemistry, 30:3128 (1991); Rajarathnam K., Biochemistry 33:6623-30 (1994)).
295. Alternatively, unprotected peptide segments are chemically linked where the bond formed between the peptide segments as a result of the chemical ligation is an unnatural (non-peptide) bond (Schnolzer, M Science, 256:221 (1992)). This technique has been used to synthesize analogs of protein domains as well as large amounts of relatively pure proteins with full biological activity (deLisle Milton RC, Techniques in Protein Chemistry IV. Academic Press, New York, pp. 257-267 (1992)).

### 3. Processes of making the compositions

296. Disclosed are processes for making the compositions as well as making the intermediates leading to the compositions. For example, disclosed are nucleic acids in SEQ ID NOs: 2, 42, 44, and 47. A cDNA construct can be assembled that includes the sequences of SEQ ID NOs: 2 and 44, and, optionally, further includes the sequence of SEQ ID NO: 42. Such cDNA constructs can further include additional elements including, for example, a hemagglutin ("HA") domain. An exemplary HA domain is provided as SEQ ID NO: 46; and an exemplary nucleic acid that encodes it is provided as SEQ ID NO: 47. A cDNA construct can be assembled that includes the sequences of SEQ ID NOs: 2, 44, and, optionally, further includes the sequence of SEQ ID NO: 42 and/or 47, or SEQ ID NOS: 4 and/or 44 such a cDNA construct could also include a nucleic acid sequence that encodes a polyhistidine tag. There are a variety of methods that can be used for making these compositions, such as synthetic chemical methods and standard molecular biology methods. It is understood that the methods of making these and the other disclosed compositions are specifically disclosed.
297. Disclosed are nucleic acid molecules produced by the process comprising linking, in an operative way, a nucleic acid comprising the sequences set forth in SEQ ID NOs: 2 (or 4), 44, 47, and/or 42, and a sequence controlling the expression of the nucleic acid.
298. Also disclosed are nucleic acid molecules produced by the process comprising linking in an operative way a nucleic acid molecule comprising a sequence having 80% identity to a sequence comprising SEQ ID NOs: 2 (or 4), 44, 47, and/or 42, and a sequence controlling the expression of the nucleic acid.
299. Disclosed are nucleic acid molecules produced by the process comprising linking in an operative way a nucleic acid molecule comprising a sequence that hybridizes under stringent hybridization conditions to a sequence that comprises SEQ ID NOs: 2 (or 4), 44, 47, and/or 42 and a sequence controlling the expression of the nucleic acid.
300. Disclosed are nucleic acid molecules produced by the process comprising linking in an operative way a nucleic acid molecule comprising a sequence encoding a combination of peptides set forth in SEQ ID NOs: 2 and 44, in the presence or absence a sequence encoding a peptide of SEQ ID NO: 42 and 47, and a sequence controlling an expression of the nucleic acid molecule.
301. Disclosed are nucleic acid molecules produced by the process comprising linking in an operative way a nucleic acid molecule comprising a sequence encoding a peptide having 80% identity to a peptide combinations set forth herein and a sequence controlling an expression of the nucleic acid molecule.
302. Disclosed are nucleic acids produced by the process comprising linking in an operative way a nucleic acid molecule comprising a sequence encoding a peptide having 80% identity to a peptide combination set forth herein, wherein any change from the provided peptide sequences are conservative changes, and a sequence controlling expression of the nucleic acid molecule.
303. Disclosed are cells produced by the process of transforming the cell with any of the disclosed nucleic acids. Disclosed are cells produced by the process of transforming the cell with any of the non-naturally occurring disclosed nucleic acids.
304. Disclosed are any of the disclosed peptides produced by the process of expressing any of the disclosed nucleic acids. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.
305. Throughout this application, various publications are referenced. The disclosures of these publications in their entireties are hereby incorporated by reference into this application in order to more fully describe the state of the art to which this invention pertains. The references disclosed are also individually and specifically incorporated by reference herein for the material contained in them that is discussed in the sentence in which the reference is relied upon.
306. It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.
307. The present invention may be understood more readily by reference to the following detailed description of preferred embodiments of the invention and the Examples included therein and to the Figures and their previous and following description.
308. Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### I. Examples

309. The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

### a) Methods for obtaining the CEM15 cDNA and for cloning it into two different systems

310. Human CEM15 (NP-068594; also known as MDS019, AAH24268) was amplified from total cellular RNA of the NALM-6 cell line (human B cell precursor leukemia) by RT-PCR.
311. Oligo-dT primed first-strand cDNA was amplified using Expand HiFi Taq DNA polymerase (Roche) with the following primers; '5'A' CACTTTAGGGAGGGCTGTCC (SEQ ID NO: 10) and '3'A' CTGTGATCAGCTGGAGATGG (SEQ ID NO: 11). The1366 bp product was reamplified with CEM15 specific PCR primers that included *Nco*I and *Xho*I restriction sites on the 5' and 3' primer respectively; '5'B' CTCCCATGGCAAAGCCTCACTTCAGAAACACAG (SEQ ID NO: 12) and '3'B' CTCCTCGAGGTTTTCCTGATTCTGGAGAATGGCCC (SEQ ID NO: 13).
312. The 1154bp PCR product was digested with *Eco*RI to remove potentially co-amplified highly homologous APOBEC3B/Phorbolin 3 (Q9UH17) sequences and the NcoI/XhoI digested product subcloned into a modified pET28a (Novagen) plasmid such that a CEM15-thrombin-HA-6His fusion protein could be expressed. The full-length human CEM15 cDNA was subcloned by PCR into a mammalian expression vector (pcDNA3) such that it is expressed with an amino terminal haemagglutinin (HA) epitope. It was also subcloned into pET28a (Novagen) to express a 6His-thrombin-CEM15 fusion protein.
313. The expression of the former clone in mammalian HepG2 cells (Human liver hepatoma line) demonstrate expression of full length protein (PAGE gel cell extracts were transferred to nitrocellulose and the presence of CEM15 was determined by reaction with anti-HA tag antibodies). This latter fusion was expressed to high levels in *E. coli* as a soluble protein and purified by nickel affinity chromatography (the expression and yield of CEM15 was determined by Coomassie blue stained PAGE gel and was approximately 700 µg per 50 mls of original E. coli culture, enough to crystallize or begin test on cells).

### 2. Example 2

### a) APOBEC-1 model.

314. The construction of the new APOBEC-1 model is based upon the hypothesis that enzymes with a common catalytic function (i.e. hydrolytic deamination of a nucleoside base) exhibit a common three-dimensional fold despite a low overall amino acid sequence identity (∼30%). This level of homology is often cited as the lower limit upon which one can reliably model the fold of a given polypeptide sequence (Burley, S.K. (2000) Nature Struct. Biol. 7:932-934.). At present, experimentally derived three-dimensional structures are available for three cytidine deaminases (CDAs) whose role in pyrimidine metabolism has been firmly established. These enzymes encompass the dimeric CDA from *E. coli* (Betts L, CW (1994) J Mol Biol. 235:635-56), the tetrameric CDA from B. subtilis (Johansson E., (2002). Biochem. 41:2563-70) and the tetrameric CDA Cdd1 from *S. cerevisiae*. The Cartesian coordinates for the former two models are available in the public Protein Data Bank (www.rcsb.org/pdb) as entries 1AF2 and 1JTK. Among the known CDA structures however, only Cdd1 exhibits RNA editing activity (Dance, G.S.C. (2001) Nuc. Acids Res. 29:1772-1780.) and therefore its coordinates have been critical in the assembly of a composite 3-D model for APOBEC-1. The latter model is a critical component in the development of a working model for RNA editing by APOBEC-1 and provides a tool to understand and manipulate its related proteins (ARPs) including AID, and CEM15.

### b) Methods for the Construction of a Structure-Based Sequence Alignment (SBSA) Leading to a New APOBEC-1 Three-dimensional Model.

### (1) Expression and Purification

315. Cdd1 was amplified by PCR from Baker's yeast. The product was cloned into a pET-28a vector (Novagen) containing N-terminal 6xHis using NdeI and EcoRI restriction sites; constructs were verified by DNA sequencing. BL21 CodonPlus (Stratagene) cells transformed with vector were grown at 37°C to an OD600 of 0.7 and induced with 1 mM IPTG at 30°C for 3 hours. Bacterial pellets were resuspended in lysis buffer (50 mM Tris-Cl pH 8.0, 10 mM β-mercaptoethanol, 1 mg/ml lysozyme, 1mM PMSF, 2 mM benzamidine and 5µg/ml each of aprotinin, leupeptin and pepstatin A), lysed, and nuclease digested (0.5% Triton X-100, 2 mM ATP, 10 mM MgSO₄, 33 µg/ml each of DNaseI and RNaseI) at 4°C. The 6xHis tagged protein was purified in batch with NiNTA agarose (Qiagen) utilizing the following wash, elution, and dialysis scheme: *wash 1*, 10 mM Tris-Cl pH 8.0, 100 mM KC1, 20 mM imidazole, 10% glycerol; *wash 2*, same as *wash 1* including 1 M KC1; *wash 3*, repeat *wash 1*; *elution*, 10 mM Tris-Cl pH 8.0, 0.5 M KC1, 0.4 M imidazole, 10% glycerol; dialyze against 2x 2 liters 10 mM Tris-C1 pH 8.0, 120 mM NaCl, 1 mM DTT). Removal of the 6xHis tag was achieved by digestion for 16 hours at 20°C with 10 U biotinylated thrombin (Pierce). Protein was dialyzed against 20 mM HEPPS pH 8.0, 0.25 M KC1, 5% glycerol, and 4 mM DTT and concentrated to 6 mg/ml as estimated by Bradford assays (BioRad) using an Ultrafree-4 spin cartridge (Millipore). Protein was utilized immediately for crystallization.

### (2) Crystallization

316. Crystals were grown at 20°C by use of the hanging drop vapor diffusion method (McPherson (1990) European J. Biochem 189, 1-23) from well solutions of 16.5% (w/v) PEG monomethylether (MME) 5K, 450 mM NH4C1, 100 mM Na-succinate pH 5.5, 10 mM DTT and 1 mM NaN₃. Four µl of well solution was added to an equal volume of protein. Crystals appeared in six days and reached a maximum size of 50 x 90 x 450 mm3 after 3-4 weeks. Single crystals were harvested with a nylon loop (Hampton Research), and cryo-protected through four serial transfers in 100 µl volumes of solutions containing 19% (*w*/*v*) PEG monomethylether 5000, 500 mM NH₄Cl, 100 mM Na-succinate pH 5.5, 1 mM DTT and either 5, 10, 15 or 17.5% (v/v) PEG 550 MME. Crystals were flash cooled by plunging into liquid nitrogen, and stored prior to X-ray data collection. In order to bind UMP, crystals were serially transferred in the presence of 10 mM UMP from pH 5.5 to 7.5 in 0.5 pH unit increments. Buffers of the appropriate pKa were chosen for each step. Crystals were subsequently cryo-adapted at elevated pH and flash frozen as described.

### (3) Structure Determination

317. Crystals of scCdd1 belong to space group C2221 with unit cell dimensions a = 78.51 Å, b = 86.32 Å and c = 156.14 Å. There is one 66 kDa tetramer (4x 145 amino acids) per asu. The structure was solved by use of MAD phasing (ref) at the Zn(II) K-absorption edge with the peak energy at 1.2828 Å, inflection = 1.28310 Å and remote energy 1.25740 Å. The positions of four zinc atoms were located by use of the software package SOLVE v2.0 (Terwilliger (2001) Acta Crystallogr. D. 57 1755-62), and phases were density modified by use of RESOLVE (Terwilliger, 2001) with 4-fold NCS averaging. The NCS averaged phases improved electron density maps significantly and allowed skeletonization by use of O (Jones et al. (1991) Acta Crystallogr. A 47 110-119). Additional NCS averaging with DM (Winn et al., (2002) Acta Crystallogr. D. 58 1929-36) improved the electron density map quality and allowed modeling of amino acids 4 to 136 in all four subunits. Upon addition of UMP, the C-terminal 6 aa's were observed in electron density maps. The present structure has been refined by use of the software package CNS (Brünger et al., 1998 Acta Crystallogr. D. 54, 095-921) using all data from 30 to 2.0 Å resolution with a crystallographic Rfactor of 23.2% (Rfree = 26.2%). The model exhibits reasonable bond and angle deviations from ideal values (0.009Å and 1.52o, respectively). More than 89% of residues are in the allowed region of the Ramachandran Plot as determined by the program PROCHECK (Laskowski et al. 1993, J. Applied Crystallogr. 26, 283-291). Coordinates and structure factor amplitudes will be deposited into the public Protein Data Bank (PDB) (www.rcsb.org/pdb).

### (4) Homology Modeling

318. The design of homology models for the ARP enzymes was based upon the observation that the enzyme Cdd1 from *Saccharomyces cerevisiae* is capable of acting on monomeric nucleoside substrates of pyrimidine metabolism, as well as larger RNA substrates such as reporter apoB mRBA expressed ectopically in yeast (Dance et al, 2001 Nucleic Acid Res. 29, 1772-1780). These results along with our X-ray crystallographic structure determination of yeast Cdd1 demonstrated that the fundamental CDA fold, typical of pyrimidine metabolism enzymes, may be sufficient for catalyzing C to U editing of RNA or dC to dU mutations on DNA. As such, the three known crystal structures of cytidine deaminases were utilized to prepare a template for homology modeling of APOBEC-1, CEM-15 and AID. The initial amino acid sequence alignment among enzymes of known structure with those of the unknown ARPs was prepared by use of the program ClustalX v1.8 (Thompson et al., 1997 Nucleic Acid Res. 24, 4876-4882). Sequences aligned included: #P19079 (B. subtilis), #NP_013346 (S. cerevisiae), #1065122 (E. coli), #4097988 (APOBEC-1 from H. sapiens), NP_065712(AID from H. sapiens) and #NP_068594 (APOBEC-3G from H. sapiens), which were retrieved from the NCBI (www.ncbi.nlm.nih.gov/Pubmed). Subsequently, manual adjustments were made to the alignments of the ARP primary sequences according to sequence constraints derived from the triple three-dimensional structural superposition of the known cytidine deaminase coordinates of yeast (i.e. scCDD1), E. coli (PDB accession number 1AF2) and B. subtilis PDB (PDB accession number 1JTK) described by Betts et al. (1994, J. Mol. Biol 235, 635-56) and Johansson et al. (2002 Biochemistry 41, 2563-70) as implemented in the program LSQKAB (Kabsch 1976 Acta Crystallogr. A 32, 922-923). When optimized to account for the conserved three-dimensional fold, the alignments between the enzymes of pyrimidine metabolism and the ARPs revealed equenece identity ranging from ∼7% to 26% in the respective catalytic and non-catalytic domains (Wedekind et al., 2003 Trends in Genetics, 19, 207-216). Despite the modest sequence identity at the amino acid level, the template appears to be accurate, because the actual three-dimensional structural homology of proteins with a common function often far exceeds the relatedeness values predicted by simple amino acid sequence alignments (Chothia & Lesk, 1986 EMBO J. 5, 823-826). In order to rigorously model the respective ARP structures with the highest degree of empirically derived structural restraints, method of comparative modeling was employed using "satisfaction of spatial restraints" as implemented in the program Modeller (Sali & Blundell 1993, J. Mol. Biol. 234, 779-815). Following model calculation, realistic model geometry is achieved through real-space optimization using enforced stereochemical refinement derived from application of the CHARM22 force field parameters (MacKerell et al., 1998 J. Phys. Chem. B. 102 3586-16). In all models, the Zn2+ ion was contrained in Modeller to be within 2.25 Å distance of each the respective putative metal ligands: 2x cyteine-S□ and 1x histidine-N□1 (as in Wedekind et al., 2003 Trends in Genetics 19, 207-216). This constraint resulted in a satisfactory and realistic tetrahedral geometry consistent with the known CDA structures, as well as the chemical requirements for base hydrolytic deamination. In order to model the location of DNA or RNA substrate binding, the edited nucleotide was modeled according to constraints derived from the known locations of CDA inhibitors in the template X-ray crystal structures: 1JKT (tetrahydrouridine ) and 1AF2 (3,4 dihydrouridine). Due to the known substrates of AID and APOBEC-1, DNA and RNA sequences were modeled as single-stranded. Additionally, the restraint that nucleotide bases flanking the edited/mutated sites maintain modest base stacking was imposed by adding additional distance restraints in the model calculation. Each monomer of a respective ARP model was also restrained to be symmetric. This method of modeling far exceeds previous standards employed to model APOBEC-1 (Navaratnam, N. et al. (1998) JMB 275:695-714.). The result of modeling is the existence of an extensive flexible linker that extends from residues 136 to 143 of human APOBEC-1 and residues 131-138 of human AID (See Figure 6b).

### (5) Mutagenesis and Construction of Chimeric Cdd1 Enzymes

319. In order to corroborate the comparative model of APOBEC-1, Cdd1 was employed as a model compound to examine: (i) the feasibility of the predicted APOBEC-1 fold, and (ii) the role of key functional elements predicted to be in the active site linker or other active site locations necessary for catalysis. (Note: mutations can be divided into two classes: those that stabilize/destabilize the structure through insertions or changes of large streches of amino acids; and those that effect function by modest changes to amino acids). A series of mutants were constructed in a manner analogous to the following method. In order to assess the importance of the predicted C-terminal "tail" ofCdd1 upon the ability to edit RNA, a 19 amino acid linker from *E. coli* was added after residue 142. Specifically, Cdd1 was PCR amplified using a 5' Cdd1-specific primer and a 3' primer encoding the 19 amino acid *E. coli* "linker" extension and subcloned into the NdeI and EcoRI sites of pET28a (Novagen). In order to assess the importance of linker flexibility Gly137 was converted to Ala using the QuikChange mutagenesis system (Stratagene) according to the manufacturer's protocols; other point mutations were constructed similarly. To assesses whether or not the CDA from *E. coli* (PDB #1AF2) was competent to edit under conditions similar to APOBEC-1 and Cdd1 in yeast (Dance et al., 2001 Nucleic Acid Res. 29, 1772-1780; Dance et al., 2000 Nucleic Acids Res. 28, 424-9), the *E. coli* CDA was PCR amplified from genomic DNA and subcloned for yeast expression as described below. In order to address the question of whether or not the proposed homology model for APOBEC-1 (above) was feasible in terms of the overall three-dimensional fold and catalytic activity, a series of Cdd1 chimeras were assembled by fusing together two Cdd1 polypeptide chains joined by a linker. The 5' monomers containing the appropriate C-terminal Apobec-1 or *E. coli* 19 amino acid linker were amplified and subcloned as described above. The amino terminally foreshortened C-terminal monomer (missing helix αl based upon homology modeling) was PCR amplified using the wild type or Glu63 to Ala Cdd1 template and ligated as an EcoRI/XhoI fragment to the appropriate 5' monomer in pET28a. The linking EcoRI site was mutagenized to restore the reading frame of the Cdd1 chimeras. All Cdd1 monomer and chimeric cDNAs were amplified using Cdd1 specific primers and subcloned via EcoRI and XbaI sites into a modified pYES2.0 vector to allow galactose regulated expression of an HA-epitope tagged protein in yeast for Western analysis. Cdd1 mutants and chimeric proteins were expressed and purified essentially as described above. The results of editing in the context of the yeast system established for APOBEC-1 and Cdd1 (Dance et al 2001 Nucleic Acid Res. 29, 1772-1780; Dance et al., 2000 Nucleic Acids Res. 28, 424-9) are summarized in Figure 11.
320. In the context of late log phase growth in yeast with galactose feeding, overexpressed Cdd1 is capable of C to U specific editing of reporter apoB mRNA at site C6666 at a level of 6.7%, which is ∼10x times greater than the negative control (empty vector - compare lanes 1 and 2, above). In contrast, the CDA from E. coli (equivalent to PDB entry 1AF2) is incapable of editing on the reporter substrate (lane 3). Similarly, the active site mutants E61A and G137A abolish detectable Cdd1 activity (lanes 4 and 5). Likewise, the addition of the E. coli linker sequence (lane 6) impairs editing function as well. In a series of chimeric constructs in which the Cdd1 tetramer was converted into a molecular dimer, the chimeric molecule appears functional, as long as an amino acid linker of 7-8 amino acids is used to join the respective Cdd1 subunits (See Right Panel lanes 1-4). However, when the longer E. coli linker is used to join Cdd1 monomers, there is no detectable activity on the reporter substrate, although the chimeric protein is expressed (See Western blot). Paradoxically, when conserved Gly residues of the APOBEC-1 linker (130 and 138) are mutated to Ala, the chimeric enzyme is still active (Lanes 3 and 4 of right panel). This suggests that these components are not an important part of the linker flexibility, or that the new chimera adopts a different fold in this region compared to that of the pyrimidine metabolism enzymes. Indeed, the ARP models suggest a restructuring of the active site linker that makes the entire region spanning from 130 to 142 (human APOBEC-1 numbering) flexible in a manner that moves to accommodate large polymeric substrates such as RNA or DNA (see Figure 12). Additional evidence of the importance of the linker sequence comes from mutagenesis on rat APOBEC-1 (highly homologous to human). When the 8 amino acid linker sequence of rat APOBEC-1 is replaced with the first 8 amino acids of the E. coli linker, the APOBEC-1 construct is unable to edit reporter apoB mRNA in the human hepatoma cell line HepG2.

### (6) Editing Activity

321. Editing activity for wild type and mutant constructs of scCdd1 were measured using the poisoned primer extension assay as described previously and subsequently.

### (7) Results

322. The hidden Markov modeling software SAM was trained with CDD1, APOBEC1, APOBEC2, AID and phorbolin 1. This identified APOBEC3A, 3B, 3C, 3E, 3F, 3G, XP_092919, PHB1, XP_115170/XP_062365.
323. PHI-BLAST, using the target pattern H[VA]-E-x-x-F-(x)19-[I/V]-[T/V]-[W/C]-x-x-S-W-[ST]-P-C=x-x-C limited the search more and misses only the 3B (Phorbolin 2) variant AAD00089 in which a single codon change GAC/T to GAA/G changes the ZDD center HxE to HxA. This is either a sequencing error or a significant SNP for psoriasis.
324. [HC]-x-E-x-x-F-x(19,30)-P-C-x(2,4)-C yields the usual suspects for human. There are a couple of novel deaminases with motif HPE....SPC......C. Also identified is a mouse gene homologous to hu APOBEC3G (CEM15). On Chro 15, position 15E2. This is highly homologous to APOBEC3B, D+E, G. There are 9 exons. Both ZDDs fall in their own exons. On the mouse gene, the start of the linker is an exon junction.
325. The multiple sequence alignment results are shown below in Table 4.

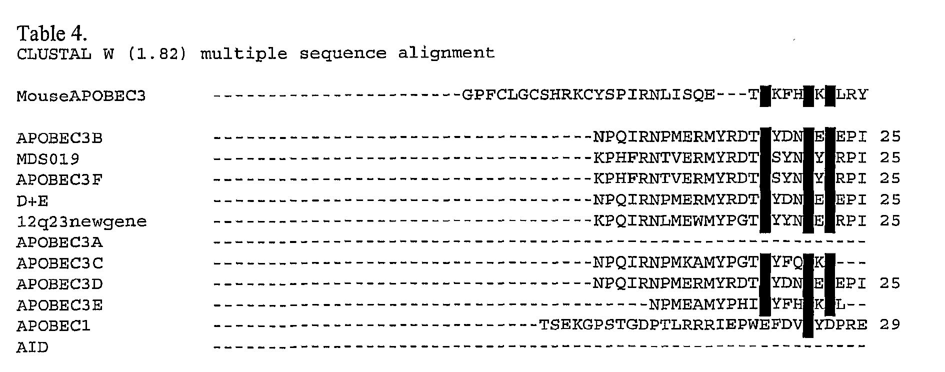

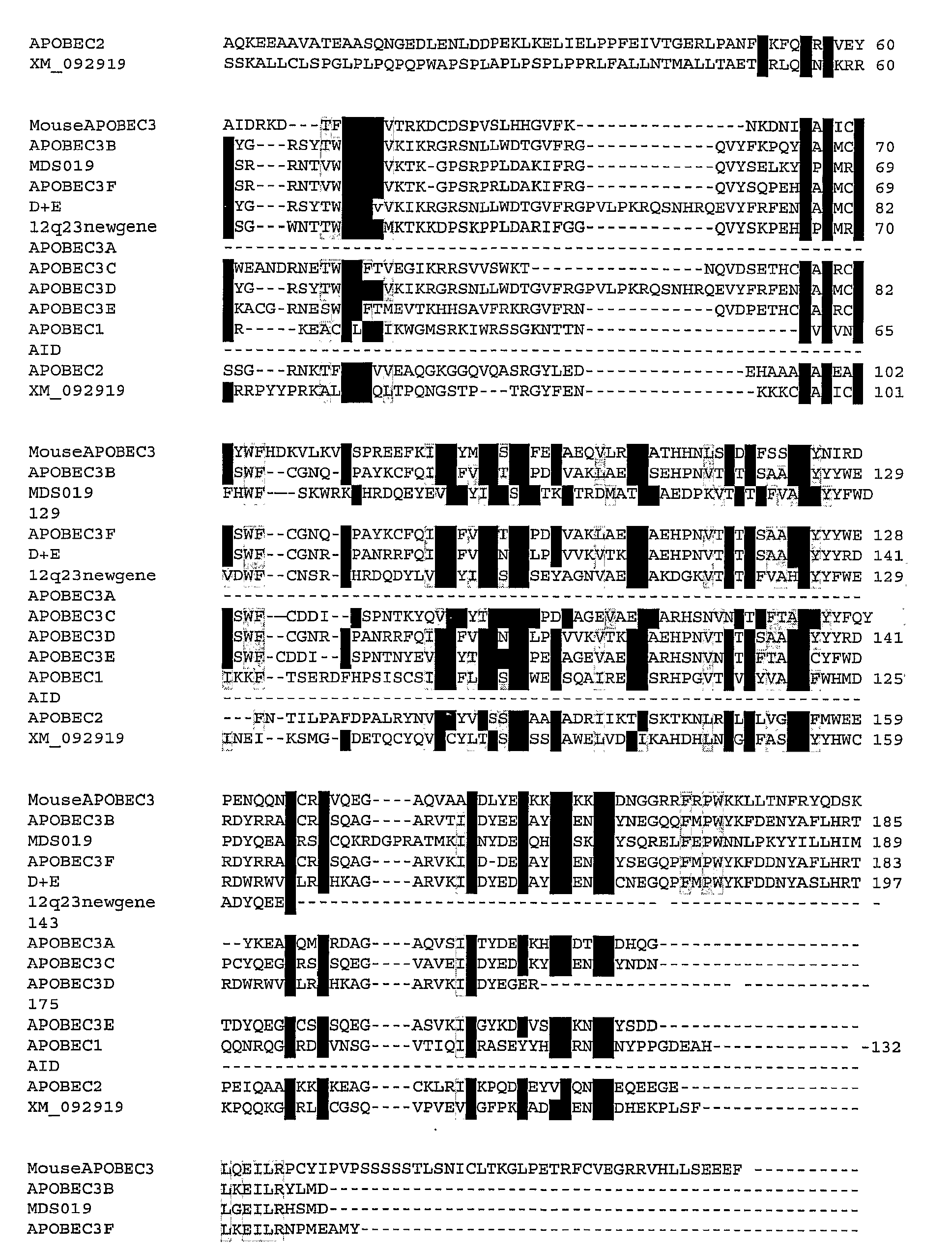

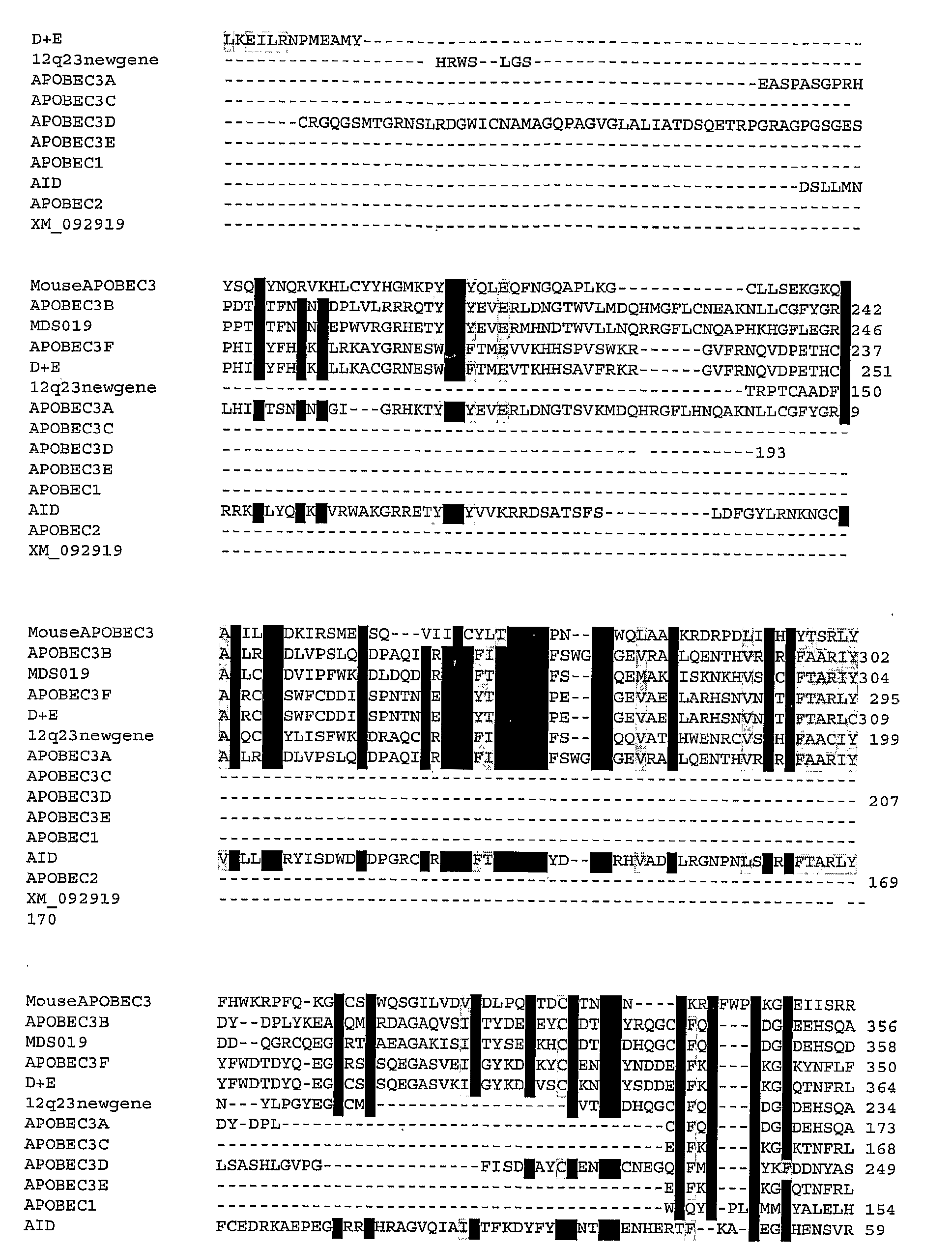

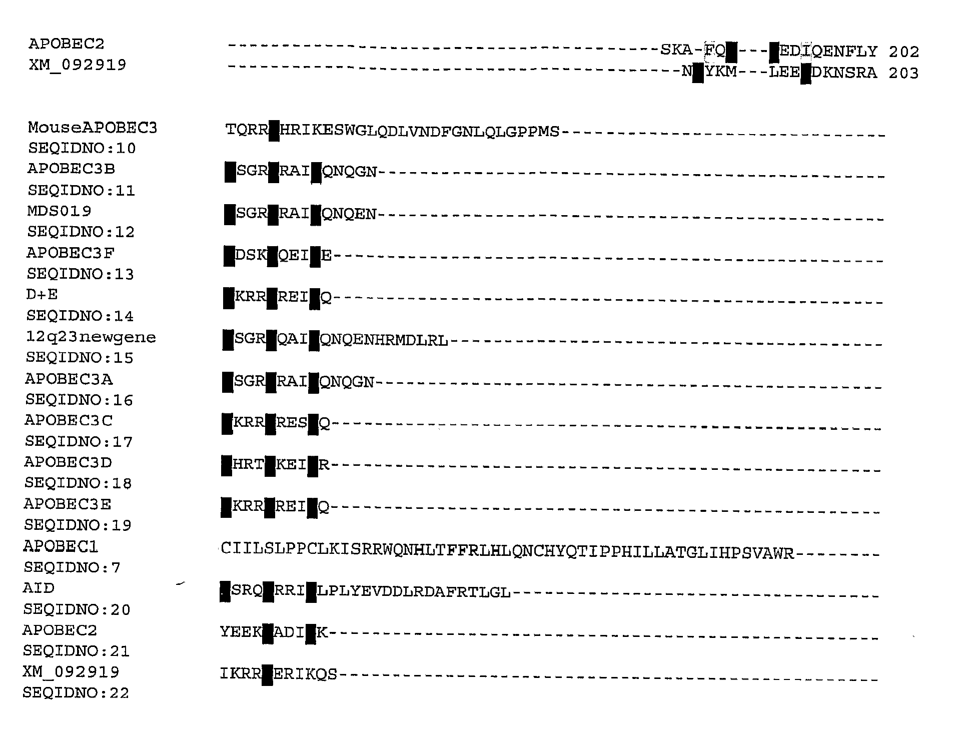

The TBLASTN results are shown in Table 5:

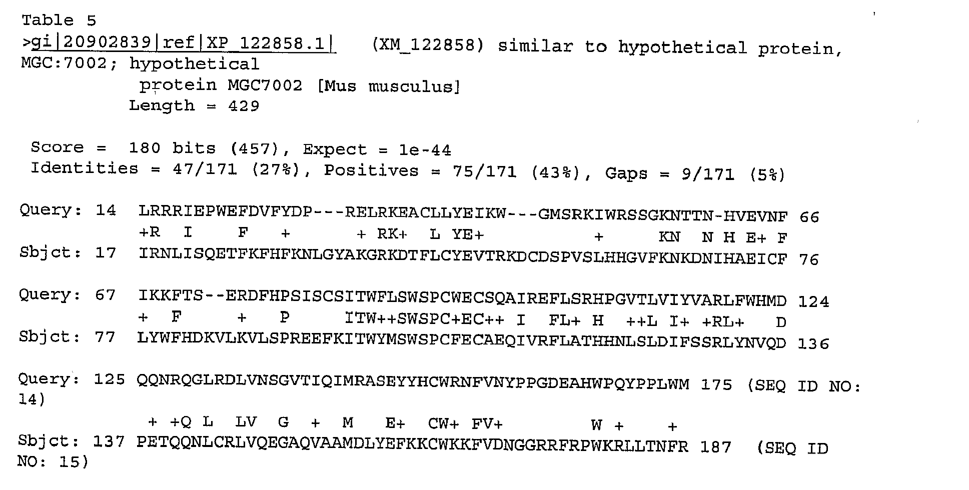

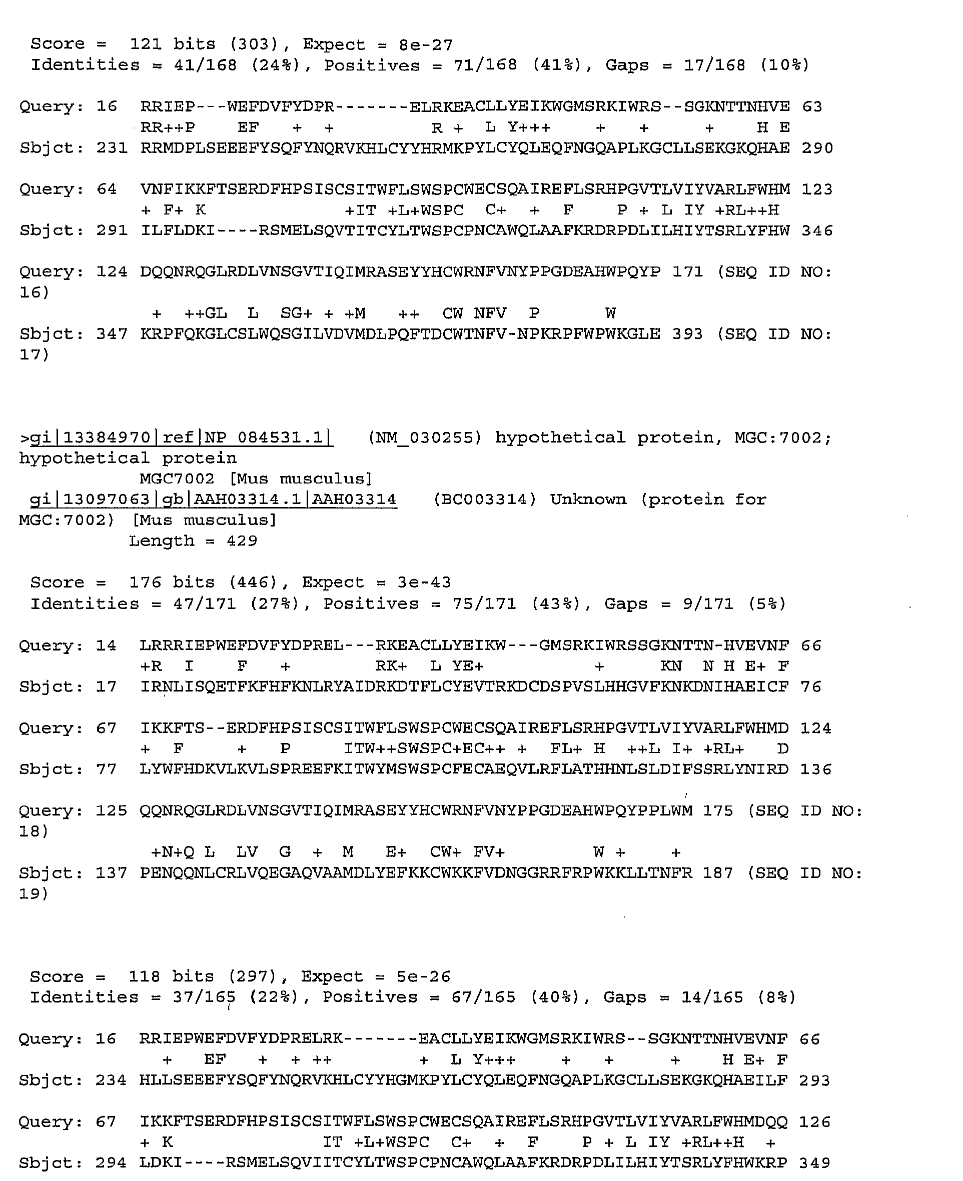

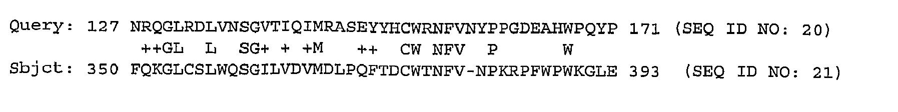

The is the BLAST alignment as shown in Table 6:

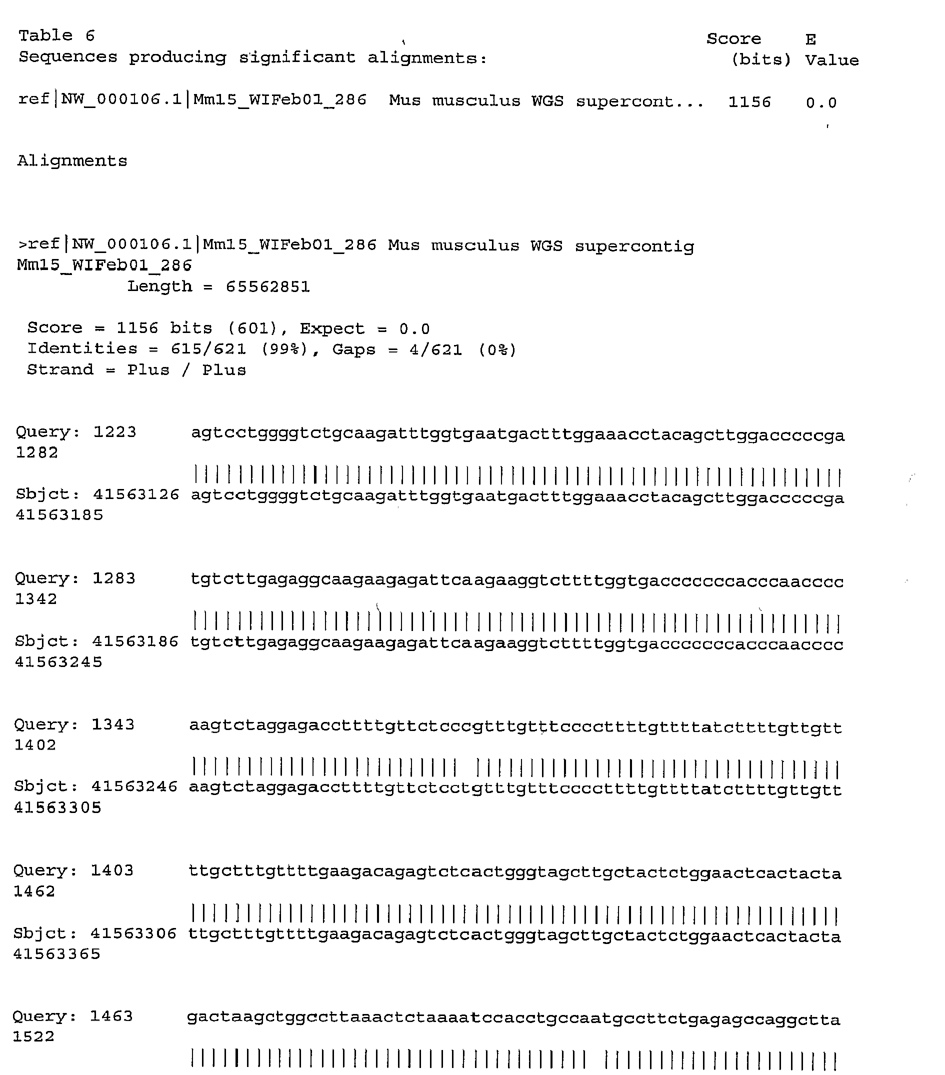

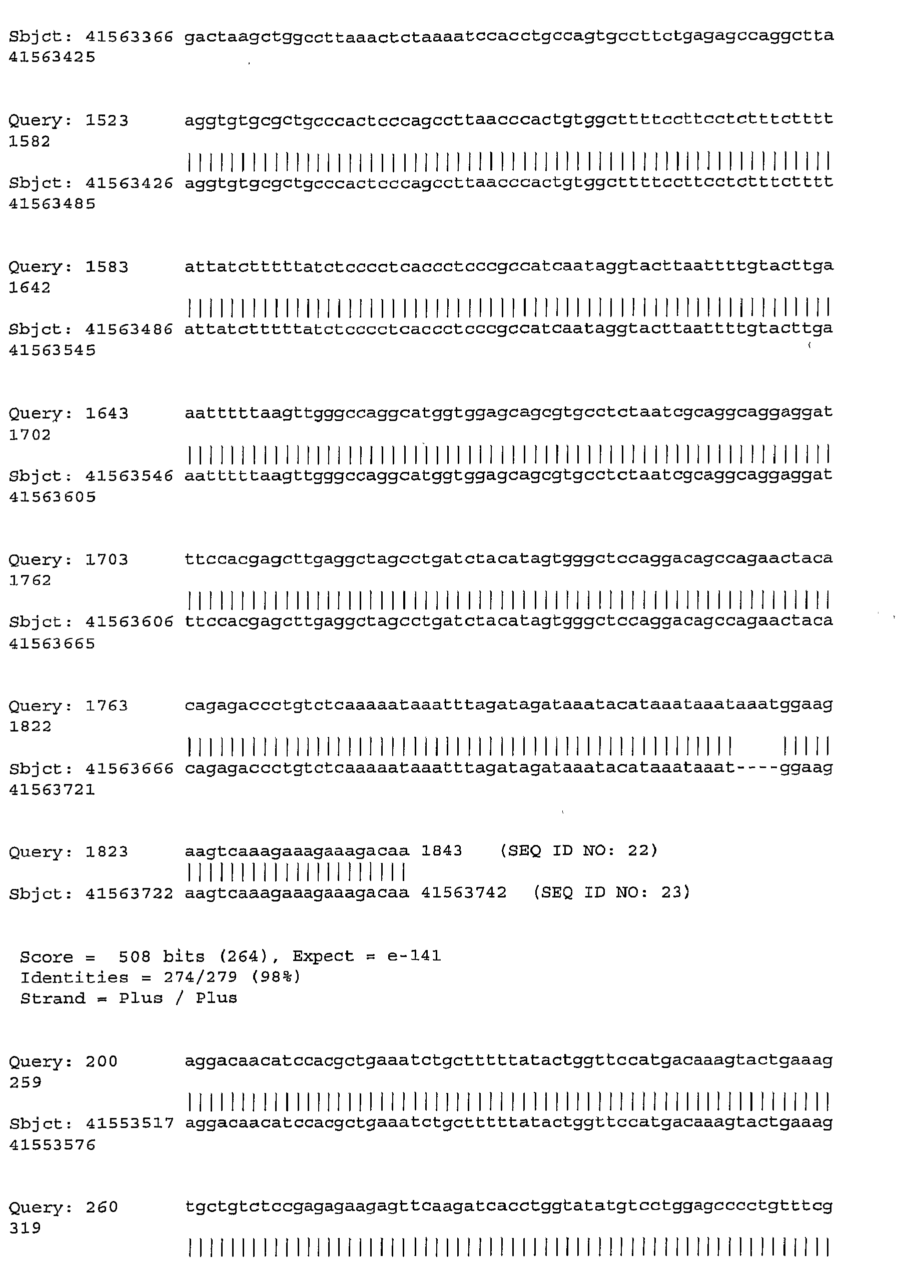

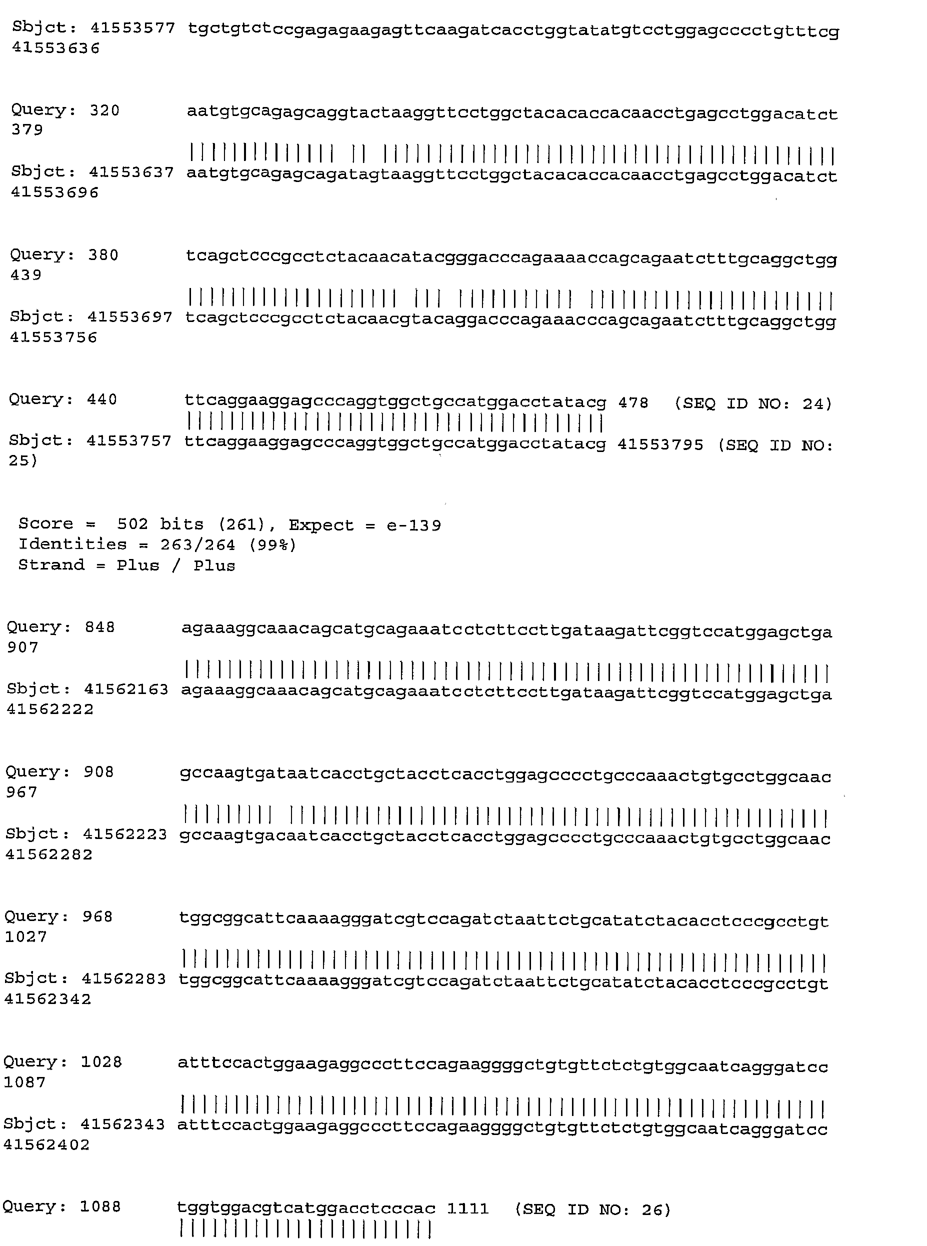

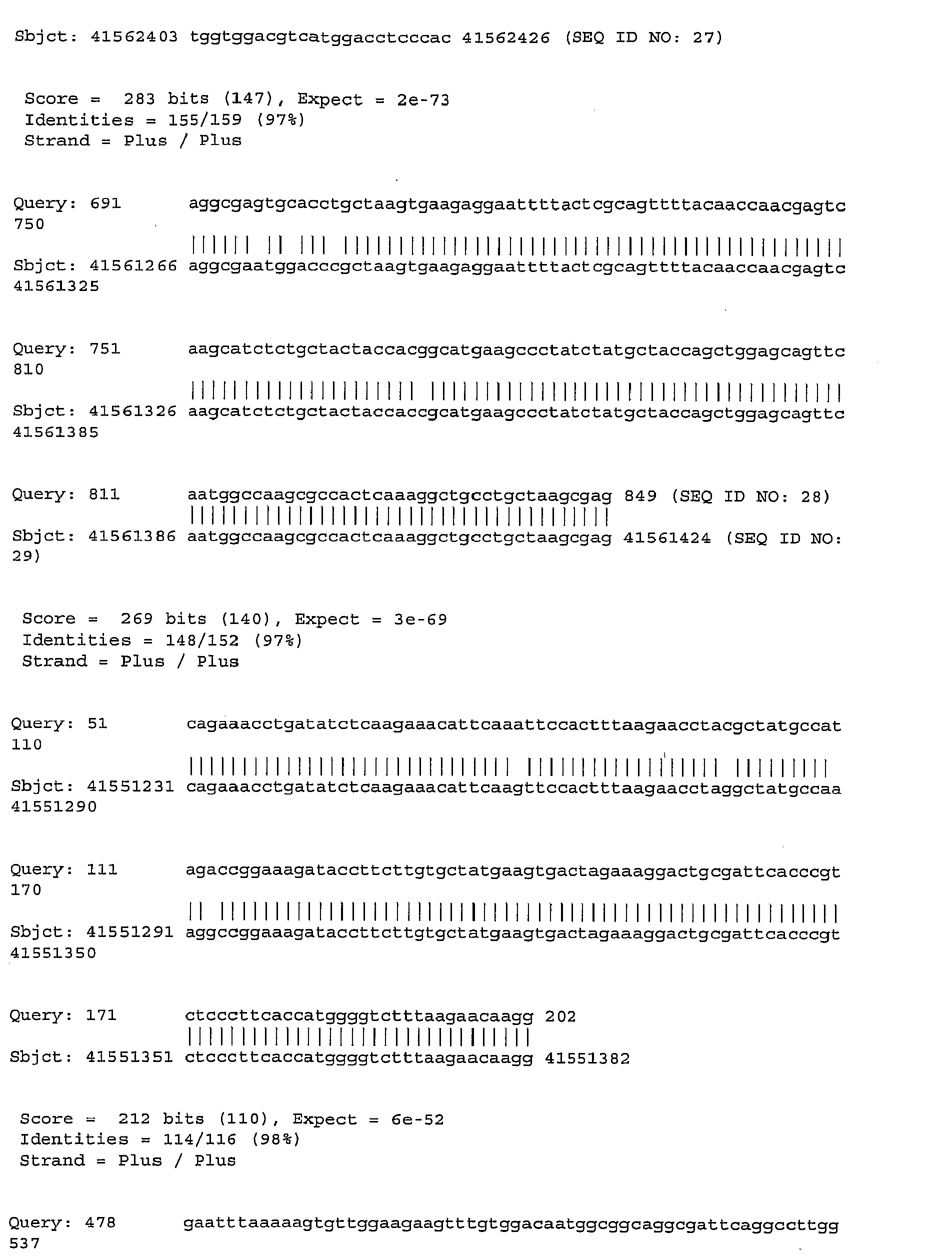

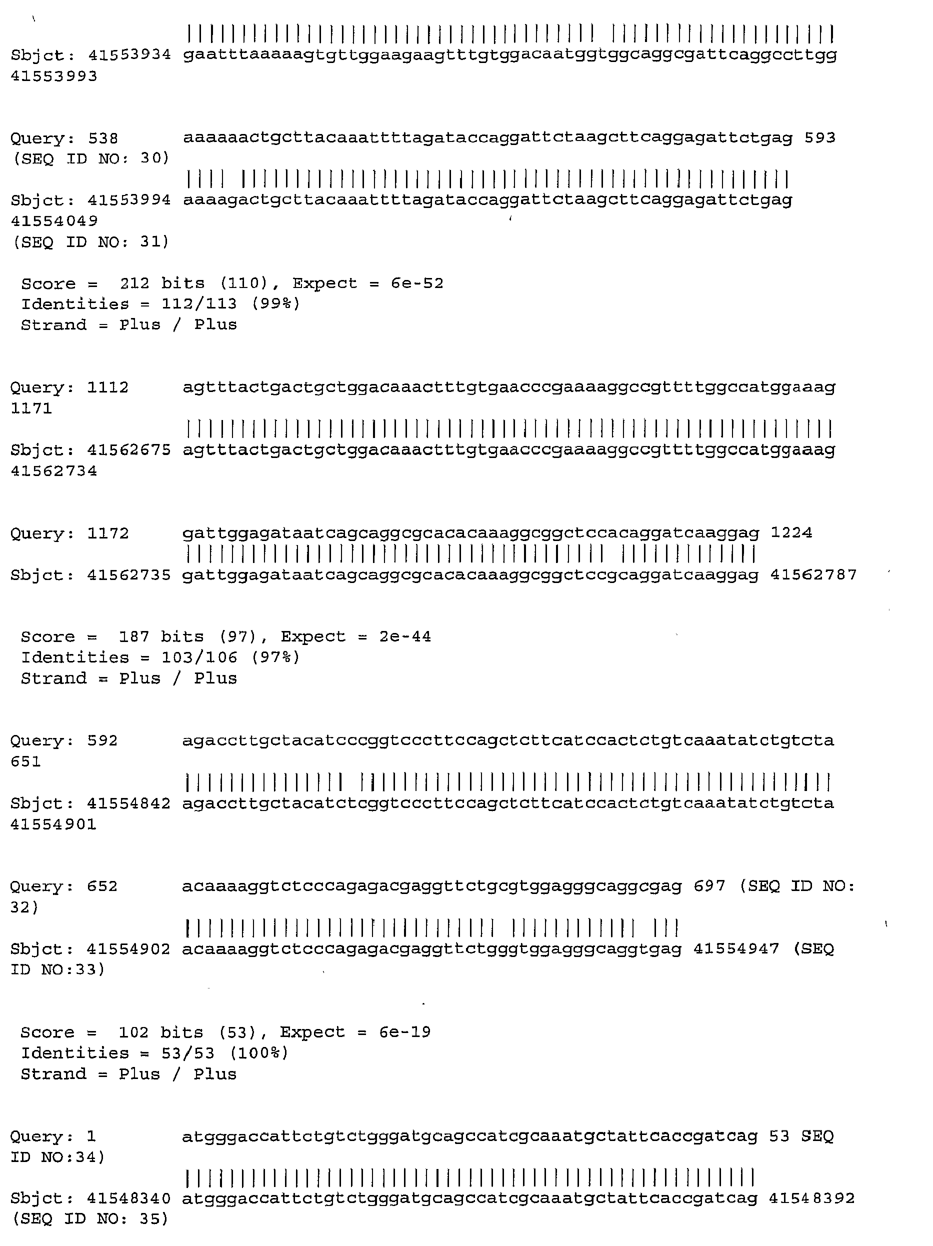

### 3. Example 3

### a) EXPERIMIENTAL

326. All plasmids were constructed by standard recombinant DNA methods and verified by DNA sequencing. The intervening sequence (IVS)-apoB construct has been described previously (Sowden, M., (1996) RNA 2, 274-288), mutation of 6 bp at the 5' splice donor sequence, including the intronic GU dinucleotide (IVS- Δ5'apoB) and deletion of 20 bp encompassing the 3' splice acceptor and polypyrimidine tract sequences (IVS- Δ3'apoB), was accomplished by 'runaround' PCR using primers that included an *Xho*I site to facilitate subsequent re-ligation of the PCR product (Fisher, C. L. (1997) BioTechniques 23, 570-574). IVS-Δ3'5'apoB was created by ligation of the appropriate halves of the above molecules.
327. McArdle RH7777 cells were maintained as previously described (Sowden, M.P. (1996) J. Biol. Chem. 271:3011-3017) and transfected in six-well clusters with 2 µg of DNA using lipofectAMINE^{®} (Gibco BRL) according to the manufacturer's recommendations. RNAs were harvested 48 h post-transfection in TriReagent (Molecular Research Center, Cincinnati, OH, U.S.A.) and subjected to reverse-transcriptase (RT)-PCR for amplification of intron-containing or exonic apoB specific transcripts using appropriate PCR primers as previously described (Sowden, M., (1996) RNA 2, 274-288) and outlined in the Figure legends. Editing efficiencies were determined by poisoned-primer-extension assay on purified PCR products (Sowden, M., (1996) RNA 2, 274-288) and quantified by analysis on a Phosphorlmager (model 425E; Molecular Dynamics).
328. The poisoned-primer-extension assay relies on the annealing of a ³²P-end-labelled primer 3' of the editing site to the heat-denatured single-stranded PCR product. Extension of this primer using RT in the presence of dATP, dCTP, dTTP and dideoxy (dd)-GTP produces an extension product eight nucleotides longer if the cytidine has not been edited (CAA in the Figures); that is, incorporation of ddGTP causes chain termination. If editing has created a uridine, then primer extension continues a further 11 nucleotides to the next 5' cytidine, where chain termination then occurs (UAA in the Figures). Quantification of the level of editing is accurately determined using laser scanning densitometry. The linear exposure range of the PhosphorImager screen is sufficiently great to permit precise determination of low counts in the UAA bands whilst the high levels of counts in the CAA band remain in the linear range. Editing percentages were calculated as the counts in the UAA band divided by the total counts in the CAA plus UAA bands times 100. This assay has a lower level of detection of 0.1% editing and remains linear up to 99.5% and is independent, between 1 ng and 500 ng, of the total amount of template PCR product used (M. P. Sowden, unpublished work).
329. Rev complementation/editing assays (Taagepera, S., (1998) Proc. Natl. Acad. Sci. U.S.A. 95:7457-7462.) were performed in duplicate in McArdle cells seeded in six-well clusters. Briefly, a total of 2 µg of DNA, comprising 1 µg of reporter DNA, 0.75 µg of transactivator DNA (pRc/CNW vector or a nucleocytoplasmic shuttling competent Rev-Rex fusion; a gift of Dr Thomas J. Hope, Infectious Disease Laboratory, Salk Institute for Biological Studies, La Jolla, CA, U.S.A.) and 0.25 µg of pRSV-β-galactosidase [internal control for chloramphenicol acetyl-transferase (CAT) assays] were introduced into McArdle cells using lipofectAMINE^{®} as described above. Cells were harvested at 48 h post-transfection, protein extracts prepared by freeze-thawing, and β-gal (Sowden, M.P., (1989) Nucleic Acids Res. 17:2959-2972) and CAT (Neumann, J.R., (1987) BioTechniques. 5:444-448) assays performed as previously described. All extracts were normalized for b-gal activity. Parallel transfections were harvested for RNA preparation and RT-PCR amplification of the apoB RNA. Editing efficiencies were quantified as described above.

### b) RESULTS

### (1) Introns interfere with editing

330. Previous studies demonstrated that the editing efficiency of apoB RNA was dramatically reduced when an intron was placed ≤ 350 nt 5' or 3' of the target cytidine (Sowden, M., (1996) RNA 2, 274-288). To provide proof that it was specifically RNA splicing and/or spliceosome assembly that had affected editing efficiency, splicing-competent and splicing-defective RNA transcripts were evaluated for their ability to support RNA editing in transfected McArdle rat hepatoma cells. The apoB pre-mRNA reporter construct contained an abbreviated splicing cassette from the adenovirus late leader sequence fused to 450 nt of wild-type apoB mRNA (Figure 1A). Unspliced pre-mRNA and spliced mRNA were amplified from total cellular McArdle cell mRNA using the MS 1/MS2 and SP6/T7 amplimer pairs respectively (Figure 1A). Consistent with previous results, the splicing cassette impaired the ability of the IVS-apoB RNA transcript to be edited, either before (pre-mRNA) or after (mRNA) it was spliced relative to a control transcript (pRc-apoB) that contained only apoB sequence (Figure 1B). These results corroborate previous findings suggesting that there is a window of opportunity for editing apoB mRNA in the nucleus and that no further editing occurs in the cytoplasm of wild-type hepatic cells. Specifically, recently published subcellular-fractionation studies have shown that the low level of editing measured on this transcript as mRNA (1 %) occurred while the RNA was still in the nucleus (Yang, Y., (2000) J. Biol. Chem. 275: 22663-22669).
331. Deletion of the polypyrimidine tract/branch point sequences and the 3' splice acceptor site in the IVS- Δ3'apoB transcript (Figure 1A) ablated the ability of this pre-mRNA to be spliced, as the SP6/T7 amplimer pair yielded only PCR products indicative of unspliced transcripts. The editing efficiency of this splicing-defective construct was higher than that of IVS-apoB (14%, S.E.M=1.0 %; Figure 1B). The IVS- Δ5'apoB transcript was also defective in splicing owing to deletion of the 5' splice donor sequence (the SP6/T7 amplimer pair failed to yield PCR products corresponding to spliced RNA), and this RNA also demonstrated markedly elevated editing compared with IVS-apoB (11%, S.E.M.=0.1 %; Figure 1B). The double-splice-site mutant IVSD3'5'apoB (Figure 1A) had an editing efficiency higher than either of the single-site mutants (20%, S.E.M.=0.2 %) and equivalent to the intron lacking RNA transcript, pRc-apoB (24%, S.E.M.=0.2 %; Figure 1B). These results indicated that it is the assembly of a fully functional spliceosome and/or RNA splicing that impedes editosome assembly and/or function, and that both 5' and 3' splicing signals contribute to the inhibitory effect.
332. Each of the constructs in Figure 1 generated pre-mRNA transcripts of equivalent length, but the presence of active or inactive introns might influence expression levels of the resultant mRNAs. However, it was previously reported that the expression level of a given apoB transcript did not affect its editing efficiency (Sowden, M., (1996) RNA 2, 274-288). Moreover, there was no competition between the editing efficiencies of exogenous and endogenous apoB transcripts, indicating that editing factors were not made to be rate-limiting by the increased concentration of apoB editing sites. These facts underscore the significance of the intron and RNA splicing on the regulation of editing efficiency.
333. In human apoB mRNA, C₆₆₆₆ is located in the middle of the 7.5 kb exon 26, significantly further from a 5' or 3' intron than in the chimeric constructs described above. Therefore it was evaluated whether the proximity of the splice donor and acceptor sites to the tripartite motif affected editing efficiency. Insertion of a monomer or a dimer of the splicing-defective intron cassette (IVS Δ3'5') increased the distance between the active intron and the editing site by 425 and 850 nt respectively (Figure 2A). This increased the effective size of the chimeric exon to nearly 1 kb or 1.4 kb respectively; the average size of an internal exon being only 200-300 nt in mammals (Robberson, B.L., (1990) Mol. Cell. Biol. 10:1084-1094).
334. ApoB pre-mRNA was amplified from each transcript expressed in McArdle cells using the MS7/MS2 amplimers and nesting with the MS2/MS3 amplimer pair. The sequence of primer MS7 is unique to the functional intron sequence and thus ensured amplification of unspliced pre-mRNA. Barely detectable levels of editing were measured on both pre-mRNA transcripts. However, a 10-fold higher level of editing was observed upon the spliced mRNA of both transcripts (6.0 %) (Figure 2B), which is 6-fold higher than the spliced mRNA derived from IVS-apoB (Figure 1B). This indicated that increasing the distance between the intron and the editing site alleviated, but was not completely capable of overcoming, the inhibitory effect of spliceosome assembly/RNA splicing on editing (i.e. compare 6 with 20% editing of IVS Δ3'5'apoB in Figure 1).

### (2) The apoB editing site is not efficiently used within an intron

335. A search of GenBank2 for apoB mooring-sequence similarities reveals numerous potential editing sites. However, many are located short distances from splice sites or within 5' or 3' untranslated regions or introns where the functional consequence(s) of a cytidine-to-uridine editing event is unclear. The release of the entire human, mouse and rat genome sequences will likely reveal more mooring-sequence similarities, although their location in introns or exons may be uncertain until these genomes are annotated. In this regard, the results indicated that mooring-sequence-dependent editing sites may not be biologically active if they are positioned too close to splice junctions.
336. In an attempt to be able to predict functional cytidine-to-uridine editing sites from these transcriptomes, it was investigated whether the apoB editing site is recognized when positioned within an intron. A 450 nt section of the apoB RNA transcript containing the editing site was placed within the intron of the adenovirus late leader sequence (IVS-apoB INT) and this construct was expressed in transfected McArdle cells. Pre-mRNA transcripts were amplified using the Ex1/Ex2 amplimers followed by nested PCR with the MS Δ5/MSΔ6 amplimer pair and were edited at an efficiency of 0.4 % (Figure 3B). Intron-containing transcripts were amplified using the MS Δ5/MS Δ6 amplimers followed by nested PCR with the MS2/MS3 amplimer pair and were edited at an efficiency of 0.5 % (Figure 3B). The use of the MS Δ5/MS Δ6 amplimer pair in the initial PCR would not distinguish between unspliced pre-mRNA or spliced-out lariat RNA, but given the rapid degradation of lariat RNA, it is unlikely that the amplified PCR products represent lariat RNA species. If, however, there were amplified lariat species present, the difference of 0.1 % between intron-containing and unspliced pre-mRNA suggests that lariat RNAs containing apoB editing sites are not efficient editing substrates.
337. Mutation of the 5' and 3' splicing signals of the above construct to generate IVS- Δ3'5'apoB INT restored editing efficiency (20%; Figure 3B) to a level equal to that of IVS- Δ3'5'apoB construct (20%; Figure 1C). A minor additional primer extension product indicative of promiscuous editing was also apparent. These results support the hypothesis that pre-mRNA is not an effective substrate for cytidine-to-uridine editing and that this likely results from interference by spliceosome assembly/RNA splicing or potentially the rapid nuclear export of spliced mRNAs into the cytoplasm.

### (3) Blocking the commitment of transcripts to the splicing pathway alleviates splice-site inhibition of editing

338. Most apoB mRNA editing substrate studies have employed cDNA transcripts which lack introns (Sowden M.P., (1998) Nucleic Acids Res. 26:1644-1652.; Driscoll, D.M., (1993) Mol. Cell. Biol. 13:7288-7294.; Bostrom, K., (1990) J. Biol. Chem. 265:22446-22452.) Wild-type apoB cDNA transcripts expressed in wild-type McArdle cells edit 2-3-fold more efficiently than the endogenous transcript (Sowden, M., (1996) RNA 2, 274-288.; Sowden M.P., (1998) Nucleic Acids Res. 26:1644-1652). It has been demonstrated that chimeric splicing-editing reporter RNAs (IVS-apoB) had low editing efficiency as nuclear transcripts, which did not change once spliced mRNAs had entered the cytoplasm (Figure 1; (Yang, Y., (2000) J. Biol. Chem. 275: 22663-22669). Hence the window of opportunity for a transcript to be edited in wild-type cells was confined to the nucleus, and when introns are proximal to the editing site, its utilization was impaired.
339. To investigate if spliceosome assembly was involved in the inhibition of editing, and by-passing the spliceosome assembly commitment step inhibition may be alleviated (in a manner similar to intronless cDNA transcripts), the processes of RNA splicing and RNA nuclear export were separated by utilizing a modification of the Rev complementation assay that has been employed to identify HIV-1 Rev-like nuclear export sequences (Taagepera, S., (1998) Proc. Natl. Acad. Sci. U.S.A. 95:7457-7462). Rev functions, by interaction with an RRE, to export unspliced RNA out of the nucleus. A reporter plasmid was constructed which contained an intron interrupted by the CAT gene and a functional apoB RNA editing cassette (Figure 4A). CAT activity could only be expressed if unspliced RNA was exported to the cytoplasm, a process wholly dependent upon an active Rev protein expressed from a co-transfected plasmid. In the presence of Rev, spliceosome assembly on the transcript does not occur and therefore should not interfere with the utilization of the apoB editing site contained with the intron.
340. McArdle cells were co-transfected with the modified reporter construct, together with either a control vector or a Rev expression vector. CAT activity was determined 48 h later (Figure 4B). In the presence of the control vector, very low levels of CAT activity were expressed, presumed to be due to splicing and degradation of the CAT transcript as a lariat RNA. Expression of the Rev protein resulted in nuclear export of unspliced intronic RNA and translation of the CAT protein, as evident in the 7-fold higher level of CAT activity in these cell extracts. These findings demonstrated that, in McArdle cells, HIV-1 Rev protein successfully diverted RNAs from the spliceosome assembly pathway and transported them into the cytoplasm.
341. Total cellular RNA was harvested from parallel transfections, the apoB sequence amplified, and the editing efficiencies were determined (Figure 4C). Consistent with the findings described above, editing of apoB RNA within an intron of the RRE construct in the absence of Rev expression was very low ('intron+exon' amplified with EF/MS2). However, the editing efficiency was enhanced 5-fold when the Rev protein was co-expressed. Given that editing in the cytoplasm has never been demonstrated in wild-type McArdle cells (Yang, Y., (2000) J. Biol. Chem. 275: 22663-22669), nor would it be driven by an increase in apoB RNA abundance in the cytoplasm (Sowden, M., (1996) RNA 2, 274-288) the enhanced editing likely occurred in the nucleus as a consequence of pre-mRNAs by-passing commitment to the spliceosome assembly and/or RNA export pathways. Editing unspliced CAT-apoB chimeric RNAs in the cytoplasm would necessitate the activation of cytoplasmically localized editing factors by Rev.
342. In addition to an enhanced editing efficiency, the unspliced CAT-apoB RNA was also promiscuously edited (additional primer extension stop labeled '1', Figure 4C). Promiscuous editing does not occur under physiological expression levels of APOBEC-1 in McArdle cells (Sowden, M., (1996) RNA 2, 274-288.; Sowden, M.P. (1996) J. Biol. Chem. 271:3011-3017.; Siddiqui, J.F., (1999) Exp Cell Res. 252:154-164), in rat tissues or under biological conditions where editing efficiencies are greater than 90%, e.g. rat intestine (Greeve, J., (1993) J. Lipid Res. 34:1367-1383). Nor does it occur when rat hepatic editing efficiencies are stimulated by metabolic or hormonal manipulations (Lau, P.P., (1995) J. Lipid Res. 36:2069-2078.; Baum, C.L.. (1990) J. Biol. Chem. 265: 19263-19270). Promiscuous editing appears to be unique to cells in which APOBEC-1 has been artificially overexpressed (Sowden, M., (1996) RNA 2, 274-288.; Sowden, M.P. (1996) J. Biol. Chem. 271:3011-3017.; Siddiqui, J.F., (1999) Exp Cell Res. 252:154-164) and is observed under these conditions on both nuclear and cytoplasmic transcripts (Yang, Y., (2000) J. Biol. Chem. 275: 22663-22669). The results presented in Figures 3 and 4 are therefore the first demonstration of promiscuous editing in the nucleus without the exogenous overexpression of APOBEC-1.

### c) DISCUSSION

343. ApoB mRNA editing, while conceptually a simple process of hydrolytic cytidine deamination to uridine (Johnson, D.F., (1993) Biochem. Biophys. Res. Commun. 195:1204-1210) has turned out to have surprising complexities in both the number of proteins involved and the cell biology involved in its regulation. It is well established that a sequence element consisting of three proximal components (enhancer, spacer and mooring sequence) comprise the cis-acting sequences required for efficient site-specific editing of C₆₆₆₆ in apoB mRNA (Smith, H.C., (1991) Proc. Natl. Acad. Sci. U.S.A. 88:1489-1493; Backus, J.W., (1992) Nucleic Acids Res. 20: 6007-6014; Smith, H.C. (1993) Semin. Cell. Biol. 4:267-278; Shah R.R., (1991) J. Biol. Chem. 266:16301-16304; Backus, J.W., (1991) Nucleic Acids Res. 19: 6781-6786; Driscoll, D.M., (1993) Mol. Cell. Biol. 13: 7288-7294). A multiple protein editosome catalyses and regulates editing of C₆₆₆₆ (Smith, H.C., (1991) Proc. Natl. Acad. Sci. U.S.A. 88:1489-1493; Harris, S.G., (1993) J. Biol. Chem. 268:7382-7392; Yang, Y., (1997) J. Biol. Chem. 272: 27700-27706). The components of the minimal editosome from defined *in vitro* system analyses are APOBEC-1 as a homodimeric cytidine deaminase (Lau, P.P., (1994) Proc. Natl. Acad. Sci. U.S.A. 91:8522-8526) bound to the auxiliary protein ACF/ASP that serves as the editing-site recognition factor through its mooring-sequence-selective RNA-binding activity (Mehta, A., (2000) Mol. Cell. Biol. 20:1846-1854; Lellek, H., (2000) J. Biol. Chem. 275:19848-19856). Several other auxiliary protein candidates have also been described that had binding affinities for APOBEC-1 and/or apoB mRNA and that demonstrated the ability to modulate editing efficiency (Giannoni, F., (1994) J. Biol. Chem. 269:5932-5936;Ymanaka, S., (1994) J. Biol. Chem. 269:21725-21734; Yang, Y., (1997) J. Biol. Chem. 272: 27700-27706; Lellek, H., (2000) J. Biol. Chem. 275:19848-19856; Teng, B., (1993) Science 260:1816-1819; Inui, Y., (1994) J. Lipid Res. 35:1477-1489; Anant, S.G., (1997) Nucleic Acids Symp. Ser. 36:115-118; Lau, P.P., (1997) J. Biol. Chem. 272:1452-1455). Although, under biological conditions, editing occurs only in the nucleus (Lau, P.P., (1991) J. Biol. Chem. 266, 20550-20554; Yang, Y., (2000) J. Biol. Chem. 275:22663-22669), nuclear and cytoplasmic distributions have been described for both APOBEC-1 and ACF (Yang, Y., (2000) J. Biol. Chem. 275:22663-22669; Yang, Y., (1997) Proc. Natl. Acad. Sci. U.S.A. 94:13075-13080; Dance, G.S.C., (2000) Nucleic Acids Res. 28:424-429). Nuclear editing has been characterized as occurring coincident with, or immediately after, pre-mRNA splicing (Lau, P.P., (1991) J. Biol. Chem. 266, 20550-20554; Yang, Y., (2000) J. Biol. Chem. 275:22663-22669; Sowden, M., (1996) RNA 2:274-288). Prior to splicing, pre-mRNA was not efficiently edited (Lau, P.P., (1991) J. Biol. Chem. 266, 20550-20554). It was not apparent, given the size of exon 26 and the nature of the cis-acting RNA sequence requirements, why there was a lag in editing activity during pre-mRNA maturation. This question was addressed in studies indicating that spliceosome assembly and/or nuclear RNA export pathways regulate the utilization of cytidine-to-uridine editing sites.
344. In reporter RNA constructs, introns within 350-1000 nt of the apoB editing site suppressed editing efficiency. This inhibition was dependent on an active 5' splice site and/or 3' splice donor site and was partially alleviated after the reporter RNA had been spliced. This indicates that the process of spliceosome assembly functionally interfered with editosome assembly and/or function. This is supported by the distance dependence of this inhibition. When the splice sites were located more distal to the editing site, editing efficiencies were increased albeit not to levels seen on RNAs that do not contain introns. The gating hypothesis (Sowden, M., (1996) RNA 2, 274-288) proposed that each apoB RNA had a temporal 'window of opportunity' to become edited during its splicing and export from the nucleus. In this model, factors involved in spliceosome and editosome assembly are thought to compete for access to the mRNA. Consequently it is predicted that there will be less steric hindrance between the spliceosome and the editosome, and editing efficiency will improve the more distal an intron is located relative to the editing site (e.g. IVS-(IVS Δ3'5')-apoB or IVS- (IVS Δ3'5')2 -apoB compared with IVS-apoB). This phenomenon might explain the lower editing efficiency of native apoB editing prior to splicing, because the native editing site is only three times further away from the 5' or 3' splice junctions than that used in our reporter RNA constructs.
345. Importantly, these results have implications for the prediction of novel mooring-sequence-dependent RNA-editing sites. Not only is there a requirement for a target cytidine to be appropriately located upstream of a mooring sequence, but for efficient utilization, the editing site should not be in close proximity to an intron. Considering that the average size of an internal exon is only 200-300 nt in mammals (Robberson, B.L., (1990) Mol. Cell. Biol. 10, 1084-1094), it is highly unlikely that a significant amount of mooring-sequence-dependent editing will be observed in mRNAs with standard sized exons. An analysis of the human, mouse and rat expressed-sequence-tag databases by Hidden Markov modeling has confirmed that the majority of mooring-sequence identities within coding sequences are located proximal to intron/exon junctions. An evaluation of select RNA transcripts revealed that they were not edited. Related to these observations are results showing that editing sites located within introns were not inefficiently utilized. Taken together, the results support the hypothesis that spliceosome assembly and editosome assembly processes are communicating a temporal and spatial relationship that ultimately determines the efficiency of mooring-sequence-dependent editing. Consistent with this communication between the spliceosome and editosome is the finding that several proteins that have a role in RNA structure and/or splicing have also been implicated in RNA editing as auxiliary factors. These include hnRNP C, hnRNP D, APOBEC-1-binding protein (which has homology with hnRNP A and B) and KSRP, a protein involved in alternative splice site utilization (Lellek, H., (2000) J. Biol. Chem. 275:19848-19856; Greeve, J., (1998) J. Biol. Chem. 379:1063-1073; Anant, S.G., (1997) Nucleic Acids Symp. Ser. 36:115-118; Lau, P.P., (1997) J. Biol. Chem. 272:1452-1455.).
346. The promiscuous editing observed on IVS- Δ3'5'apoB INT was unexpected, given the nature of the transcript, i.e., a cDNA equivalent to IVS-Δ3'5'apoB in Figure 1 on which no promiscuous editing was observed at equivalent editing at C⁶⁶⁶⁶. A possibility for this could be the fortuitous introduction of a pair of tandem UGAU (SEQ ID NO: 36) sequences within the intronic sequence 3' of the editing site, a motif that has been previously shown to promote promiscuous editing (Sowden, M.P., (1998) Nucleic Acids Res. 26:1644-1652).
347. The description of the relationship of RNA splicing and editing is unique for apoB cytidine-to-uridine mRNA editing. However, an emerging theme in RNA processing is an interdependence of multiple steps in RNA maturation. Perhaps the most relevant to apoB editing is the adenine-to-inosine editing of glutamate and 5-hydroxytryptamine receptors. In contrast with apoB mRNA editing, mRNA substrates that undergo adenine-to-inosine editing all require the presence of a complementary intron sequence to form a partially double-stranded RNA structure that is recognized by the appropriate ADAR1 or ADAR2 enzyme Simpson, L., (1996) Annu. Re. Neurosci. 19:27-52; Maas, S., (1997) Currr. Opin. Cell. Biol. 9:343-349; Rueter, S. M. and Emeson, R. B. (1998) Modification and Editing of RNA (Grosjean, H. and Benne, R., eds.), pp. 343-361). The critical role of cis-acting intronic sequences indicates deamination is a nuclear event, and as the editing site is frequently located close to a 5' splice acceptor site (Higuchi, M., (1993) Cell. 75:1361-1370; Egebjerg, J., (1994) Proc. Natl. Acad. Aci. U.S.A. 91:10270-10274) suggests that the level of editing maybe influenced by interference or interaction with RNA splicing. For example, endogenously expressed GluR2 mRNA from neuronal cell lines is always edited to 100% at the Gln/Arg site, whereas unspliced GluR2 transcripts are edited to only 70-90% (Higuchi, M., (1993) Cell 75:1361-1370.), indicating a partial inhibition of splicing until editing has occurred. Conversely, the transcript of the *Glu-R6* gene contains three exonic editing sites (Ile/Val, Tyr/Cys and Gln/Arg) which are edited to different extents, indicating that there must be a tightly regulated and coordinated action of the appropriate ADAR and the spliceosome at each editing site (Kohler, M., (1993) Neuron 10:491-500; Seeburg, P.H., (1998) Brain Res. Rev. 26:217-229). In crosses of ADAR2 +/- with GluR-B (R) +/+ mice, an influence from the editing status of the Gln/Arg site on subsequent splicing of the downstream intron was observed (Higuchi, M., (2000) Nature 405:78-81), indicating that these RNA processing events do not occur independently. The major steps in pre-mRNA processing, capping, splicing, 3'-end cleavage and polyadenylation are coupled to transcription through recruitment of the necessary processing factors to the largest subunit of the RNA polymerase II. This represents an efficient process for increasing local concentrations of related processing and transcription factors on pre-mRNAs as and when they are needed (Lewis, J.D., (2000) Science 288:1385-1389). Many analyses of RNA processing have attempted to identify active versus inactive populations of processing factors and have postulated that the greatest concentration of factors may or may not correspond to sites of function, dependent upon metabolic activity (Spector, D. (1993) Annu. Rev. Cell. Biol. 9:265-315). Specifically, recent photobleaching studies (Lewis, J.D., (2000) Science 288:1385-1389. and references cited therein) suggested that 'speckles' correspond to sites where free small nuclear RNPs transiently assemble before recruitment by the C-terminal domain of RNA polymerase II and transfer to nascent transcripts. It is easily conceivable, therefore, that the processes of RNA editing and RNA splicing should be tightly coordinated, and the observation of nuclear and cytoplasmically localized APOBEC-1 and ACF corresponds to active and inactive complexes respectively. These two components of the minimal editosome, together with other editosomal proteins if necessary, could be rapidly recruited to newly synthesized apoB mRNA transcripts by a coordinated action of RNA polymerase II and spliceosome assembly.
348. Most, if not all, known RNA processing reactions can occur *in vitro*, but they are not as efficient as *in vivo*. This is also true for *in vitro* apoB RNA editing reactions. However, IVS-apoB RNA transcripts were edited with the same efficiency as intronless apoB transcripts *in vitro*. This indicates that the presence of an intron *per se* does not interfere with editing, but, as was shown, there is a clear interdependence of splicing and editing for editing site regulation and fidelity *in vivo*. Such interdependence is also exhibited in mammalian nonsense-mediated decay ('NMD') of RNA, wherein only RNAs that contain nonsense codons and that have passed through the spliceosome are 'marked' and targeted for decay (Le Hir, H., (2000) EMBO J. 19:6860-6869). This imprinting of nuclear pre-mRNA by proteins that remain bound in the cytoplasm is a means of mRNAs 'communicating their history' (Kataoka, N., (2000) Mol. Cell. 6:673-682) and/or perhaps ensuring that no further RNA processing/editing occurs in the cytoplasm (Maquat, L., (2001) Cell 104:173-176).
349. In conclusion, it has been demonstrated a spatial and temporal relationship between RNA splicing and apoB RNA editing. The suppression of editing-site utilization by proximal introns can explain the uniquely large size of exon 26 and/or the scarcity of other mooring-sequence-dependent cytidine-to-uridine editing sites. Moreover, these studies highlight the need to consider apoB RNA editing as an integrated process with RNA transcription and splicing, potentially expanding the number of auxiliary factors that should be considered as involved in apoB RNA editing.

### 4. Example 4

### Isolation and identification of edited mRNAs or mutated DNA sequences

350. Edited mRNA or mutated DNA is identified through an adaptation of a bacterial DNA mismatch detection system (Faham et al. Hum. Mol. Genet. (2001) which was originally developed to detect single nucleotide polymorphisms in genomic DNA. In this system, DNA repair confers a positive selection through antibiotic resistance for clones containing an insert bearing the edited or mutated nucleotide (Faham et al., 2001). Unedited and edited cDNA prepared from mRNA (or restriction endonuclease fragments of genomic DNA) isolated from wild type and AID-expressing NIH3T3 is used to form heteroduplexes that go into the selection system. The identity of the tetracycline resistant (selected) clone(s) is determined by DNA sequencing. The location of the edited nucleotide(s) will be determined by comparing selected cDNA(s) to genomic and EST sequence databases. Similar selection for edited or mutated mRNA or DNA sequences are carried out on appropriate material isolated from CEM15 expressing 293T cells infected with Vif- or Vif+ HIV-1 pseudotyped virus.
351. Edited mRNAs are also identified through a complementary approach that selects for mRNAs associated with affinity purified AID editing complexes. 6His-tagged AID is expressed in NIH3T3 cells (or 6His tagged CEM15 expressed in 293T cells infected with Vif- pseudotyped HIV-1) to promote editing complex assembly on mRNA and then affinity purified on nickel resin. The associated mRNA substrates are isolated and cDNAs cloned. The identity of the selected mRNAs is determined by DNA sequencing and the location of edited nucleotides in the candidate mRNA(s) is determined. A similar approach can be used to select for mutated DNA and can be applied to identify RNA or DNA associated with all members of the ARP family when expressed in a relevant cell context.
352. Mouse and human homologs of mRNAs that are edited are expressed in wild type and AID expressing NIH3T3 cells. RT-PCR products containing the predicted editing sites are amplified and sequenced for C/U changes by primer extension. Next, the relevant region encompassing the editing site is amplified by RT-PCR from human tonsil B cell mRNA and DNA (cells in which AID has already acted on mRNA and/or DNA) and verified to have C/U changes by primer extension DNA sequencing. Finally, full length human cDNA(s) encoding edited mRNAs are expressed in hybridoma cells and activated splenic B cells from AID -/- mice to assess their ability to induce SHM and CSR in the absence of AID expression.

### 5. Example 5

### Identification of protein-protein and protein-RNA interactions

353. The homology of AID and CEM15 with APOBEC-1 suggested that ARPs functions as an RNA editing enzyme and/or DNA mutating enzymes through a multi-protein-containing editosome or mutasome. Both edited and unedited apoB mRNA co-purified with the APOBEC-1 editosome (Smith, Proc Natl Acad Sci U S A, (1991) 88(4):1489-93). RNA binding proteins (RBP) involved in apoB mRNA editing were first identified through ultraviolet light (UV) crosslinking of RNA-protein interactions in liver and intestinal cell extracts (Navaratnam Proc Natl Acad Sci U S A, 1993. 90(1): p. 222-6; Harris J. Biol. Chem., 1993. 268(10):7382-921; Smith Methods (1998)15(1):27-39). Subsequently, ACF was isolated and cloned using biochemical fractionation and yeast two hybrid genetic selection. Overexpression of 6His-tagged APOBEC-1 in mammalian cells enabled intracellular assembled editosomes to be affinity purified (Yang J. Biol. Chem (1997) 272(44):27700-6). These studies demonstrated four RBP (100 kDa, 66 kDa, 55 kDa and 44 kDa) in the affinity purified editosome. P100, p66 and p55 were mooring sequence selective RBP that remained bound to apoB mRNA even in the presence of a 100-fold molar excess of competitor RNA lacking a mooring sequence (Steinburg, Biochem Biophys Res Commun (1999) 263(1):81-6). P44 was more readily displaced in RNA excess competition analyses. P66 has been shown to be ACF (Blanc, RNA, 2002) and ACF pre-mRNA has been shown to code for multiple RBP (including the 44 kDa RBP) through alternative mRNA splicing (Dance, J. Biol. Chem. (2002) 277:12703-9). Novel edited mRNAs are identified using AID or CEM15 to affinity select editosomes (mutasomes) in which RBP are selectively bound to mRNAs or DNA (respectively) of interest.

### 6. Example 6

### Identification of edited mRNAs or mutated DNA sequences

### a) The cellular source of edited and unedited mRNAs or mutated DNAs

354. The high throughput bacterial genetic selection of this example represents the primary approach for identifying edited mRNAs (or mutated DNAs) and determining the nucleotides that are edited and has been modified from that described by Faham et al. (2001). The bacterial selection system relies upon the high specificity and sensitivity of the *E. coli* DNA mismatch repair. The system is adapted from an approach developed to localize DNA variations associated with human disease susceptibility alleles. C→U mRNA editing events (but also A→I if present) generates single nucleotide mismatches within in vitro constructed heteroduplexes whose complementary strands are derived from cDNAs amplified from mRNA that has been isolated from cells that either do or do not express AID (see Figure 9). Consequently, the cellular context from which the mRNAs are isolated is an important consideration. The single nucleotide polymorphism between individual mice of the same strain are significant and therefore can cause high backgrounds in the DNA mismatch selection systems. NIH3T3 fibroblasts serve as the cell context (rather than AID-/- mouse splenic cells) because they have been shown to support CSR upon transfection with AID expression vectors (Okazaki, Nature (2002) 416(6876):340-5) (CEM15 edited mRNAs or mutated DNAs can be evaluated in 293T human cells infected with pseudotyped virus using the same experimental strategy.)

### b) Selection of AID edited mRNAs (or mutated DNA) by E. coli mismatch repair and Cre recombinase

355. AID (or other ARP) editing target sites are identified as outlined in Figure 8. Double-stranded cDNA are synthesized and PCR amplified (SMART PCR cDNA synthesis kit; Clontech) from mRNA isolated from wild type NIH3T3 cells and from transfected NIH3T3 cells that have expressed AID for 48-72 h (a time period in which CSR was observed on an artificial switch construct. Control experiments with APOBEC-1 have shown that mRNA expression begins from the transgene within 6 h and continues linearly for 48 h. The two separate double stranded cDNA pools are digested with DpnII to generate approximately 300 bp fragments with GATC overhangs. cDNAs from wild type NIH3T3 cells are cloned into BamHI digested (GATC overhang) Cre expression vector (pCre100), transformed into dam minus *E. coli* and unmethylated, single-stranded DNA isolated using helper phage M13K07 (New England Biolabs), according to the manufacturer's recommendations. The pool of cDNA fragments prepared from RNA isolated from AID-transfected NIH3T3 cells are methylated using TaqI methylase (NEB) and then combined with BamHI linearized, methylated pCre200 (identical to pCre100 except for an inactivating 5 bp deletion within the Cre recombinase gene). The resultant methylated, Cre-deficient, edited cDNA pool is combined with the single-stranded, unmethylated, active-Cre+, unedited cDNA library, denatured and then reannealed to form heteroduplexes. Taq DNA ligase (NEB) is used to form closed circles of hemi-methylated heteroduplexes. Addition of exonuclease III converts DNA that has not been closed with Taq ligase to single stranded DNA, which is then removed. The heteroduplex mixture is transformed into an electrocompetent *E. coli* strain (Editing Site Identifier; ESI) engineered to carry on its episome (F' factor) a tetracycline resistance gene flanked by two lox sites. The heteroduplex mixture contains: (i) perfect cDNA homoduplexes from mRNAs that are not AID substrates from the two cell sources (not shown) and (ii) four different possible cDNA duplexes resulting from AID mRNA or DNA substrates in their unedited (homoduplex) and edited/mutated (heteroduplex) forms (shown). These appear in Fig. 8 as two homoduplexes with C:G and G:C base pairs at the editing site and two heteroduplexes with mismatched base pairs at the editing site corresponding to A:C and T:G.
356. The genetic selection within the ESI strain then proceeds as follows. Heteroduplex molecules carrying no mismatch (i.e., identical or unedited cDNAs) replicate normally and both plasmids carrying the active and inactive Cre recombinase are present. The Cre protein expressed from the wild type allele (purple circles) recombines the F' cassette between the two lox sites leading to the loss of the tetracycline resistance gene rendering the cell tetracycline sensitive and unable to grow. However, the presence of a mismatch in the heteroduplex molecule leads to the repair of such a mismatch. In the repair process, the unmethylated strand carrying the active Cre gene (and the unedited base) is degraded and the strand carrying the inactive Cre (and the edited base) is used as a template to be copied. As a result, the cell transformed with a heteroduplex becomes devoid of a functional Cre gene (through DNA repair), permitting the cell to retain tetracycline resistance and grow. These bacteria will only harbor plasmid encoding the DpnII cDNA fragment corresponding to edited transcripts. Once these clones (plasmids) are isolated and sequenced, the identity of the cDNA will be compared, to EST (Expressed Sequence Tag) and genomic DNA databases, and the location of the edited base will be apparent as a C/U polymorphism.
357. The mismatch repair detection method was selected after an extensive search of techniques for detecting single nucleotide polymorphisms (SNPs). The bacterial selection system involves a robust biological selection for edited sequences, does not rely upon knowledge of the editing mechanism or edited sequences a priori and has the capacity for high throughput.
358. The selected clones are DpnII restriction fragments from cDNAs of edited mRNAs. The number of different edited mRNAs (or mutated DNAs), their relative expression level, the number of editing sites per mRNA (or genome) and the efficiency of AID's (or other ARP's) editing/mutating activity are variables that can affect the number of positive clones. Given the precedent of APOBEC-1 having few known mRNA substrates, only a limited number of mRNAs or mutated DNAs are edited by AID in NIH3T3 cells (and other ARPs in relevant cell context) and therefore only a very few clones are selected.
359. To test for the possibility that a large number of clones is due to a high background, heteroduplexes are formed from wild type NIH3T3 cells alone and processed through the bacterial mismatched detection system. This yields a low number or no clones. If a high background is observed then an NIH3T3 line can be cloned and re-tested. A high background can also be due to inefficient mismatch repair activity and/or the failure to express sufficient Cre recombinase. This potential problem can be avoided by utilizing a new generation of plasmids that express higher levels of Cre. The APOBEC-1 editing system serves as a control for the selection of true positives and to assess the background in the system. The human liver cell line HepG2 is used because it does not express APOBEC-1 and hence no editing of the endogenous apoB mRNA occurs. An APOBEC-1 overexpressing HepG2 cell line edits approximately 50% of its apoB mRNAs and is used as a source of edited mRNAs. cDNAs synthesized from RNA isolated from these two cell lines are prepared, heteroduplexed and analyzed in the mismatch selection system as described in Figure 8. The control selection contains clones representing the primary (cytidine 6666) and secondary (cytidine 6802) apoB mRNA editing sites and known promiscuous editing sites (Sowden, Nuc. Acid Res. (1998) 26(7):1644-52). Few or no unedited apoB cDNAs corresponding to the same sites or cDNAs encoding other mRNAs exist. There also exists commercially available systems for selecting heteroduplex single base mismatches (e.g. MutS). The MutS protein binds to base mismatches with high affinity and when coupled to paramagnetic beads (GeneCheck, Fort Collins, CO) can be used to select for mismatched heteroduplexes from cDNAs prepared from NIH3T3 in which AID is or is not expressed.
360. As described above, mRNA is harvested for NIH3T3 cells 48-72 h following transfection with AID. AID expressed in NIH3T3 cells has a V5 epitope tag so that the level of expression of full length protein can be assessed by western blotting of whole cell protein lysates. APOBEC-1 expression kinetics demonstrated that high levels of editing occurred within 48h. The detection of edited mRNAs in the bacterial selection system does not require that all the mRNA molecules of a given type be edited because positive clones are selected for growth and edited cDNA is identified from literally thousands of cfu plated onto selection media.
361. If the bacterial selection system does not yield positive clones, higher levels of editing activity or greater transfection efficiencies can be necessary. An APOBEC-1-GFP chimera retained editing activity (Siddiqui, Exp Cell Res 252:154) and GFP-AID has been shown to induce SHM in Ramos cells (Rada, Proc. Nat1 Acad Sci 99(10):7003-5). Fluorescence activated cell sorting (FACS) distinguished transfected from non-transfected cells, yielding cell populations with distinct levels of APOBEC-1-GFP or AID-GFP expression with corresponding levels of editing activity or SHM rate (respectively). A sufficient number of cells transiently expressing a high level of AID-GFP can be isolated by FACS from which to make RNA.
362. AID and other ARPs can also be overexpressed in NIH3T3 cells (or other appropriate cell contexts). Overexpression of proteins carries the risk that the expression level can exceed the capacity of cells to regulate the protein's activity and subcellular distribution. Studies in apoB mRNA editing demonstrated that APOBEC-1 and ACF assumed a normal cellular distribution even at the highest levels of expression tested, but that editing activity was hyperactive (Yang, J. Biol. Chem. (2000) 275:22663-9). High levels of APOBEC-1 expression can lead to promiscuous editing of additional sites within apoB mRNA (Sowden, Nuc Acids Res 26:1644; Sowden, J. Biol Chem. 271(6):3011-17) and of other mRNAs (Yamananka, J. Biol. Chem. 271:11506-10). Although this can occur when AID is expressed, the data from studies with APOBEC-1 show that even the promiscuous editing sites were mooring sequence dependent and that the wild type editing site was always utilized with greater efficiency than the promiscuous sites. If promiscuous AID editing occurs, the correct site (the biologically relevant one) is more frequently represented in selected clones than the promiscuous sites.

### 7. Example 7

### Isolation and characterization of edited mRNA(s)

363. Candidate edited mRNAs are isolated from affinity purified editosomes assembled in NIH3T3 cells expressing 6His-tagged AID (or other similarly tagged ARPs in appropriate cell contexts). Editosome-associated RNAs are evaluated for AID editing. The AID editosome affinity approach for isolating candidate edited mRNAs has been selected because it requires no prior knowledge of which RNA binding protein (RBP) complements AID editing activity and is based only on the assumption that AID must interact (directly or through an RBP) with mRNAs to carry out site-specific editing. Candidate mRNAs isolated through AID affinity purification are compared to those isolated directly in Example 6.
364. Expression of 6His tagged APOBEC-1 in hepatoma cells stimulated apoB mRNA editing through the assembly of functional editosomes on apoB transcripts (Yang, J. Biol Chem (1997) 272:27700). APOBEC-1 editing is a nuclear event but proteins involved in editing were distributed throughout the cell and were bound to substrate mRNA in both compartments of the cell when APOBEC-1 was overexpressed. Interestingly, AID-GFP induced SHM in transfected Ramos cells but was predominantly found in the cytoplasm (Rada,Proc Natl Acad Sci (2002) 99(10):7003-7008). AID shuttles between cellular compartments, explaining the dichotomy that SHM must occur in the nucleus yet AID appeared to be cytosolic.
365. Extracts are prepared using a hypotonic cell lysis method, followed by nonionic detergent disruption of membranes, addition of KCl to 300 mM and clearing of particulate material by centrifugation at 100,000 x g, 20 min. This protocol has been used with several cell types to produce a combined nuclear and cytoplasmic S100 extract that is competent for *in vitro* editosome assembly and apoB mRNA editing (Yang, J. Biol. Chem (1997)). S100 extracts have been used to nickel affinity purify editosomes through 6His tagged APOBEC-1. This approach also enabled the co-purification and characterization of ACF and the characterization of APOBEC-1 homodimers (Lau, Proc Natl Acad Sci (1994) 91:8522-26). An S100 extract from 6His-tagged, AID expressing NIH3T3 cells is used as a source of affinity purified editosomal mRNA for RT-PCR amplification of cDNAs.
366. The published protocol for isolating editosomes assembled on 6His-tagged APOBEC-1 is followed (Yang, J. Biol. Chem (1997). Whole cell extracts are prepared from transient or stable AID transfected NIH3T3 cells (as described in Example 6) and bound to nickel resin (NTA resin, Qiagen) for one hour. Bulk protein and nonspecific protein interactions with the column are removed by sequential washes with copious volumes of phosphate buffered saline (PBS), PBS containing 0.4% Triton X100, PBS containing 300 mM KC1 and PBS containing 20 mM imidazole. The editosome is eluted with 300 mM imidazole and extracted with TriReagent (MRC, Inc) to liberate the associated mRNA(s). Oligo dT primer cDNA is synthesized and if specific mRNA sequences have been identified as candidate editing substrates from studies in Example 6, then appropriate primer pairs will be used to RT-PCR amplify a region corresponding to the editing site. Poisoned primer extension is used to determine the occurrence of edited mRNA. The analysis therefore provides confirmatory information. Alternatively, the mRNAs extracted from AID-affinity purified editosomes can be used to synthesize double stranded DNA, heteroduplexed to control NIH3T3 cDNA and selected for edited nucleotides as described in Figure 8.
367. Alternatively, yeast two hybrid (Y2H) selection strategy can be used, based on the hypothesis that AID (or other ARP) editing/mutational activity requires an RBP (or DNA binding protein) editing/mutation site interaction. Y2H selection has been successful used to identify RBP for APOBEC-1 (Blanc, J. Biol. Chem. 276:46386; Lellek, J. Biol. Chem. 275(26):19848-56). It is a positive selection system based on the affinity of AID for a yeast clone expressing the cDNA encoding a cognate RBP. It has established criteria for selecting and verifying stable interactions which provide both the selectivity and sensitivity required for identifying AID-RBP interactions. Y2H selection is however an indirect approach for identifying edited mRNAs and requires five steps: identifying proteins that interact with AID, selecting those that are RNA binding proteins, using the RBPs to affinity select mRNAs isolated from NIH3T3 cells +/- AID expression and then applying the analytical system described in Example 6 to validate substrate mRNAs. The advantage of this approach is that once RBP have been identified they can be combined with mRNA substrates at significantly higher concentrations than can be achieved in cells, thereby shifting the equilibrium in favor of association. Although this increases the potential for nonspecific interactions, RBP mRNA binding is carried out in the presence of tRNA as a competitor for nonspecific interactions.
368. AID serves as 'bait' in the MatchMaker two hybrid system (Clontech) and the cognate RBP ('prey') will be expressed from a mouse spleen cDNA library (Clontech). In this selection system, robust growth of yeast via histidine prototrophy and *lacZ* reporter gene expression (blue colonies) is dependent on the activation of transcription through the interaction of the bait DNA binding domain gene fusion with a prey transactivating domain gene fusion. These exist as AID-fusion proteins and proteins expressed from the cDNA library respectively, and can only activate transcription if there is a stable interaction between the AID and its cognate RBP. The expression of full length epitope-tagged AID in the yeast strain expressing the bait plasmid is confirmed by western blotting. Additionally, the inability of AID alone to activate transcription will be evaluated as an important negative control.
369. The MatchMaker system includes specific protocols for setting up the yeast two hybrid selection, for verifying true His+, *LacZ*+ transformants and ruling out false positives. Both the selection scheme and verification of true positives follow the manufacturer's recommendations using cDNAs encoding APOBEC-and ACF (Figure 9). Success with this system in selecting appropriate interactions is evident as robust growth under his- selection (left) and appearance of blue colonies on filter 'lifts' (right) for APOBEC-1 interaction as homodimers and heterodimers with ACF. The positive control (p53 binds to SV40T antigen) and negative control (lamin C does not bind to APOBEC-1) confirmed the stringency of the selection system. It appears that if AID interacts with an RBP, it is possible to select for these interactions through the yeast two hybrid system. A mouse spleen cDNA library has been obtained for the MatchMaker system. As AID can activate SHM in fibroblasts (Okazaki, Nature (2002) 416(6878):921-6), the RBP of interest is broadly and constitutively expressed and therefore if no cDNAs are isolated from spleen libraries, then a fibroblast library can be evaluated.
370. Once candidate RBPs for AID have been selected by yeast two hybrid analysis and verified for their affinity for AID, their cDNAs are isolated from yeast, amplified through *E. coli* using protocols provided by Clontech, and sequenced. The cDNA's identity is established through DNA database BLAST search analysis. A variety of protein motifs serve as RNA binding domains. These are identified as a routine feature of Genbank and SwissProt databases searches and are readily apparent if they occur in the selected RBPs for AID.
371. mRNA isolated from AID transfected NIH3T3 cells is bound to all candidate RBPs for 1 hour at 30°C in editosome assembly buffer containing an RNase inhibitor (Promega) as described for the assembly of apoB RNA-ACF complexes (Harris, Biochem Biophys Res Commun 183(2):899-903) and then slowly filtered through nitrocellulose. Nondenatured RNAs are only retained by the nitrocellulose filter if they are bound to protein (Economidis, Proc Natl Acad Sci (1983) 80(14):4296-300). Non-specific, low affinity interactions in this assay are blocked by the inclusion of 100-fold mass excess of yeast tRNA. The filter binding assay (commercially available acetylated bovine serum albumin) is used as a non-binding, negative control protein and recombinant ACF as a positive control for the amount of RNA that is expected from a *bona fide* interaction with hepatocyte mRNA or *in vitro* apoB transcript. RNA retained on the filter by ACF (or RBPs selected through AID affinity) is eluted in TriReagent and analyzed for edited mRNA as described in Example 6.
372. RBPs bind to only a few unique mRNA sequences and therefore the bulk of the mRNA flow through the nitrocellulose filter. The amount of mRNA retained on the filter by RBPs falls between the baseline established with BSA and a significant signal seen from ACF interaction with apoB mRNA. There is a low recovery for RBPs whose cognate mRNAs are of low abundance in total cellular mRNA.

### 8. Example 8

### Validation that candidate editing substrates are edited by AID

### a) Verification that Candidate mRNAs support C→U Editing in AID Expressing NIH3T3 Cells

373. The bacterial mismatch detection system has selected *Dpn*II fragments of cDNAs that contained heteroduplex mismatches. Those C/U polymorphisms that are due to AID mRNA editing and not genomic polymorphism are confirmed by comparing the sequence of the selected fragments to the mouse and human genomic and EST sequence databases. The presumption that these C/U polymorphisms are due to AID-specific mRNA editing is validated by expressing the unedited mRNA candidate in NIH3T3 cells that either express AID or do not (a negative control for nonspecific base modifications). RNA is isolated and RT-PCR amplified using cDNA- and vector-specific primers. Editing of the target C is determined by 'poisoned' primer extension sequencing of the RT-PCR products and comparing the results obtained from NIH3T3 cells that either express AID or do not. This method uses reverse transcriptase to extend a -end-labeled primer (that anneals to the PCR product downstream and proximal to the editing site) with dATP, dCTP, TTP and ddGTP. C→U changes result in different length primer extension products that can be resolved by P.A.G.E. and quantified by phosphorimager scanning densitometry. This method is widely used for detecting edited nucleotides due to its high specificity, sensitivity and linearity (Smith, H.C. Methods (1998) 15(1):27-39).

### b) Editing of Candidate mRNAs in Human B Lymphocytes

374. The next step in verification is to determine whether the identified mRNA(s) is edited in human B cells that are undergoing CSR and SHM. Purified human tonsil B lymphocytes is isolated and then fluorescence- activated cell sorted (FACS) into populations of naïve, germinal center, and memory B cells using the cell surface markers IgD, CD38, and CD19, respectively (Hu, J Immunol (1997) 159(3):1068-71). The editing site within the mRNA(s) of interest is amplified by RT-PCR from oligo dT-primed first strand cDNA synthesized from RNA isolated from the B cell subpopulations. Primers specific for the mRNA of interest are designed to amplify a PCR 400-500 bp product that encompasses the editing site (modeled after the apoB editing analysis). The poisoned primer extension assay is used to determine the proportion of PCR products that contained the edited nucleotide.
375. It is not possible to predict what proportion of the mRNAs of a given sequence will be edited (i.e., the editing efficiency) as this depends on the expression level of AID and other regulatory factors (Yang, J. Biol. Chem. (2002) 275(30):22663-9). The poisoned primer extension assay has a detection limit of 0.3% edited mRNA (Sowden, Nuc Acids Res (1999) 26(7):1644-52) and therefore even low levels of editing can be detected. Edited transcripts are only be detected in the IgD-CD38+, CD19+ germinal center B cells. The poisoned primer extension data from mRNAs isolated from naïve B lymphocytes serves as an important negative control for mRNA modification, and is important for establishing the background at the predicted editing, which can be due to very low levels of dGTP contamination of some commercially available deoxyribonucleotide stocks.
376. To further evaluate the induction of editing on select mRNAs in human B cells, CSR and SHM are induced and editing of select mRNAs determined as described above. Human naïve peripheral blood and tonsil B cells is activated *in vitro* by culturing with CD40 ligand-transfected fibroblasts in the presence of IL-4, which activates AID expression and SHM. Transcripts expressed by pre- and post-activated B cells is compared for editing, as described above.

### c) Induction of CSR and SHM Through the Expression of Edited mRNAs

377. The consequence of C→U editing for protein expression is determined through sequence analysis for missense and nonsense mutations. Amino acids substitutions due to codon sense changes or protein truncation due to editing of a sense codon to a translation stop codon (nonsense) are apparent. Less certain is whether the introduction of a stop codon will induce mRNA degradation known as nonsense mediated decay (Hilleren, RNA (1999) 5(6):711-9) or alterations within exon splicing enhancers that could affect exon skipping (Liu, Nat. Genet. (2001) 27(1):55-8). Consequently, a variety of validation analyses involving protein expression, mRNA ablation and cDNA sequence analysis are required.
378. The Quickchange® mutagenesis system from Stratagene is used to mutate the C at the editing site to a T in full length cDNAs encoding the edited mRNAs. These 'pre-edited' cDNAs are expressed in the N89 and N114 mouse hybridoma lines and the ability of these cells to carry out SHM is determined. To evaluate the induction of SHM, a minor modification of the methods described in the literature (Martin, Nature (2002) 415(6873):802-6) is used wherein N89 and N114 mouse hybridoma lines, bearing early stop codons in the variable region segments of their heavy chain genes, revert to normal Ig production at detectable frequency upon expression of exogenous AID. Briefly, a retroviral system based on the pMIG vector (Van Parijs, Immunity (1999) 11(3):281-8) is used to express complete cDNAs encoding the edited candidate transcript in conjunction with a green fluorescent protein (GFP) marker gene in the N89 and N114 hybridomas. An AID-expressing pMIG vector is used as a positive control for SHM induction and transduction with pMIG containing the unedited cDNA serves as the negative control.
379. For retroviral transduction, hybridomas are cultured in 5 µg/ml polybrene-supplemented medium with virus-containing supernatant from the Phoenix packaging cell line (virus/cell multiplicity of 10:1), and cells analyzed for GFP expression by FACS at 48-72 hr. Retrovirus-infected hybridomas are sorted on the basis of GFP co-expression, and tested for IgM secretion after 2 weeks from infection by standard ELISA and ELISPOT assays. To confirm the presence of AID-induced mutations, individual transduced Ig-secreting subclones are isolated in some experiments, and their variable region segments amplified by PCR from genomic DNA with primers 5'TTACCTGGGTCTATGGCAGT3' (SEQ ID NO: 37) and 5'TGAAGGCTCAGAATCCCCC3' (SEQ ID NO: 38) 30 cycles at 95°C 15 s, 56°C 15s, 72°C 30s, using Pfu polymerase. PCR products from independent hybridoma subclones (at least 40/hybridoma) are cloned into a pBluescript plasmid and sequenced.
380. The ability of candidate AID substrates to complement switch function in AID-deficient B cells activated *in vitro*, in which class switch activity is blocked (Muramatsu, Cell (2000) 102(5):553-63) are also tested. Ig switching is induced in primary splenic B lymphocytes by culture in the presence of 20 µg/ml bacterial lipopolysaccharide (LPS), 10 µg/ml dextran sulfate for 5 days, and switching evaluated by flow cytometry and PCR-based assays, as previously described (Kuzin, J Immunol (2000) 164(3):1451-7). *In vitro* activated B cells from AID-deficient and control mice are transduced with AID- or candidate AID substrate-expressing retroviruses by supplementing the culture medium with 5 µg/ml polybrene and viral supernatants (10:1 1 multiplicity) at day 1.5 of culture. Under these conditions, >5% of B cells are transduced (GFP-positive by FACS at day 5 of culture). Cells are stained at day 5 for secondary Ig isotypes (IgG2b and IgG3) using phycoerythrin-labeled monoclonal antibodies (Pharmingen), and the expression of secondary isotypes in GFP-positive and -negative cells is evaluated by 2-color flow cytometry. Since normal LPS-stimulated B cells switch to IgG production at a rate of 10-20% by day 5, while AID-deficient cells are completely blocked (Muramatsu, Cell (2000) 102(5):553-63), detectable IgG expression in retrovirally-transduced, GFP-positive AID-deficient cells provide unequivocal evidence of complementation of the switch defect in these cells.
381. Direct molecular evidence of DNA recombination of Sµ-Sγ3 regions by CSR is obtained by a modified digestion-circularization PCR method (DC-PCR), already described in a prior publication (Kuzin, J Immunol (2000) 164(3):1451-7). Briefly, genomic DNA from target cells (in this case, sorted GFP-positive AID-deficient and control LPS-activated B cells) is cut with the XbaI restriction enzyme, and re-ligated in diluted conditions that favor re-circularization. PCR with primers flanking the re-ligation site, specific for regions upstream of Sµ and downstream of Sγ3, amplifies products in which the two S regions have been joined by CSR, while the non-rearranged, unlinked configurations are not circularized and do not yield any product.

### d) Evaluating the Role of Edited mRNAs in Gene Conversion

382. Edited mRNAs confirmed to mediate CSR and SHM in mouse B cells are ideal candidates for transfection into chicken DT40 AID^{-/-E} cells in which AID has been disrupted (Arakawa, Science (2000) 295(5558):1301-6). This cell line was derived from a DT40 variant that does not express sIgM. This allows sIgM reversion that is mediated by AID-induced Ig light chain gene conversion to be readily quantified. DT40 AID^{-/-E} cells and the positive control AID knock-in AID^{-/-R} cell line. The AID knock-in cell induces GC in this cell background. Induction of Ig light chain gene conversion by chicken substrate candidates is evaluated after transfection of pre-edited candidate cDNAs by analyzing sIgM reversion rates by FACS, as described (Arakawa, Science (2000) 295(5558):1301-6). The edited form rescues the AID^{-/-} phenotype with respect to gene conversion, whereas the unedited form does not. Revertant clones are sequenced to confirm the presence of gene conversion. Whenever possible, the chicken homolog to the mouse or human cDNA is identified, its editing site confirmed and used in the DT40 cell transfections.

### e) Results

383. The mRNA edited by AID can be identified and their ability to be edited in mouse and human B lymphocytes can reveal whether one or more mRNAs are edited at single or multiple sites each. Theoretically, C→U editing could occur anywhere along the length of pre-mRNA. Sowden, Biochem J (2001) 359:697-705 demonstrates that C→U mRNA editing is restricted to exon sequence. Editing in the 5' and 3' untranslated region of mRNAs has not been documented but modifications in this region could affect mRNA stability, mRNA 3' end formation. Editing within coding exons that are predicted to have a silent effect at the codon level could affect exon skipping (Liu, Nat Genet 27(1):55-8; Cartegni Nat Rev Genet (2002) 3(4):285-98).
384. There is a possibility that C→U editing could change a CAA or CGA codon to a translation stop codon (nonsense codon). If a stop codon is introduced >50 nucleotides of the terminal exon junction, referred to as a premature stop codon, cellular surveillance mechanisms identify the messages as aberrant and the mRNA is destroyed through a process known as nonsense mediated decay (NMD). Edited apoB mRNA (CAA→UAA occurs in the middle of the mRNA) does not undergo NMD in liver and in fact the protein encoded by edited mRNA is preferentially expressed and secreted (Greeve, J Lipid Res (1993) 34(8)1367-83). The CGA→UGA editing event in NF 1 mRNA also occurs within its coding region (Skuse, Nucleic Acids Res (1996) 24(3):478-85).
385. If edited mRNA is subjected to NMD, the encoded protein can become reduced in abundance as well. CSR and SHM are therefore induced in this case by the reduction of a specific protein. If premature stop codons are detected in edited mRNAs, alterations in their abundance is evaluated by RNase Protection Assay (RPA) using commercially available kits from Ambion. The mRNA's abundance in NIH3T3 cells expressing AID is compared to that measured on RNA from wild type NIH3T3 (normalized against the transcript of a house keeping gene). If the edited mRNA is less abundant than unedited mRNA, NMD is suggested. In this case experiments can be conducted for the ablation of the target mRNA in addition to overexpressing the protein from edited mRNA. The ablation of mRNA is induced through RNAi expression. RNAi vectors are the current technology of choice as mRNA ablation does not depend on the expression of RNase H nor the empirical positioning of antisense oligonucleotides along the target sequence (Paddison Genes Dev (2002) 16(8):948-58; Bernstein RNA (2001) 7(11):1509-21; Paddison Proc Natl Acad Sci (2002) 99:31443-8). The mammalian RNAi expression vector is constructed to express short targeting RNAs (shRNA) for the mRNA of interest. Ablation of the target mRNA is confirmed by RPA (using RNA from cells transfected with empty vector alone as a negative control). CSR and SHM end points are assessed in RNAi treated cells as described above.
386. Co-expression of multiple cDNAs is required if editing of more than one mRNA is necessary for any given function. Co-expression can be achieved by modified retroviral vectors or co-transfection experiments. The efficiency of each edited mRNA (or combinations thereof) to rescue the AID-/- phenotype in CSR, SHM and GC is determined relative to the findings with AID replacement.
387. In addition, an important proof that one or more edited mRNAs can induce CSR and SHM is their ability to rescue immune function in AID -/knockout mice, as well as specific targeted inactivation of the relevant genes. Suitable vectors containing the immunoglobulin 3' IgH enhancer elements able to drive restricted transgene expression in activated B cells are available in the Bottaro lab, which also has extensive experience with the generation of transgenic lines. An array of gene-targeting techniques can be used, including the RAG2-/- blastocyst complementation system, which allows rapid and efficient analysis of targeted mutations in mature lymphocytes.

### 9. Example 9

### Molecular identification of non-Ig gene AID targets in lymphomas.

388. A small number of oncogenes (c-myc, Pim1, Pax5, RhoH/TTF) have been found to bear hallmarks of SHM in human lymphoma samples. Additional important targets can exist whose mutation contributes to neoplastic development. In this experiment, a mutation screening method based on a genetic selection strategy that exploits bacterial DNA mismatch repair is used. This method has been used to identify single nucleotide polymorphism in human genomic DNA and has been modified herein.
389. These experiments take advantage of the mismatch repair detection (MRD) system, a novel, high-throughput bacterial positive genetic selection strategy for human disease related single nucleotide polymorphisms. In this example, the selection system is used as it was originally intended for screen mismatches in genomic DNA sequences.
390. Genomic DNA isolated from a non-B cell source (e.g. fibroblasts) and from lymphomas from AID-transgenic mice is digested with DpnII (average size ∼0.3kb) and cloned separately into two different plasmids. Unmethylated plasmids (grown in a dam methylase-deficient E. coli strain) containing the 'control' inserts (from normal tissue DNA) also encode an intact Cre recombinase, whereas the methylated plasmids contain putative mutated fragments from lymphoma cells and encode an inactive 5 nucleotide deletion mutant of Cre. Heteroduplexes formed in vitro between the two plasmid libraries by melting and reannealing are transformed into a bacterial strain that harbors an F' episome carrying a 'floxed' tetracycline resistance gene. Repair of the mismatch uses the methylated strand as template, resulting in loss of the functional Cre recombinase gene and retention of the 'flexed' tetracycline resistance gene. Non-mismatched heteroduplexes, instead, induce no repair, express functional Cre, and result in Tet^{R} LoxP-mediated deletion. The Tet^{R} clones obtained through the MRD process therefore contain exclusively fragments displaying sequence heterogeneity between the original samples, and are subject to further selection and identification steps.

### 10. Example 10

### CEM15

### a) Expression of proteins and the nucleoside/nucleotide deaminase assay

391. Wild type and mutant CEM15 can be expressed from cloned cDNAs in a coupled transcription-translation system (Promega's® TNT™). APOBEC-1 serves as a positive control; when translated in vitro it retains both deaminase activity as described below, and when added to a source of auxiliary factors, supports apoB mRNA editing (Muramatsu, M., J Biol Chem, (1999) 274(26): p. 18470-6). Deaminase activity of in vitro translated APOBEC-1 and CEM15 was determined in 25 mM Tris pH 7 with 1 mM nucleotide or nucleoside at 30°C followed by precipitation of the protein with 0.5 M perchloric acid (Neuhard, J J Bacteriol, 1968. 96(5): p. 1519-27). Deaminase activity can be monitored as the reduction in absorbance at 280 nm or 290 nm for C and dC or CMP and dCMP, respectively. CEM15 and APOBEC-1 deaminated 180 pmols and 25 pmols of CMP per hour, respectively. Assaying mutant CEM 15 in parallel with wild type determines the effects of mutations in CEM15 on deaminase activity. To ensure the addition to the assay of equivalent amounts of wild type and mutant forms of CEM15, the expression of each protein is determined from ³⁵S methionine incorporation calculations (normalizing for the number of methionines in each protein). Mutations that inhibit CEM15 nucleoside/nucleotide deaminase activity are, by analogy to APOBEC-1 predicted to inhibit CEM15's deaminase activities on DNA or RNA substrates as well. To address the effect of Vif on CEM15 deaminase activity, in vitro translated Vif is titrated into the assays. The molar ratios of Vif to CEM15 are determined by quantifying protein expression as described above.

### b) The role of CEM15 deaminase activity in HIV infectivity suppression and the ability of Vif to suppress deaminase activity in vivo

392. The inhibitory effect of CEM15 on the infectivity of vif+ and vif- HIV-1 particles by transient cotransfection of appropriate HIV-1 proviral DNA and CEM15 expression plasmids has been established (Sheehy Nature, (2002) 418: p. 646-650). A similar assay has been developed using VSV G-protein pseudotyped lentiviral particles that (1) confirmed this result and (2) is amenable to the rapid demarcation of the regions of HIV-1 DNA (or RNA) that is the target for CEM15 catalytic activity. Briefly, an Env-deleted HIV-1 proviral DNA vector (derived from pNL43; AIDs Reagent Repository) was modified by replacement of Nef with a GFP reporter gene and two in-frame stop codons were inserted that abolished vif production (pHR-GFPΔVif) (confirmed by western blotting with anti-Vif antibodies (AIDs Reagent Repository). Stable, HA-tagged CEM15 expressing 293T cell lines were selected with puromycin and verified by western blotting with a HA specific monoclonal antibody (HA.11; BabCo). The expression of similar levels of full-length HA-tagged CEM15 (or mutant derivative thereof) can be assayed in future stable cell lines. The addition of this epitope tag has no effect on the ability of CEM15 to suppress infectivity. Isogenic HIV-1 pro-viral DNAs are packaged into pseudotyped lentiviral particles by cotransfection with a plasmid encoding the VSV G-protein into 293T cells that lack endogenous CEM15 (-) or expressed wild type CEM15 (+). The resulting pseudotyped particles contain HIV-1 RNA of near full-length (with only a ∼2kb deletion) were quantitated by reverse transcriptase (RT) assay. p24Gag protein content can also be assayed by ELISA to normalize viral particles. A defined number (1x10⁵ cpm of RT activity) of these particles were added to target, virus susceptible MT2 cells (5x10⁵). To assess their infectivity, the percentage of cells that expressed the GFP indicator gene encoded by the packaged recombinant HIV-1 genome was quantified 24 hours later by flow cytometry (University of Rochester Core Facility). The results indicate that the expression of CEM15 in 293T cells resulted in at least a 100-fold decrease in Vif- viral infectivity compared to particles generated in parental 293T cells. The low level of GFP expression from vif-, CEM15+ particles is indistinguishable from background fluorescence in control cells [0.2%]. This assay can be extended to include Vif + proviral DNA controls and the use of deaminase inactivated CEM15 mutants in stable 293T cell lines. Most significantly however, the assay is amenable to the use of several existing HIV-1 proviral isotyped vectors that are deleted for different regions and different amounts of the HIV-1 genome. Deleted genes can be provided in trans by cotransfection of suitable expression plasmids. A recent comprehensive examination of viral proteins and host tRNA^{Lys3} derived fromvif- virions revealed no significant biochemical or priming defects.

### c) Determine the DNA and/or RNA substrate(s) for CEM15 and determine the effect of Vif expression on substrate utilization

393. Evaluate the ability of CEM15 to deaminate dC on HIV-1 DNA substrates using an Apyrimidinic Endonuclease (APE) DNA cleavage assay in which apyrimidinic sites are created by DNA Uracil N-Glycosylase (UNG) activity at sites of dC to dU mutation (Tom, S., J Biol Chem, (2001) 276(52): p. 48781-9). By analogy to APOBEC-1 and AID, CEM15 can exhibit activity on ssDNA substrates. ssDNA substrates corresponding to either strand of the HIV-1 genome is prepared by asymmetric PCR using ³²P end labeled primers from a series of plasmids containing overlapping fragments (derived from pBRU3; of the regions of HIV-1 DNA that respond to CEM15 inhibition). Purified ssDNA is treated with in vitro translated wild type or mutant CEM15 and repurified. An unlabelled complementary asymmetric PCR product (derived from the same plasmid) is annealed and the duplex exposed to recombinant UNG (NEB, MA) and APE (Novus Biologicals, CO) according to the manufacturer's recommendations. 5' end-labeled cleavage products are analyzed on 6% polyacrylamide gels by comparison to a DNA sequencing ladder generated from the same parental plasmid and primer used for asymmetric PCR. Specific cleavage sites are determined by comparison to untreated or CEM15 deaminase mutant treated ssDNA controls. The ability of Vif to block CEM15 DNA modifications is tested by titration of Vif into the reactions at known molar ratios to CEM15. Confirmed CEM15 deaminase-dependent sites are evaluated for their effect on infectivity by creating dC to dT mutations in HIV-1 proviral DNAs at these site(s).
394. Alternatively, the more conventional APE assay can be employed on dsDNA substrates. Briefly DNAs digested from the plasmid series described above are ³²P end-labeled using T4 polynucleotide kinase and incubated with CEM15. Purified DNAs is treated with recombinant UNG and APE and 5' end-labeled cleavage products analyzed by agarose gel electrophoresis and by comparison to untreated DNA controls. Cleaved fragments are isolated, 3' A-tails added by Taq DNA polymerase and inserted into a TA cloning plasmid (Invitrogen, CA). Sequencing of the junctions at the cloning sites identifies the ends of fragment(s), thereby locating the site of dC to dU modification in the HIV-1 DNA. If CEM15 requires auxiliary proteins (like APOBEC-1 for apoB mRNA editing), cellular extracts that provide auxiliary protein(s) can be added to the DNA cleavage assay. Their source is 293T cells or derivatives that express high levels of CEM15. The APE assay is specific to DNA substrates; consequently, analysis of CEM15-mediated editing/modification events on HIV-1 genomic RNA can be assessed via a high-throughput screening assay.
395. It can be determined whether tRNA^{lys3} C to U is edited by CEM15 in vitro and map the sites of modification by the poisoned primer extension analysis established for quantifying apoB mRNA editing. tRNA^{lys3} is transcribed in vitro (MEGAshortscript, Ambion) purified, boiled and renatured. An aliquot of CEM15 known to support in vitro deamination (and a mutant thereof as control) is added to twenty fmols tRNA^{lys3} in editing buffer (10 mM Hepes pH 8, 10% glycerol, 50 mM KCl, 30 mM EDTA and 0.25 mM DTT; or its optimized derivative) at 30 °C for 1-3 h [96]. C to U editing of tRNA^{lys3} purified from the reaction is determined using ³²P end labeled deoxyoligonucleotide primers complementary to sequences of tRNA^{lys3} immediately 3' of C residues in separate poisoned primer extension assays. Primer extension products are resolved by 12% PAGE and quantified by Phosphorimager analysis. The ability of Vif to block tRNA^{lys3} editing is determined by titration of Vif into the editing assay.
396. tRNAs are highly modified and it is conceivable that CEM15 dependent deamination relies on a pre-existing modification of tRNA^{lys3} The in vitro editing assay can also be performed on purified human tRNA^{lys3} (BioS&T, Canada) that contains all appropriate modifications. Many of these modifications cause reverse transcriptase to stall, thereby precluding the RT-PCR amplification of tRNA^{lys3} from CEM15 transfected cells and sequencing of the products to identify sites of C to U conversion.

### d) Analysis of CEM15-mediated modification of HIV-1 genomic RNA and DNA

397. Initial screens target HIV-1 genomic RNA and HIV-1 dsDNA since their modification most likely explains CEM15's inhibition of viral replication. The following HIV-1 genomic RNA species are isolated from cell-free pseudotyped virions produced in the 293T/CEM15 transfection system: vif-minus genomes generated in the absence (A) or presence (B) of CEM15, (representing unmodified or modified HIV-1 genomic RNA respectively) and (C) vif+ genomes generated in the presence of CEM15 (a control also representing unmodified HIV-1 genomes). Full-length cDNAs are synthesized using SuperScript™ III RT (Invitrogen®) and modifications to maximize first-strand synthesis fidelity followed by PCR amplification using high fidelity Taq DNA polymerase (Roche, IN) and assayed in the mismatch repair screen. Viral reverse transcripts, stimulated by addition of dNTPs and physiologic polyamine are isolated from pseudotyped particles generated from the above transfection scenarios and assayed in the mismatch repair screen. To analyze CEM15 dependent modification of HIV-1 proviral DNA extrachromosomal (Hirt) DNA extracts are prepared 48 hours post DNA transfection (following scenarios A, B and C above), overlapping 2-4 kb fragments of HIV-1 DNA amplified by PCR and then assayed by the mismatch repair screen. The high throughput bacterial DNA repair screen: DNAs (or cDNAs) prepared from the transfections described above are digested with DpnII (average size -0.3kb) and cloned separately into two different plasmids. Unmethylated plasmids containing the control (A or C) inserts encode an intact Cre recombinase whereas the methylated plasmids containing methylated putative CEM15 modified (dC to dU in DNA or C to U in RNA inserts (B) encode an inactive 5 nucleotide deletion mutant of Cre. Heteroduplexes formed in vitro between the two plasmid libraries by melting and reannealing are transformed into a bacterial strain that harbors an F' episome carrying a 'floxed' tetracycline resistance gene. Repair of the mismatch to the strand with the modified base results in retention of the plasmid borne inactive Cre recombinase and the 'floxed' tetracycline resistance gene is retained and expressed. Non-mismatched heteroduplexes express functional Cre and Tet^{R} is lost. HIV-1 DNA inserts from resulting clones are sequenced and compared to the wild type viral DNA.
398. Error-prone HIV-1 replication generates approximately 0.3 - 1 mutation per genome, distributed randomly, per replication cycle. CEM15-induced mutations are largely site-specific. Statistical analysis of the number of site-specific dC to dU (or dG to dA) changes observed identifies sites of CEM15 dependent modification. CEM15 could block viral integration by recruitment of CEM15 not to a specific sequence, but to a specific DNA conformation or structure (e.g. the unique structure formed during viral DNA integration).
399. HIV genomic RNA, proviral DNA (dsDNA) and host cell mRNA and genomic DNA can also be analyzed for CEM15-dependent modifications. This experiment exploits a high throughput, bacterial positive genetic selection strategy for human disease related SNPs.

### J. References

Abad JL, Serrano F, San Roman AL, Delgado R, Bernad A, Gonzalez MA. Single-step, multiple retroviral transduction of human T cells. J Gene Med4: 27-37 (2002).
Alberts, B., D. Bray, J. Lewis, M. Raff, K. Roberts and J.D. Watson Molecular Biology of the Cell. (3rd ed.) Garland Pub. Inc. NY, NY (1994).
Alizadeh AA, Eisen MB, Davis RE, Ma C, Lossos IS, Rosenwald A, Boldrick JC, Sabet H, Tran T, Yu X, Powell JI, Yang L, Marti GE, Moore T, Hudson JJ, Lu L, Lewis DB, Tibshirani R, Sherlock G, Chan WC, Greiner TC, Weisenburger DD, Armitage JO, Warnke R, Levy R, Wilson W, Grever MR, Byrd JC, Botstein D, Brown PO, Staudt LM. Distinct types of diffuse large B-cell lymphoma identified by gene expression profiling. Nature403:503-511 (2000).
Alt FW, Oltz EM, Young F, Gorman J, Taccioli G, Chen. VDJ recombination. Immunol. Today13: 306-314 (1992)
Anant, S. and Davidson, N.O. (2000) An AU-rich sequence element (UUUN[A/U]U) downstream of the edited C in apolipoprotein B mRNa is a high affinity binding site for APOBEC-1: binding of APOBEC-1 to this motif in the 3' untranslated region of c-myc increase mRNA stability. Mol Cell. Biol. 20:1982-(1992).
Anant, S., MacGinnitie, A.J. and Davidson, N.O. APOBEC-1, the catalytic subunit of the mammalian apoB B mRNA editing enzyme, is a novel RNA-binding protein. J. Biol. Chem. 270:14762-14767 (1995).
Anant, S. G., Giannoni, F., Antic, D., DeMaria, C. T., Keene, J. D., Brewer, G. and Davidson, N. O. AU-rich RNA binding proteins Hel-N1 and AUF1 bind apolipoprotein B mRNA and inhibit posttranscriptional C to U editing. Nucleic Acids Symp. Ser. 36, 115-118 (1997).
Anant, S., et al. Evolutionary origins of the mammalian apolipoprotein B RNA editing enzyme, apobec-1: structural homology inferred from analysis of a cloned chicken small intestinal cytidine deaminase. Biol Chem. 379:1075-81 (1998).
Anant S, Davidson NO. 2000. An AU-rich sequence element (UUUN[A/U]U) downstream of the edited C in apolipoprotein B mRNA is a high-affinity binding site for Apobec-1: binding of Apobec-1 to this motif in the 3' untranslated region of c-myc increases mRNA stability. Mol. Cell. Biol. 20:1982-1992 (2000)
Anant, S., et al. ARCD-1, an apobec-1-related cytidine deaminase, exerts a dominant negative effect on C to U RNA editing. Am J Physiol Cell Physiol. 281:C1904-16 (2001).
Anant, S., Mukhopadhyay, D., Hirano, K.-I., Brasitus, T.A. and Davidson, N.O. APOBEC-1 transcription in rat colon cancer: dereased apobec-1 protein production through alterations in polysome distribution and mRNA translation associated with upstream AUGs. Biochim. Biophys Acta 1571:54-62 (2002).
Andersson, T., C. Furebring, C.A. Borrebaeckand S. Pettersson, Temporal expression of a V(H) promoter-Cmu transgene linked to the IgH HS1,2 enhancer. Mol Immunol. 36(1):19-29 (1999).
Arakawa, H., J. Hauschildand J.M. Buerstedde, Requirement of the activation-induced deaminase (AID) gene for immunoglobulin gene conversion. Science. 295(5558):1301-6 (2002).
Arulampalam, V., C. Furebring, A. Samuelsson, U. Lendahl, C. Borrebaeck, I. Lundkvistand S. Pettersson, Elevated expression levels of an Ig transgene in mice links the IgH 3' enhancer to the regulation of IgH expression. Int Immunol 8(7):1149-57 (1996).
Bachl J, Wab1 M. Enhancers of hypermutation. Immunogenet. 45: 59-64 (1996).
Bachl J, Olsson C, Chitkara N, Wab1 M. The Ig mutator is dependent on the presence, position, and orientation of the large intron enhancer. Proc Natl Acad Sci U S A 95: 2396-2399 (1998).
Bachl J, Olsson C. Hypermutation targets a green fluorescent protein-encoding transgene in the presence of immunoglobulin enhancers. Eur. J. Immunol. 29: 1383-1389 (1999).
Bachl J, Carlson C, Gray-Schopfer V, Dessing M, Olsson C. Increased transcription levels induce higher mutation rates in a hypermutating cell line. J Immunol 166:5051-5057 (2001).
Backus, J.W. and Smith, H.C. Apolipoprotein B mRNA sequences 3' of the editing site are necessary and sufficient for editing and editosome assembly. Nucleic Acids Res. 19:6781-6786 (1991).
Backus, J.W. and Smith, H.C. Three distinct RNA sequence elements are required for efficient apoB RNA editing in vitro. Nucleic Acids Res. 22, 6007-6014 (1992).
Backus, J.W. and Smith, H.C. Specific 3' sequences flanking a minimal apoB mRNA editing 'cassette' are critical for efficient editing in vitro. Biochim. Biophys. Acta 1217, 65-73 (1994).
Backus, J.W., Schock, D. and Smith, H.C. Only cytidines 5' of the apoB mRNA mooring sequence are edited. Biochim. Biophys. Acta 1219:1-14 (1994).
Barchi JJ, Jr., Cooney DA, Hao Z, Weinberg ZH, Taft C, Marquez VE, Ford H, Jr. Improved synthesis of zebularine [1-(beta-D-ribofuranosyl)-dihydropyrimidin-2-one] nucleotides as inhibitors of human deoxycytidylate deaminase. J Enzyme Inhib 9:147-162 (1995).
Baum, C.L., Teng, B.B. and Davidson, N.O. Apolipoprotein B messenger RNA editing in the rat liver: modulation by fasting and refeeding a high carbohydrate diet. J. Biol. Chem. 265, 19263-19270 ( 1990).
Betts L, Xiang S, Short SA, Wolfenden R, Carter CW, Jr. Cytidine deaminase. The 2.3 A crystal structure of an enzyme: transition-state analog complex. J Mol Biol 235: 635-656 (1994).
Bernstein, E., A.M. Denliand G.J. Hannon, The rest is silence. RNA. 7(11):1509-21 (2001).
Betts L., Xiang S, Short SA, Wolfenden R, Carter CW Cytidine deaminase. The 2.3 A crystal structure of an enzyme: transition-state analog complex. J Mol Biol. 235:635-56 (1994).
Betz AG, Milstein C, Gonzalez-Fernandez A, Pannell R, Larson T, Neuberger MS. Elements regulating somatic hypermutation of an immunoglobulin kappa gene: critical role for the intron enhancer/matrix attachment region. Cell 77: 239-248 (1994).
Blanc. V., Navaratnam, N., Henderson, J.O., Anant, S., Kennedy, S., Jarmuz, A., Scott, J. and Davidson, N.O. Identification of GRY-RBP as an apo B mRNA binding protein that interacts with both apobec-1 and with apobec-1 complementation factor (ACF) to modulate C to U editing. J. Biol. Chem. 276, 10272-10283 (2001).
Blanc, V., et al. Mutagenesis of apobec-1 complementation factor reveals distinct domains that modulate RNA binding, protein-protein interaction with apobec-1, and complementation of C to U RNA-editing activity. J Biol Chem. 276:46386-93 (2001).
Blanc V, Davidson NO. C-to-U RNA editing: mechanisms leading to genetic diversity. J. Biol. Chem. 278: 1395-1398 (2003).
Bostrom, K., Garcia, Z., Poksay, K. S., Johnson, D. F., Lusis, A. J. and Innerarity, T. L. Apolipoprotein B mRNA editing. Direct determination of the edited base and occurrence in non-apolipoprotein B producing cell lines. J. Biol. Chem. 265, 22446-22452 (1990).
Bransteitter R, Pham P, Scharff MD, Goodman MF. Activation-induced cytidine deaminase deaminates deoxycytidine on single-stranded DNA but requires the action of RNase. Proc. Natl. Acad. Sci. USA 100:4102-4107 (2003).
Bronner CE, Baker SM, Morrison PT, Warren G, Smith LG, Lescoe MK, Kane M, Earabino C, Lipford J, Lindblom A, et al. Mutation in the DNA mismatch repair gene homologue hMLH1 is associated with hereditary non-polyposis colon cancer. Nature 368: 258-261 (1994).
Bross, L., M. Muramatsu, K. Kinoshita, T. Honjoand H. Jacobs, DNA Double-Strand Breaks: Prior to but not Sufficient in Targeting Hypermutation. J Exp Med. 195(9):1187-1192 (2002).
Burley, S.K. An overview of structural genomics. Nature Struct. Biol. 7:932-934 (2000).
Cartegni, L., S.L. Chewand A.R. Krainer, Listening to silence and understanding nonsense: exonic mutations that affect splicing. Nat Rev Genet 3(4):285-98 (2002).
Casellas, R., A. Nussenzweig, R. Wuerffel, R. Pelanda, A. Reichlin, H. Suh, X.F. Qin, E. Besmer, A. Kenter, K. Rajewskyand M.C. Nussenzweig, Ku80 is required for immunoglobulin isotype switching. Embo J. 17(8):2404-11 (1998).
Chaudhuri J, Tian M, Khuong C, Chua K, Pinaud E, Alt FW. Transcription-targeted DNA deamination by the AID antibody diversification enzyme. Nature 422: 726-730 (2003).
Chen, S.H., Habib, G., Yang, C.Y., Gu, Z.W., Lee, BR., Weng, S.A., Silberman, S.R., Cai, S.J., Deslypere, J.P., Rosseneu, M., Gotto, A.M.J.R., Li, W.H. and Chan, L. Apolipoprotein B-48 is the product of a messenger RNA with an organ-specific in-frame stop codon. Science 238:363-366(1987).
Chen, J., R. Lansford, V. Stewart, F. Youngand F.W. Alt, RAG-2-deficient blastocyst complementation: an assay of gene function in lymphocyte development. Proc Natl Acad Sci USA 90(10):4528-32(1993.)
Cho DS, Yang W, Lee JT, Shiekhattar R, Murray JM, Nishikura K. Requirement of dimerization for RNA editing activity of adenosine deaminases acting on RNA. J Biol Chem 278: 17093-17102 (2003).
Chua, K.F., F.W. Altand J.P. Manis, The Function of AID in Somatic Mutation and Class Switch Recombination: Upstream or Downstream of DNA Breaks. JExp Med. 195(9):F37-41 (2002).
Chuck AS, Palsson BO. Consistent and high rates of gene transfer can be obtained using flow-through transduction over a wide range of retroviral titers. Hum Gene Ther 7: 743-750 (1996).
Damle RN, Wasil T, Fais F, Ghiotto F, Valetto A, Allen SL, Buchbinder A, Budman D, Dittmar K, Kolitz J, Lichtman SM, Schulman P, Vinciguerra VP, Rai KR, Ferrarini M, Chiorazzi N.. Ig V gene mutation status and CD38 expression as novel prognostic indicators in chronic lymphocytic leukemia. Blood 94:1840-1847 (1999).
Dance, G. S. C., Sowden, M. P., Yang, Y. and Smith, H. C. APOBEC-1 dependent cytidine to uridine editing of apolipoprotein B RNA in yeast. Nucleic Acids Res. 28, 424-429 (2000).
Dance, G.S.C., Beemiller, P., Yang, Y., Van Mater, D. Mian, S.I. and Smith, H.C. Identification of the yeast cytidine deaminase CDD1 as an orphan C to U RNA editase. Nucleic Acids Res. 29,1772-1780 (2001).
Dance, G.S.C., Sowden,M.P., Cartegni, L., Cooper, E., Krainer, A.R., Smith, H.C., Two proteins essential for apolipoprotein B mRNA editing are expressed from a single gene through alternative splicing. J. Biol. Chem. 277:12703-09 (2002).
Davidson, N.O., Powell, L.M., Wallis, S.C. and Scott, J. Thyroid hormone modulates the introduction of a stop codon in rat liver apolipoprotein B messenger RNA. J. Biol. Chem. 263:13482-13485 (1988).
de la Chapelle A, Peltomaki P. Genetics of hereditary colon cancer. Annu Rev Genet 29:329-348 (1995).
Dickerson SK, Market E, Besmer E, Papavasiliou FN. AID Mediates Hypermutation by Deaminating Single Stranded DNA. J Exp Med 197:1291-1296 (2003).
Di Noia J, Neuberger MS. Altering the pathway of immunoglobulin hypermutation by inhibiting uracil-DNA glycosylase. Nature 419: 43-48 (2002).
Doi T, Kinoshita K, Ikegawa M, Muramatsu M, Honjo T. De novo protein synthesis is required for the activation-induced cytidine deaminase function in class-switch recombination. Proc. Natl. Acad. Sci. USA 100:2634-2638 (2003).
Driscoll, D.M. and E. Casanova, Characterization of the apolipoprotein B mRNA editing activity in enterocyte extracts. J Biol Chem. 265(35): 21401-3 (1990).
Driscoll JS, Marquez VE, Plowman J, Liu PS, Kelley JA, Barchi JJ, Jr. Antitumor properties of 2(1H)-pyrimidinone riboside (zebularine) and its fluorinated analogues. J Med Chem 34:3280-3284 (1991).
Driscoll, D. M., Lakhe-Reddy, S., Oleksa, L. M. and Martinez, D. Induction of RNA editing at heterologous sites by sequences in apolipoprotein B mRNA. Mol. Cell. Biol. 13:7288-7294 (1993).
Economidis, I.V. and T. Pederson, In vitro assembly of a pre-messenger ribonucleoprotein. Proc Natl Acad Sci USA 80(14):4296-300 (1983).
Egebjerg, J., Kukekov, V. and Heinemann, S. F. Intron sequence directs RNA editing of the glutamate receptor subunit GluR2 coding sequence. Proc. Natl. Acad. Sci. U.S.A. 91:10270-10274 (1994).
Ehrenstein, M.R. and M.S. Neuberger, Deficiency in Msh2 affects the efficiency and local sequence specificity of immunoglobulin class-switch recombination: parallels with somatic hypermutation. Embo J. 18(12):3484-90 (1999).
Faham M, Cox DR. A novel in vivo method to detect DNA sequence variation. Genome Res 5:474-482 (1995).
Faham, M., S. Baharloo, S. Tomitaka, J. De Youngand N.B. Freimer, Mismatch repair detection (MRD): high-throughput scanning for DNA variations. Hum Mol Genet. 10:1657-64.
Faham, M., S. Baharloo, S. Tomitaka, J. DeYoungand N.B. Freimer, Mismatch repair detection (MRD): high-throughput scanning for DNA variations. Hum Mol Genet. 10(16):1657-64 (2001).
Faustino NA, Cooper TA. Pre-mRNA splicing and human disease. Genes Dev 17: 419-437 (2003).
Fishel R, Lescoe MK, Rao MR, Copeland NG, Jenkins NA, Garber J, Kane M, Kolodner R. The human mutator gene homolog MSH2 and its association with hereditary nonpolyposis colon cancer. Cell 75: 1027-1038 (1993).
Fisher, C. L. and Pei, K. P. Modification of a PCR-based site-directed mutagenesis method. BioTechniques 23, 570-574 (1997).
Frick L, Yang C, Marquez VE, Wolfenden R. Binding of pyrimidin-2-one ribonucleoside by cytidine deaminase as the transition-state analogue 3,4-dihydrouridine and the contribution of the 4-hydroxyl group to its binding affinity. Biochemistry 28:9423-9430 (1989).
Fugmann, S.D. and Schatz, D.G., Immunology. One AID to unite them all. Science. 295:1244-5 (2002).
Funahashi, T., Giannoni, F., DePaoli, A.M., Skarosi, S.F. and Davidson, N.O. Tissue-specific, developmental and nutritional regulation of the gene encoding the catalytic subunit of the rat apoB mRNA editing enzyme: functional role in the modulation of apoB mRNA editing. J. Lipid Res. 36:414-428 (1995).
Gabay C, Ben-Bassat H, Schlesinger M, Laskov R. Somatic mutations and intraclonal variations in the rearranged Vkappa genes of B-non-Hodgkin's lymphoma cell lines. Eur J Haematol 63:180-191 (1999).
Gaidano G, Pasqualucci L, Capello D, Berra E, Deambrogi C, Rossi D, Larocca LM, Gloghini A, Carbone A, Dalla-Favera R. Aberrant somatic hypermutation in multiple subtypes of AIDS-associated non-Hodgkin lymphoma. Blood: in press (2003).
Gerber, A.P. and Keller, W. RNA editing by base deamination: more enzymes, more targets, new mysteries. TIBS 26:376-384 (2001).
Giannoni, F., Bonen, D. K., Funahashi, T., Hadjiagapiou, C., Burant, C. F. and Davidson, N. O. Complementation of apolipoprotein B mRNA editing by human liver accompanied by secretion of apolipoprotein B48. J. Biol. Chem. 269, 5932-5936 (1994).
Giannoni, F., Chou, S.C., Skarosi, S.F., Verp, M.S., Field, F.J., Coleman, R.A. and Davidson, N.O. Developmental regulation of the catalytic subunit of the apoB mRNA editing enzyme (APOBEC-1) in human small intestine. J. Lipid Res. 36, 1664-1675 (1995).
Gott, J. M. and Emeson, R. B. Functions and mechanisms of RNA editing. Annu. Rev. Genet. 34:499-531 (2000).
Greeve, J., Altkemper, I., Dieterich, J-H., Greten, H. and Winder, E. Apolipoprotein B mRNA editing in 12 different mammalian species: hepatic expression is reflected in low concentrations of apoB-containing plasma lipoproteins. J. Lipid Res. 34:1367-1383 (1993).
Greeve, J., Lellek, H., Rautenberg, P. and Greten, H. Inhibition of the apolipoprotein B mRNA editing enzyme-complex by hnRNP C1 protein and 40S hnRNP complexes. Biol. Chem. 379:063-1073 (1998).
Greeve, J., Lellek, H., Apostel, F., Hundoegger, K/. Barialai, A., Kirsten, R., Welker, S and Greten, H. Absense of APOBEC-1 mediated mRNA editing in human carcinomas. Oncogene 18:6357-66 (1999).
Greeve J, Philipsen A, Krause K, Klapper W, Heidorn K, Castle BE, Janda J, Marcu KB, Parwaresch R. Expression of activation-induced cytidine deaminase in human B-cell non-Hodgkin's lymphomas. Blood 101:3574-3580 (2003).
Grosjean, H. and Benne, R. Modification and Editing of RNA. ASM Press, Washington D.C. (1998)
Hamblin TJ, Davis Z, Gardiner A, Oscier DG, Stevenson FK. Unmutated Ig V(H) genes are associated with a more aggressive form of chronic lymphocytic leukemia. Blood 94:1848-1854 (1999).
Harris, S.G. and Smith, H.C. In vitro apoB mRNA editing activity can be modulated by fasting and refeeding rats with a high carbohydrate diet. Biochem. Biophys. Res. Commun. 183:899-903 (1992).
Harris, S. G., Sabio, I., Mayer, E., Steinburg, M. F., Backus, J. W., Sparks, J. D., Sparks, C. E. and Smith, H. C. Extract-specific heterogeneity in high-order complexes containing apolipoprotein B mRNA editing activity and RNA-binding proteins. J. Biol. Chem. 268, 7382-7392 (1993).
Hersberger, M. and Innerarity, T. L. Two efficiency elements flanking the editing site of cytidine 6666 in the apolipoprotein B mRNA support mooring dependent editing. J. Biol. Chem. 273, 9435-9442 (1998).
Hersberger, M., Patarroyo-White, S., Arnold, K. S. and Innerarity, T. L. Phylogenetic analysis of the apolipoprotein B mRNA editing region. Evidence for a secondary structure between the mooring sequence and the 3' efficiency element. J. Biol. Chem. 274, 34590-34597 (1999).
Higuchi, M., Single, F.N., Köhler, M., Sommer, B., Sprengel, R. and Seeburg, P.H. RNA editing of AMPA receptor subunit GluR-B: a base-paired intron-exon structure determines position and efficiency. Cell 75, 1361-1370 (1993).
Higuchi, M., Maas, S., Single, F. N., Hartner, J., Rozov, A., Burnashev, N., Feldmeyer, D., Sprengel, R. and Seeburg, P. H. Point mutation in an AMPA receptor gene rescues lethality in mice deficient in the RNA editing enzyme ADAR2. Nature (London) 405, 78-81 (2000).
Hilleren, P. and R. Parker, mRNA surveillance in eukaryotes: kinetic proofreading of proper translation termination as assessed by mRNP domain organization? RNA 5(6): p. 711-9 (1999).
Hirano, K.I., Young, S.G., Farese, R.V., Ng, J., Sande, E., Warburton, C., Powell-Braxton, L.M. and Davidson, N.O. Targeted disruption of the mouse apobec-1 gene abolishes apoB mRNA editing and eliminates ApoB48. J. Biol. Chem. 271, 9887-9890 (1996).
Honjo, T., et al. Molecular Mechanism of Class Switch Recombination: Linkage with Somatic Hypermutation. Annu Rev Immunol. 20:165-96 (2002).
Hu, B.T., S.C. Lee, E. Marin, D.H. Ryanand R.A. Insel, Telomerase is up-regulated in human germinal center B cells in vivo and can be re-expressed in memory B cells activated in vitro. J Immunol 159(3):1068-71 (1997).
Inui, Y., Giannoni, F., Funahashi, T. and Davidson, N.O. REPR and complementation factor(s) interact to modulate rat apolipoprotein B mRNA editing in response to alterations in cellular cholesterol flux. J. Lipid Res. 35, 1477-1489 (1994).
Jansen B, Zangemeister-Wittke U. Antisense therapy for cancer -- the time of truth. Lancet Oncol 3: 672-683 (2002).
Jarmuz, A., et al. An Anthropoid-Specific Locus of Orphan C to U RNA-Editing Enzymes on Chromosome 22. Genomics. 79(3):285-96 (2002).
Johansson E, Mejlhede N, Neuhard J, Larsen S. Crystal structure of the tetrameric cytidine deaminase from Bacillus subtilis at 2.0 Å resolution. Biochem. 41 2563-70 (2002).
Juliano RL, Yoo H.. Aspects of the transport and delivery of antisense oligonucleotides. Curr Opin Mol Ther 2:297-303 (2000).
Kataoka, N., Yong, J., Kim, V. N., Velazquez, F., Perkinson, R. A., Wang, F. and Dreyfuss, G. Pre-mRNA splicing imprints mRNA in the nucleus with a novel RNA-binding protein that persists in the cytoplasm. Mol. Cell 6, 673-682 (2000).
Keegan, L.P., et al. The many roles of an RNA editor. Nat Rev Genet. 2:869-78 (2001).
Khamlichi AA, Pinaud E, Decourt C, Chauveau C, Cogne M. The 3' IgH regulatory region: a complex structure in a search for a function. Adv Immunol 75: 317-345 (2000).
Kinoshita K, Honjo T. Unique and unprecedented recombination mechanisms in class switching. Curr. Opin. Immunol. 12: 195-198 (2000).
Kohler, M., Burnashev, N., Sakmann, B. and Seeburg, P. H. Determinants of Ca 2+ permeability in both TM1 and TM2 of high affinity kainate receptor channels : diversity by RNA editing. Neuron 10, 491-500 (1993).
Kong Q, Harris RS, Maizels N. Recombination-based mechanisms for somatic hypermutation. Immunol Rev 162: 67-76 (1998).
Kong Q, Maizels DNA breaks in hypermutating immunoglobulin genes: evidence for a break-and-repair pathway of somatic hypermutation. Genetics 158:369-378 (2001).
Krogh, A., Brown, M., Mian, I. S., Sjolander, K. and Haussler, D. Hidden Markov models in computational biology. Applications to protein modeling, J Mol Biol. 235, 1501-31 (1994).
Kuzin, II, G.D. Ugine, D. Wu, F. Young, J. Chenand A. Bottaro, Normal isotype switching in B cells lacking the I mu exon splice donor site: evidence for multiple I mu-like germline transcripts. J Immunol 164(3):1451-7 (2000).
Kuzin, I.I., J. E. Snyder, G. D. Ugine, D. Wu, S. Lee, T. J. Bushnell, R. A. Insel, F. M. Young, Bottaro, A., Tetracyclines inhibit activated B cell function. Int. Immunol. 12: 921-931 (2001).
Lau, P. P., Xiong, W. J., Zhu, H. J., Chen, S. H. and Chan, L. Apolipoprotein B mRNA editing is an intranuclear event that occurs post-transcriptionally coincident with splicing and polyadenylation. J. Biol. Chem. 266, 20550-20554 (1991).
Lau, P.P., Zhu, H.J., Baldini, A., Charnsangavej, C. and Chan, L. Dimeric structure of a human apolipoprotein B mRNA editing protein and cloning and chromosomal localization of its gene. Proc. Natl. Acad. Sci. USA 91, 8522-8526 (1994).
Lau, P.P., Cahill, D.J., Zhu, H.J. and Chan, L.. Ethanol modulates apoB mRNA editing. J. Lipid Res. 36, 2069-2078 (1995).
Lau, P.P., Zhu, H.J., Nakamuta, M. and Chan, L. Cloning of an Apobec-1-binding protein that also interacts with apolipoprotein B mRNA and evidence for its involvement in RNA editing. J. Biol. Chem. 272, 1452-1455 (1997).
Lau, P.P, Chang, B.H.J. and Chan, L. Two-hybrid cloning identifies an RNA-binding protein GRY-RBP, as a component of apobec-1 editosome. Biochem. Biophys. Res. Commun. 282, 977-983 (2001).
Le Hir, H., Izaurralde, E., Maquat, L. E. and Moore, M. J. The spliceosome deposits multiple proteins 20-24 nucleotides upstream of mRNA exon-exon junctions. EMBO J. 19:6860-6869 (2000).
Lee, R.M., et al., An alternatively spliced form of apobec-1 messenger RNA is overexpressed in human colon cancer. Gastroenterology. 115:1096-103 (1998).
Lellek, H., Kirsten, R., Diehl, I., Apostel, F., Buck, F. and Greeve, J. Purification and Molecular cloning of a novel essential component of the apolipoprotein B mRNA editing Enzyme-complex. J. Biol. Chem., 275:19848-19856 (2000).
Lewis, J. D. and Tollervey, D. Like attracts like: getting RNA processing together in the nucleus. Science 288:1385-1389 (2000).
Liao, W., Hong, S.H., Chan, B.H.J., Rudolph, F.B., Clark, S.C. and Chan, L. APOBEC-2, a cardiac- and skeletal muscle-specific member of the cytidine deaminase supergene family. Biochem. Biophys. Res. Commun. 260:398-404 (1999).
Liu, H.X., L. Cartegni, M.Q. Zhangand A.R. Krainer, A mechanism for exon skipping caused by nonsense or missense mutations in BRCA1 and other genes. Nat Genet 27(1):55-8 (2001).
Liu, H.X., M. Zhangand A.R. Krainer, Identification of functional exonic splicing enhancer motifs recognized by individual SR proteins. Genes Dev 12(13):1998-2012 (1998).
Loeb LA. Mutator phenotype may be required for multistage carcinogenesis. Cancer Res 51:3075-3079 (1991).
Loeb LA. A mutator phenotype in cancer. Cancer Res 61: 3230-3239 (2001).
Loeb LA, Loeb KR, Anderson JP. Multiple mutations and cancer. Proc Natl Acad Sci U S A 100:776-781 (2003).
Longacre, A. and U. Storb, A novel cytidine deaminase affects antibody diversity. Cell. 102(5): p. 541-4 (2000).
Lossos IS, Alizadeh AA, Eisen MB, Chan WC, Brown PO, Botstein D, Staudt LM, R. L. Ongoing immunoglobulin somatic mutation in germinal center B cell-like but not in activated B cell-like diffuse large cell lymphomas. Proc. Natl. Acad. Sci. USA 97:10209-10213 (2000).
MacGinnitie, A.J., Anant, S. and Davidson, N.O. Mutagenesis of APOBEC-1, the catalytic subunit of the mammalian apolipoprotein B mRNA editing enzyme, reveals distinct domains that mediate cytosine nucleoside deaminase, RNA-binding, and RNA editing activity. J. Biol. Chem. 270:14768-14775 (1995).
McCahill, A., Lankester, D.J., Park, S., Price, N.T. and Zammit, V.A. Acute modulation of the extent of apoB mRNA editing and relative rates of synthesis of apoB48 and apoB100 in cultured rat hepatocytes by osmotic and other stresses. Molec. Cell. Biochem. 208, 77-87 (2000).
McCarthy H, Wierda WG, Barron LL, Cromwel CC, Wang J, Coombes KR, Rangel R, Elenitoba-Johnson KS, Keating MJ, Abruzzo LV. High Expression of Activation-Induced Cytidine Deaminase (AID) and Splice Variants is a Distinctive Feature of Poor Prognosis Chronic Lymphocytic Leukemia. Blood: Feb 13 [epub ahead of print] (2003)
Maas, S., Melcher, T., Herb, A., Seeburg, P.H., Keller, W., Krause, S., Higuchi, M. and O'Connell, M.A. Structural requirements for RNA editing in glutamate receptor pre-mRNA by recombinant double-stranded RNA adenosine deaminase. J. Biol. Chem. 271, 12221-12226 (1996).
Maas, S., Melcher, T. and Seeburg, P. H. Mammalian RNA-dependent deaminases and edited mRNAs. Curr. Opin. Cell. Biol. 9:343-349 (1997).
Maas, S. and Rich, A. Changing genetic information through RNA editing. BioEssays 22, 790-802 (2000).
Madsen P., Anant S., Rasmussen, H.H., Gromov, P., Vorum, H., Dumanski, J.P., Tommerup, N., Collins, J.E., Wright, C.L., Dunham, I., MacGinnitie, A.J., Davidson, N.O. and Celis, J.E. Psoriasis upregulated phorbolin-1 shares structural but not functional similarity to the mRNA-editing protein apobec-1. J. Invest. Dermatol. 113, 162-169 (1999).
Manis, J.P., Y. Gu, R. Lansford, E. Sonoda, R. Ferrini, L. Davidson, K. Rajewskyand F.W. Alt, Ku70 is required for late B cell development and immunoglobulin heavy chain class switching. J Exp Med. 187(12):2081-9 (1998).
Manis JP, Tian M, Alt FW. Mechanism and control of class-switch recombination. Trends Immunol 23: 31-39 (2002).
Maquat, L. and Carmichael, G. G. Quality control of mRNA function. Cell 104, 173-176 (2001).
Marinettii, G.V., Disorders of Lipid Metabolism. New York: Plenum Press (1990).
Martin, A., P.D. Bardwell, C.J. Woo, M. Fan, M.J. Shulmanand M.D. Scharff, Activation-induced cytidine deaminase turns on somatic hypermutation in hybridomas. Nature. 415(6873):802-6 (2002).
Mathews DH, Turner DH. Dynalign: an algorithm for finding the secondary structure common to two RNA sequences. J Mol Biol 317: 191-203 (2002).
Mehta, A., Kinter, M.T., Sherman, N.E. and Driscoll, D.M. Molecular cloning of apobec-1 complementation factor, a novel RNA- binding protein involved in the editing of apolipoprotein B mRNA, Mol Cell Biol., 20:1846-54 (2000).
Mehta, A., Driscoll, D.M., Identification of Domains in APOBEC-1 Complementation Factor Required for RNA Binding and Apolipoprotein B mRNA editing. RNA. 8:69-82 (2002).
Meyer J, Jack HM, Ellis N, Wab1 M. High rate of somatic point mutation in vitro in and near the variable-region segment of an immunoglobulin heavy chain gene. Proc Natl Acad Sci U S A 83:6950-6953 (1986)
Mian, I.S., Moser, M.J., Holley, W.R. and Chatterjee, A. Statistical modeling and phylogenetic analysis of a deaminase domain, J Comput. Biol., 5:57-72 (1988).
Minegishi, Y., et al., Mutations in activation-induced cytidine deaminase in patients with hyper IgM syndrome. Clin Immunol. 97:203-10 (2000).
Morrison, J.R., Paszty, C., Stevens, M.E., Hughes, S.D., Forte, T. and Scott, J. ApoB RNA editing enzyme-deficient mice are viable despite alterations in lipoprotein metabolism. Proc. Natl. Acad. Sci. USA 93, 7154-7159 (1996).
Muramatsu, M., Sankaranand, V.S., Anant, S., Sugai, M., Kinoshita, K., Davidson, N.O. and Honjo, T. Specific expression of activation-induced cytidine deaminase (AID), a novel member of the RNA-editing deaminases family in germinal center B cells. J. Biol. Chem. 274, 18740-18476 (1999).
Muramatsu M, Sankaranand VS, Anant S, Sugai M, Kinoshita K, Davidson NO, Honjo T. Specific expression of activation-induced cytidine deaminase (AID), a novel member of the RNA-editing deaminase family in germinal center B cells. J. Biol. Chem. 274: 18470-18476 (1999).
Muramatsu, M., Kinoshita, K., Fagarasan, S., Yamada, S., Shinkai, Y. and Honjo, T. Class switch recognition and hypermutation require activation-induced cytidine deaminase (AID), a potential RNA editing enzyme. Cell 102, 553-564 (2000).
Mukhopadhyay, D., S. Anant, R.M. Lee, S. Kennedy, D. Viskochiland N.O. Davidson, C-->U editing of neurofibromatosis 1 mRNA occurs in tumors that express both the type II transcript and apobec-1, the catalytic subunit of the apolipoprotein B mRNA-editing enzyme. Am J Hum Genet. 70(1):38-50 (2002).
Muschen M, Re D, Jungnickel B, Diehl V, Rajewsky K, Kuppers R. Somatic mutation of the CD95 gene in human B cells as a side-effect of the germinal center reaction. J Exp Med 192:1833-1840 (2000).
Muschen, M., K. Rajewsky, M. Kronkeand R. Kuppers, The origin of CD95-gene mutations in B-cell lymphoma. Trends Immunol. 23(2):75-80 (2002).
Nagaoka, H., M. Muramatsu, N. Yamamura, K. Kinoshitaand T. Honjo, Activation-induced deaminase (AID)-directed hypermutation in the immunoglobulin Smu region: implication of AID involvement in a common step of class switch recombination and somatic hypermutation. J Exp Med, 195(4): p. 529-34 (2002).
Nakamuta, M., Chang, B.H.J., Zsigmond, E., Kobayashi, K., Lei, H., Ishida, B.Y., Oka, K., Li, E. and Chan, L. Complete phenotypic characterization of apobec-1 knockout mice with a wild-type genetic background and a human apoB transgenic background, and restoration of apoB mRNA editing by somatic gene transfer of APOBEC-1. J. Biol. Chem. 271:25981-25988 (1996).
Navaratnam, N., D., Patel, R.R., Shah, J.C., Greeve L.M., Powell, T.J., Knott, J., Scott, An additional editing site is present in apolipoprotein B mRNA. Nucleic Acids Res.. 19:1741-1744 (1991).
Navaratnam, N., R. Shah, D. Patel, V. Fayand J. Scott, Apolipoprotein B mRNA editing is associated with UV crosslinking of proteins to the editing site. Proc Natl Acad Sci U S A. 90(1): 222-6 (1993).
Navaratnam, N., Bhattacharya, S., Fujino, T., Patel, D., Jarmuz, A.L. and Scott, J. Evolutionary origins of apoB mRNA editing: catalysis by a cytidine deaminase that has acquired a novel RNA-binding motif at its active site. Cell 81:187-195 (1995).
Navaratnam, N., Fujino, T., Bayliss, J., Jarmuz, A., How, A. Richardson, N., Somasekaram, A. Bhattacharya, S., Carter, C. & Scott, J. Escherichia coli cytidine deaminase provides a molecular model for ApoB RNA editing and a mechanism for RNA substrate recognition JMB 275:695-714 (1998).
Neuberger MS, Ehrenstein MR, Klix N, Jolly CJ, Yelamos J, Rada C, Milstein C. Monitoring and interpreting the intrinsic features of somatic hypermutation. Immunol Rev 162:107-116 (1998).
Neumann, J. R., Morency, C. A. and Russian, K. O. A novel rapid assay for chloramphenicol acetyltransferase gene expression. BioTechniques 5:444-448 (1987).
Nicolaides NC, Papadopoulos N, Liu B, Wei YF, Carter KC, Ruben SM, Rosen CA, Haseltine WA, Fleischmann RD, Fraser CM, et al. Mutations of two PMS homologues in hereditary nonpolyposis colon cancer. Nature 371: 75-80 (1994).
O'Connell, M.A. RNA Editing: Rewriting Receptors. Current Biology 7:R437-R439 (1997).
Oka, K., Kobayashi, K., Sullivan, M., Martinez, J., Teng, B.B., Ishimura-Oka, K. and Chan, L. Tissue-specific inhibition of apoB B mRNA editing in the liver by adenovirus-mediated transfer of a dominant negative mutant APOBEC-1 leads to increased low density lipoprotein in mice. J. Biol. Chem. 272, 1456-1460 (1997).
Okazaki, I.M., et al., The AID enzyme induces class switch recombination in fibroblasts. Nature. 416:340-5 (2002)
Okazaki I, Hiai H, Kakazu N, Yamada S, Muramatsu M, Kinoshita K, Honjo T. Constitutive expression of AID leads to tumorigenesis. J. Exp. Med. 197:1173-1181 (2003).
Oppezzo P, Vuillier F, Vasconcelos Y, Dumas G, Magnac C, Payelle-Brogard B, Pritsch O, Dighiero G. Chronic lymphocytic leukemia B cells expressing AID display a dissociation between class switch recombination and somatic hypermutation. Blood: Jan 9 [epub ahead of print] (2003).
Paddison, P.J., A.A. Caudy, E. Bernstein, G.J. Hannonand D.S. Conklin, Short hairpin RNAs (shRNAs) induce sequence-specific silencing in mammalian cells. Genes Dev 16(8):948-58 (2002).
Paddison, P.J., A.A. Caudyand G.J. Hannon, Stable suppression of gene expression by RNAi in mammalian cells. Proc Natl Acad Sci U S A 99(3):1443-8 (2002).
Papadopoulos N, Nicolaides NC, Wei YF, Ruben SM, Carter KC, Rosen CA, Haseltine WA, Fleischmann RD, Fraser CM, Adams MD, et al. Mutation of a mutL homolog in hereditary colon cancer. Science 263: 1625-1629 (1994).
Papavasiliou, F.N. and D.G. Schatz, Cell-cycle-regulated DNA double-stranded breaks in somatic hypermutation of immunoglobulin genes. Nature 408(6809):216-21 (2000).
Papavasiliou, F.N. and D.G. Schatz, The Activation-induced Deaminase Functions in a Postcleavage Step of the Somatic Hypermutation Process. J Exp Med 195(9):1193-1198 (2002).
Pasqualucci L, Migliazza A, Fracchiolla N, William C, Neri A, Baldini L, Chaganti RS, Klein U, Kuppers R, Rajewsky K, Dalla-Favera R. BCL-6 mutations in normal germinal center B cells: evidence of somatic hypermutation acting outside Ig loci. Proc Natl Acad Sci U S A 95:11816-11821 (1998).
Pasqualucci L, Neumeister P, Goossens T, Nanjangud G, Chaganti RS, Kuppers R, Dalla-Favera R. Hypermutation of multiple proto-oncogenes in B-cell diffuse large-cell lymphomas. Nature 412: 341-346 (2001).
Pasqualucci L, Migliazza A, Basso K, Houldsworth J, Chaganti RS, Dalla-Favera R. Mutations of the BCL6 proto-oncogene disrupt its negative autoregulation in diffuse large B-cell lymphoma. Blood 101:2914-2923 (2003).
Pear WS, Miller JP, Xu L, Pui JC, Soffer B, Quackenbush RC, Pendergast AM, Bronson R, Aster JC, Scott ML, Baltimore D. Efficient and rapid induction of a chronic myelogenous leukemia-like myeloproliferative disease in mice receiving P210 bcr/abl-transduced bone marrow. Blood 92: 3780-3792 (1998).
Petersen-Mahrt, S.K., et al., AID mutates E. coli suggesting a DNA deamination mechanism for antibody diversification. Nature. 418:99-104 (2002).
Petersen-Mahrt SK, Neuberger MS. 2003. In vitro deamination of cytosine to uracil in single-stranded DNA by APOBEC1. J. Biol. Chem. in press (2003).
Phung, T.L., Sowden, M.P., Sparks, J.D., Sparks, C.E. and Smith, H.C. Regulation of hepatic apoB RNA editing in the genetically obese Zucker rat. Metabolism 45, 1056-1058 (1996).
Powell, L.M., Wallis, S.C., Pease, R.J., Edwards, Y.H., Knott, T.J. and Scott, J. A novel form of tissue-specific RNA processing produces apolipoprotein-B48 in intestine. Cell 50:831-840 (1996).
Puck, J.M., A disease gene for autosomal hyper-IgM syndrome: more genes associated with more immunodeficiencies. Clin Immunol. 97(3):191-2 (2000).
Qian, X., Balestra, M.E., Yamanaka, S., Boren, J., Lee, I. And Innerarity, T.L. Low expression of the aplolipoprotein B mRN A-editing transgene in mice reduces LDL level but does not cause liver dysplasia or tumors. Arterioscler. Thromb. Vasc. Biol. 18:1013-20 (1998).
Rada, C., et al. (2002) AID-GFP chimeric protein increases hypermutation of Ig genes with no evidence of nuclear localization. Proc. Natl. Acad. Sci USA. 99(10):7003-7008 (2002)
Rada C, Williams GT, Nilsen H, Barnes DE, Lindahl T, Neuberger MS. Immunoglobulin isotype switching is inhibited and somatic hypermutation perturbed in UNG-deficient mice. Curr. Biol. 12: 1748-1755 (2002).
Ramiro AR, Stavropoulos P, Jankovic M, Nussenzweig MC. Transcription enhances AID-mediated cytidine deamination by exposing single-stranded DNA on the nontemplate strand. Nat Immunol in press (2003).
Revy, P, Muto, R., Levy, Y., Geissmann, f., Plebani, A., Sanal, O., Catalan, N., Forveille, M., Dufourcq-Lagelouse, R., Gennery, A., Tezcan, I., Ersoy, F., Kayserili, H., Ugazio, A.G., Brousse, N., Muramatsu, M., Notarangelo, L.D., Kinoshita, K., Honjo, T., Fisher, A. and Durandy, A. Activation-induced cytidine deaminase (AID) deficiency causes the autosomal recessive form of the hyper-IgM syndrome (HIGM2). Cell 102(5):565-576 (2000).
Richardson, N., Navaratnam, N. and Scott, J. Secondary structure for the apolipoprotein B mRNA editing site. AU binding proteins interact with a stem loop. J. Biol Chem. 273, 31707-31717 (1998).
Robberson, B. L., Cote, G. J. and Berget, S. M. Exon definition may facilitate splice site selection in RNAs with multiple exons. Mol. Cell. Biol. 10, 1084-1094 (1990).
Rolink, A., F. Melchersand J. Andersson, The SCID but not the RAG-2 gene product is required for S mu-S epsilon heavy chain class switching. Immunity 5(4):319-30 (1996).
Rosenwald A, Wright G, Chan WC, Connors JM, Campo E, Fisher RI, Gascoyne RD, Muller-Hermelink HK, Smeland EB, Giltnane JM, Hurt EM, Zhao H, Averett L, Yang L, Wilson WH, Jaffe ES, Simon R, Klausner RD, Powell J, P.L. D, Longo DL, Greiner TC, Weisenburger DD, Rueter, S. M. and Emeson, R. B. Adenosine-to-inosine conversion in mRNA. In Modification and Editing of RNA (Grosjean, H. and Benne, R., eds.), pp. 343-361, American Society for Microbiology Press, Washington (1998). Rueter, S.M., Dawson, T.R. and Emeson, R.B. Regulation of alternative splicing by RNA editing. Nature 399, 75-80 (1999).
Sakashita, E. and H. Sakamoto, Protein-RNA and protein-protein interactions of the Drosophila sex-lethal mediated by its RNA-binding domains. Journal of Biochemistry 120(5):1028-33 (1996).
Sale, J.E., D.M. Calandrini, M. Takata, S. Takedaand M.S. Neuberger, Ablation of XRCC2/3 transforms immunoglobulin V gene conversion into somatic hypermutation. Nature. 412(6850): 921-6 (2001).
Sanger WG, Dave BJ, Lynch JC, Vose J, Armitage JO, Montserrat E, Lopez-Guillermo A, Grogan TM, Miller TP, LeBlanc M, Ott G, Kvaloy S, Delabie J, Holte H, Krajci P, Stokke T, Staudt LM. The use of molecular profiling to predict survival after chemotherapy for diffuse large-B-cell lymphoma. New Engl. J. Med. 346: 1937-1947 (2002).
Schock, D., Kuo, S.R., Steinburg, M.F., Bolognino, M., Sparks, J.D., Sparks, C.E. and Smith, H.C. An auxiliary factor containing a 240 kDa protein is involved in apoB RNA editing. Proc. Natl. Acad. Sci. USA 93, 1097-1102 (1996).
Schrader CE, Edelman W, Kuchelapati R, Stavnezer J. Reduced isotype switching in splenic B cells from mice deficienct in mismatch repair. J. Exp. Med. 190: 323-330 (1999).
Schrader C, E., Vardo J, Stavnezer J. Role for mismatch repair proteins Msh2, Mlh1, and Pms2 in immunoglobulin class switching shown by sequence analysis of recombination junctions. J. Exp. Med. 195: 367-373 (2002).
Scott, J. The molecular and cell biology of apolipoprotein-B..J. Mol. Med. 6:65-80 (1989).
Seeburg, P. H., Higuchi, M. and Sprengel, R. RNA editing of brain glutamate receptor channels : mechanism and physiology. Brain Res. Rev. 26:217-229 (1998).
Shah, R. R., Knott, T. J., Legros, J. E., Navaratnam, N., Greeve, J. C. and Scott, J. Sequence requirements for the editing of apolipoprotein B mRNA. J. Biol. Chem. 266:16301-16304 (1991).
Sheehy, A.M., et al., Isolation of a human gene that inhibits HIV-1 infection and is suppressed by the viral Vif protein. Nature. 418:646-650 (2002).
Shen HM, Peters A, Baron B, Zhu X, Storb U. Mutation of BCL-6 gene in normal B cells by the process of somatic hypermutation of Ig genes. Science 280: 1750-1752 (1998).
Shinkura R, Tian M, Smith M, Chua K, Fujiwara Y, Alt FW. The influence of transcriptional orientation on endogenous switch region function. Nat Immunol 4: 435-441 (2003).
Siddiqui, J. F. M., Van Mater, D., Sowden, M. P. and Smith, H. C. Disproportionate relationship between APOBEC-1 expression and apolipoprotein B mRNA editing activity. Exp. Cell Res. 252, 154-164 (1999).
Simpson, L. and Emeson, R. B. RNA editing. Annu. Rev. Neurosci. 19, 27-52 (1996).
Skuse, G.R., A.J. Cappione, M. Sowden, L.J. Methenyand H.C. Smith, The neurofibromatosis type I messenger RNA undergoes base-modification RNA editing. Nucleic Acids Res.. 24(3):478-86 (1996).
Smith, H. C., Kuo, S. R., Backus, J. W., Harris, S. G., Sparks, C. E. and Sparks, J. D. In vitro mRNA editing : identification of a 27 S editing complex. Proc. Natl. Acad. Sci. U.S.A. 88:1489-1493 (1991).
Smith, H.C. Apo B mRNA editing: the sequence to the event. Seminars in Cell Biology (Stuart, K., ed.) Saunders Sci. Publications/Academic Press, London, 4, 267-278 (1993).
Smith, H.C. and Sowden, M.P. Base modification RNA editing Trends in Genetics 12:418-424 (1996).
Smith, H.C., Gott, J.M. and Hanson, M.R. A guide to RNA editing. RNA, 3, 1105-1123 (1997).
Smith, H.C., Analysis of protein complexes assembled on apolipoprotein B mRNA for mooring sequence-dependent RNA editing. Methods. 15(1):27-39 (1998).
Sowden, M. P., Harrison, S. M., Ashfield, R. A., Kingsman, A. J. and Kingsman, S. M. Multiple cooperative interactions constrain BPV-1 E2 dependent activation of transcription. Nucleic Acids Res. 17, 2959-2972 (1989).
Sowden, M. P., Hamm, J. K. and Smith, H. C. Over-expression of APOBEC-I results in mooring sequence dependent promiscuous RNA editing. J. Biol. Chem. 271, 3011-3017 (1996).
Sowden, M.P., Hamm, J.K., Spinelli, S. and Smith, H.C. Determinants involved in regulating the proportion of edited apolipoprotein B RNAs. RNA 2, 274-288 (1996).
Sowden, M.P., Eagleton, M.J. and Smith, H.C. ApoB RNA sequence 3' of the mooring sequence and cellular sources of auxiliary factors determine the location and extent of promiscuous editing. Nucleic Acids Res. 26, 1644-1652 (1998).
Sowden, M.P. and H.C. Smith, Commitment of apolipoprotein B RNA to the splicing pathway regulates cytidine-to-uridine editing-site utilization. Biochem J 359(Pt 3):697-705 (2001).
Sowden, M.P., Ballatori, N., de Mesy Jensen, K.L., Hamilton Reed, L., Smith, H.C., The editosome for cytidine to uridine mRNA editing has a native complexity of 27S: identification of intracellular domains containing active and inactive editing factors. J. Cell Science 115:1027-1039 (2002).
Spector, D. Macromolecular domains within the cell nucleus. Annu. Rev. Cell Biol. 9, 265-315 (1993).
Steinburg, M. F., Schock, D., Backus, J. W. and Smith, H. C. Tissue-specific differences in the role of RNA 3' of the apolipoprotein B mRNA mooring sequence in editosome assembly. Biochem. Biophys. Res. Commun. 263, 81-86 (1999).
Storb U, Peters A, Klotz E, Kim N, Shen HM, Hackett J, Rogerson B, Martin TE. Cis-acting sequences that affect somatic hypermutation of Ig genes. Immunol Rev 162: 153-160 (1998).
Strasser A, Harris AW, Cory S. The role of bc1-2 in lymphoid differentiation and neoplastic transformation. Curr Top Microbiol Immunol 182:299-302 (1992).
Stull RA, Hyun WC, Pallavicini MG. Simultaneous flow cytometric analyses of enhanced green and yellow fluorescent proteins and cell surface antigens in doubly transduced immature hematopoietic cell populations. Cytometry 40:126-134 (2000).
Schlissel, M. S., L. M. Corcoran, and D. Baltimore. Virus-transformed pre-B cells show ordered activation but not inactivation of immunoglobulin gene rearrangement and transcription. J. Exp. Med. 173:711-720 (1991).
Taagepera, S., McDonald, D., Loeb, J. E., Whitaker, L. L., McElroy, A. K., Wang, J. Y. J. and Hope, T. J. Nuclear-cytoplasmic shuttling of C-ABL tyrosine kinase. Proc. Natl. Acad. Sci. U.S.A. 95, 7457-7462 (1998).
Tashiro J, Kinoshita K, Honjo T. Palindromic but not G-rich sequences are targets of class switch recombination. Int. Immunol. 13: 495-505 (2001).
Teng, B. and N.O. Davidson, Evolution of intestinal apolipoprotein B mRNA editing. Chicken apolipoprotein B mRNA is not edited, but chicken enterocytes contain in vitro editing enhancement factor(s). J Biol Chem. 267(29):21265-72 (1992).
Teng, B., Burant, C. F. and Davidson, N.O. Molecular cloning of an apolipoprotein B messenger RNA editing protein, Science, 260:1816-1819 (1993).
Teng, B.B., Blumenthal, S., Forte, T., Navaratnam, N., Scott, J., Gotto, A.M. and Chan, L. Adenovirus-mediated gene transfer fo rat apolipoprotein B mRNA-editing protein in mice virtually eliminates apolipoprotein B100 and normal low density lipoprotein production. J. Biol. Chem. 269:29395-29404 (1994).
van Engelen BG, Hiel JA, Gabreels FJ, van den Heuvel LP, van Gent DC, Weemaes CM. Decreased immunoglobulin class switching in Nijmegen Breakage syndrome due to the DNA repair defect. Hum. Immunol. 62: 1324-1327 (2001).
Van Mater, D., Sowden, M.P., Cianci, J., Sparks, J.D., Sparks, C.E., Ballitori, N. and Smith, H.C.. Ethanol increases apoB mRNA editing in rat primary hepatocyte and McArdle cells. Biochem. Biophys Res. Commun. 252, 334-339 (1998).
Van Parijs, L., Y. Refaeli, J.D. Lord, B.H. Nelson, A.K. Abbasand D. Baltimore, Uncoupling IL-2 signals that regulate T cell proliferation, survival, and Fas-mediated activation-induced cell death. Immunity 11(3):281-8 (1999).
Vaux DL, Cory S, Adams JM. Bc1-2 gene promotes haemopoietic cell survival and cooperates with c-myc to immortalize pre-B cells. Nature 335: 440-442 (1988).
von Wronski, M.A., Hirano, K.I., Cagen, L.M., Wilcox, H.G., Raghow, R., Thomgate, F.E., Heimberg, M., Davidson, N.O. and Elam, M.B. Insulin increases expression of apobec-1, the catalytic subunit of the apoB B mRNA editing complex in rat hepatocytes. Metabolism Clinical & Exp. 7:869-873 (1998).
Wab1 M, Burrows PD, von Gabain A, Steinberg C. Hypermutation at the immunoglobulin heavy chain locus in a pre-B-cell line. Proc Natl Acad Sci U S A 82: 479-482 (1985).
Wedekind JE, McKay DB. Purification, crystallization, and X-ray diffraction analysis of small ribozymes. Methods Enzymol 317:149-168 (2000).
Wedekind JE, Dance GS, Sowden MP, Smith HC. Messenger RNA editing in mammals: new members of the APOBEC family seeking roles in the family business. Trends Genet. 19: 207-216 (2003).
Willerford DM, Swat W, Alt FW. Developmental regulation of V(D)J recombination and lymphocyte differentiation. Curr Opin Genet Dev 6: 603-609 (1996).
Woo PC, Tsoi HW, Wong LP, Leung HC, Yuen KY. Antibiotics modulate vaccine-induced humoral immune response. Clin Diagn Lab Immunol 6:832-837 (1999).
Wu, J.H., Semenkovish, C.F., Chen, S.H., Li, W.H. and Chan, L. ApoB mRNA editing: validation of a sensitive assay and developmental biology of RNA editing in the rat. J. Biol. Chem. 265, 12312-12316 (1990).
Wuerffel RA, Du J, Thompson RJ, Kenter AL. Ig Sgamma3 DNA-specifc double strand breaks are induced in mitogen-activated B cells and are implicated in switch recombination. J Immunol 159: 4139-4144 (1997).
Vora KA, Tumas-Brundage KM, Lentz VM, Cranston A, Fishel R, Manser T. Severe attenuation of the B cell immune response in Msh2-deficient mice. J. Exp. Med. 189: 471-482 (1999).
Yamanaka, S., Poksay, K. S., Balestra, M. E., Zeng, G. Q. and Innerarity, T. L. Cloning and mutagenesis of the rabbit apoB mRNA editing protein. J. Biol. Chem. 269:21725-21734 (1994).
Yamanaka, S., Balestra, M., Ferrell, L., Fan, J., Arnold, K.S., Taylor, S., Taylor, J.M. and Innerarity, T.L. Apolipoprotein B mRNA-editing protein induces hepatocellular carcinoma and dysplasia in transgenic animals. Proc. Natl. Acad. Sci. USA 92:8483-8487 (1995).
Yamanaka, S., K. S. Poksay, D. M. Driscoll, Innerarity, T. L., Hyperediting of multiple cytidines of apolipoprotein B mRNA by APOBEC-1 requires auxiliary protein(s) but not a mooring sequence motif. J. Biol. Chem. 271:11506-11510 (1996).
Yamanaka, S., Poksay, K.S., Arnold, K.S. and Innerarity, T.L. A novel translational repressor mRNA is edited extensively in livers containing tumors caused by the transgene expression of the apoB mRNA- editing enzyme, Genes Dev., 11:321-33 (1997).
Yang, Y. and Smith, H. C. In vitro reconstitution of apolipoprotein B RNA editing activity from recombinant APOBEC-1 and McArdle cell extracts. Biochem. Biophys. Res. Commun. 218, 797-801 (1996).
Yang, Y., Kovalski, K. and Smith, H.C. Partial characterization of the auxiliary factors involved in apoB mRNA editing through APOBEC-1 affinity chromatography, J Biol. Chem., 272:27700-27706 (1997).
Yang, Y., Yang, Y. and Smith, H. C. Multiple protein domains determine the cell type-specific nuclear distribution of the catalytic subunit required for apolipoprotein B mRNA editing. Proc. Natl. Acad. Sci. U.S.A. 94:13075-13080 (1997).
Yang, Y., Sowden, M. P. and Smith, H. C. Induction of cytidine to uridine editing on cytoplasmic apolipoprotein B mRNA by overexpressing APOBEC-1. J. Biol. Chem. 275, 22663-22669 (2000).
Yang, Y., M.P., Sowden Y., Yang, H.C., Smith, Intracellular Trafficking Determinants in APOBEC-1, the Catalytic Subunit for Cytidine to Uridine Editing of Apolipoprotein B mRNA. Exp. Cell Res. 267:153-164 (2001).
Yang, Y., Ballatori, N., Smith, H.C., Synthesis and secretion of the atherogenic risk factor apoB100 is reduced through TAT-mediated protein transduction of an mRNA editase into hepatocytes. Molec. Pharm. 61:269-276 (2002).
Yelamos J, Klix N, Goyenechea B, Lozano F, Chui YL, Gonzalez Fernandez A, Pannell R, Neuberger MS, Milstein C. Targeting of non-Ig sequences in place of the V segment by somatic hypermutation. Nature 376: 225-229 (1995).
Yoshikawa, K., et al. AID enzyme-induced hypermutation in an actively transcribed gene in fibroblasts. Science 296:2033-2036 (2002)
Yu K, Chedin F, Hsieh CL, Wilson TE, Lieber MR. 2003. R-loops at immunoglobulin class switch regions in the chromosomes of stimulated B cells. Nat Immunol 4: 442-451 (2002)
Zhao Q, Zhou R, Temsamani J, Zhang Z, Roskey A, Agrawal S. Cellular distribution of phosphorothioate oligonucleotide following intravenous administration in mice. Antisense Nucleic Acid Drug Dev 8:451-458 (1998).
Zhou L, Cheng X, Connolly BA, Dickman MJ, Hurd PJ, Hornby DP. Zebularine: a novel DNA methylation inhibitor that forms a covalent complex with DNA methyltransferases. J Mol Biol 321:591-599 (2002).

A chimeric protein comprising:
a protein transduction domain; and
a deaminase domain, wherein the deaminase edits viral RNA.

The chimeric protein as defined above, wherein the protein transduction domain is selected from the group consisting of poly-arginine, poly-lysine peptide, third alpha helix of Antennapedia homeodomain protein, HSV-1 virion protein (VP) 22, HIV-1 Vpr, and HIV TAT protein.

The chimeric protein as defined above, wherein the protein transduction domain is an HIV Tat domain.

The chimeric protein as defined above, wherein the Tat domain comprises SEQ ID NO: 43.

The chimeric protein as defined above, wherein the deaminase domain comprises CEM15.

The chimeric protein as defined above, wherein the CEM15 domain comprises SEQ ID NO: 1.

The chimeric protein as defined above, wherein the deaminase domain is a fragment or derivative of CEM15 having deaminase function.

The chimeric protein as defined above, wherein the CEM15 fragment or derivative has at least 70% amino acid similarity with CEM15.

The chimeric protein as defined above, further comprising an epitope tag.

The chimeric protein as defined above, wherein the epitope tag is hemagglutinin.

The chimeric protein as defined above, further comprising a polyhistidine tag.

The chimeric protein as defined above, further comprising a polypeptide domain that enhances solubility of the chimeric protein.

The chimeric protein as defined above, wherein the polypeptide domain is a chicken muscle pyruvate kinase.

The chimeric protein as defined above, wherein the chicken muscle pyruvate kinase comprises the amino acid sequence of SEQ ID NO: 41.

The chimeric protein as defined above, further comprising a protein cleavage site.

A chimeric protein comprising a protein transducing domain; and a deaminase domain that edits DNA.

The chimeric protein as defined above, wherein the deaminase domain edits viral DNA.

The chimeric protein as defined above, wherein the deaminase is a cytidine deaminase.

A chimeric protein comprising a protein transducing domain; and a deaminase domain, wherein the deaminase is not APOBEC-1.

The chimeric protein as defined above, wherein the deaminase has less than 70% amino acid similarity with APOBEC-1.

The chimeric protein as defined above, wherein the deaminase has more than 70% amino acid similarity with Cem15.

A chimeric protein comprising a protein transducing domain; and a deaminase, wherein the deaminase does not edit ApoB1 mRNA.

A chimeric protein comprising a protein transducing domain; and a deaminase domain, wherein the deaminase comprises more than two CTD-1 repeats.

The chimeric protein as defined above, wherein more than one of the CTD-repeats has a deaminating function.

A chimeric protein comprising a protein transducing domain; a deaminase domain, wherein the deaminase comprises a CTD-1; and an anchor oligonucleotide.

A CEM15 mimetic, wherein the mimetic binds viral infectivity factor.

A chimeric protein comprising
a protein transducing domain; and
the CEM15 mimetic of as defined above.

A method of interrupting HIV infectivity comprising contacting an HIV-infected cell or a cell prior to HIV infection with the chimeric protein as defined above, under conditions that allow delivery of the chimeric protein into the cell, wherein the chimeric protein binds with vif to interrupt HIV infectivity.

A method of treating a subject with an HIV infection or at risk for an HIV infection comprising administering to the subject an effective amount of the chimeric protein as defined above.

The method as defined above, wherein the administration step is dose-dependent.

The method as defined above, wherein the administration step is transient.

The method as defined above, further comprising administering to the subject an agent that enhancing the efficiency of mRNA editing function of the chimeric protein.

An isolated nucleotide sequence that encodes the chimeric protein as defined above.

A vector comprising the nucleotide sequence as defined above.

A recombinant host cell comprising the vector as defined above.

A composition comprising the chimeric protein as defined above and a pharmaceutical carrier.

A method of screening for a viral RNA deaminase mimetic comprising adding the agent to be screened to a virally infected mammalian system; and detecting levels of edited viral RNA, elevated levels of edited viral RNA indicating a viral RNA deaminase mimetic.

The method as defined above, wherein the virus is a retrovirus.

The method as defined above, wherein the retrovirus is HIV.

The method as defined above, wherein the viral RNA deaminase mimetic is a CEM15 mimetic.

The method as defined above, further comprising detecting binding of the agent to be screened to a virion infectivity factor.

A method of screening for a viral DNA deaminase mimetic comprising adding the agent to be screened to a virally infected mammalian system; and detecting levels of edited viral DNA, elevated levels of edited viral RNA indicating a viral RNA deaminase mimetic.

The method as defined above, wherein the virus is a retrovirus.

The method as defined above, wherein the retrovirus is HIV.

The method as defined above, wherein the viral DNA deaminase mimetic is a CEM15 mimetic.

The method as defined above, further comprising detecting binding of the agent to be screened to a viral integration factor.

A chimeric protein comprising:
a first polypeptide comprising a protein transduction domain; and
a second polypeptide comprising Activation Induced Deaminase or a fragment thereof which can deaminate cytidine to form uridine in an mRNA molecule or deaminate cytidine to form thymidine in a DNA molecule.

The chimeric protein as defined above wherein the protein transduction domain is selected from the group consisting of poly-arginine, poly-lysine peptide, third alpha helix of Antennapedia homeodomain protein, HSV-1 virion protein (VP) 22, HIV-1 Vpr, and HIV TAT protein.

The chimeric protein as defined above, wherein the protein transduction domain is an HIV Tat domain.

The chimeric protein according as defined above, wherein the HIV TAT protein transduction domain comprises an amino acid sequence of SEQ ID NO: 43.

The chimeric protein according as defined above wherein the AID or fragment thereof comprises an amino acid sequence of SEQ ID NO: 3 or fragments thereof.

The chimeric protein as defined above, wherein the AID fragment or derivative has at least 70% amino acid similarity with SEQ ID NO: 3.

The chimeric protein as defined above further comprising: a third polypeptide comprising a cytoplasmic localization protein or a fragment thereof which enhances localization of the chimeric protein to the cytoplasm.

The chimeric protein as defined above wherein the cytoplasmic localization protein or fragment thereof is chicken muscle pyruvate kinase or a fragment thereof.

The chimeric protein as defined above wherein the chicken muscle pyruvate kinase or a fragment thereof comprises an amino acid sequence of SEQ ID NO: 41 or fragments thereof.

The chimeric protein as defined above, wherein the third polypeptide enhances solubility.

The chimeric protein as defined above wherein, within the chimeric protein, the third polypeptide is C-terminal of the second polypeptide.

The chimeric protein as defined above, further comprising an epitope tag.

The chimeric protein as defined above, wherein the epitope tag is hemagglutinin.

The chimeric protein as defined above further comprising a polyhistidine tag.

The chimeric protein as defined above, wherein the chimeric protein comprises an amino acid sequence of SEQ ID NO: 3.

The chimeric protein as defined above, wherein the chimeric protein is in isolated form.

A composition comprising:
a pharmaceutically acceptable carrier and the chimeric protein as defined above.

The composition as defined above, wherein the chimeric protein is present in an amount which is effective to edit mRNA or deaminate cytidines in DNA of B lymphoblastic or any cells in which mRNA or DNA will serve as a substrate for the enzyme and which uptake the chimeric protein.

The composition as defined above, wherein the composition is in the form of a tablet, capsule, powder, solution, suspension, or emulsion.

A nucleic acid molecule encoding the chimeric protein as defined above.

The nucleic acid molecule as defined above, wherein the nucleic acid is DNA.

The nucleic acid molecule as defined above, wherein the nucleic acid is RNA.

An expression vector comprising the nucleic acid molecule as defined above.

The expression vector as defined above, wherein the expression vector is operable in prokaryotic cells.

A recombinant host cell comprising the expression vector as defined above.

A recombinant host cell comprising the nucleic acid molecule as defined above.

A DNA construct comprising:
the DNA molecule as defined above;
a promoter sequence operably connected 5' to the DNA molecule; and
a 3' regulatory sequence operably connected 3' of the DNA molecule.

An expression vector comprising the DNA construct as defined above.

The expression vector as defined above, wherein the expression vector is operable in prokaryotic cells.

A recombinant host cell comprising the expression vector as defined above.

A recombinant host cell comprising the DNA construct as defined above.

An isolated B lymphoblastic cell or other receptive cell which has taken up the chimeric protein as defined above.

A method of inducing production of immunoglubulins of the various classes and their subtypes comprising:
contacting a B lympohoblast with the chimeric protein as defined above under conditions effective to cause cellular uptake of the chimeric protein, and thereby induce antibody production in the B lymphoblast.

The method as defined above wherein the B lymphoblast is *in vitro.*

The method as defined above wherein the B lymphoblast is *in vivo.*

The method as defined above wherein the antibody production includes IgG production.

The method as defined above wherein the antibody production includes IgA production.

The method as defined above wherein the antibody production includes IgE production.

The method as defined above wherein the chimeric protein comprises an amino acid sequence of SEQ ID NO: 3.

A method of inducing class switch recombination in a B lymphocyte cell comprising:
contacting a B lymphocyte cell with the chimeric protein as defined above under conditions effective to cause cellular uptake of the chimeric protein, and thereby induce class switch recombination during antibody production in the B lymphocyte cell.

The method as defined above wherein the B lymphocyte cell is *in vitro.*

The method as defined above wherein the B lymphocyte cell is *in vivo.*

The method as defined above wherein the chimeric protein comprises an amino acid sequence of SEQ ID NO: 3.

The method as defined above wherein the B lymphocyte cell, prior to said contacting, is deficient in an ability to exhibit class switch recombination during antibody production.

The method as defined above wherein the B lymphocyte cell, prior to said contacting, exhibits normal levels of class switch recombination during antibody production.

A method of inducing somatic hypermutation in a B lymphocyte cell comprising:
contacting a B lymphocyte cell with the chimeric protein as defined above under conditions effective to cause cellular uptake of the chimeric protein, and thereby induce somatic hypermutation during antibody production in the B lymphocyte cell.

The method as defined above wherein the B lymphocyte cell is *in vitro.*

The method as defined above wherein the B lymphocyte cell is *in vivo.*

The method as defined above wherein the chimeric protein comprises an amino acid sequence of SEQ ID NO: 3.

The method as defined above wherein the B lymphocyte cell, prior to said contacting, is deficient in an ability to exhibit somatic hypermutation during antibody production.

The method as defined above wherein the B lymphocyte cell, prior to said contacting, exhibits normal levels of somatic hypermutation during antibody production.

A method of inducing an immune response in response to an antigen in a subject comprising:
contacting a B lymphocyte cell with the chimeric protein as defined above under conditions effective to cause cellular uptake of the chimeric protein, and thereby induce antibody production in the B lymphocyte cell to afford a stronger immune response to an antigen in the subject.

The method as defined above wherein said contacting is carried out *in vitro,* said method further comprising:
introducing the B lymphocyte cell into the subject.

The method as defined above wherein said contacting is carried out *in vivo.*

The method as defined above wherein the antibody production includes IgG production.

The method as defined above wherein the antibody production includes IgA production.

The method as defined above wherein the antibody production includes IgE production.

The method as defined above wherein the chimeric protein comprises an amino acid sequence of SEQ ID NO: 3.

A method of treating a subject for hyper-IgM syndrome comprising:
administering to a subject exhibiting hyper-IgM syndrome an effective amount of a chimeric protein as defined above, wherein the chimeric protein taken up by B lymphocyte cells induces antibody production sufficient to treat the hyper-IgM syndrome.

The method as defined above wherein said administering is carried out orally, topically, transdermally, parenterally, subcutaneously, intravenously, intramuscularly, intraperitoneally, by intracavitary or intravesical instillation, intraocularly, intraarterially, intralesionally, by application to mucous membranes, or by implantation.

The method as defined above wherein the antibody production includes IgG production.

The method as defined above wherein the antibody production includes IgA production.

The method as defined above wherein the antibody production includes IgE production.

The method as defined above wherein the chimeric protein comprises an amino acid sequence of SEQ ID NO: 3.

A method of treating a subect for hyper-IgM syndrome comprising:
administering to a subject exhibiting hyper-IgM syndrome a population ofB lymphocyte cells as defined above, wherein the administered B lymphocyte cells exhibit antibody production sufficient to treat the hyper-IgM syndrome.

The method as defined above wherein said administering is carried out intravenously, intramuscularly, or intraarterially.

The method as defined above wherein the antibody production includes IgG production.

The method as defined above wherein the antibody production includes IgA production.

The method as defined above wherein the antibody production includes IgE production.

The method as defined above further comprising prior to said administering:
removing the population ofB lymphocyte cells from the subject and
exposing the B lymphocyte cells to the chimeric protein under conditions effective to cause cellular uptake of the chimeric protein.

The method as defined above wherein the chimeric protein comprises an amino acid sequence of SEQ ID NO: 3.

A method of treating a subject for B lymphocyte cell lymphoma comprising:
administering to a subject exhibiting B lymphocyte cell lymphoma an effective amount of a chimeric protein as defined above, wherein the chimeric protein taken up by cancerous B lymphocyte cells, and inhibits blunt cell growth thereof, thereby treating the lymphoma.

The method as defined above wherein said administering is carried out orally, topically, transdermally, parenterally, subcutaneously, intravenously, intramuscularly, intraperitoneally, by intracavitary or intravesical instillation, intraocularly, intraarterially, intralesionally, by application to mucous membranes, or by implantation.

The method as defined above wherein the chimeric protein comprises an amino acid sequence of SEQ ID NO: 3.

A delivery device comprising a chimeric protein as defined above.

The delivery device as defined above, wherein the delivery device is in the form of a liposome, a niosome, a transdermal patch, an implant, or a syringe.

A delivery device comprising a composition as defined above.

The delivery device as defined above, wherein the delivery device is in the form of a liposome, a niosome, a transdermal patch, an implant, or a syringe.

## Claims

1. A method of identifying an inhibitor of an interaction between CEM15 and Vif, the method comprising:
(a) administering a composition to a system, wherein the system comprises CEM15,
(b) assaying the effect of the composition on a CEM15-Vif interaction; and
(c) selecting a composition which inhibits a CEM15-Vif interaction.

2. The method of claim 1, wherein the system comprises a batch processing container.

3. The method of claim 1, wherein a library of compositions are administered to the system.

4. The method of claim 1, wherein inhibition of a CEM15-Vif interaction by the composition is determined by assaying the amount of CEM15-Vif binding in the presence of the compound compared to the amount of CEM15-Vif binding in the absence of the test compound.

5. The method of claim 1, wherein if the amount of CEM15-Vif binding is reduced in the presence of the composition as compared to the amount of CEM15-Vif binding in the absence of the test compound, the composition can be said to inhibit CEM15-Vif binding.

6. The method of claim 1, wherein inhibition of a CEM15-Vif interaction is a result of the composition blocking binding of Vif to CEM15.

7. The method of claim 1, wherein inhibition of a CEM15-Vif interaction is a result of the composition competing with CEM15-Vif binding.

8. The method of claim 1, wherein inhibition of a CEM15-Vif interaction is determined by measuring viral infectivity.

9. The method of claim 8, wherein the viral infectivity is HIV-1 infectivity.

10. The method of claim 8, wherein the viral infectivity is measured by contacting the composition with one or more CEM15-positive cells in the presence of HIV-1 virus expressing Vif.

11. A method of identifying an inhibitor of CEM15-Vif binding, the method comprising:
assaying the amount of CEM15-Vif in the presence of a composition and the amount of CEM15-Vif binding in the absence of the composition, wherein if the amount of CEM15-Vif binding is reduced in the presence of the composition as compared to the amount of CEM15-Vif binding in the absence of the composition, the composition is identified as an inhibitor of CEM15-Vif binding.

12. A method of identifying an inhibitor of an interaction between CEM15 and Vif, the method comprising incubating a library of molecules with CEM15 to form a mixture, and identifying the molecules that disrupt the interaction between CEM15 and Vif.
